(19) Europäisches Patentamt
European Patent Office
Office européen des brevets

(11) **EP 2 046 789 B1**

(12) **EUROPEAN PATENT SPECIFICATION**

(45) Date of publication and mention of the grant of the patent:
**05.09.2012 Bulletin 2012/36**

(21) Application number: **07805258.6**

(22) Date of filing: **27.07.2007**

(51) Int Cl.:
***C07D 471/04*** *(2006.01)* ***C07D 219/06*** *(2006.01)*
***A61K 31/165*** *(2006.01)* ***C07D 209/42*** *(2006.01)*
***C07D 215/54*** *(2006.01)* ***C07D 217/26*** *(2006.01)*
***C07D 235/24*** *(2006.01)* ***C07D 237/26*** *(2006.01)*
***C07D 333/68*** *(2006.01)* ***C07D 233/62*** *(2006.01)*

(86) International application number:
**PCT/IB2007/052992**

(87) International publication number:
**WO 2008/012782 (31.01.2008 Gazette 2008/05)**

(54) **LABELLED ANALOGUES OF HALOBENZAMIDES AS RADIOPHARMACEUTICALS**

MARKIERTE HALOBENZAMID-ANALOGA ALS RADIOPHARMAZEUTIKA

ANALOGUES MARQUES D'HALOBENZAMIDES UTILISES COMME PRODUITS RADIOPHARMACEUTIQUES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **27.07.2006 FR 0653138**

(43) Date of publication of application:
**15.04.2009 Bulletin 2009/16**

(60) Divisional application:
**11167370.3 / 2 363 399**

(73) Proprietors:
- **INSTITUT NATIONAL DE LA SANTE ET DE LA RECHERCHE MEDICALE (INSERM)
75013 Paris (FR)**
- **Université d'Auvergne
63001 Clermont-Ferrand Cedex 1 (FR)**

(72) Inventors:
- **MADELMONT, Jean-Claude
F-63540 Romagnat (FR)**
- **CHEZAL, Jean-Michel
F-63400 Chamalieres (FR)**
- **CHAVIGNON, Oliver
63730 Les Martres De Veyre (FR)**
- **TEULADE, Jean-Claude
34430 Saint Jean De Vedas (FR)**
- **MOINS, Nicole
63000 Clemont-Ferrand (FR)**

(74) Representative: **Tanty, François
Cabinet Nony
3, rue de Penthièvre
75008 Paris (FR)**

(56) References cited:
**EP-A- 0 082 665** **EP-A1- 0 458 886**
**WO-A-2005/042505** **US-A- 4 764 522**

- **SHOWALTER H D ET AL: "Tyrosine kinase inhibitors. 6. Structure-activity relationships among N- and 3-substituted 2,2'-diselenobis(1H-indoles) for inhibition of protein tyrosine kinases and comparative in vitro and in vivo studies against selected sulfur congeners." JOURNAL OF MEDICINAL CHEMISTRY, vol. 40, no. 4, 14 February 1997 (1997-02-14), pages 413-426, XP002469707 ISSN: 0022-2623**
- **OLSSON R ET AL: "Microwave-assisted solvent-free parallel synthesis of thioamides" TETRAHEDRON LETTERS, ELSEVIER, AMSTERDAM, NL, vol. 41, no. 41, 7 October 2000 (2000-10-07), pages 7947-7950, XP004235906 ISSN: 0040-4039**
- **BEER H-F ET AL: "[<123>I<18>F] N-(2-aminoethyl)-5-halogeno-2-pyridinecarb oxamides, site specific tracers for MAO-B mapping with SPET and PET", NUCLEAR MEDICINE AND BIOLOGY, ELSEVIER, NY, US, vol. 22, no. 8, 1 November 1995 (1995-11-01), pages 999-1004, XP004051704, ISSN: 0969-8051, DOI: 10.1016/0969-8051(95)02022-5**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 046 789 B1

Remarks:

The file contains technical information submitted after the application was filed and not included in this specification

## Description

The present invention relates to the use of aromatic and heteroaromatic analogues of halobenzamides labelled with a suitable radioactive isotope as radiopharmaceuticals for the diagnosis by gamma scintigraphic and positron imaging or for the internal radiotherapeutic treatment of melanoma, and also to some novel aromatic and heteroaromatic analogues of halobenzamides.

Melanoma is one of the most dangerous skin tumours with a steadily increasing incidence. Specifically, the current incidence of cutaneous melanoma is close to 10 000 new cases diagnosed per year in France. This is a highly invasive cancer, the development of which is rapidly fatal at the metastatic stage. 5-year survival does not exceed 14%, except in the case where the thickness of the tumour is less than 0.76 mm. In the case where the lesion exceeds this thickness, this tumour gives metastases in an unpredictable and silent fashion. This is why a search is currently underway for a method of investigation which makes possible the early evaluation both of the local extension and of the distant extension of the tumour.

During the last decade, a number of radiopharmaceutical products, selected for their potential melanin affinity, have been experimented with but few have had a satisfactory clinical development.

It should be explained that radiopharmaceutical products comprise two functional components, one being radioactive and the other not being radioactive. The radioactive component makes possible the detection of the product in the context of the diagnosis and it constitutes the active agent in the case of therapeutic use. It is composed of a radionuclide with appropriate physical properties. The nonradioactive component, for its part, is composed of a molecule or tracer, optionally biological, intended to accumulate in the target organ, in this case, in the context of the present invention, in the melanoma, and to ensure the absorption of radioactivity by the latter. This nonradioactive component is determining for the biological behaviour of the radiopharmaceutical product in the body, in particular regarding the specificity and the pharmacokinetic profile.

More particularly in imaging, monoclonal antibodies which specifically target melanoma have been developed. In addition, various molecules exhibiting an affinity for melanoma have been studied in man, after labelling with $^{123}$I : methylene blue, *N*-[3-(4-morpholino)propyl]-*N*-methyl-2-hydroxy-5-iodo-3-methylbenzylamine (ERC9) and benzamide structures.

Furthermore, the document EP 458 886 describes compounds of use in the diagnosis and treatment of malignant melanoma. *N*-(2-Diethylarninoethyl)-4-iodobenzamide (BZA) forms in particular the subject of more detailed studies, and also *N*-(2-diethylaminoethyl)-2-iodobenzamide (BZA2), in the medical imaging application and more particularly for the scintigraphic detection of primary ocular melanoma and metastases of cutaneous and ocular melanomas.

The document US 5 911 970 for its part describes, inter alia, other benzamide-derived compounds exhibiting a high specificity and affinity with regard to the surface of cancer cells.

There thus exists, on the one hand, a need to have available a specific tracer which makes it possible, from the time of the diagnosis, to carry out an assessment of extension of the disease and then, subsequently, monitoring. Such a tracer should advantageously make possible the differential diagnosis of ocular melanoma, primary lesion, which is often difficult to identify.

On the other hand, treatments remain defeated with regard to disseminated melanoma and the development of novel specific therapeutic approaches for the treatment of melanoma is essential.

This is because, while the surgical treatment of melanoma remains the best weapon (operable primary cutaneous tumours and isolated secondary sites), the low effectiveness of the treatments provided to date with regard to the disseminated disease illustrates the need to develop a treatment of melanoma by vectorized internal radiotherapy. To date, the first experimental results, carried out with an $\alpha$-MSH analogue, arc very preliminary and the ideal tracer remains to be determined.

Thus, the object of the present invention is the targeting of melanotic lesions by the development of molecules which, administered to the body, will make it possible to vectorize a radioisotope. This object results in two fields of applications, namely imaging and radiotherapy.

The compounds in accordance with the invention are thus of advantage both for their use in imaging, namely for the diagnosis of malignant melanoma, and in vectorized internal radiotherapy, targeting more particularly secondary lesions and primary ocular lesions. One of the major advantages of the compounds according to the present invention lies precisely in their mixed potentiality. In other words, according to the chemical variations under consideration, their respective behaviours in the body destine them more particularly for use in medical imaging, for use in radiotherapy or else, and this category of compounds may prove to be particularly attractive, for use both in medical imaging and in radiotherapy.

The examples reported below clearly illustrate, for a few compounds, these various behaviours in terms of specificity, of duration of radioactivity or of antitumour effectiveness.

Finally, some compounds corresponding to the general formula of the compounds in accordance with the invention, when they are unlabelled, may also exhibit an innate antitumour activity. Thus, in this specific case, this

amounts to saying that the nonradioactive component defined above can play, in addition to its role of targeting the melanoma and/or of promoting absorption of radioactivity, a role of cytotoxic agent per se.

Consequently, this particular category of compounds, the use of which is the subject-matter of the present invention, for some already known for their anticancer activity when they are devoid of radionuclides, in particular via the mode of action of intercalating agents, may exhibit the advantage of making possible a radiotherapeutic application which can be combined with an application in chemotherapy. The reference is then to compounds intended for an application in radiochemotherapy. However, it is clearly the activity via labelling by a radioactive isotope which is targeted in the present invention. In other words, the possible activity related to the mechanism of actions innate to the unlabelled compound is generally only to be regarded as an additional advantage.

Mention may in particular be made, among unlabelled compounds exhibiting an innate cytotoxic activity which can, after labelling, be used in the radiochemotherapy of melanoma, of certain compounds described in the documents WO 93/24096, WO 98/17649 or US 6 821 983 as analogues of acridinecarboxamide ("DACA"). The targeting of melanoma had not until now been demonstrated for these compounds. The two following publications :

- Osman, S., Rowlinson-Busza, G., Luthra, K. S., Aboagye, E. O., Brown, G. D., Brady, F., Myers, R., Gamage, S. A., Denny, W. A., Baguley, B. C., Price, P. M., Cancer Res., 2001, 61, 2935-2944, and
- Saleem, A., Harte, R. J., Matthews, J. C., Osman, S., Brady, F., Luthra, S. K., Brown, G. D., Bleehen, N., Connors, T., Jones, T., Price, P. M., and Aboagye, E. O., J. Clin. Oncology, 2001,19, 1421-1429,

describe the biodistribution of DACA labelled with $^{11}C$. However, these two experiments relate to short times (< 1 h) and do not demonstrate a specific fixing of DACA for the melanotic tumours evaluated.

Derivatives of phenazine type are described in particuar in the document WO 2005/118580 and in the publication S. A. Gamage et al., Bioorganic & Medicinal Chemistry, 2006, 14, 1160-1168, for their innate anticancer activity. However, the use of such phenazine derivatives for the targeting of melanoma has never been demonstrated.

According to a first aspect, a subject-matter of the present invention is the use of a compound of formula (I):

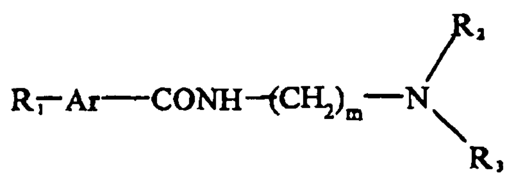

(I)

in which

$R_1$ represent a radionuclide,

Ar is a heteroaryl group,

m is an integer varying from 2 to 4, $R_2$ and $R_3$ represent, independently of one another, a hydrogen atom, a $(C_1-C_6)$ alkyl group, a $(C_1-C_6)$alkenyl group in which the heteroaryl group is a 5- or 6-membered aromatic ring comprising 1 or 2 nitrogen atoms or a bi- or tricyclic aromatic nucleus comprising from 1 to 4 nitrogen atoms or comprising a sulphur atom, at least one of the rings of which has 6 ring members, the other fused ring or rings having 5 or 6 ring members, it being possible for the said heteroaryl group to be monosubstituted by:

- an optionally labelled halogen atom,
- a $(C_1-C_4)$alkoxy group,
- a $(C_1-C_4)$alkyl group
- an $-NO_2$ group,
- an oxo group, or
- an anilino group which can itself be substituted by 1 to 3 groups which can be chosen from a $(C_1-C_4)$alkyl or $(C_1-C_4)$ alkoxy group, a hydroxyl group, a halogen atom or an $NHR^e$ group where $R^e$ represents a hydrogen, or a $COR^a$ group, a $COOR^a$ group or an $SO_2R^a$ group, where $R^a$ represents an aryl group or a $(C_1-C_{10})$alkyl group optionally substituted by an oxo group,

and in which $R_1$ is bonded to the aromatic nucleus as such or, when the substituent of the aromatic nucleus is an anilino group, $R_1$ can be bonded to the phenyl group of the anilino group,

and in which Ar is chosen from a pyridyl, phenazinyl, naphthyridinyl, indolyl, imidazopyridyl, benzimidazolyl, quinolyl, quinolonyl, isoquinolyl, quinoxalinyl, benzothienyl, acridinyl or acridonyl group, it being possible for the said group to be monosubstituted by a methyl group, a methoxy group or an optionally labelled halogen atom, and an acridinyl group substituted by an anilino group which can itself be substituted by 1 to 3 groups which can be chosen from a $(C_1-C_4)$alkyl

or ($C_1$-$C_4$)alkoxy group, a hydroxyl group, a halogen atom or an $NHR^e$ group where $R^e$ represents a hydrogen, or a $COR^a$ group, a $COOR^a$ group or an $SO_2R^a$ group, where $R^a$ represents an aryl group or a ($C_1$-$C_{10}$)alkyl group optionally substituted by an oxo group,
and their addition salts with pharmaceutically acceptable acids, for the use in a radiopharmaceutical composition intended for the diagnosis and/or treatment of melanoma.

In the context of the present invention, the term "halogen" is understood to mean chlorine, fluorine, bromine, iodine or astatine.

It is specified that, in the context of the present invention, the term "aromatic nucleus" is distinct from a phenyl group.

The term "heteroatom" is understood to mean nitrogen, oxygen or sulphur.

Within the meaning of the present invention, the term "radionuclide" is understood to mean an isotope of natural or artificial origin which demonstrates radioactive properties, The radionuclide can be a radioisotope chosen from $^{123}I$, $^{124}I$, $^{125}I$, $^{131}I$, $^{75}Br$, $^{76}Br$, $^{77}Br$, $^{18}F$, $^{210}At$ or $^{211}At$.

Advantageously, $R_1$ is an iodine atom chosen from $^{123}I$, $^{124}I$, $^{125}I$ and $^{131}I$.

Mention may be made, as example of 5- or 6-membered aromatic ring comprising 1 or 2 heteroatom(s), of pyridine.

Mention may be made, as example of bi- or tricyclic aromatic nucleus in accordance with the invention, of the nuclei having benzene as one of the rings, including indole, quinoline, isoquinoline, quinoxaline or benzimidazole for the bicycles and acridine and phenazine for the tricycles.

Mention may be made, as example of bi- or tricyclic aromatic nucleus in accordance with the invention, each of the rings of which, taken in isolation, is an aromatic nucleus comprising at least one heteroatom, of naphthyridine or imidazopyridine for the bicycles.

Within the meaning of the present invention, the heteroaryl can be partially hydrogenated. However, for the bi- or tricycles, each of the rings forming them, taken in isolation, comprises at least one double bond. In the context of the present invention, the terms "aromatic nucleus", "aryl", and "heteroaryl" include all the positional isomers.

According to another preferred embodiment, the compound of formula (I) exhibits a bi- or tricyclic aromatic nucleus as defined above and the $R_1$ group is bonded to one of the rings and the group

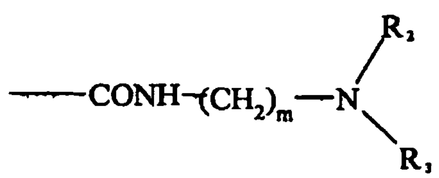

is bonded to the other ring or to one of the other rings constituting the bi- or tricyclic group.

According to a favoured embodiment of the present invention, when Ar comprises only one ring, the preferred compound of formula (I) exhibits the $R_1$ group in the para position with respect to the group

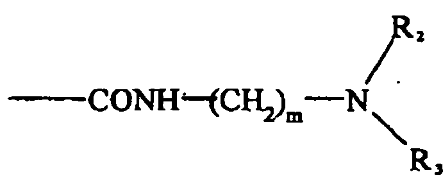

According to another favoured embodiment of the present invention, when Ar is a bi- or tricycle, the preferred compound of formula (I) exhibits the $R_1$ group bonded to one of the rings and the group

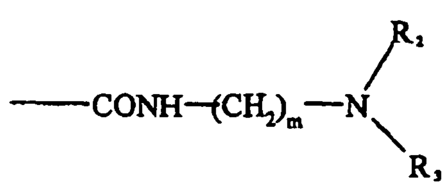

bonded to the other ring or to one of the other rings.

According to yet another favoured embodiment of the present invention, preference is given to the use of a

compound of formula (I), characterized in that Ar is a bior tricyclic heteroaryl as defined above and in that $R_1$ is bonded to the ring, taken in isolation, not comprising a heteroatom or comprising the least thereof and the group

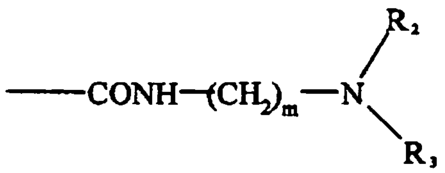

is bonded to another ring comprising the greater number of heteroatom(s).

An additional subject-matter of the present invention is a compound of formula (I')

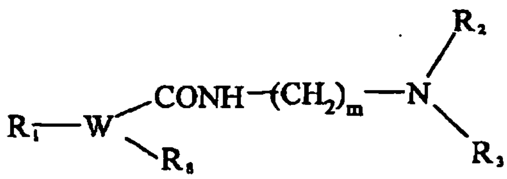

(I')

in which

W is chosen from a phenazinyl, imidazopyridyl, quinolyl, quinoxalinyl, acridinyl and acridonyl group, it being possible for the said acridinyl group to be substituted by an anilino group itself substituted by three groups,

- at least one of the substituents representing the $(C_1-C_4)$alkoxy group,
- at least one of the substituents being chosen from an $NHR^e$ group where $R^e$ represents a hydrogen, or a $COR^a$ group, a $COOR^a$ group or an $SO_2R^a$ group, where $R^1$ represents an aryl group or a $(C_1-C_{10})$alkyl group optionally substituted by an oxo group, and

  - the remaining substituent representing a hydrogen or halogen atom,

$R_1$, $R_2$, $R_3$ and m have the same meaning as above, and

$R_8$ represents a hydrogen atom, a $(C_1-C_4)$alkyl or $(C_1-C_4)$alkoxy group, an optionally labelled halogen atom, an -SH group, an -OH group or an $-NR_5R_6$ group where $R_5$ and $R_6$ can independently represent a hydrogen atom or a $(C_1-C_4)$alkyl group,

for the use in a composition intended for the diagnosis and/or treatment of melanoma,

In the formulae which follow, the groups of variable definition, in particular the $R_1$ and $R_8$ radicals, can take any position on the heterocycle. According to a specific embodiment, the $R_1$ group is bonded to one of the rings and the group

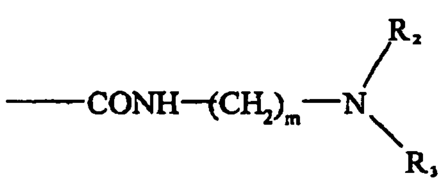

is bonded to the other ring or to one of the other rings.

According to yet another embodiment, the $R_8$ radical is situated, when this is possible, on a ring other than that carrying the $R_1$ group.

A subject-matter of the present invention is more particularly a compound of formula (Ia)

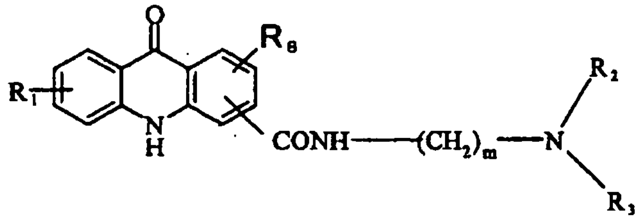

(Ia)

in which
$R_1$, $R_2$, $R_3$ and m have the same meaning as above, and
$R_8$ represents a hydrogen atom, a $(C_1-C_4)$alkyl group, a $(C_1-C_4)$alkoxy group, an optionally labelled halogen atom, an -SH group, an -OH group or an $NR_5R_6$ group where $R_5$ and $R_6$ can independently represent a hydrogen atom or a $(C_1-C_4)$alkyl group,
for use in a composition intended for the diagnosis and/or treatment of melanoma.

In addition, a subject-matter of the present invention is more particularly the a compound of formula (Ib)

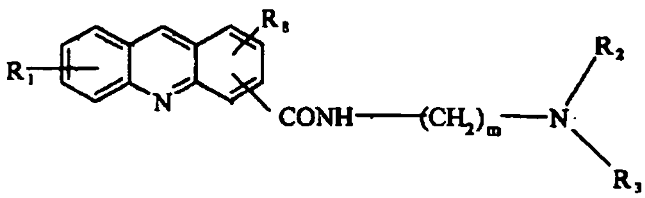

(Ib)

in which
$R_1$, $R_2$, $R_3$ and m have the same meaning as above, and
$R_8$ is as defined above,
for use in a composition intended for the diagnosis and/or treatment of melanoma.

A subject-matter of the present invention is also more particularly a compound of formula (Ic)

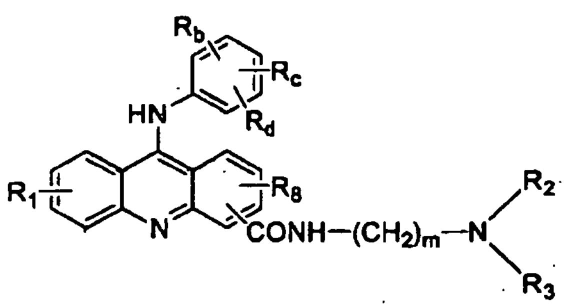

(Ic)

in which
$R_1$, $R_2$, $R_3$ and m have the same meaning as above, and
$R_8$ is as defined above,
at least one of the substituents chosen from $R^b$, $R^c$ and $R^d$ represents a $(C_1-C_4)$alkoxy group,
at least one of the substituents chosen from $R^b$, $R^c$ and $R^d$ is chosen from an $NHR^c$ group where $R^e$ represents a hydrogen, or a $COR^a$ group, a $COOR^a$ group or an $SO_2R^a$ group, where $R^a$ represents an aryl group or a $(C_1-C_{10})$alkyl group optionally substituted by an oxo group, and
the remaining substituent, chosen from $R^b$, $R^c$ and $R^d$, represents a hydrogen or halogen atom,
for use in a composition intended for the diagnosis and/or treatment of melanoma.

Another subject-matter of the present invention is more particularly a compound of formula (If)

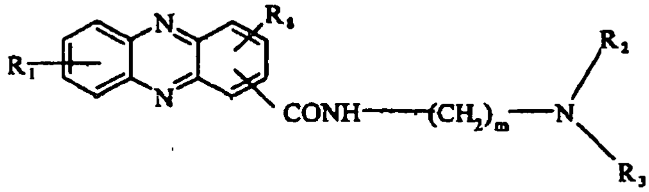

(If)

in which
$R_1$, $R_2$, $R_3$ and m have the same meaning as above, and
$R_8$ is as defined above,
for use in a composition intended for the diagnosis and/or treatment of melanoma.

In addition, a subject-matter of the present invention is more particularly a compound of formula (Ig)

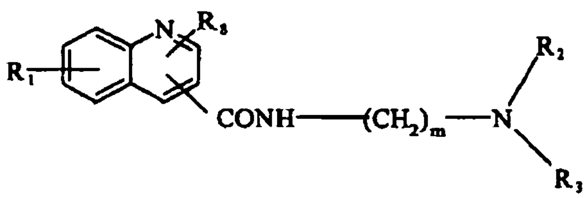

(Ig)

in which
$R_1$, $R_2$, $R_3$ and m have the same meaning as above, and
$R_8$ is as defined above,
for use in a composition intended for the diagnosis and/or treatment of melanoma.

A subject-matter of the present invention is also more particularly a compound of formula (Ih)

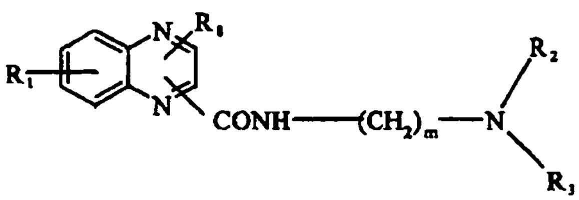

(Ih)

in which
$R_1$, $R_2$, $R_3$ and m have the same meaning as above, and
$R_8$ is as defined above,
for use in a composition intended for the diagnosis and/or treatment of melanoma.

A subject-matter of the present invention is also more particularly a compound of formula (Ii)

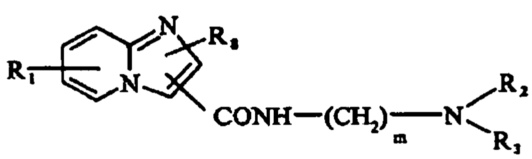

(Ii)

in which
$R_1$, $R_2$, $R_3$ and m have the same meaning as above, and
$R_8$ is as defined above,
for use in a composition intended for the diagnosis and/or treatment of melanoma.

In addition, a subject-matter of the present invention is more particularly a compound of formula (Io)

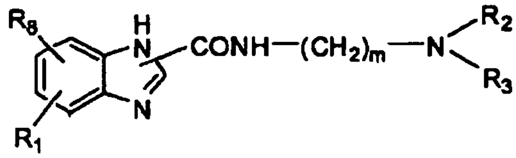

(Io)

in which
$R_1$, $R_2$, $R_3$ and m have the same meaning as above, and
$R_8$ is as defined above,
for use in a composition intended for the diagnosis and/or treatment of melanoma.

Another subject-matter of the present invention is more particularly a compound of formula (Ip)

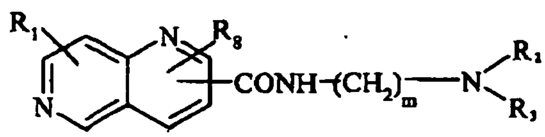

(Ip)

in which
$R_1$, $R_2$, $R_3$ and m have the same meaning as above, and
$R_8$ is as defined above,
for use in a composition intended for the diagnosis and/or treatment of melanoma.

Finally, according to a second aspect, a subject-matter of the present invention is novel compounds of formula (II)

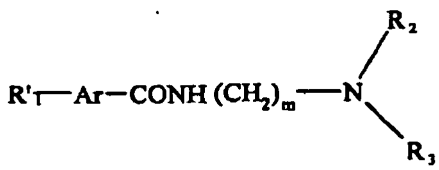

(II)

in which
$R'_1$ represents a labelled halogen atom,
m has the same meaning as above,
$R_2$ and $R_3$ represent, independently of one another, a $(C_1-C_6)$alkyl group or a $(C_1-C_6)$alkenyl group, and
Ar is chosen from the pyridyl, indolyl, imidazopyridinyl, benzimidazolyl, quinolyl, quinolonyl, isoquinolyl, quinoxalinyl, naphthyridinyl and benzothienyl group.

The preferences relating to the respective positions of the $R_1$ substituent and of the group

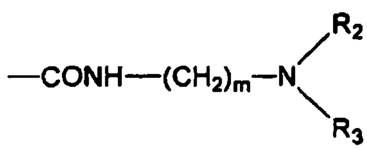

described above relating to the compounds of formula (I) also apply in defining the compounds of formula (II).

In addition, a subject-matter of the present invention is more particularly, according to a third aspect, novel compounds of formula (I")

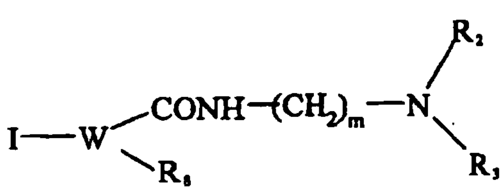

(I")

in which
$R_2$, $R_3$ and m have the same meaning as above,
$R_8$ is as defined above and
W has the same meaning as above, it being possible for the iodine atom to be labelled $^{123}I$, $^{124}I$, $^{125}I$ or $^{131}I$.

A subject-matter of the present invention is more particularly novel compounds of formula (Id)

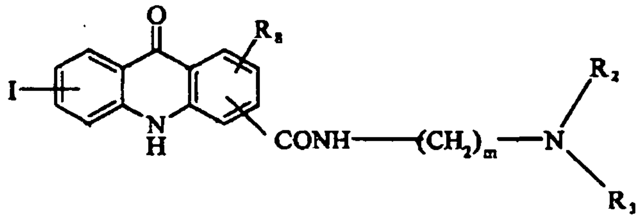
(Id)

in which
$R_2$, $R_3$ and m have the same meaning as above, and
$R_8$ is as defined above, it being possible for the iodine atom to be labelled.

In addition, a subject-matter of the present invention is more particularly novel compounds of formula (Ie)

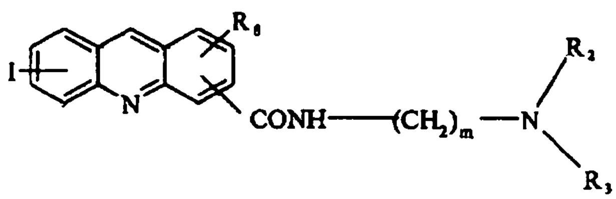
(Ie)

in which
$R_2$, $R_3$ and m have the same meaning as above, and
$R_8$ is as defined above.

Another subject-matter of the present invention is more particularly novel compounds of formula (Ij)

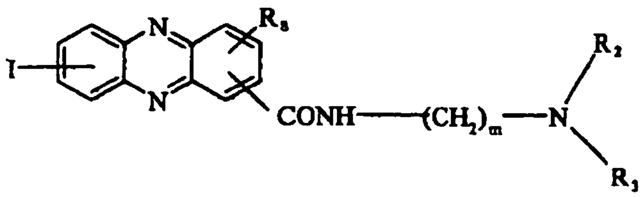
(Ij)

in which
$R_2$, $R_3$ and m have the same meaning as above, and
$R_8$ is as defined above, it being possible for the iodine atom to be labelled.

In addition, a subject-matter of the present invention is more particularly novel compounds of formula (Ik)

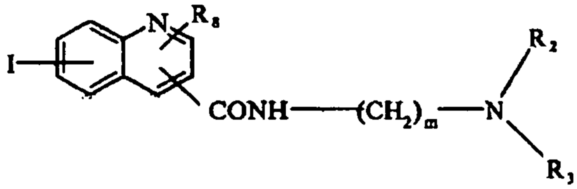
(Ik)

in which
$R_2$, $R_3$ and m have the same meaning as above, and
$R_8$ is as defined above, it being possible for the iodine atom to be labelled.

In addition, a subject-matter of the present invention is more particularly novel compounds of formula (Im)

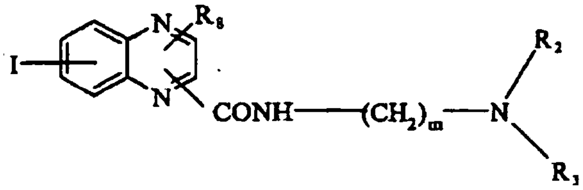

(Im)

in which
$R_2$, $R_3$ and m have the same meaning as above, and
$R_8$ is as defined above, it being possible for the.iodine atom to be labelled.

In addition, a subject-matter of the present invention is more particularly novel compounds of formula (In)

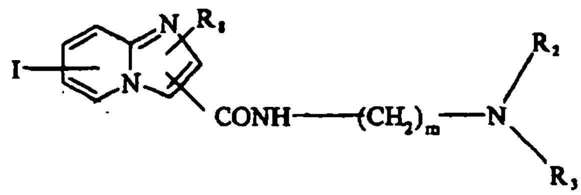

(In)

in which
$R_2$, $R_3$ and m have the same meaning as above, and
$R_8$ is as defined above, it being possible for the iodine atom to be labelled.

Another subject-matter of the present invention is more particularly novel compounds of formula (Iq)

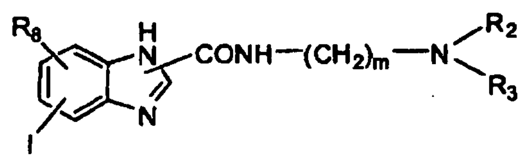

(Iq)

in which
$R_2$, $R_3$ and m have the same meaning as above, and
$R_8$ is as defined above, it being possible for the iodine atom to be labelled.

In addition, a subject-matter of the present invention is more particularly novel compounds of formula (Ir)

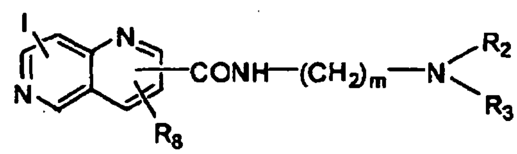

(Ir)

in which
$R_2$, $R_3$ and m have the same meaning as above, and
$R_8$ is as defined above, it being possible for the iodine atom to be labelled.

All the substitutions, including those represented for the compounds of formulae (I), (I'), (I"), (Ia), (Ib), (Ic), (Id), (Ie), (If), (Ig), (Ib), (Ii), (Ij), (Ik), (Im), (In), (Io), (Ip), (Iq) and (Ir) by the $R_1$ and $R_8$ groups, the iodine atom or

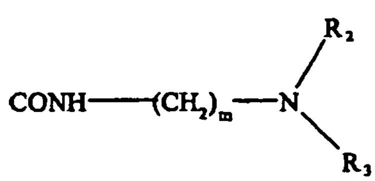

can take place in all the possible positions of the Ar group.

For the compounds of formulae (Ia), (Ib), (Ic), (Id) and (Ie), the substitution by the group

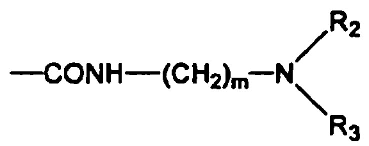

can take place in particular in the 4-position or in the 9-position of the acridine or in the 4-position of the acridone, which are the preferred positions.

For these same compounds, the $R_1$ group or the iodine atom is preferably in the 2-, 5- or 7-position of the acridine or of the acridone.

According to a preferred embodiment of the present invention, the compound is chosen from:

- N-(2-diethylaminoethyl)-6-iodonicotinamide dihydrochloride (7);
- N-(2-diethylaminoethyl)-5-iodoindole-2-carboxamide hydrochloride (10);
- N-(2-diethylaminoethyl)-4-iodobenzo[b]thiophene-2-carboxamide hydrochloride (13);
- N-(2-diethylaminoethyl)-3-iodoimidazo[1,2-a]pyridine-2-carboxamide dihydrochloride (16);
- N-(2-diethylaminoethyl)-6-iodoimidazo[1,2-a]pyridine-2-carboxamide dihydrochloride (21);
- N-(2-diethylaminoethyl)-5-iodobenzimidazole-2-carboxamide dihydrochloride (26);
- N-(2-diethylaminoethyl)-6-iodoquinoline-2-carboxamide dihydrochloride (31);
- *N*-(2-diethylaminoethyl)-1,4-dihydro-6-iodo-4-oxoquinoline-3-carboxamide hydrochloride (35);
- *N*-(2-diethylaminoethyl)-5-iodoisoquinoline-3-carboxamide dihydrochloride (39);
- *N*-(2-diethylaminoethyl)-6-iodoquinoxaline-2-carboxamide dihydrochloride (46);
- *N*-(4-dipropylaminobut.yl)-6-iodoquinoline-2-carboxamide dihydrochloride (49);
- *N*-(2-diethyllaminoethyl)-9,10-dihydro-1-iodo-9-oxacridine-4-carboxamide hydrochloride (55);
- *N*-(2-diethylaminoethyl)-9,10-dihydro-2-iodo-9-oxoacridine-4-carboxamide hydrochloride (60);
- *N*-(2-diethylaminoethyl)-9,10-dihydro-3-iodo-9-oxoacridine4-carboxamide hydrochloride (65);
- *N*-(2-diethylaminoethyl)-9,10-dihydro-5-iodo-9-oxoacridine-4-carboxamide hydrochloride (69);
- *N*-(2-diethylaminoethyl)-9, 10-dihydro-6-iodo-9-oxoacridine-4-carboxamide hydrochloride (78);
- *N*-(2-diethylaminoethyl)-9,10-dihydro-7-iodo-9-oxoacridine-4-carboxamide hydrochloride (84);
- *N*-(2-diethylaminoethyl)-9,10-dihydro-8-iodo-9-oxoacridine-4-carboxamide hydrochloride (87);
- *N*-(2-diethylaminoethyl)-1-iodoacridine-4-carboxamide dihydrochloride (91);
- *N*-(2-diethylaminoethyl)-2-iodoacridine-4-carboxamide dihydrochloride (95);
- *N*-(2-diethylaminoethyl)-3-iodoacridine-4-carboxamide dihydrochloride (98);
- *N*-(2-diethylaminoethyl)-5-iodoacridine-4-carboxamide dihydrochloride (102);
- *N*-(2-diethylaminoethyl)-6-iodoacridine-4-carboxamide dihydrochloride (107);
- *N*-(2-diethylaminoethyl)-7-iodoacridine-4-carboxamide dihydrochloride (111);
- *N*-(2-diethylaminoethyl)-8-iodoacridine-4-carboxamide dihydrochloride (115);
- *N*-(2-diethylaminoethyl)-2-iodoacridine-9-carboxamide dihydrochloride (120);
- *N*-(2-diethylaminoethyl)-2,7-diiodoacridine-9-carboxamide dihydrochloride (122);
- *N*-(2-diethylaminoethyl)-2-iodo-9-(4-methanesulphonamido-2-methoxyanilino)acridine-4-carboxamide dihydrochloride (125);
- *N*-(2-diethylaminoethyl)-5-iodo-9-(4-methanesulphonamido-2-methoxyanilino)acridine-4-carboxamide dihydrochloride (127);
- *N*-(2-diethylaminoethyl)-7-iodo-9-(4-methanesulphonamido-2-methoxyanilino)acridine-4-carboxamide dihydrochloride (129);
- *N*-(2-diethylaminoethyl)-9-(5-iodo-4-methanesulphonamido-2-methoxyanilino)acridine-4-carboxamide dihydrochloride (137);
- *N*-(2-diethylaminoethyl)-8-iodo-1,6-naphthyridine-2-carboxamide dihydrochloride (142);
- *N*-(4-dipropylaminobutyl)-6-iodoquinoxaline-2-carboxamide dihydrochloride (144);
- *N*-(2-diethylaminoethyl)-7-iodophenazine-1-carboxamide dihydrochloride (151);
- and their pharmaceutically acceptable salts.

For the group of the compounds of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II), m is preferably equal to 2 or 4.

The compounds of formulae (I), (I'), (I"), (Ia), (Ib), (Ic), (Id), (Ie), (If), (Ig), (Ih), (Ii), (Ij), (Ik), (Im), (In), (Io), (Ip),

(Iq), (Ir) and (II) can comprise one or more asymmetric carbon atoms. They can thus exist in the form of enantiomers or of diastereoisomers. These enantiomers, diastereoisomers and their mixtures, including the racemic mixtures, form part of the invention.

The pharmaceutically acceptable salts of the compounds of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) include the addition salts with pharmaceutically acceptable acids, such as inorganic acids, for example hydrochloric, hydrobromic, phosphoric or sulphuric acid, and organic acids, such as acetic, trifluoroacetic, propionic, oxalic, succinic, fumaric, malic, tartaric, citric, ascorbic, maleic, glutamic, benzoic, toluenesulphonic, methanesulphonic, stearic and lactic acid.

The compounds of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) or their salts can form solvates (namely hydrates); the invention includes such solvates.

In comparison with the compounds described in the document EP 458 886 and more particularly with *N*-(2-diethylaminoethyl)-4-iodobenzamide (BZA), improvements in terms of specificity and/or of affinity for the target organ, namely the melanoma, and/or in terms of pharmacokinetic profile and/or of antitumour effectiveness could be observed for some labelled compounds in accordance with the present invention.

When the compounds in accordance with the invention of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) are used for medical imaging purposes, $R_1$ is preferably a radionuclide possessing $\gamma$ or $\beta^+$ emission which can be detected according to conventional radioimaging techniques, for example scintigraphic imaging by single photon emission tomography (SPET) and by positron emission tomography (PET).

Such a radionuclide possessing $\gamma$ or $\beta^+$ emission advantageously exhibits an optimum energy for the measurement by means of a $\gamma$-camera or PET camera. Mention may in particular be made, as radionuclides acceptable for medical imaging, of $^{123}I$, $^{124}I$, $^{125}I$, $^{131}I$, $^{18}F$, $^{75}Br$, $^{76}Br$ and $^{77}Br$.

$^{123}I$ is particularly suitable for SPET scintigraphic diagnosis and $^{124}I$ for a PET scintigraphic diagnosis.

When the compounds of the invention of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) are used for therapeutic purposes, $R_1$ is preferably a radionuclide possessing $\alpha$, $\beta^-$ or Auger electron emission. The radionuclides suitable in this context, capable of providing a cytotoxic effect, can be chosen from $^{131}I$, $^{125}I$, $^{211}At$ and $^{210}At$.

$^{131}I$ is particularly suitable for an application in the treatment of melanoma in internal radiotherapy. Furthermore, $^{125}I$, due to its Auger electron emission, can be used in internal radiotherapy provided that it is internalized in the cell.

The compounds of formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) can be prepared by condensation of an ester of formula (III)

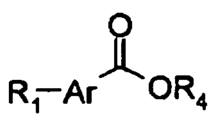

**(III)**

in which Ar has the same meaning as in the formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (II) and $R_4$ represents a $(C_1-C_6)$alkyl, aryl or heteroaryl group, for example chosen from benzene, pentafluorobenzene, p-nitrobenzene, triazole; benzotriazole, 7-azabenzotriazole and succinimide, with a diamine of formula (IV)

$$H_2N-(CH_2)_m-NR_2R_3 \qquad (IV)$$

in which m, $R_2$ and $R_3$ have the same meaning as in the formulae (I), (I'), (I"), (Ia), (Ib), (Ic), (Id), (Ie), (If), (Ig), (Ih), (Ii), (Ij), (Ik), (Im), (In), (Io), (Ip), (Iq), (Ir) and (III).

The condensation of the amine (IV) with the ester (III) can preferably be carried out in the presence or absence of trimethylaluminium by heating at reflux of the dichloromethane, of the toluene or of any other appropriate solvent.

This process of synthesis forms part of the invention as regards the compounds of formulae (I") and (II), according to a fourth aspect.

The compounds of formulae (I) and (II) in which $R_2$ or $R_3$ is a hydrogen can preferably be prepared by condensation of the amine (IV) with the ester (III) in which $R_4$ is a *p*-nitrophenyl. This reaction can preferably be carried out in tetrahydrofuran at ambient temperature.

The compounds of formula (III) can be synthesized from the compounds (V) according to various methodologies:

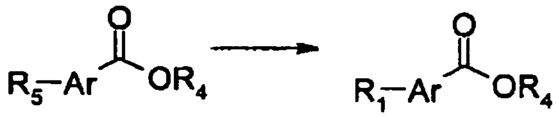

(V) (III)

When $R_5$ is a hydrogen atom: by direct iodination of the aromatic or heteroaromatic part preferably using *N*-iodosuccinimide at reflux of the acetonitrile or of any other appropriate solvent. This iodination can also be carried out in the presence of diiodine, sodium periodate and sulphuric acid. The iodination can also be obtained after formation of an organolithium compound at low temperature, followed by treatment with diiodine.

When $R_5$ is a halogen atom: by direct exchange in the presence of alkali metal iodide, in an acidic medium, and in the presence or absence of a catalyst, such as copper sulphate. The halogen/iodine exchange can also be obtained after passing through an organometallic compound (organolithium compound, organomagnesium compound, and the like) at low temperature, followed by treating the latter with diiodine.

When $R_5$ is an $NH_2$ group: by a diazotization reaction, the amine being treated at 0°C with sodium nitrite in an acidic medium, and then, after formation of the diazonium salt, by addition of alkali metal iodide and heating. This diazotization can also be carried out in an organic medium, the amino derivative being treated with tert-butyl nitrite in the presence of diiodomethane or of diiodine.

When $R_5$ is an $NO_2$ or NO group: by reduction, either by catalytic hydrogenation or by using a metal (Fe, Sn, and the like) in the presence of an acid (HCl, HBr, acetic acid, and the like). The amine thus obtained is treated according to the protocol described above.

In the case where Ar is an acridinyl group, the compounds (IIIb) are preferably obtained by reduction of the acridone (IIIa) to give acridane (VI) in the presence of a borane/tetrahydrofuran complex or of any other complexing agent, such as tert-butylamine, diethylamine, dimethylamine, morpholine, pyridine, trimethylamine, triethylamine, triphenylphosphine, dimethyl sulphoxide or dimethyl sulphide and at reflux of the tetrahydrofuran or of any other appropriate solvent. This methodology exhibits the advantage of reducing acridone to acridane without affecting the iodine or the ester functional group, in contrast to the conventional methods using, for example, as reducing agent, lithium aluminium hydride ($LiAlH_4$), which reduces the ester to alcohol, or the Al/Hg (or Na/Hg or Na/ROH) amalgam, which results in the substitution of the iodine by a hydrogen.

The acridanes (VI) are subsequently oxidized to acridines (IIIb) by ferric chloride hexahydrate at 50°C or by any other appropriate oxidizing agent, according to the scheme which follows:

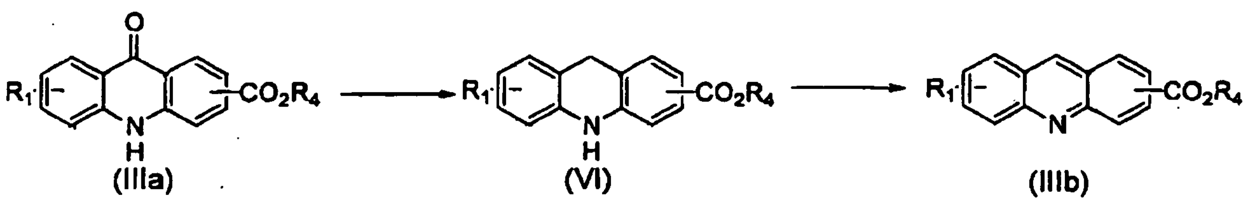

(IIIa) (VI) (IIIb)

This process is novel and also forms part of the invention.

Thus, the present invention relates, according to a fifth aspect, to the process for the preparation of a compound of formula (VI)

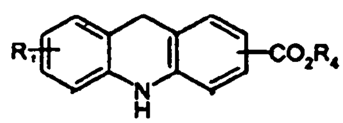

in which $R_1$ is a halogen atom and $R_4$ represents a (C1-C4)alkyl, aryl or heteroaryl group of use as synthetic intermediate in the preparation of the compounds of formula (Ia) in which W is an acridonyl group,
characterized in that it comprises a stage of reduction of the acridone of formula (IIIa)

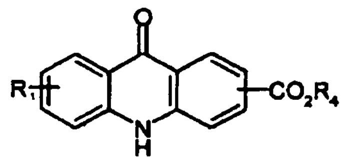

in which $R_1$ and $R_4$ are as defined above, in the presence of a complexing.

The process can be followed by a stage of oxidation of the acridanes of formula (VI) thus obtained in order to obtain an acridine of formula (IIIb).

The labelling of the compound of formulae (I), (I'), (I''), (Ia), (Ib), (Ic), (Id), (Ie), (If), (Ig), (Ih), (Ii), (Ij), (Ik), (Im), (In), (Io), (Ip), (Iq), (Ir) and (II) can be carried out by several techniques. For example, it can be carried out by exchange in an acidic medium between the nonradioactive iodinated molecule and a radioactive alkali metal halide. The exchange can be carried out by heating, at reflux, an aqueous solution of the compound of formulae (I), (I'), (I''), (Ia), (Ib), (Ic), (Id), (Ie), (If), (Ig), (Ih), (Ii), (Ij), (Ik), (Im), (In), (Io), (Ip), (Iq), (Ir) and (II) in a buffered medium or of the compound of formulae (I), (I'), (I''), (Ia), (Ib), (Ic), (Id), (Ie), (If), (Ig), (Ih), (Ii), (Ij), (Ik), (Im), (In), (Io), (Ip), (Iq), (Ir) and (II) in acetic acid and of the radioactive halide, in the presence or absence of copper sulphate. The labelling can also take place between a trialkylstannane precursor of the compound of formulae (I), (I'), (I''), (Ia), (Ib), (Ic), (Id), (Ie), (If), (Ig), (Ih), (Ii), (Ij), (Ik), (Im), (In), (Io), (Ip), (Iq), (Ir) and (II) and an alkali metal halide, such as $Na^{125}I$ or $Na^{131}I$, in the presence of an oxidizing agent, such as chloramine-T, peracetic acid or aqueous hydrogen peroxide solution, and an acid, such as hydrochloric acid, acetic acid or an acidic buffer, preferably at ambient temperature and in an appropriate solvent.

The trialkylstannane precursor compounds of the compounds of formulae (I), (I'), (I''), (Ia), (Ib), (Ic), (Id), (Ie), (If), (Ig), (Ih), (Ii), (Ij), (Ik), (Im), (In), (Io), (Ip), (Iq), (Ir) and (II) also form part of the present invention. They are defined in the same way as the compounds of the abovementioned formulae, except for the $R_1$ group, which is an $-Sn[(C_1-C_6)alkyl]_3$ group and which can in particular be an $-SnBu_3$ group. In the continuation of the description, they are referred to as compounds of formula (VII)

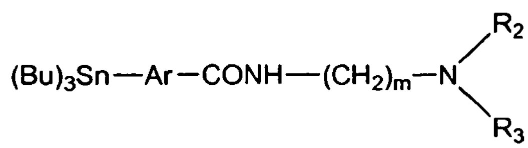
(VII)

in which $R_2$, $R_3$ and m have the same meaning as above.

The following examples illustrate the invention.

The melting points, uncorrected, were determined on an Electrothermal model IA 9300 capillary apparatus. The NMR spectra were recorded on an Avance DRX-200 (200 MHz for $^1H$ and 50 MHz for $^{13}C$) and a Bruker AC 400 (400 MHz for $^1H$ and 100 MHz for $^{13}C$). The chemical shifts $\delta$ are expressed in ppm (parts per million) and the coupling constants in Hz. The following abbreviations are used: s (singlet), bs (broad singlet), d (doublet), dd (split doublet), t (triplet), q (quartet), st (sextet), td (split triplet), m (multiplet). The infrared spectra were recorded on a Nicolet Impact 410 FTIR and Vector 22 FT spectrophotometer. The direct introduction mass spectra were recorded on a Hewlett Packard 5989A device coupled with a 5890 series 2 GC. The electrospray ionization mass spectra (ESI-MS) were obtained on an Esquire-LC, Brucker. In the case of the organotin compounds, the tin fragments are given for the 120 isotope of tin (isotopic abundance = 33%). The elemental analyses for C, H and N were carried out at the Service Central d'Analyse [Central Analytical Service] (CNRS, Vernaison, France). The thin layer chromatograms (TLC) were run on alumina plates (60 F254, type E, Merck) or on silica plates (Kieselgel 60 F254, Merck) and visualized under a UV lamp or with sublimed iodine. The column purification of the compounds was carried out using alumina (Merck aluminium oxide 90, standardized (activity stage II-III)) or silica (SDS, 60Å C.C, 35-70 μm, Chromagel) as support.

## EXAMPLE 1

Synthesis of *N*-(2-diethylaminoethyl)-6-iodo-2-naphthamide hydrochloride (3)

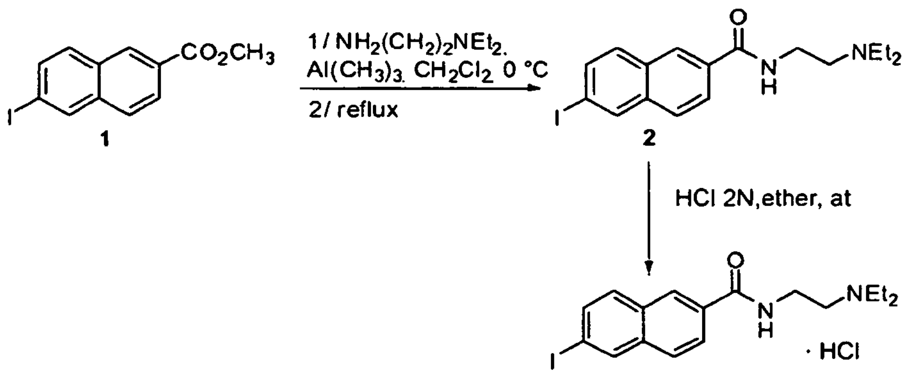


1 2 3

**Stage A: *N*-(2-diethylaminoethyl)-6-iodo-2-naphthamide (2)**

A 2M solution of trimethylaluminium in toluene (7.2 ml, 14.4 mmol) is added to a solution of *N*,*N*-diethylethylenediamine (2 ml, 14.1 mmol) in anhydrous dichloromethane (110 ml) under argon at 0˚C. Methyl 6-iodo-2-naphthoate **(1)** (Adcock, W. and Wells, P. R., Aust. J. Chem., 1965, 18, 1351-1364) (3.12 g, 10 mmol), in solution in anhydrous dichloromethane (88 ml), is rapidly added. The medium is heated at reflux for 16 hours and then the reaction is halted with water (130 ml). The reaction mixture is extracted with dichloromethane (3 x 100 ml). The organic phase is dried over magnesium sulphate and evaporated under vacuum. The crude reaction product is purified by chromatography on alumina eluted with a dichloromethane/ethanol (97/3, v/v) mixture, to result in the amide **2** (1.58 g, 3.99 mmol). Yield : 40%; Rf: 0.58 ($Al_2O_3$, dichloromethane/ethanol (97/3, v/v)); melting point: 105-107˚C; $^1$H NMR (400 MHz, $CDCl_3$) δ 1.05 (t, 6H, *J* = 7 Hz), 2.58 (q, 4H, *J* = 7 Hz), 2.68 (t, 2H, *J* = 6 Hz), 3.53 (q, 2H, *J* = 6 Hz), 7.40 (m, 1H), 7.54 (d, 1 H, *J* = 8.5 Hz), 7.68 (d, 2H, 8,5 Hz), 7.83 (d, 1H, *J* = 8.5 Hz), 8.18 (s, 1H), 8.23 (s, 1H); $^{13}$C NMR (100 MHz, $CDCl_3$) δ 11.8 (2C), 37.3, 46.6 (2C), 51.1, 93.6, 124.4, 127.0, 127.3, 130.2, 131.1, 132.3, 135.0, 135.7, 136.2, 166.9; IR (KBr) ν $cm^{-1}$ : 1537, 1614, 1630, 3303; MS (m/z, %) 281 ($M^+$-115.6), 126 (9), 86 (100), 58 (14).

**Stage B: *N*-(2-diethylaminoethyl)-6-iodo-2-naphthamide hydrochloride (3)**

*N*-(2-diethylaminoethyl)-6-iodo-2-naphthamide **(2)** (1.00 g, 2.52 mmol) is dissolved in anhydrous dichloromethane (22 ml) under argon at ambient temperature. A 2N solution of hydrochloric acid in ether (22 ml) is added to the reaction medium with stirring. The latter is stirred for 10 minutes and then the solvent is evaporated. The solid obtained is taken up in anhydrous ether (50 ml). The solution is stirred under argon and at ambient temperature for 24 hours. The precipitate obtained is filtered off, to result in the hydrochloride **3** (1.03 g, 2.38 mmol). Yield : 94%; melting point: 151-152˚C; $^1$H NMR (200 MHz, $d_6$-DMSO) δ 1.22 (t, 6H, *J* = 7 Hz), 3.19 (m, 6H), 3.68 (m, 2H), 7.83 (m, 1H), 7.97 (m, 2H), 8.46 (s, 1H), 8.53 (s, 1H), 9.13 (m, 1 H), 10.33 (m, 1H); IR (KBr) ν $cm^{-1}$: 1297, 1533, 1655, 3252. Anal. Calculated for $C_{17}H_{21}IN_2O$-HCl-$H_2O$: C, 45.30; H, 5.37; N, 6.22. Found: C, 45.26; H, 5.16; N, 6.40.

**EXAMPLE 2**

Synthesis of *N*-(2-diethylaminoethyl)-6-iodonicotinamide dihydrochloride **(7)**

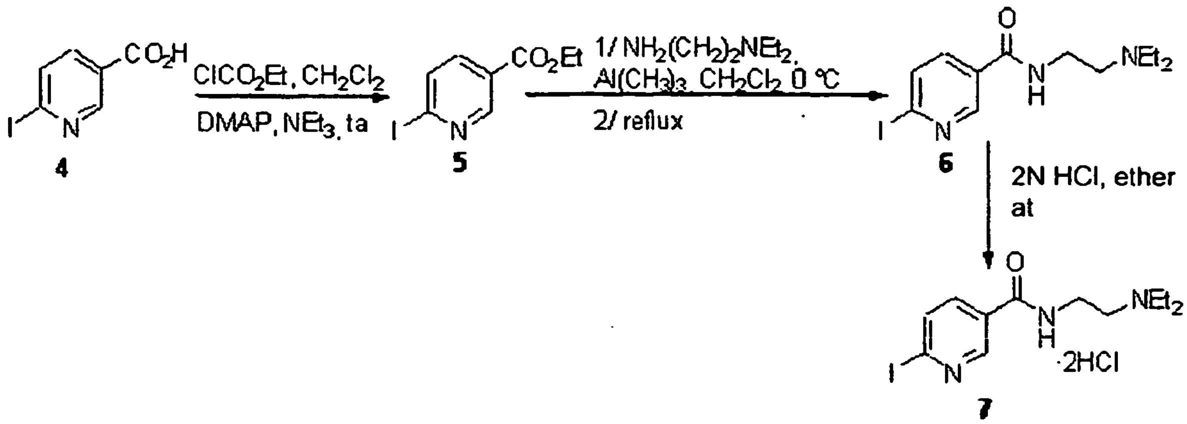


4 5 6 7

Stage A: Ethyl 6-iodonicotinate (5)

Triethylamine (1.67 μl, 1.19 mmol), ethyl chloroformate (0.70 ml, 7.32 mmol) and 4-dimethylaminopyridine (0.45 g, 3.68 mmol) are added, at 0°C, to a solution of 6-iodonicotinic acid (4) (Newkome, G. R., Moorfield, C. N. and Sabbaghian, B., J. Org. Chem., 1986, 51, 953-954) (250 mg, 1.00 mmol) in anhydrous dichloromethane (25 ml). After returning to ambient temperature, the reaction mixture is brought to reflux for 2 hours. The solution is evaporated to dryness and then the residue is purified on a column of alumina eluted with dichloromethane, to result in the ester 5 (211 mg, 0.76 mmol) ; yield: 76%; Rf: 0.92 ($Al_2O_3$, dichloromethane); melting point: 46-48°C; $^1H$ NMR (200 MHz, $CDCl_3$) δ 1.32 (t, 3H, *J* = 3 Hz), 4.32 (q, 2H, *J* = 7 Hz), 7.78 (m, 2H), 8.82 (d, 1H, J = 2 Hz); $^{13}C$ NMR (50 MHz, $CDCl_3$) δ 14.2, 61.6, 123.3, 125.7, 134.7, 138.0, 151.4, 164.7; IR (KBr) ν $cm^{-1}$: 1268, 1292, 1577, 1711, 3082; MS (m/z, %) 277 ($M^+$, 61), 232 (21), 204 (20), 150 (100), 127 (16), 77 (43), 51 (22).

**Stage B: *N*-(2-diethylaminoethyl)-6-iodonicotinamide (6)**

The compound **6** was prepared according to the procedure described for the preparation of the compound **2,** using ethyl 6-iodonicotinate **(5)** as starting material and heating the reaction medium at reflux for 20 hours. Yield: 98%; viscous liquid: Rf: 0.60 ($Al_2O_3$, dichloromethane/ethanol (97/3, v/v)); $^1H$ NMR (200 MHz, $CDCl_3$) δ 0.83 (t, 6H, *J* = 7 Hz), 2.37 (q, 4H, *J* = 7 Hz), 2.47 (t, 2H, *J* = 6 Hz), 3.28 (q, 2H, *J* = 6 Hz), 7.38 (m, 1 H), 7.78 (m, 2H), 8.73 (m, 1 H); $^{13}C$ NMR (50 MHz, $CDCl_3$) δ 11.7 (2C), 37.2, 46.4 (2C), 50.8, 120.9, 129.5, 134.5, 136.1, 148.6, 164.4; IR ($CCl_4$) ν $cm^{-1}$: 1538, 1630, 2927, 2965, 3301; MS (m/z, %) 348 ($M^+$+1. 1), 86 (100), 77 (8), 58 (16).

**Stage C: *N*-(2-diethylaminoethyl)-6-iodonicotinamide dihydrochloride (7)**

The compound **7** was prepared according to the procedure described for the preparation of the compound **3,** using *N*-(2-diethylaminoethyl)-6-iodonicotinamide **(6)** as starting material. Yield: 70%; melting point: 127-129°C; $^1H$ NMR (200 MHz, $d_6$-DMSO) δ 1.26 (t, 6H, *J* = 7 Hz), 3.25 (m, 6H), 3.68 (m, 4H), 8.00 (m, 2H), 8.87 (m, 1H), 9.25 (m, 1H), 10.24 (m, 1H); IR (KBr) ν $cm^{-1}$: 1278, 1529, 1588, 1661, 2661, 3232. Anal. Calculated for $C_{12}H_{18}IN_3O \cdot 2HCl$: C, 34.31; H, 4.80; N, 10.00. Found: C, 34.43; H, 4.76; N, 10.08.

**EXAMPLE 3**

Synthesis of *N*-(2-diethylaminoethyl)-5-iodoindole-2-carboxamide hydrochloride **(10)**

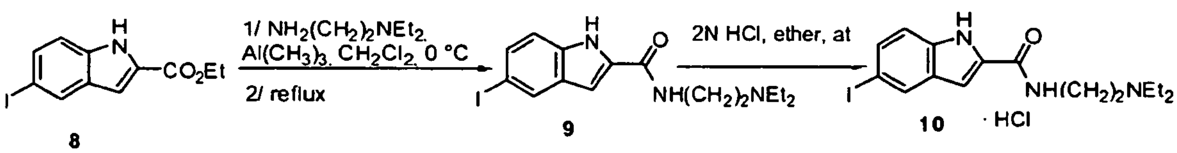


8 9 10

**Stage A: *N*-(2-diethylaminoethyl)-5-iodoindole-2-carboxamide (9)**

The compound **9** was prepared according to the procedure described for the preparation of the compound **2,** using ethyl 5-iodoindole-2-carboxylate **(8)** (Beshore, D. C. and Dinsmore, C: J., Synth. Commun., 2003, 33, 2423-2427) as starting material and heating the reaction medium at reflux for 3 hours. Yield: 50%; melting point: 208-210˚C; Rf: 0.53 ($Al_2O_3$, dichloromethane/ethanol (97/3, v/v)); $^1H$ NMR (200 MHz, $d_6$-DMSO) δ 1.03 (t, 6H, *J* = 7 Hz), 2.57 (m, 6H), 3.37 (m, 2H), 7.11 (s, I H), 7.42 (AB spectrum, 2H, *J* = 8.5 Hz), 8.09 (s, 1H), 8.52 (t, 1H, *J* = 6 Hz), 11.82 (s, 1H); $^{13}C$ NMR (50 MHz, $d_6$-DMSO) δ 11.9 (2C), 37.3, 46.8 (2C), 51.5, 83.3, 101.2, 114.7, 129.8, 129.9, 131.0, 132.8, 135.3, 160.6; IR (KBr) ν $cm^{-1}$ : 1411, 1547, 1635, 2801, 2965, 3250, 3418; MS (m/z, %) 385 ($M^+$, 2), 86 (100), 58 (2).

**Stage B: *N*-(2-diethylaminoethyl)-5-iodoindole-2-carboxamide hydrochloride (10)**

The compound **10** was prepared according to the procedure described for the preparation of the compound **3,** using *N*-(2-diethylaminoethyl)-5-iodoindole-2-carboxamide **(9)** as starting material. Yield: 81%; melting point: 217-219˚C; $^1H$ NMR (400 MHz, $CDCl_3$) δ 1.29 (m, 6H), 3.28 (m, 6H), 3.72 (m, 2H), 7.22 (s, 1H), 7.31 (d, 1H, *J* = 8.5 Hz), 7.48 (d, 1H, *J* = 8. MHz), 8.06 (s, 1H), 9.16 (bs, 1H), 10.59 (bs, 1H), 11.94 (s, 1H); IR (KBr) ν $cm^{-1}$: 1544, 1646, 2468, 2569, 3228. Anal. Calculated for $C_{15}H_{20}IN_3O·HCl$ ; C, 42.72; H, 5.02; N, 9.96. Found: C, 43.47; H, 5.20; N, 10.12.

## EXAMPLE 4

Synthesis of *N*-(2-diethylaminoethyl)-4-iodobenzo[b]thiophene-2-carboxamide hydrochloride **(13)**

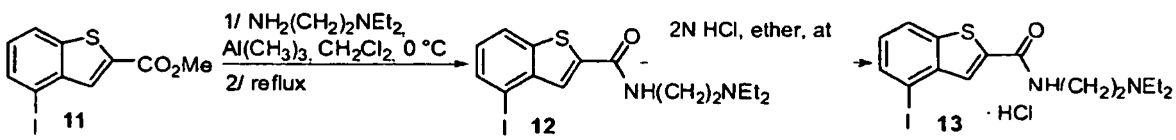


**Stage A: *N*-(2-diethylaminoethyl)-4-iodobenzo[b]thiophene-2-carboxamide (12)**

The compound **12** was prepared according to the procedure described for the preparation of the compound **2,** using methyl 4-iodobenzo[*b*]thiophene-2-carboxylate **(11)** (Bridges, A. J., Lee, A., Maduakor, E. C. and Schwartz, C. E., Tetrahedron Lett., 1992, 33, 7499-7502) as starting material and heating the reaction medium at reflux for 24 hours. Yield: 72%; Rf: 0.58 ($Al_2O_3$, dichloromethane/ethanol (97/3, v/v)); melting point: 76-78˚C; $^1H$ NMR (400 MHz, $CDCl_3$) δ 1.09 (t, 6H, *J* = 7 Hz), 2.60 (q, 4H, *J* = 7 Hz), 2.68 (t, 2H, *J* = 7 Hz), 3.50 (q, 2H, *J* = 7 Hz), 7.08 (bs, 1H), 7.1 (t, 1H, *J* = 8 Hz), 7.80 (m, 3H); $^{13}C$ NMR (100 MHz, $CDCl_3$) δ 12.2 (2C), 37.5, 46.9 (2C), 51.1, 91.4, 122.7, 127.2, 128.4, 134.9, 139.5, 140.0, 142.2, 161.7; IR (KBr) ν $cm^{-1}$: 1560, 1625, 2963, 3287; MS (m/z, %) 402 ($M^+$, 1), 86 (100).

**Stage B: *N*-(2-diethylaminoethyl)-4-iodobenzo[b]thiophene-2-carboxamide hydrochloride (13)**

The compound **13** was prepared according to the procedure described for the preparation of the compound **3,** using *N*-(2-diethylaminoethyl)-4-iodobenzo[b]thiophene-2-carboxamide **(12)** as starting material. Yield: 72%; melting point: 163-165˚C; $^1H$ NMR (200 MHz, $d_6$-DMSO) δ 1.28 (t, 6H, *J* = 7 Hz), 3.25 (m, 6H), 3.70 (m, 2H), 7.26 (t, 1H, *J* = 8 Hz), 7.92 (d, 1H, *J* = 8 Hz), 8.00 (d, 1H, *J* = 8 Hz), 8.22 (s, 1H), 9.42 (m, 1H), 10.29 (m, 1H); IR (KBr) ν $cm^{-1}$: 1535, 1648, 3249. Anal. Calculated for $C_{15}H_{19}IN_2OS·HCl$: C, 41.06; H, 4.59; N, 6.39. Found: C, 41.14; H, 4.79; N, 6.21.

## EXAMPLE 5

Syntheses of *N*-(2-diethylaminoethyl)-3-iodoimidazo[1,2-a]pyridine-2-carboxamide dihydrochloride **(16)** and of *N*-(2-diethylaminoethyl)-3-(tributylstannyl)imidazo[1,2-*a*]pyridine-2-carboxamide **(17)**

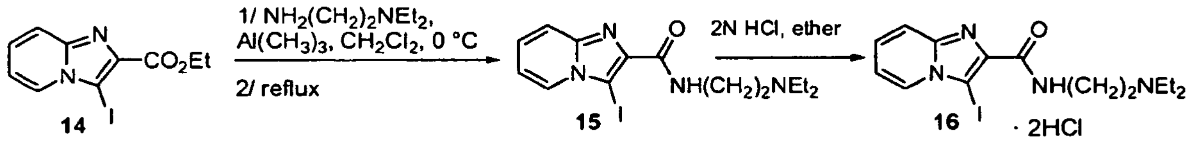


14 15 16

**Stage A: *N*-(2-diethylaminoethyl)-3-iodoimidazo[1,2-a]pyridine-2-carboxamide (15)**

The compound **15** was prepared according to the procedure described for the preparation of the compound **2,** using ethyl 3-iodoimidazo[1,2-*a*]pyridine-2-carboxylate **(14)** (Enguchard, C., Renou, J. L., Collot, V., Hervet, M., Rault, S. and Gueiffier, A., J. Org. Chem., 2000, 65, 6572-6575) as starting material and heating the reaction medium at reflux for 5 hours. Yield: 70%; viscous liquid; Rf: 0.41 ($Al_2O_3$, dichloromethane/ethanol (98/2, v/v)); $^1H$ NMR (200 MHz, $CDCl_3$) δ 0.99 (t, 6H, *J* = 7 Hz), 2.54 (q, 4H, *J* = 7 Hz), 2.63 (t, 2H, *J* = 6.5 Hz), 3.48 (q, 2H, *J* = 6.5 Hz), 6.89 (t, 1H, *J* = 7 Hz), 7.24 (m, 1H), 7.48 (d, 1H, *J* = 9 Hz), 7.77 (m, 1H), 8.19 (d, 1H, *J* = 7 Hz); $^{13}C$ NMR (50 MHz, $CDCl_3$) δ 12.2 (2C), 37.2, 47.2 (2C), 51.9, 64.1, 113.7, 117.9, 126.4, 126.6, 139.1, 146.7, 161.5; IR ($CCL_4$) ν $cm^{-1}$: 1217, 1250, 1546, 1662, 2924, 3450; MS (m/z, %): 387 ($M^+$ + 1, 1), 271 (10), 259 (12), 243 (5), 116 (6), 99 (6), 86 (100), 58 (13).

Stage B: *N*-(2-diethylaminoethyl)-3-iodoimidazo[1,2-a]pyridine-2-carboxamide dihydrochloride (16)

The compound 16 was prepared according to the procedure described for the preparation of the compound 3, using *N*-(2-diethylaminoethyl)-3-iodoimidazo[1,2-a]pyridine-2-carboxamide (15) as starting material. Yield: 60%; melting point: 128-130˚C; $^1H$ NMR (200 MHz, $d_6$-DMSO) δ 1.31 (t, 6H, J= 7 Hz), 3.25 (m, 6H), 3.72 (m, 2H), 7.25 (t, 1H, J = 7 Hz), 7.59 (m, 1H), 7.72 (d, 1H, J = 9 Hz), 8.56 (d, 1H, J = 7 Hz), 8.92 (bs, 1H), 10.49 (bs, 1H); IR (KBr) ν $cm^{-1}$: 1535, 1669, 2660, 2974, 3265, 3422. Anal. Calculated for $C_{14}H_{19}IN_4O\cdot 2HCl$: C, 36.62; H, 4.61; N, 12.20. Found: C, 36.40; H, 4.92; N, 11.97.

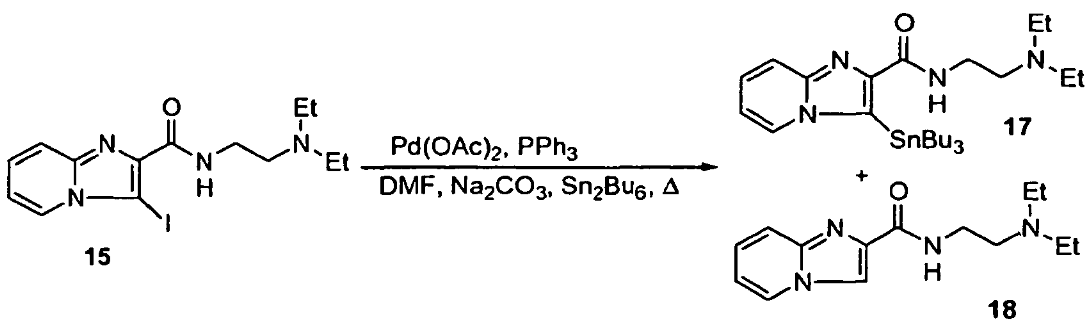


15 17 18

**Stage C: *N*-(2-diethylaminoethyl)-3-(tributylstannyl)imidazo[1,2-a]pyridine-2-carboxamide (17)**

Bis(tributyltin) (1.30 ml, 2.57 mmol), triphenylphosphine (36 mg, 0.14 mmol), sodium carbonate (149 mg, 1.41 mmol) and palladium diacetate (20 mg, 0.09 mmol) are added, under argon, to a solution of iodinated compound **15** (546 mg, 1.41 mmol) in anhydrous dimethylformamide (8 ml). The solution is heated at 100˚C for 4 hours and then at 135˚C for 24 hours (the solution becomes black). After returning to ambient temperature, the solution is filtered through Celite® 521 and the filter residue is washed with dimethylformamide (8 ml). The filtrate is evaporated under vacuum. The residue obtained is chromatographed on a column of alumina eluted with a dichloromethane/ethanol (99/1, v/v) mixture, to result, in order to elution, in:

***N*-(2-diethylaminoethyl)-3-(tributylstannyl)imidazo[1,2-a]pyridine-2-carboxamide (17)**

(45 mg, 0.08 mmol); yield: 6%; viscous liquid; Rf: 0.26 ($Al_2O_3$, dichloromethane/ethanol: 99/1 (v/v)); $^1H$ NMR (400 MHz, $CDCl_3$) δ 0.86 (t, 9H, *J* = 7 Hz), 1.04 (t, 6H, *J* = 7 Hz), 1.37-1.17 (m, 12H), 1.53 (m, 6H), 2.62 (q, 4H, *J* = 7 Hz), 2.69 (t, 2H, *J* = 6 Hz), 3.51 (q, 2H, *J* = 6 Hz), 6.74 (t, 1H, *J* = 7 Hz), 7.17 (m, 1H), 7.56 (d, 1H, *J* = 9 Hz), 7.63 (bs, 1H), 8.20 (d, 1H, *J* = 7 Hz); $^{13}C$ NMR (100 MHz, $CDCl_3$) δ 11.8 (2C), 11.9 (3C, $^1J_{Sn-C}$= 365 Hz), 13.7 (3C), 27.3 (3C, $^3J_{Sn-C}$= 65 Hz), 29.1 (3C, $^2J_{Sn-C}$= 21 Hz), 37.0, 46.9 (2C), 51.9, 112.4, 118.1, 125.2, 127.7, 128.3, 148.1, 148.3, 164.1; MS (m/z, %): 493 ($M^+$-57.9), 100 (41), 86 (100), 58 (20).

***N*-(2-diethylaminoethyl)imidazo[1,2-a]pyridine-2-carboxamide (18)**

(193 mg, 0.74 mmol); yield: 52%; viscous liquid; Rf: 0.16 ($Al_2O_3$, dichloromethane/ethanol (99/1, (v/v)); $^1H$ NMR (400 MHz, $CDCl_3$) δ 1.09 (t, 6H, *J* = 7 Hz), 2.69 (m, 6H), 3.58 (q, 4H, *J* = 6 Hz),), 6.87 (t, 1H, *J*= 7 Hz), 7.28 (m, 1H), 7.60 (d, 1H, *J* = 9 Hz), 7.80 (bs, 1H), 8.19 (m, 2H); $^{13}C$ NMR (100 MHz, $CDCl_3$) δ 10.6 (2C), 35.9, 45.9 (2C), 50.8, 111.8, 113.2, 117.1, 124.9, 125.5, 139.1, 143.5, 161.8; IR ($CCl_4$) ν $cm^{-1}$: 1489, 1565, 1669, 2971; MS (m/z, %): 260 ($M^+$, 2), 145 (9), 99 (21), 86 (100), 58 (18).

**EXAMPLE 6**

Synthesis of *N*-(2-diethylaminoethyl)-6-iodoimidazo[1,2-a]pyridine-2-carboxamide dihydrochloride (21)

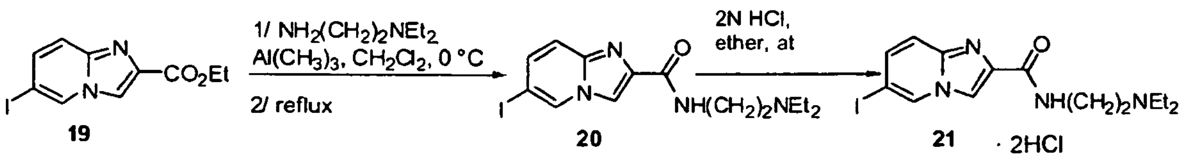


19 20 21 · 2HCl

**Stage A: *N*-(2-diethylaminoethyl)-6-iodoimidazo[1,2-a]pyridine-2-carboxamide (20)**

The compound **20** was prepared according to the procedure described for the preparation of the compound **2,** using ethyl 6-iodoimidazo[1,2-*a*]pyridine-2-carboxylate **(19)** (Sunberg, R. J., Biswas, S., Murthi, K. K., Rowe, D., McDall, J. W. and Dzimianski, M. T., J. Med. Chem., 1998, 41, 4317-4328) as starting material and heating the reaction medium at reflux for 72 hours. Yield: 83%; viscous liquid; Rf: 0.55 ($Al_2O_3$, dichloromethane/ethanol (97/3, v/v)); $^1H$ NMR (200 MHz, $CDCl_3$) δ 1.03 (t, 6H, *J* = 7 Hz), 2.57 (q, 4H, *J* = 7 Hz), 2.66 (t, 2H, *J* = 6.5 Hz), 3.51 (q, 2H, *J* = 6.5 Hz), 7.34 (m, 2H), 7.68 (m, 1H), 8.06 (s, 1H), 8.39 (t, 1H, *J* = 1.5 Hz); $^{13}C$ NMR (50 MHz, $CDCl_3$) δ 12.2 (2C), 37.3, 47.2 (2C), 51.9, 76.5, 113.4, 118.9, 130.9, 133.5, 140.2, 142.6, 161.8; IR ($CCl_4$) ν $cm^{-1}$: 1218, 1251, 1562, 1670, 2971, 3430; MS (m/z, %): 99 ($M^+$ - 287, 11), 86 (100), 58 (11).

**Stage B: *N*-(2-diethylaminoethyl)-6-iodoimidazo[1,2-a]pyridine-2-carboxamide dihydrochloride (21)**

The compound **21** was prepared according to the procedure described for the preparation of the compound **3,** using *N*-(2-diethylaminoethyl)-6-iodoimidazo[1,2-a]pyridine-2-carboxamide **(20)** as starting material. Yield: 80%; melting point: 208-210°C; $^1H$ NMR (200 MHz, $d_6$-DMSO) δ 1.21 (t, 6H, *J* = 7 Hz), 3.17 (m, 6H), 3.58 (m, 2H), 7.57 (m, 2H), 8.41 (s, 1H), 9.00 (t, 1H, *J* = 5 Hz), 9.09 (s, 1H), 9.95 (m, 1H); IR (KBr) ν $cm^{-1}$ : 1289, 1600, 1659, 2650, 3100-2900, 3426. Anal. Calculated for $C_{14}H_{19}IN_4O{\cdot}2HCl{\cdot}2H_2O$: C, 33.96; H, 5.09; N, 11.31. Found: C, 34.33; H, 5.20; N, 11.06.

**EXAMPLE 7**

Synthesis of *N*-(2-diethylaminoethyl)-5-iodobenzimidazole-2-carboxamide dihydrochloride **(26)**

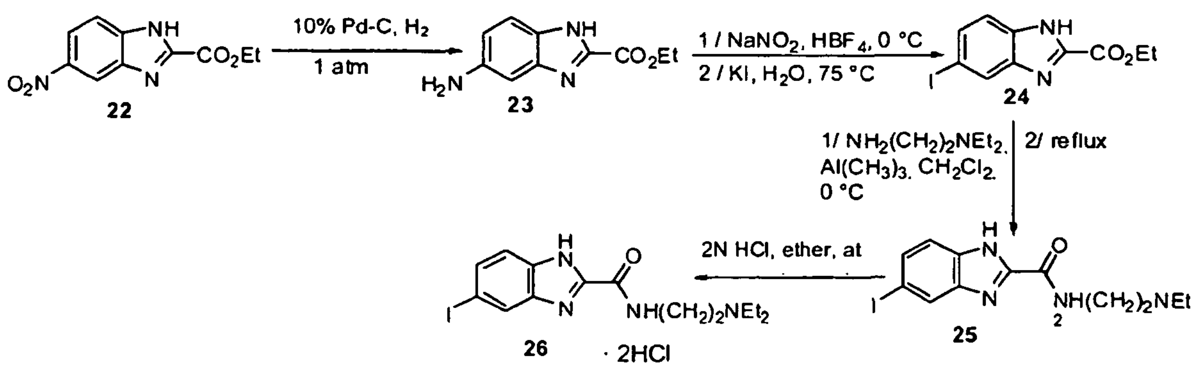


22 23 24 25 26 · 2HCl

**Stage A: Ethyl 5-aminobenzimidazole-2-carboxylate (23)**

10% palladium-on-charcoal (760 mg) is added to a solution of ethyl 5-nitrobenzimidazole-2-carboxylate **(22)** (Buchel, K. H. Z., *Naturforsch.,* B. **1970,** *25*, 945-953) (7.00 g, 29.7 mmol) in ethyl acetate (213 ml). The solution is hydrogenated at atmospheric pressure for 18 hours. The reaction medium is filtered through Celite® 521, the filter residue is washed with ethanol and the filtrates are then evaporated to dryness. The orange solid obtained is purified by chromatography on alumina eluted with a dichloromethane/ethanol (97/3, v/v) mixture, to result in the pale orange solid **23** (2.22 g, 10.8 mmol). Yield: 36%; Rf: 0.25 ($Al_2O_3$, dichloromethane/ethanol (97/3, v/v)); melting point: 147-149°C; $^1H$ NMR (200 MHz, $CDCl_3$) δ 1.41 (t, 3H, *J*= 7 Hz), 4.46 (q, 2H, *J*= 7 Hz), 6.76 (m, 2H), 7.58 (d, 1H, *J* = 9 Hz); IR (KBr) ν $cm^{-1}$: 1269, 1701, 3370; MS (m/z, %) 205 ($M^+$, 53), 159 (100), 133 (52), 131 (44), 105 (35), 83 (22), 78 (20), 52 (21).

**Stage B: Ethyl 5-iodobenzimidazole-2-carboxylate (24)**

A solution of sodium nitrite (750 mg, 11.0 mmol) in water (3 ml) is added dropwise to a solution of the amine **23** (2.20 g, 10.7 mmol) in 50% tetrafluoroboric acid (40 ml, 32.1 mmol) at 0°C. The solution is stirred at 0°C for 1 hour and then filtered, to result in a white precipitate corresponding to the diazonium salt (3.26 g, 10.7 mmol). The latter is dissolved in water (67 ml) and then a solution of potassium iodide (2.79 g, 16.8 mmol) in water (14 ml) is added. The mixture is heated to 70°C. When evolution of gas has ceased (2 hours) and after returning to ambient temperature, the medium is basified using a saturated aqueous sodium carbonate solution (pH = 8-9). The solution is extracted with dichloromethane (2 x 80 ml) and the combined organic extracts are washed with a 5% aqueous sodium hydrogensulphite solution (2 × 40 ml), dried over magnesium sulphate, filtered and evaporated to give a brown powder. The product is purified by chromatography on alumina eluted with a dichloromethane/ethanol (99/1, v/v) mixture, to result in the yellow precipitate **24** (407 mg, 1.29 mmol). Yield: 12%; Rf: 0.57 ($Al_2O_3$, dichloromethane/ethanol (99/1 v/v)); melting point: 166-168°C; $^1H$ NMR (200 MHz, $d_6$-DMSO) δ 1.40 (t, 3H, *J* = 7 Hz), 4.50 (q, 2H, *J* = 7 Hz), 7.5 (d, 1H, *J* = 9 Hz), 7.66 (dd, 1H, *J* = 9, 2 Hz), 8.09 (d, 1H, *J* = 2 Hz); IR (KBr) ν $cm^{-1}$: 1246, 1310, 1515, 1697, 3287; MS (m/z, %) 316 ($M^+$, 56), 270 (30), 244 (100), 117 (38), 90 (25), 63 (19).

**Stage C: *N*-(2-diethylaminoethyl)-5-iodobenzimidazole-2-carboxamide** (25)

The compound **25** was prepared according to the procedure described for the preparation of the compound **2,** using ethyl 5-iodobenzimidazole-2-carboxylate **(24)** as starting material and heating the reaction medium at reflux for 6 hours. Yield 82%; Rf: 0.38 ($Al_2O_3$, dichloromethane/ethanol (97/3, v/v)); melting point: 136-138°C; $^1H$ NMR (200 MHz, $CDCl_3$) δ 1.04 (t, 6H, *J* = 7 Hz), 2.64 (q, 4H, *J*= 7 Hz), 2.76 (m, 2H), 3.62 (m, 2H), 7.36 (d, 1H, *J* = 8.5 Hz), 7.52 (d, 1H, *J* = 8.5), 7.96 (bs, 1H), 8.28 (m, 1H); IR ν $cm^{-1}$: 1420, 1551, 1664, 2966, 3175; MS (m/z, %) 386 ($M^+$, 1), 86 (100), 58 (11).

**Stage D: *N*-(2-diethylaminoethyl)-5-iodobenzimidazole-2-carboxamide dihydrochloride (26)**

The compound **26** was prepared according to the procedure described for the preparation of the compound **3,** using *N*-(2-diethylaminoethyl)-5-iodobenzimidazole-2-carboxamide **(25)** as starting material. Yield: 82%; melting point: 217-219°C; $^1H$ NMR (200 MHz, $d_6$-DMSO) δ 1.27 (t, 6H, *J* = 7 Hz), 3.22 (m, 6H), 3.74 (m, 2H), 7.52 (d, 1H, *J* = 8.5 Hz), 7.64 (dd, 1H, *J* = 8.5, 1 Hz), 8.03 (d, 1H, *J* = 1 Hz), 9.36 (m, 1H), 10.45 (m, 1H); IR (KBr) ν $cm^{-1}$: 1593, 1683, 2979. Anal. Calculated for $C_{14}H_{19}IN_4O\cdot 2HCl\cdot H_2O$: C, 35.24; H, 4.86; N, 11.74. Found: C, 35.38; H, 4.77; N, 11.79.

## EXAMPLE 8

Syntheses of *N*-(2-diethylaminoethyl)-6-iodoquinoline-2-carboxamide dihydrochloride **(31)** and of *N*-(2-diethylaminoethyl)-6-(tributylstannyl)quinoline-2-carboxamide **(32)**

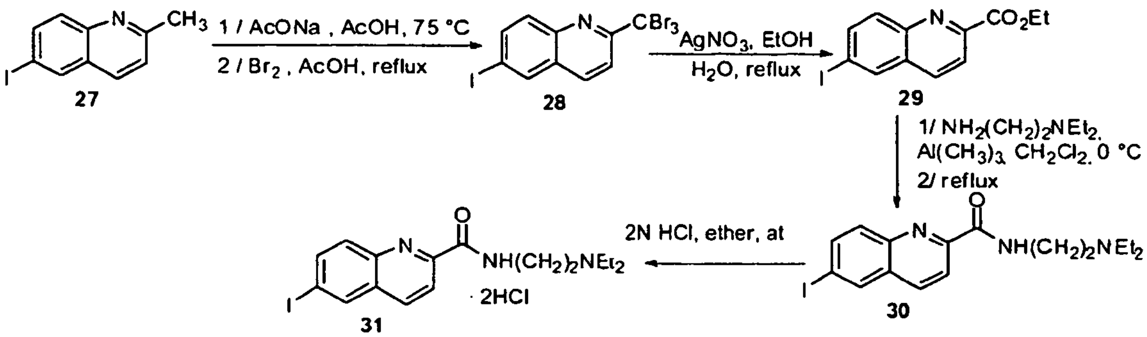


**Stage A: 2-tribromomethyl-6-iodoquinoline (28)**

6-Iodo-2-methylquinoline **(27)** (Petrow, V. and Sturgeon, B., J. Chem. Soc., 1954, 570-574) (7.12 g, 26.5 mmol) and sodium acetate (12.1 g) are dissolved in acetic acid (15 ml). The mixture is heated under argon at 75˚C for 10 minutes. A solution of dibromine (4.45 ml, 86.6 mmol) in acetic acid (13 ml) is added dropwise. On completion of the addition, the reaction medium is heated at reflux for 2 hours and then cooled to ambient temperature. The contents of the round-bottomed flask are poured onto crushed ice (200 g) and stirred at ambient temperature for 18 hours. The precipitate formed is filtered off, washed with water (60 ml) and placed in a dessicator for 4 hours, to result in the orange solid **28** (9.10 g, 18.0 mmol). Yield: 68%; Rf: 0.86 ($Al_2O_3$ cyclohexane/ethyl acetate (8/2, v/v)); melting point: 124-126˚C; $^1$H NMR (400 MHz, $CDCl_3$) δ 7.92 (d, 1H, *J* = 8.5 Hz), 8.03 (d, 1H, *J* = 8.5 Hz), 8.14 (d, 1H, *J* = 9 Hz), 8.26 (m, 2H); $^{13}$C NMR (100 MHz, $CDCl_3$) δ 40.8, 94.5, 118.6, 129.1, 131.7, 135.9, 136.4, 139.3, 144.0, 159.0; IR (KBr) ν $cm^{-1}$: 1293, 1480, 1584; MS (m/z, %) 509 ($M^+$+6, 1), 507 ($M^+$+4, 2), 505 ($M^+$+2, 3), 503 ($M^+$, 1), 428 (40), 426 (77), 424 (45), 348 (81), 346 (81), 255 (100), 140 (97), 127 (90), 82 (90), 57 (89).

**Stage B: Ethyl 6-iodoquinoline-2-carboxylate (29)**

A solution of siliver nitrate (9.05 g, 53.8 mmol) in water (83 ml) is added dropwise to a solution of the iodinated compound **28** (9.10 g, 18.0 mmol) in ethanol (132 ml). The mixture is heated at reflux for 30 minutes. The solution is cooled to ambient temperature, filtered and acidified with an aqueous hydrochloric acid solution (1.5M, 5 ml). The filtrate is evaporated by half, basified with a saturated aqueous sodium carbonate solution (100 ml) and extracted with ether, and the ether extract is dried over magnesium sulphate and evaporated. The product is purified by chromatography on alumina eluted with an ethyl acetate/cyclohexane (8/2, v/v) mixture, to result in a dark yellow solid **29** (3.96 g, 12.1 mmol). Yield: 67%; Rf: 0.97 ($Al_2O_3$, ethyl acetate/cyclohexane (8/2, v/v)); melting point: 119-121˚C; $^1$H NMR (200 MHz, $d_6$-DMSO) δ 1.42 (t, 3H, *J* = 7 Hz), 4.46 (q, 2H, *J* = 7 Hz), 7.97 (d, 1H, *J* = 9 Hz), 8.16 (m, 2H), 8.55 (d, 1H, *J* = 9 Hz), 8.64 (s, 1H); $^{13}$C NMR (100 MHz, $CDCl_3$) δ 14.4, 62.4, 95.0, 121.8, 130.7, 132.2, 135.6, 136.4, 139.1, 146.4, 148.7, 165.0; IR (KBr) ν $cm^{-1}$: 1307, 1714; MS (m/z, %) 327 ($M^+$, 17), 283 (19), 255 (100), 127 (28), 100 (11).

**Stage C: *N*-(2-diethylaminoethyl)-6-iodoquinoline-2-carboxamide (30)**

The compound **30** was prepared according to the procedure described for the preparation of the compound **2,** using ethyl 6-iodoquinoline-2-carboxylate **(29)** as starting material and heating the reaction medium at reflux for 6 hours. Yield: 69%; Rf: 0.56 ($Al_2O_3$, dichloromethane/ethanol (97/3, v/v)); melting point: 75-77˚C; $^1$H NMR (400 MHz, $CDCl_3$) δ 1.09 (t, 6H, *J* = 7 Hz), 2.63 (q, 4H, *J* = 7 Hz), 2.73 (t, 2H, *J* = 7 Hz), 3.58 (q, 2H, *J* = 7 Hz), 7.81 (d, 1H, *J* = 9 Hz), 7.98 (dd, 1H, *J* = 9 and 2 Hz), 8.16 (d, 1H, *J* = 8.5 Hz), 8.26 (d, 1H, J = 2 Hz), 8.30 (d, 1H, J = 8.5 Hz), 8.57 (bs, 1H); $^{13}$C NMR (100 MHz, $CDCl_3$) δ 12.1 (2C), 37.5, 47.2 (2C), 51.7, 93.7, 119.6, 130.7, 131.8, 136.1, 136.5, 139.4, 145.4, 150.5, 164.1; IR (KBr) ν $cm^{-1}$: 1526, 1663, 2963, 3400; MS (m/z, %) 386 ($M^+$, 1), 86 (100), 58 (11).

Stage D: N (2-diethylaminoethyl)-6-iodoquinoline-2-carboxamide dihydrochloride (31)

The compound 31 was prepared according to the procedure described for the preparation of the compound 3, using N-(2-diethylaminoethyl)-6-iodoquinoline-2-carboxamide (30) as starting material. Yield: 69%; melting point: 104-106˚C; $^1$H NMR (200 MHz, $d_6$-DMSO) δ 1.28 (t, 6H, J = 7 Hz), 3.24 (m, 6H), 3.77 (q, 2H, J = 7 Hz), 7.92 (d, 1H, *J* = 9 Hz), 8.21 (m, 2H), 8.57 (d, 1H, *J* = 9 Hz), 8.65 (s, 1H), 9.34 (m, 1H), 10.08 (m, 1H); IR ν $cm^{-1}$: 1384, 1523, 1684, 3415. Anal. Calculated for $C_{16}H_{20}IN_3O\cdot 2HCl\cdot 2.5H_2O$: C, 37.30; H, 5.28; N, 8.16. Found: C, 37.35; H, 5.15; N, 8.23.

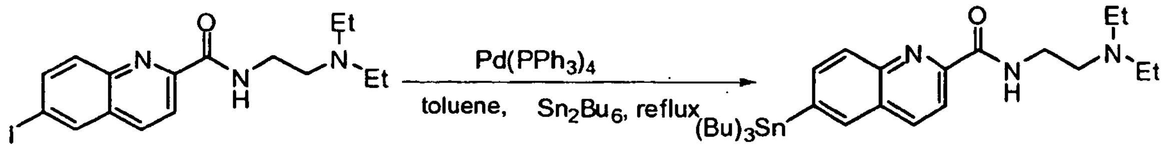


30 32

**Stage E: *N*-(2-diethylaminoethyl)-6-(tributylstannyl)quinoline-2-carboxamide (32)**

Bis(tributyltin) (443 μl, 1.18 mmol) and a spatula tip of tetrakis(triphenylphosphine)palladium are added, under argon, to a solution of the iodinated compound **30** (332 mg, 0.87 mmol) in anhydrous toluene (15 ml) degassed beforehand under argon. The solution is heated at reflux for 8 hours. After returning to ambient temperature, the solution is filtered through Celite® 521 and the filtrate is evaporated under vacuum. The residue obtained is chromatographed on a column of alumina eluted with an ethyl acetate/cyclohexane (6/4, v/v) mixture, to result in the tributylstannane **32** (282 mg, 0.50 mmol); yield: 58%; viscous liquid; Rf: 0.86 (ethyl acetate/cyclohexane (6/4, v/v)); $^1$H NMR (400 MHz, $CDCl_3$) δ 0.90 (t, 9H, *J* = 7 Hz), 1.14 (m, 12H), 1.36 (m, 6H), 1.57 (m, 6H), 2.69 (q, 4H, *J* = 7 Hz), 2.80 (t, 2H, *J* = 6 Hz), 3.63 (q, 2H, *J* = 6 Hz), 7.84 (d, 1H, *J* = 8.5 Hz), 7.96 (s, 1H), 8.04 (d, I H, *J* = 8.5 Hz), 8.29 (AB spectrum, 2H, *J* = 8.5 Hz), 8.67 (m, 1H); $^{13}$C NMR (100 MHz, $CDCl_3$ ) δ 10.0 (3C, $^1J_{Sn-C}$= 333 Hz), 11.8 (2C), 13.7 (3C), 27.4 (3C, $^3J_{Sn-C}$= 56 Hz), 29.2 (3C, $^2J_{Sn-C}$ 20 Hz), 37.4, 47.4 (2C), 51.8, 118.7, 128.2, 128.9, 136.1, 136.9, 137.3, 143.4, 146.5, 149.6, 164.8; IR ($CCl_4$) ν $cm^{-1}$: 1519, 1680, 2926, 2960; ESI-MS m/z 562.2 $[M+H]^+$.

<u>**EXAMPLE 9**</u>

Synthesis of *N*-(2-diethylaminoethyl)-1,4-dihydro-6-iodo-4-oxoquinoline-3-carboxamide hydrochloride **(35)**

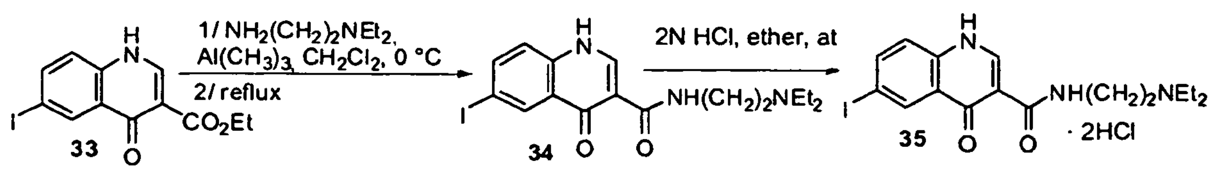


33 34 35

**Stage A: *N*-(2-diethylaminoethyl)-1,4-dihydro-6-iodo-4-oxoquinoline-3-carboxamide (34)**

The compound **34** was prepared according to the procedure described for the prpearation of the compound **2,** using ethyl 1,4-dihydro-6-iodo-4-oxoquinoline-3-carboxylate **(33)** (Lin, A. J. and Loo, T. L., J. Med. Chem., 1978, 21, 268-272) as starting material and heating the reaction medium at reflux for 5 hours. Yield: 81%; Rf: 0.04 ($Al_2O_3$, dichloromethane/ethanol (97/3, v/v)); melting point: 234-236°C; $^1$H NMR (400 MHz, *d*$_6$-DMSO) δ 1.13 (t, 6H, *J* = 7 Hz), 2.85 (m, 6H), 3.69 (q, 2H, *J* = 6 Hz), 7.33 (d, 1H, *J* = 8.5 Hz), 7.87 (d, 1H, *J* = 8.5 Hz), 8.49 (s, 1H), 8.67 (s, 1H), 10.32 (bs, I H); $^{13}$C NMR (100 MHz, *d*$_6$-DMSO) δ 11.4 (2C), 36.4, 46.6 (2C), 51.4, 89.6, 111.3, 121.5, 127.9, 133.8, 138.7, 140.5, 144.0, 164.2, 174.4; IR (KBr) ν $cm^{-1}$: 1507, 1551, 1633, 2965, 3061; MS (m/z, %) 413 ($M^+$, 1), 86 (100), 58 (10).

**Stage B: *N*-(2-diethylaminoethyl)-1,4-dihydro-6-iodo-4-oxoquinoline-3-carboxamide dihydrochloride (35)**

The compound **35** was prepared according to the procedure described for the preparation of the compound **3,** using *N*-(2-diethylaminoethyl)-1,4-dihydro-6-iodo-4-oxoquinoline-3-carboxamide **(34)** as starting material. Yield: 92%; melting point: 164-166°C; $^1$H NMR (200 MHz, *d*$_6$-DMSO) δ 1.27 (t, 6H, *J* = 7 Hz), 3.24 (m, 6H), 3.73 (m, 2H), 7.62 (d, 1H, *J* = 9 Hz), 8.11 (dd, 1H, *J* = 9 and 1.5 Hz), 8.51 (d, 1H, *J* = 1.5 Hz), 8.80

(d, 1H, *J* = 6.5 Hz), 10.07 (m, 2H); IR (KBr) ν $cm^{-1}$: 1521, 1618, 1654, 3028, 3514. Anal. Calculated for $C_{16}H_{20}IN_3O_2 \cdot 2HCl \cdot 1.5H_2O$: C, 37.45; H, 4.91; N, 8.19. Found: C, 37.42; H, 5.08; N, 8.12.

<u>**EXAMPLE 10**</u>

Synthesis of *N*-(2-diethylaminoethyl)-5-iodoisoquinoline-3-carboxamide dihydrochloride **(39)**

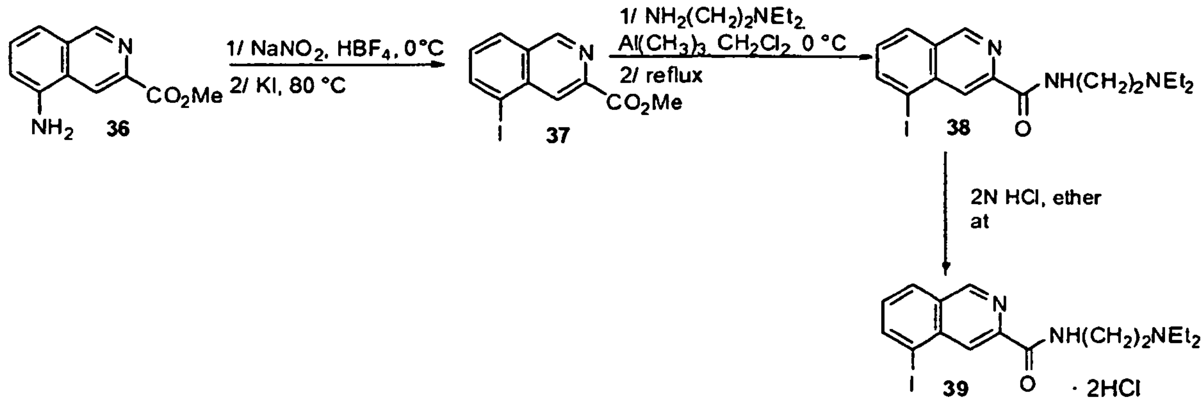


**Stage A: Methyl 5-iodoisoquinoline-3-carboxylate (37)**

Concentrated hydrochloric acid (576 μl) is added to a suspension of methyl 5-aminoisoquinoline-3-carboxylate **(36)** (Lee, C. H., Bayburt, E. K., DiDomenico, S., Drizin, I., Gomtsyan, A. R., Koenig, J. R., Pemer, R. J., Schmidt, R. G., Turner, S. C., White, T. K. and Zheng, G. Z., US Patent 656417, 2003) (500 mg, 2.47 mmol) in water (4 ml). The solution is cooled to 0°C and then a solution of sodium nitrite (166 mg, 2.47 mmol) in water (3 ml) is rapidly added. After stirring at 0°C for 30 minutes, 50% tetrafluoroboric acid (311 μl, 2.47 mmol) is added. Stirring is continued for 30 minutes. The precipitate formed is filtered off. The diazonium salt obtained is dissolved in water (12 ml) and then a solution of potassium iodide (453 mg, 3.10 mmol) in water (5 ml) is rapidly added. The reaction medium is heated to 80°C. When the evolution of gas has ceased (2 hours) and after returning to ambient temperature, the reaction mixture is extracted with dichloromethane (3 × 30 ml) and the organic extract is washed with a 5% aqueous sodium hydrogensulphite solution (40 ml), dried over magnesium sulphate, filtered and evaporated. The product is purified by chromatography on alumina eluted with a dichloromethanol/ethanol (99/1, v/v) mixture, to result in an orange precipitate **37** (190 mg, 0.61 mmol). Yield: 25%; Rf: 0.64 ($Al_2O_3$, dichloromethane/ethanol (99/1, v/v)); melting point: 165-167°C; $^1$H NMR (400 MHz, $d_6$-DM-SO) δ 4.00 (s, 3H), 7.65 (t, 1H, *J* = 8 Hz), 8.32 (d, 1H, *J* = 8 Hz), 8.50 (d, 1H, *J* = 8 Hz), 8.55 (s, 1H), 9.37(s, 1H); $^{13}$C NMR (100 MHz, $d_6$-DMSO) δ 52.6, 99.6, 126.1, 128.5, 130.5, 131.3, 136.4, 142.2, 142.8, 153.8, 165.2; IR (KBr) ν $cm^{-1}$: 1249, 1304, 1710; MS (m/z, %) 313 ($M^4$, 313), 283 (20), 255 (100), 127 (41), 100 (18), 74 (11).

**Stage B: *N*-(2-diethylaminoethyl)-5-iodoisoquinoline-3-carboxamide (38)**

The compound **38** was prepared according to the procedure described for the preparation of the compound **2,** using methyl 5-iodoisoquinoline-3-carboxylate **(37)** as starting material and heating the reaction medium at reflux for 4 hours. Yield: 72%; Rf: 0.47 ($Al_2O_3$, dichloromethane/ethanol (97/3, v/v)); viscous liquid; $^1$H NMR (400 MHz, $CDCl_3$) δ 1.08 (t, 6H, *J* = 7 Hz), 2.64 (q, 4H, *J* = 7 Hz), 2.74 (t, 2H, *J* = 7 Hz), 3.62 (q, 2H, *J* = 7 Hz), 7.40 (t, 1H, *J* = 8 Hz), 8.00 (d, 1H, *J* = 8 Hz), 8.31 (d, 1H, *J* = 8 Hz), 8.57 (m, 1H), 8.76 (s, 1H), 9.05 (s, 1H); $^{13}$C NMR (100 MHz, $CDCl_3$) δ 11.9 (2C), 37.5, 47.3 (2C), 51.8, 99.4, 123.8, 128.3, 129.7, 130.6, 138.1, 142.0, 145.8, 152.0, 164.5; IR (KBr) ν $cm^{-1}$: 1516, 1677, 2971; MS (m/z, %) 397 ($M^+$, 1), 86 (100), 58 (7).

**Stage C: *N*-(2-diethylaminoethyl)-5-iodoisoquinoline-3-carboxamide dihydrochloride (39)**

The compound **39** was prepared according to the procedure described for the preparation of the compound **3,** using *N*-(2-diethylaminoethyl)-5-iodoisoquinoline-2-carboxamide **(38)** as starting material. Yield: 72%; melting point: 152-154°C; $^1$H NMR (200 MHz, $d_6$-DMSO) δ 1.22 (t, 6H, *J* = 7 Hz), 3.25 (m, 6H), 3.74 (m, 2H), 7.63 (t, 1H, *J* = 8 Hz), 8.33 (d, 1H, *J* = 8 Hz), 8.49 (d, 1H, *J* = 8 Hz), 8.56 (s, 1H), 9.38 (m, 2H), 9.76 (m, 1H); IR (KBr) ν $cm^{-1}$: 1526, 1684, 3447, 3927. Anal. Calculated for $C_{16}H_{20}IN_3O \cdot 2HCl$: C, 40.87; H, 4.72; N, 8.94. Found: C, 40.91; H, 4.75; N, 8.84.

**EXAMPLE 11**

Synthesis of *N*-(2-diethylaminoethyl)-6-iodoquinoxaline-2-carboxamide dihydrochloride **(46)** and of *N*-(2-diethylaminoethyl)-6-(tributylstannyl)quinoxaline-2-carboxamide **(47)**

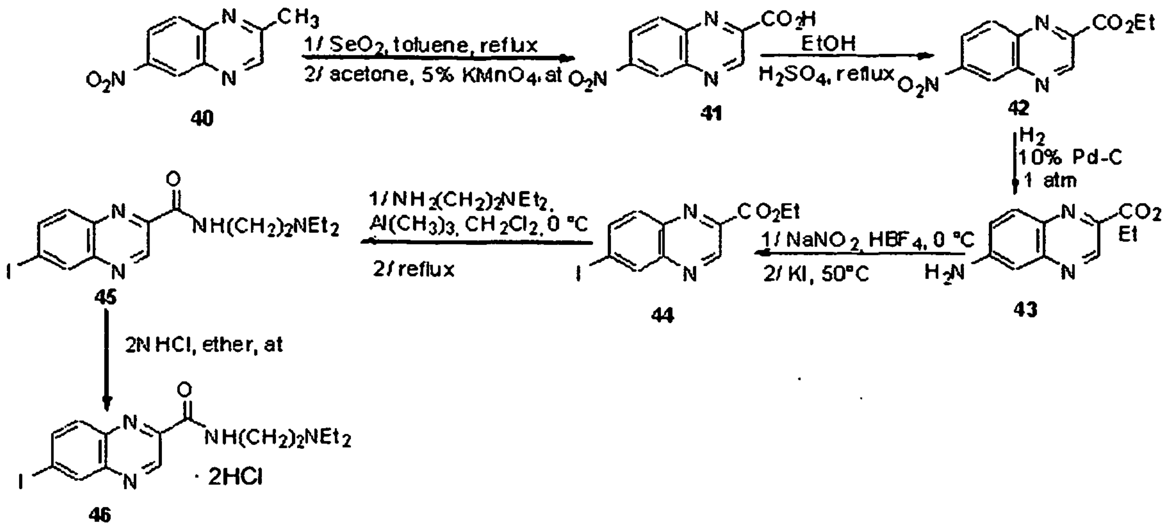


**Stage A: 6-nitroquinoxaline-2-carboxylic acid (41)**

Selenium dioxide (16.0 g, 0.14 mol) is added to a solution of 2-methyl-6-nitroquinoxaline **(40)** (Higashida, S., Sakurai, M., Yabe, Y., Nishihgaki, T., Komai, T. and Handa, H. ; Patent EP 0 587 311, 1994) (15.14 g, 80.1 mmol) in toluene (240 ml). The solution is heated at reflux for 2 hours. After returning to ambient temperature, the precipitate obtained is filtered off and washed with toluene (40 ml) and dichloromethane (50 ml). The precipitate (aldehyde) obtained is dissolved in acetone (400 ml) and a 5% aqueous sodium permanganate solution (540 ml) is added fractionwise. The solution is stirred at ambient temperature for 3 hours and then filtered. The filtrate is reduced to half under vacuum and the solution is extracted with ether. The aqueous phase is acidified (pH = 1) with concentrated hydrochloric acid. The precipitate thus obtained is filtered off and placed in a dessicator for 4 days, to result in the acid **41** (5.26 g, 24.0 mmol). Yield: 30%; melting point: 212-214°C; $^1$H NMR (200 MHz, $d_6$-DMSO) δ 8.51 (d, 1H, *J* = 9 Hz), 8.67 (dd, 1H, *J* = 2.5, 9 Hz), 9.01 (d, 1H, *J* = 2.5 Hz), 9.62 (s, 1H); $^{13}$C NMR (100 MHz, ($d_6$-DMSO) δ 124.3, 124.9, 132.1, 141.6, 143.2, 146.3, 147.3, 148.8, 164.6; IR (KBr) ν $cm^{-1}$: 1147, 1347, 1526, 1706, 3200-3500; MS (m/z, %) 219 ($M^+$, 37), 175 (100), 129 (37), 102 (39), 75 (37).

**Stage B: Ethyl 6-nitroquinoxaline-2-carboxylate (42)**

Concentrated sulphuric acid (750 μl) is added to a solution of the acid **41** (5.00 g, 22.8 mmol) in anhydrous ethanol (50 ml) under argon. The solution is heated at reflux for 7 hours. After returning to ambient temperature, the solution is evaporated under vacuum, a saturated aqueous sodium bicarbonate solution (50 ml) is added and then the mixture is extracted with dichloromethane. The organic phase is dried over magnesium sulphate and evaporated under vacuum, to produce the ester **42** (3.79 g, 15.3 mmol). Yield: 67%; melting point: 221-223°C; $^1$H NMR (200 MHz, $CDCl_3$) δ 1.58 (t, 3H, *J* = 7 Hz), 4.68 (q, 2H, *J* = 7 Hz), 8.53 (d, 1H, *J* = 9 Hz), 8.68 (dd, 1H, *J* = 2.5, 9 Hz), 9.14 (d, 1H, *J* = 2.5 Hz), 9.72 (s, 1H); IR (KBr) ν $cm^{-1}$: 1282, 1347, 1531, 1741; MS (m/z, %) 248 ($M^+$+1, 4), 203 (36), 175 (100), 128 (23), 101 (32), 75 (24).

**Stage C: Ethyl 6-aminoquinoxaline-2-carboxylate (43)**

10% palladium-on-charcoal (300 mg) is added to a solution of the nitrated ester **42** (2.93 g, 11.9 mmol) in ethanol (500 ml). The solution is hydrogenated at atmospheric pressure for 3 hours 45. The reaction medium is filtered through Celite® 545, the filter residue is washed with ethanol and then the filtrates are evaporated to dryness. The orange solid obtained is purified by chromatography on alumina eluted with a dichloromethane/ethanol (99/1, v/v) mixture, to result in the amine **43** (1.69 g, 7.79 mmol). Yield: 66%; Rf: 0.55 ($Al_2O_3$, dichloromethane/ethanol (99/1, v/v)); melting point: 181-183°C; $^1$H NMR (200 MHz, $CDCl_3$) δ 1.49 (t, 3H, *J* = 7 Hz), 4.38 (m, 2H), 4.56 (q, 2H, *J* = 7 Hz), 7.16 (d, 1H, *J* = 2.5 Hz), 7.26 (dd, 1H, *J* = 2.5, 9 Hz), 8.05 (d, 1H, *J* = 9 Hz), 9.35 (s, 1H); $^{13}$C NMR (100 MHz, $CDCl_3$) δ 14.4, 62.1, 106.9, 123.1, 131.9, 136.6, 138.4, 145.6, 145.9, 150.4, 164.7; IR (KBr) ν $cm^{-1}$: 1299, 1487, 1613, 1701, 3202, 3431; MS (m/z, %) 217 ($M^+$, 23), 145 (100), 117 (19), 90 (16), 63 (14).

**Stage D: Ethyl 6-iodoquinoxaline-2-carboxylate (44)**

A solution of sodium nitrite (480 mg, 6.96 mmol) in water (3 ml) is added dropwise at 0°C to a solution of the amine **43** (1.37 g, 6.31 mmol) in 50% tetrafluoroboric acid (10 ml). The mixture is stirred at 0°C for one hour and then a solution of potassium iodide (1.57 g, 9.46 mmol) in water (5 ml) is added. The solution is stirred at 0°C for one hour and then at 50°C for one hour. After returning to ambient temperature, the reaction mixture is basified with a saturated aqueous sodium bicarbonate solution (60 ml). The solution is extracted with dichloromethane. The organic phase is washed with a 5% aqueous sodium hydrogensulphite solution (2 × 30 ml). The organic phase is dried over magnesium sulphate and evaporated under vacuum. The residue is deposited on a column of alumina eluted with dichloromethane, to result in the iodinated ester **44** (0.68 g, 2.07 mmol). Yield: 33%; Rf: 0.80 ($Al_2O_3$, dichloromethane); melting point: 160-162°C; $^1H$ NMR (400 MHz, $CDCl_3$) δ 1.51 (t, 3H, *J*= 7 Hz), 4.59 (q, 2H, *J* = 7 Hz), 7.99 (d, 1H, *J* = 9 Hz), 8.10 (dd, 1H, *J* = 2 and 9 Hz), 8.62 (d, 1H, *J* = 2 Hz), 9.51 (s, 1H); $^{13}C$ NM R (100 MHz, $CDCl_3$) δ 14.3, 62.7, 99.2, 131.6, 138.5, 140.0, 140.7, 143.0, 144.1, 145.7, 163.9; IR (KBr) ν $cm^{-1}$: 1103, 1151, 1230, 1237, 1717; MS (m/z, %) 328 ($M^+$, 19), 284 (43), 256 (100), 128 (29), 101 (50), 75 (30).

**Stage E: *N*-(2-diethylaminoethyl)-6-iodoquinoxaline-2-carboxamide (45)**

The compound **45** was prepared according to the procedure described in the preparation of the compound **2,** using ethyl 6-iodoquinoxaline-2-carboxylate **(44)** as starting material and heating the reaction medium at reflux for 7 hours. Yield: 77%; melting point: 60-62°C; Rf: 0.64 ($Al_2O_3$, dichloromethane/ethanol (97/3, v/v)); $^1H$ NMR (400 MHz, $CDCl_3$) δ 1.00 (t, 6H, *J* = 7 Hz), 2.54 (q, 4H, *J* = 7 Hz), 2.64 (t, 2H, *J* = 6 Hz), 3.49 (q, 2H, *J* = 6 Hz), 7.71 (d, 1H, *J* = 9Hz), 7.96 (dd, 1H, *J* = 2 and 9 Hz), 8.31 (m, 1H), 8.49 (d, 1H, *J* = 2 Hz), 9.55 (s, 1H); $^{13}C$ NMR (100 MHz, $CDCl_3$) δ 12.1 (2C), 37.4, 47.1 (2C), 51.5, 97.9, 130.8, 138.5, 139.5, 139.6, 144.1, 144.3, 144.6, 162.9; IR (KBr) ν $cm^{-1}$: 1517, 1661, 3397; MS (m/z, %) 398 ($M^+$, 1), 86 (100), 58 (15).

**Stage F: *N*-(2-diethylaminoethyl)-6-iodoquinoxaline-2-carboxamide dihydrochloride (46)**

The compound **46** was prepared according to the procedure described for the preparation of the compound **3,** using *N*-(2-diethylaminoethyl)-6-iodoquinoxaline-2-carboxamide **(45)** as starting material. Yield: 76%; melting point: 201-203°C; $^1H$ NMR (200 MHz, $d_6$-DMSO) δ 1.28 (t, 6H, *J* = 7 Hz), 3.28 (m, 6H), 3.78 (m, 2H), 7.97 (d, 1H, *J* = 9 Hz), 8.30 (d, 1H, *J* = 9 Hz), 8.68 (s, 1H), 9.47 (m, 2H), 10.43 (bs, 1H); IR (KBr) ν $cm^{-1}$: 1516, 1674, 2459, 3326. Anal. Calculated for $C_{15}H_{19}IN_4O\cdot 2HCl$: C, 38.24; H, 4.49; N, 11.89. Found: C, 38.25; H, 4.45; N, 11.81.

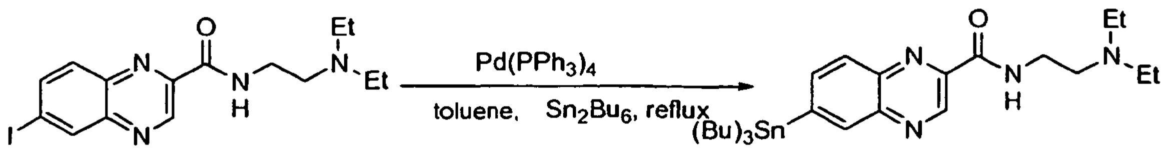


45 47

**Stage G: *N*-(2-diethylaminoethyl)-6-(tributylstannyl)quinoxaline-2-carboxamide (47)**

The compound **47** was prepared according to the procedure described for the preparation of the compound **32,** using *N*-(2-diethylaminoethyl)-6-iodoisoquinoxaline-2-carboxamide (**45**) as starting material. The residue obtained is chromatographed on a column of alumina eluted with an ethyl acetate/cyclohexane (7/3, v/v) mixture; yield: 47%; viscous liquid; Rf: 0.84 ($Al_2O_3$, ethyl acetate/cyclohexane (7/3, v/v)); $^1H$ NMR (200 MHz, $CDCl_3$) δ 0.82 (t, 9H, *J* = 7 Hz), 1.07 (t, 6H, *J*= 7 Hz), 1.19 (m, 6H), 1.30 (m, 6H), 1.51 (m, 6H), 2.58 (q, 4H, *J* = 7 Hz), 2.68 (t, 2H, *J* = 6 Hz), 3.54 (q, 2H, *J* = 6 Hz) 7.85 (d, 1 H, *J* = 8 Hz), 7.98 (d, 1 H, *J* = 8 Hz), 8.24 (s, 1 H), 8.41 (t, 1 H, *J* = 6 Hz), 9.59 (s, 1 H); $^{13}C$ NMR (100 MHz, $CDCl_3$) δ 9.9 (3C, $^1J_{Sn-C}$= 348 Hz), 11.8 (2C), 13.6 (3C), 27.3 (3C, $^3J_{Sn-C}$= 56 Hz), 29.0 (3C, $^2J_{Sn-C}$= 21 Hz), 37.2, 47.3 (2C), 51.6, 128.2, 137.7, 137.9, 140.3, 142.9, 143.4, 143.6, 149.0, 163.6; IR ($CCl_4$) *ν* $cm^{-1}$: 1521, 1684, 2929, 2961; ESI-MS m/z 563.3 $[M+H]^+$.

**<u>EXAMPLE 12</u>**

Synthesis of *N*-(4-dipropylaminobutyl)-6-iodoquinoline-2-carboxamide dihydrochloride (**49**)

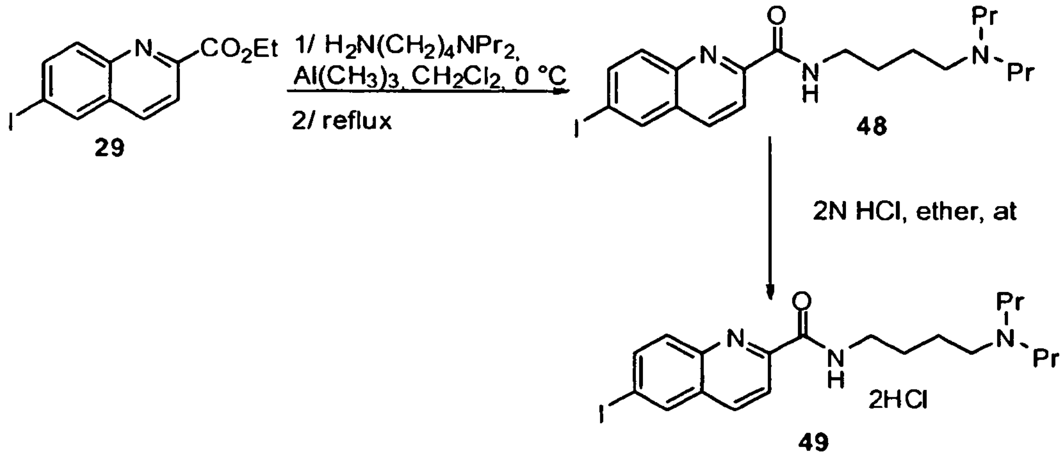


29 48 49

**Stage A : *N*-(4-dipropylaminobutyl)-6-iodoquinoline-2-carboxamide (48)**

The compound **48** was prepared according to the procedure described for the preparation of the compound **2**, using ethyl 6-iodoquinoline-2-carboxylate (**29**) and 4-amino-*N*,*N*-dipropylbutylamine (Seguin, H., Gardette, D., Moreau , M. F., Madelmont, J. C. and Gramain, J. C., Synth. Commun., 1998, 28, 4257-4272) as starting materials. The reaction medium is heated at reflux for 8 hours (360 mg, 0.82 mmol). Yield: 39%; viscous liquid; Rf: 0.68 ($Al_2O_3$, dichloromethane / ethanol (97/3, v/v)); $^1H$ NMR ($CDCl_3$, 400 MHz) δ 0.80 (t, 6H, *J* = 7 Hz), 1.44 (st, 4H, *J* = 7 Hz), 1.60 (m, 4H), 2.39 (t, 4H, *J* = 7 Hz), 2.50 (t, 2H, *J* = 7 Hz), 3.47 (q, 2H, *J* = 6.5 Hz), 7.74 (d, 1 H, *J* = 9 Hz), 7.90 (dd, 1 H, *J* = 9.2 Hz), 8.10 (d, 1 H, *J* = 8 Hz), 8.19 (d, I H, *J* = 2 Hz), 8.22 (d, 1 H, *J* = 8 Hz); $^{13}C$ NMR ($CDCl_3$, 100 MHz) δ 11.9 (2C), 19.5 (2C), 24.1, 27.6, 39.4, 53.5, 55.8 (2C), 93.9, 119.6, 130.7, 131.2, 136.2, 136.5, 138.8, 145.4, 150.3, 164.1; IR (KBr) ν $cm^{-1}$: 1532, 1668, 2956; MS (m/z, %) 453 ($M^+$, 5), 424 (33), 353 (20), 254 (34), 127 (23), 114 (100).

**Stage B : *N*-(4-dipropylaminobutyl)-6-iodoquinoline-2-carboxamide dihydrochloride (49)**

The compound **49** was prepared according to the procedure described for the preparation of the compound **3**, using *N*-(4-dipropylaminobutyl)-6-iodoquinoline-2-carboxamide (**48**) as starting material. Yield: 97%; melting point: 133-135 °C; $^1H$ NMR (400 MHz, $d_6$-DMSO) δ 0.87 (t, 6H, *J* = 7 Hz), 1.66 (m, 8H), 2.94 (m, 4H), 3.05 (m, 2H), 3.38 (m, 2H), 7.89 (d, 1H, *J* = 9 Hz), 8.12 (d, 1H, *J* = 9 Hz), 8.16 (d, 1H, *J*= 8.5 Hz), 8.51 (d, 1H, *J* = 8.5 Hz), 8.58 (s, 1H), 9.05 (m, 1H), 10.55 (bs, 1H); IR (KBr) *ν* $cm^{-1}$: 1534, 1663, 2962, 3250-3600. Anal. Calculated for $C_{20}H_{28}IN_3O \cdot 2HCl$: C, 45.64; H, 5.75; N, 7.98. Found: C, 46.51; H, 5.99; N, 8.16.

**EXAMPLE 13**

Synthesis of *N*-(2-diethylaminoethyl)-9,10-dihydro-1-iodo-9-oxoacridine-4-carboxamide hydrochloride (**55**)

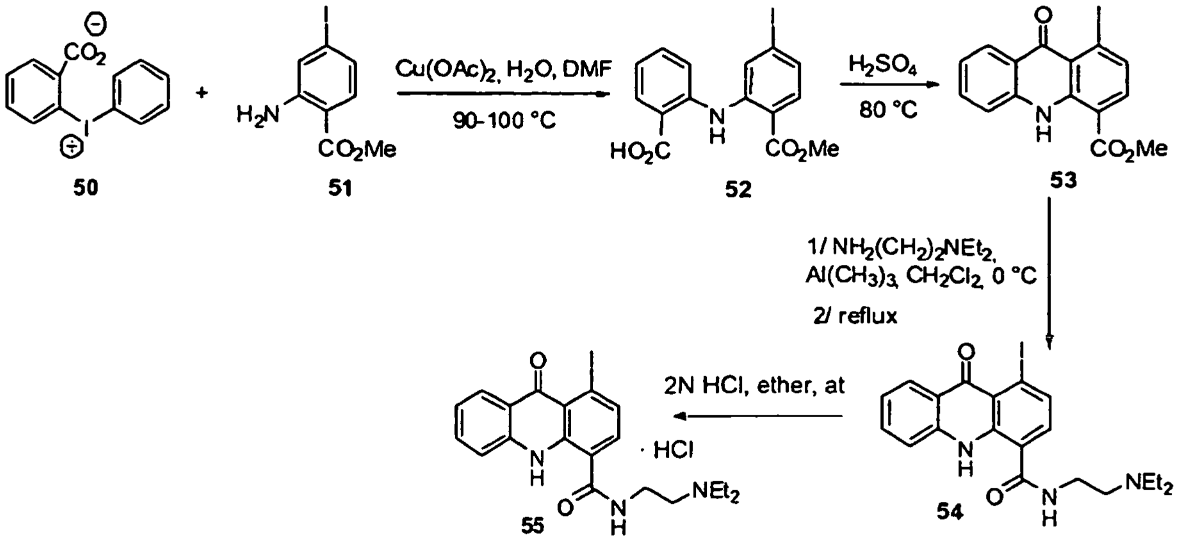


**Stage A: 2-(5-iodo-2-(methoxycarbonyl)phenylamino)benzoic acid (52)**

Diphenyliodonium-2-carboxylate (**50**) (Fieser, L. F.; Haddadin, M. J. Org. Synth. 1966, 46, 107-112) (0.88 g, 2.72 mmol) and then copper acetate monohydrate (12 mg) are added, at ambient temperature and with moderate stirring, to a solution of methyl 4-iodoanthranilate (**51**) (Allison, B. D., Hack, M. D., Phuong, V. K., Rabinowitz, M. H. and Rosen, M. D., U.S. Patent 2005/0038032, 2005) (0.50 g, 1.80 mmol) in dimethylformamide (30 ml). The green mixture obtained is heated at 90-100°C, with stirring, for 12 hours. The solvent is subsequently evaporated under vacuum. Ethyl acetate (18 ml) and then a 0.1N hydrochloric acid solution (18 ml) are successively added to the crude product. The organic phase is then separated by settling and then extracted with a 0.1N aqueous ammonia solution (2 x 9 ml). The aqueous phase recovered is acidified to pH = 6 with a 0.1N hydrochloric acid solution. The mixture is then cooled in an ice bath and then filtered under vacuum. The precipitate obtained is washed with hot water (5 ml). Yield: 80%; melting point: 206-208°C; $^1$H NMR (400 MHz, $d_6$-DMSO) δ 3.83 (s, 3H), 7.04 (t, 1H, *J* = 7.5 Hz), 7.30 (d, 1H, *J* = 8.5 Hz), 7.47 (m, 2H), 7.62 (d, 1H, *J* = 8.5 Hz), 7.74 (s, 1H), 7.92 (d, 1H, *J* = 7.5 Hz), 10.70 (s, 1H), 13.20 (m, 1H); $^{13}$C NMR (100 MHz, $d_6$-DMSO) δ 52.1, 101.9, 115.6, 118.2 (2C), 121.1, 125.3, 128.5, 131.8, 132.8, 133.5, 142.4, 144.4, 166.5, 168.3; IR (KBr) *v* cm$^{-1}$: 1220, 1254, 1575, 1685, 3000-3600; MS (m/z, %) 397 (M$^+$, 100), 321 (88), 194 (59), 166 (95), 139 (62), 63 (77), 55 (61).

**Stage B: Methyl 9,10-dihydro-1-iodo-9-oxoacridine-4-carboxylate (53)**

A solution of 2-(5-iodo-2-(methoxycarbonyl)phenylamino)benzoic acid (**52**) (1.00 g, 2.52 mmol) in concentrated sulphuric acid (2 ml) is heated, under argon, in an oil bath at 80°C for 30 minutes. After returing to ambient temperature, the green solution is cooled with an ice bath and diluted with water (15 ml). The green precipitate obtained is then filtered off under vacuum, washed with water (4 ml), taken up in anhydrous ethanol and then dried by evaporation under vacuum. The product is purified by chromatography on silica eluted with a dichloromethane / ethanol (99.5/0.5, v/v) mixture, to result in the yellow precipitate **53** (92 mg, 0.24 mmol). Yield: 85%; Rf: 0.65 ($SiO_2$, dichloromethane/ethanol (99.5/0.5, v/v)); melting point: 189-191°C; $^1$H NMR (200 MHz, $CDCl_3$) δ 4.01 (s, 3H), 7.29 (m, 2H), 7.67 (t, 1H, *J* = 8.5 Hz), 7.91 (m, 2H), 8.42 (d, 1H, *J* = 8.5 Hz), 12.07 (bs, 1H); $^{13}$C NMR (100 MHz, $CDCl_3$) δ 52.8, 102.4, 113.4, 117.3, 119.8, 121.4, 122.9, 127.5, 134.1, 135.1, 135.3, 138.7, 142.4, 168.6, 176.2; IR (KBr) *v* cm$^{-1}$: 1273, 1508, 1613, 1640, 1685, 3217; MS (m/z, %) 379 (M$^+$, 71), 347 (81), 192 (24), 164 (100), 127 (24), 88 (24), 76 (24), 63 (24).

**Stage C: *N*-(2-diethylaminoethyl)-9,10-dihydro-1-iodo-9-oxoacridine-4-carboxamide (54)**

The compound **54** was prepared according to the procedure described for the preparation of the compound **2,** using methyl 9,10-dihydro-1-iodo-9-oxoacridine-4-carboxylate (**53**) as starting material and heating the reaction medium at reflux for 5 hours. Yield: 64%; Rf: 0.40 ($Al_2O_3$, dichloromethane / ethanol (99/1, v/v)); melting point: 248-250°C; $^1$H NMR (400 MHz, $CDCl_3$) δ 1.04 (t, 6H, *J* = 6.5 Hz), 2.60 (q, 4H, *J*= 6.5 Hz), 2.70 (m, 2H), 3.48 (m, 2H), 7.19 (t, 1H, *J* = 7 Hz), 7.25 (d, 1H, *J* = 8.5 Hz), 7.36 (d, 1H, *J* = 8 Hz), 7.57 (m, 2H), 7.81 (d, 1H, *J* = 8 Hz), 8.34 (d, 1H, *J* = 8 Hz), 12.75 (s, 1H); $^{13}$C NMR (100 MHz, $CDCl_3$) δ 11.7 (2C), 37.1, 46.9 (2C), 51.2, 99.0, 117.5 (2C), 119.9, 121.3, 122.5, 127.4, 130.9, 134.0, 134.8, 139.1, 142.1, 168.4, 176.5; IR (KBr) *v* cm$^{-1}$: 1513, 1615, 3281; MS (m/z, %) 463 (M$^+$, 1), 86 (100),

58 (16).

**Stage D: *N*-(2-diethylaminoethyl)-9,10-dihydro-1-iodo-9-oxoacridine-4-carboxamide hydrochloride (55)**

The compound **55** was prepared according to the procedure described for the preparation of the compound **3**, using *N*-(2-diethylaminoethyl)-9,10-dihydro-1-iodo-9-oxoacridine-4-carboxamide (**54**) as starting material. Yield: 81%; melting point: 269-271˚C; $^1$H NMR (400 MHz, $d_6$-DMSO) δ 1.26 (t, 6H, *J* = 7 Hz), 3.22 (m, 4H), 3.33 (m, 2H, *J* = 7 Hz), 3.75 (m, 2H), 7.33 (t, 1H, *J* = 7 Hz), 7.62 (d, 1H, *J* = 8 Hz), 7.76 (d, 1H, *J* = 7 Hz), 7.96 (m, 2H), 8.20 (d, 1H, *J* = 8 Hz), 9.49 (bs, 1**H),** 10.39 (bs, 1H), 12.70 (bs, 1H); $^{13}$C NMR (100 MHz, $d_5$-DMSO-) δ 8.3 (2C), 34.3, 46.5 (2C), 49.6, 99.0, 117.7, 118.0, 118.8, 120.4, 122.3, 126.3, 133.0, 134.1, 134.3, 138.6, 141.3, 168.2, 175.2; IR (KBr) *v* cm$^{-1}$: 1513, 1577, 1613, 3057, 3100-3500. Anal. Calculated for $C_{20}H_{22}IN_3O_2{\cdot}HCl{\cdot}3H_2O$ : C, 43.38; H, 5.28; N, 7.59. Found : C, 43.74; H, 4.99; N, 7.68.

**EXAMPLE 14**

Synthesis of *N*-(2-diethylaminoethyl)-9,10-dihydro-2-iodo-9-oxoacridine-4-carboxamide hydrochloride (**60**)

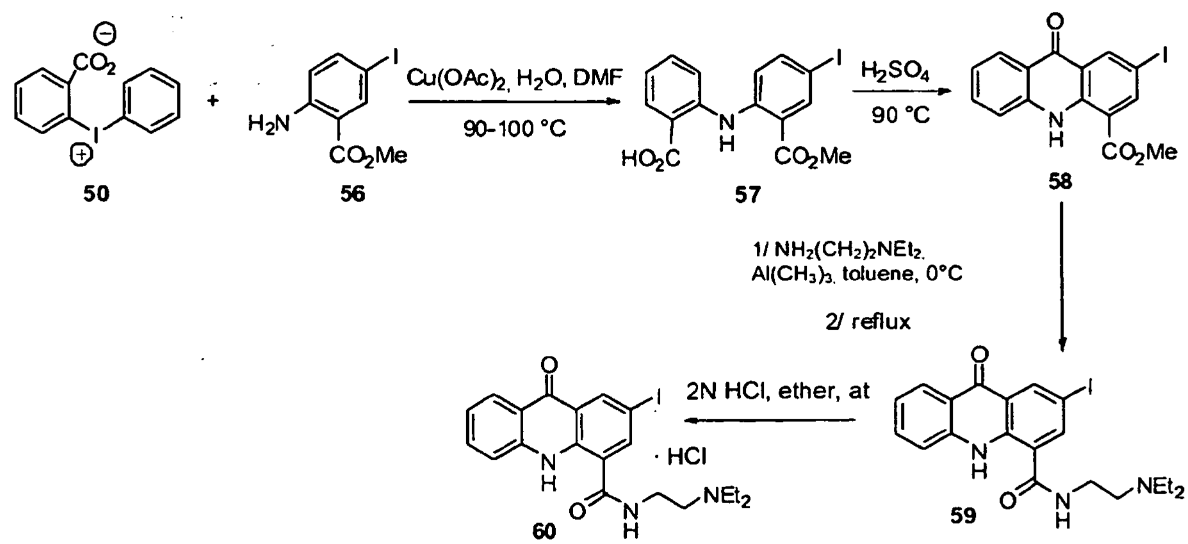

50
56
57
58
59
60

**Stage A: 2-(4-iodo-2-(methoxycarbonyl)phenylamino)benzoic acid (57)**

The compound **57** was prepared according to the procedure described for the preparation of the compound **52**, using methyl 5-iodoanthranilate (**56**) (Sy, W. W., Synth. Commun., 1992, 22, 3215-3219) as starting material. Yield: 59%; Rf: 0.86 ($Al_2O_3$, dichloromethane / ethanol (9/1, v/v)); melting point: 230-232˚C; $^1$H NMR (400 MHz, $d_6$-DMSO) δ 3.86 (s, 3H), 7.00 (m, 1H), 7.33 (d, 1H, *J* = 9 Hz), 7.46 (m, 2H), 7.74 (d, 1H, *J* = 9 Hz), 7.93 (d, 1H, *J* = 8 Hz), 8.14 (s, 1H), 10.78 (s, 1H), 13.21 (m, 1H); $^{13}$C NMR (100 MHz, $d_6$-DMSO) δ 52.2, 81.5, 117.8, 118.0, 118.7, 119.9, 120.7, 131.7, 133.4, 139.2, 141.8, 142.7, 143.1, 165.5, 168.4; IR (KBr) *v* cm$^{-1}$: 1209, 1269, 1508, 1577, 1683, 1719, 2800-3200; MS (m/z, %) 397 (M$^+$, 100), 320 (67), 194 (54), 166 (77), 139 (59), 63 (72).

**Stage B: methyl 9,10-dihydro-2-iodo-9-oxoacridine-4-carboxylate (58)**

The compound **58** was prepared according to the procedure described for the preparation of the compound **53,** using 2-(4-iodo-2-(methoxycarbonyl)phenylamino)-benzoic acid (**52**) as starting material. The residue obtained is chromatographed on a column of silica eluted with a dichloromethane/ethanol (99.5/0.5, v/v) mixture, to result, in order of elution, in:

**Methyl 9,10-dihydro-2-iodo-9-oxoacridine-4-carboxylate (58)**

Yield: 85%; Rf: 0.41 ($SiO_2$, dichloromethane / ethanol (99.5/0.5, v/v)); melting point: 275-277˚C; $^1$H NMR (200

MHz, $CDCl_3$) δ 4.03 (s, 3H), 7.32 (t, 1H, *J* = 8.5 Hz), 7.38 (d, I H, *J* = 8.5 Hz), 7.70 (t, 1H, *J* = 8.5 Hz), 8.42 (d, 1H, *J* = 8.5 Hz), 8.65 (d, 1H, *J* = 2 Hz), 8.99 (d, 1H, *J* = 2 Hz), 11.65 (bs, 1H); $^{13}C$ NMR (100 MHz, $CDCl_3$) δ 52.9, 81.9, 115.6, 117.8, 121.7, 122.8, 124.2, 127.3, 134.4, 139.9, 140.9, 142.4, 144.5, 167.3, 176.5; IR (KBr) ν $cm^{-1}$: 1282, 1430, 1508, 1694, 3262; MS (m/z, %) 379 ($M^+$, 100), 347 (52), 164 (30).

**Methyl 9,10-dihydro-9-oxoacridine-4-carboxylate** (Rewcasttle, G. W. and Denny, W. A., Synthesis, 1985, 220-222).

Yield: 8%; Rf: 0.21 ($SiO_2$, dichloromethane / ethanol (99.5/0.5, v/v)); melting point: 170-172°C (lit.: 172°C).

**Stage C: *N*-(2-diethylaminoethyl)-9,10-dihydro-2-iodo-9-oxoacridine-4-carboxamide (59)**

The compound **59** was prepared according to the procedure described for the preparation of the compound **2**, using methyl 9,10-dihydro-2-iodo-9-oxoacridine-4-carboxylate (**58**) as starting material and heating the reaction medium at reflux for 1.5 hours in anhydrous toluene. Yield: 88%; Rf: 0.43 ($Al_2O_3$, dichloromethane / ethanol (99/l, v/v)); melting point: 250-252°C; $^1H$ NMR (200 MHz, $CDCl_3$) δ 1.08 (t, 6H, *J* = 7 Hz), 2.67 (q, 4H, *J* = 7 Hz), 2.75 (t, 2H, *J* = 6 Hz), 3.56 (m, 2H), 7.21 (t, 1H, *J* = 8 Hz), 7.30 (d, 1H, *J* = 8 Hz), 7.61 (t, 1H, *J* = 8 Hz), 7.65 (bs, 1H), 8.08 (d, 1H, *J* = 2 Hz), 8.32 (d, 1H, *J* = 8 Hz), 8.75 (d, 1H, *J* = 2 Hz), 12.15 (bs, 1H); $^{13}C$ NMR (100 MHz, $CDCl_3$) δ 11.8 (2C), 37.4, 46.9 (2C), 51.2, 82.0, 117.9, 120.0, 121.2, 122.3, 124.0, 126.8, 134.0, 139.7, 139.8, 140.0 (2C), 166.9, 176.5; IR (KBr) ν $cm^{-1}$ : 1512, 1617, 3422; MS (m/z, %) 463 ($M^+$, 6), 348 (18), 320 (14), 193 (11), 164 (11), 86 (100), 58 (23).

**Stage D: *N*-(2-diethylaminoethyl)-9,10-dihydro-2-iodo-9-oxoacridine-4-carboxamide hydrochloride (60)**

The compound **60** was prepared according to the procedure described for the preparation of the compound **3** using *N*-(2-diethylaminoethyl)-9,10-dihydro-2-iodo-9-oxoacridine-4-carboxamide (**59**) as starting material. Yield: 71%; melting point: 298-300°C; $^1H$ NMR (400 MHz, $d_6$-DMSO) δ 1.27 (m, 6H), 3.23 (m, 4H), 3.33 (m, 2H), 3.75 (m, 2H), 7.33 (t, 1H, *J* = 7 Hz), 7.75 (m, 2H), 8.23 (d, 1H, *J* = 8 Hz), 8.60 (s, 1H), 8.68 (s, 1H), 9.42 (bs, 1H), 10.27 (bs, 1H), 12.19 (bs, 1H); $^{13}C$ NMR (100 MHz, $d_6$-DMSO) δ 8.3 (2C), 34.2, 46.4 (2C), 49.5, 83.2, 118.6, 120.5 (2C), 122.3, 123.3, 125.9, 134.3, 138.2, 139.3, 139.8, 140.7, 166.8, 175.3; IR (KBr) *v* $cm^{-1}$: 1300, 1517, 1560, 1617, 3200-3400. Anal. Calculated for $C_{20}H_{22}IN_3O_2 \cdot HCl\text{-}1.75H_2O$ : C, 45.21; H, 5.03; N, 7.91. Found : C, 44.91; H, 4.66; N, 8.07.

**EXAMPLE** 15

Synthesis of *N*-(2-diethylaminoethyl)-9,10-dihydro-3-iodo-9-oxoacridine-4-carboxamide hydrochloride (**65**)

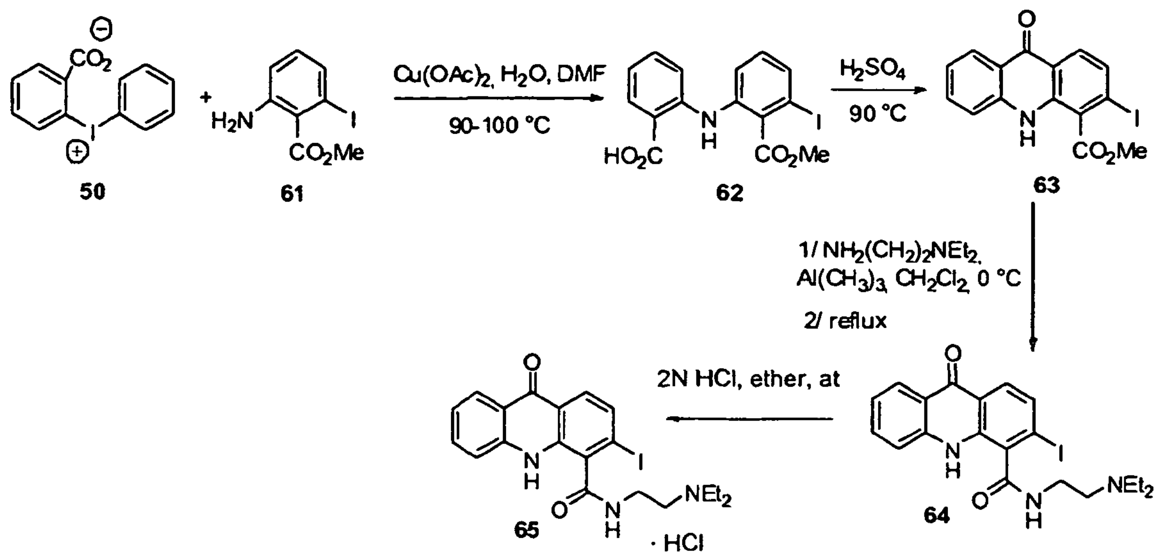


**Stage A: 2-(3-iodo-2-methoxycarbonylphenylamino)benzoic acid (62)**

The compound **62** was prepared according to the procedure described for the preparation of the compound **52**, using methyl 6-iodoanthranilate (**61**) (Seltzman, H. H. and Berrang, B. D., Tetrahedron Lett., 1993, 34, 3083-3086)

as starting material. Yield: 67%; melting point: 191-193°C; $^1$H NMR (200 MHz, $d_6$-DMSO) δ 3.85 (s, 3H), 6.85 (t, 1H, *J* = 8 Hz), 7.18 (m, 2H), 7.40 (t, 1H, *J*= 8 Hz), 7.50 (d, 1H, *J*= 8 Hz), 7.59 (d, 1H, *J*= 8 Hz), 7.90 (d, 1H, *J* = 8 Hz), 9.93 (bs, 1H); $^{13}$C NMR (100 MHz, $d_6$-DMSO) δ 52.6, 93.6, 113.6, 1 14.4, 118.7, 121.1, 131.8, 131.9, 133.0, 133.4, 134.2, 139.0, 145.8, 167.6, 169.9; IR (KBr) ν cm$^{-1}$: 1247, 1269, 1444, 1575, 1657, 1740, 2700-3200, 3318; MS (m/z, %) 397 (M, 73), 321 (100), 194 (36), 166 (41), 139 (18).

**Stage B: methyl 9,10-dihydro-3-iodo-9-oxoacridine-4-carboxylate (63)**

The compound **63** was prepared according to the procedure described for the preparation of the compound **53**, using 2-(3-iodo-2-(methoxycarbonyl)phenylamino)benzoic acid (**62**) as starting material. Yield: 65%; Rf: 0.41 ($SiO_2$, dichloromethane / ethanol (99.5/0.5, v/v)); melting point: 202-204°C; $^1$H NMR (200 MHz, $CDCl_3$) δ 4.07 (s, 3H), 7.31 (m, 2H), 7.70 (t, 1H, *J* = 7 Hz), 7.88 (d, 1H, *J* = 8.5 Hz), 8.23 (d, 1H, *J* = 8.5 Hz), 8.41 (d, 1H, *J* = 8 Hz), 10.56 (bs, 1H); $^{13}$C NMR (100 MHz, $d_6$-DMSO) δ 53.3, 102.0, 118.1, 120.4, 120.5, 122.2, 125.8, 127.8, 128.9, 131.1, 134.1, 137.4, 140.7, 167.0, 176.1; IR (KBr) ν cm$^{-1}$: 1281, 1431, 1583, 1617, 3000-3600; MS (m/z, %) 379 ($M^+$, 60), 347 (100), 164 (54), 139 (25), 63 (29).

**Stage C: *N*-(2-diethylaminoethyl)-9,10-dihydro-3-iodo-9-oxoacridine-4-carboxamide (64)**

The compound **64** was prepared according to the procedure described for the preparation of the compound **2**, using methyl 9,10-dihydro-3-iodo-9-oxoacridine-4-carboxylate (**63**) as starting material and heating the reaction medium at reflux for 6 hours. Yield: 38%; Rf: 0.40 ($Al_2O_3$, dichloromethane / ethanol (99/1, v/v)); melting point: 215-217°C; $^1$H NMR (200 MHz, $CDCl_3$) δ 1.49 (t, 6H, *J* = 7 Hz), 3.37 (m, 4H), 3.52 (m, 2H), 4.08 (m, 2H), 7.00 (d, 1H, *J* = 8 Hz), 7.19 (t, 1H, *J* = 8 Hz), 7.59 (m, 3H), 8.21 (d, 1H, *J* = 8 Hz), 8.77 (bs, 1H), 10.09 (s, 1H); $^{13}$C NMR (100 MHz, $d_6$-DMSO) δ 10.8 (2C), 39.5, 46,8 (2C), 50.3, 102.3, 118.2, 120.3, 120.4, 121.9, 125.7, 127.6, 130.9, 132.0, 133.9, 137.2, 140.9, 166.5, 176.3; IR (KBr) ν cm$^{-1}$: 1556, 1614, 2960-3230; MS (m/z, %) 463 ($M^+$, 1), 86 (100), 58 (12).

**Stage D: *N*-(2-diethylaminoethyl)-9,10-dihydro-3-iodo-9-oxoacridine-4-carboxamide hydrochloride (65)**

The compound **65** was prepared according to the procedure described for the preparation of the compound **3**, using *N*-(2-diethylaminoethyl)-9,10-dihydro-3-iodo-9-oxoacridine-4-carboxamide (**64**) as starting material. Yield: 87%; melting point: 262-264°C; $^1$H NMR (400 MHz, $d_6$-DMSO) δ 1.27 (t, 6H, *J* = 7 Hz), 3.23 (m, 4H), 3.32 (m, 2H), 3.76 (m, 2H), 7.32 (t, 1H, *J* = 8 Hz), 7.75 (m, 2H), 7.95 (d, 1H, *J* = 8.5 Hz), 7.99 (d, 1H, *J* = 8.5 Hz), 8.21 (d, 1H, *J* = 8 Hz), 9.05 (m, 1H), 10.17 (bs, 1H), 10.79 (bs, 1H); $^{13}$C NMR (100 MHz, $d_6$-DMSO) δ 8.4 (2C), 34.5, 46.6 (2C), 49.3, 102.6, 118.3, 120.4 (2C), 121.9, 125.7, 127.8, 130.9, 131.6, 133.8, 137.2, 141.0, 166.9, 176.3; IR (KBr) *v* cm$^{-1}$: 1427, 1590, 1618, 3200-3400. Anal. Calculated for $C_{20}H_{22}IN_3O_2 \cdot HCl\text{-}H_2O$: C, 46.39; H, 4.87; N, 8.12. Found : C, 46.59; H, 4.89; N, 8.06.

**EXAMPLE 16**

Synthesis of *N*-(2-diethylaminoethyl)-9,10-dihydro-5-iodo-9-oxoacridine-4-carboxamide hydrochloride (**69**)

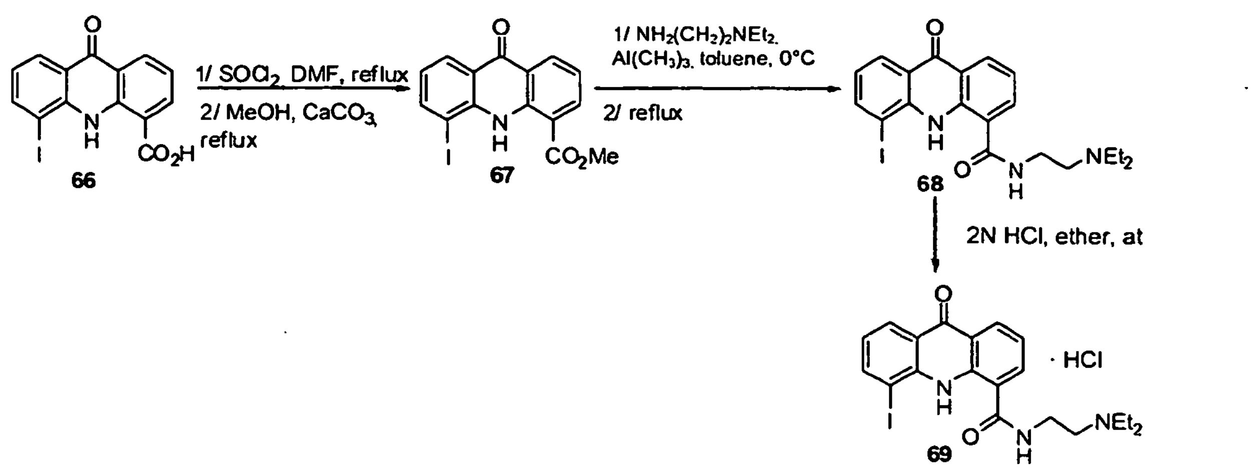

**Stage A: methyl 9,10-dihydro-5-iodo-9-oxoacridine-4-carboxylate (67)**

Dimethylformamide (3 drops) is added to a solution of 9,10-dihydro-5-iodo-9-oxoacridine-4-carboxylic acid (**66**) (Rewcastle, G. W. and Denny, W. A., Synthesis, 1985, 2, 217-220) (1.00 g, 2.74 mmol) in thionyl chloride (30 ml). The reaction medium is heated at reflux for 15 minutes and then the thionyl chloride is driven off by evaporation under vacuum. The acid chloride is taken up in anhydrous toluene (20 ml) in order to be once again evaporated to dryness. The precipitate is dissolved in anhydrous methanol (30 ml) and then calcium carbonate is added (1.20 g). The mixture is subsequently brought to reflux for 3 hours. After returning to ambient temperature, the calcium carbonate is filtered off. The precipitate is taken up in a 1N hydrochloric acid solution (25 ml) and filtered. The aqueous phase is extracted with dichloromethane (3 x 50 ml) and then all the organic phases arc combined, dried over magnesium sulphate, filtered and evaporated to dryness. The residue obtained is chromatographed on a column of alumina eluted with dichloromethane, to result in the ester **67** (0.59 g, 1.56 mmol). Yield: 57%; Rf: 0.78 ($Al_7O_3$, dichloromethane); melting point: 174-176°C; $^1$H NMR (400 MHz, $CDCl_3$) δ 4.07 (s, 3H), 7.06 (t, 1H, *J* =*7.5* Hz), 7.30 (t, 1H, *J* = 8Hz), 8.17 (d, 1H, *J* = 7.5 Hz), 8.46 (m, 2H), 8.69 (d, 1H, *J* = 8 Hz), 12. 11 (s, 1H); $^{13}$C NMR (100 MHz, $CDCl_3$) δ 52.9, 86.2, 114.3, 120.8, 121.9, 122.5, 123.6, 127.7, 133.8, 137.0, 140.7, 141.5, 144.0, 168.0, 177.6; IR (KBr) *v* $cm^{-1}$: 1141, 1279, 1430, 1518, 1608, 3197; MS (m/z, %) 379 ($M^+$, 80), 347 (100), 164 (99), 75 (71), 63 (40).

**Stage B: *N*-(2-diethylaminoethyl)-9,10-dihydro-5-iodo-9-oxoacridine-4-carboxamide (68)**

The compound **68** was prepared according to the procedure described for the preparation of the compound **2**, using methyl 9,10-dihydro-5-iodo-9-oxoacridine-4-carboxylate (**67**) as starting material and heating the reaction medium at reflux for 2 hours in anhydrous toluene. Yield: 73%; Rf: 0.23 ($Al_2O_3$, dichloromethane / ethanol (99/1, v/v)); melting point: 148-150°C; $^1$H NMR (400 MHz, $CDCl_3$) δ 1.05 (t, 6H, *J* = 7 Hz), 2.61 (q, 4H, *J* = 7 Hz), 2.72 (m, 2H), 3.56 (m, 2H), 6.95 (t, 1H, *J* = 8 Hz), 7.21 (t, 1H, *J* = 8 Hz), 7.58 (bs, 1H), 7.92 (d, 1H, *J* = 8 Hz), 8.08 (d, 1H, *J* = 8 Hz), 8.36 (d, 1H, *J* = 8 Hz), 8.53 (d, 1H, *J* = 8 Hz), 12.80 (s, 1H); $^{13}$C NMR (100 MHz, $CDCl_3$) δ 11.8 (2C), 37.3, 46.9 (2C), 51.2, 86.3, 118.1, 120.6, 122.2, 122.3, 123.2, 127.6, 131.8, 132.0, 141.1, 141.2, 143.9, 167.9, 177.8; IR (KBr) *v* $cm^{-1}$: 1521, 1610, 3200-3500; MS (m/z, %) 463 ($M^+$, 1), 86 (100), 58 (7).

**Stage C: *N*-(2-diethylaminoethyl)-9,10-dihydro-5-iodo-9-oxoacridine-4-carboxamide hydrochloride (69)**

The compound **69** was prepared according to the procedure described for the preparation of the compound **3**, using *N*-(2-diethylaminoethyl)-9,10-dihydro-5-iodo-9-oxoacridine-4-carboxamide (**68**) as starting material. Yield: 82%; melting point: 251-253°C; $^1$H NMR (400 MHz, *$d_6$*-DMSO) δ 1.27 (t, 6H, *J* = 7 Hz), 3.24 (m, 4H), 3.36 (m, 2H), 3.79 (m, 2H), 7.12 (t, 1H, *J* = 8 Hz), 7.43 (t, 1H, *J* = 7.5 Hz), 8.26 (d, 1H, *J* = 8 Hz), 8.32 (d, 1H, *J* = 7 Hz), 8.46 (d, 1H, *J* = 8 Hz), 8.55 (d, 1H, *J* = 7 Hz), 9.55 (bs, I H), 10.34 (bs, 1H), 12.97 (bs, 1H); $^{13}$C NMR (100 MHz, *$d_6$*-DMSO) δ 8.3 (2C), 34.2, 46.5 (2C), 49.8, 87.5, 117.4, 120.7, 121.1, 121.3, 123.5, 126.5, 130.8, 133.8, 140.5 (2C), 144.0, 168.1, 176.3; IR (KBr) ν $cm^{-1}$: 1301, 1428, 1522, 1611, 2650, 2946, 3200-3400. Anal. Calculated for $C_{20}H_{22}IN_3O_2 \cdot HCl \cdot H_2O$ : C, 46.39; H, 4.87; N, 8.12. Found : C, 46.03; H, 4.80; N, 8.06.

<u>**EXAMPLE 17**</u>

Synthesis of *N*-(2-diethylaminoethyl)-9,10-dihydro-6-iodo-9-oxoacridine-4-carboxamide hydrochloride (**78**)

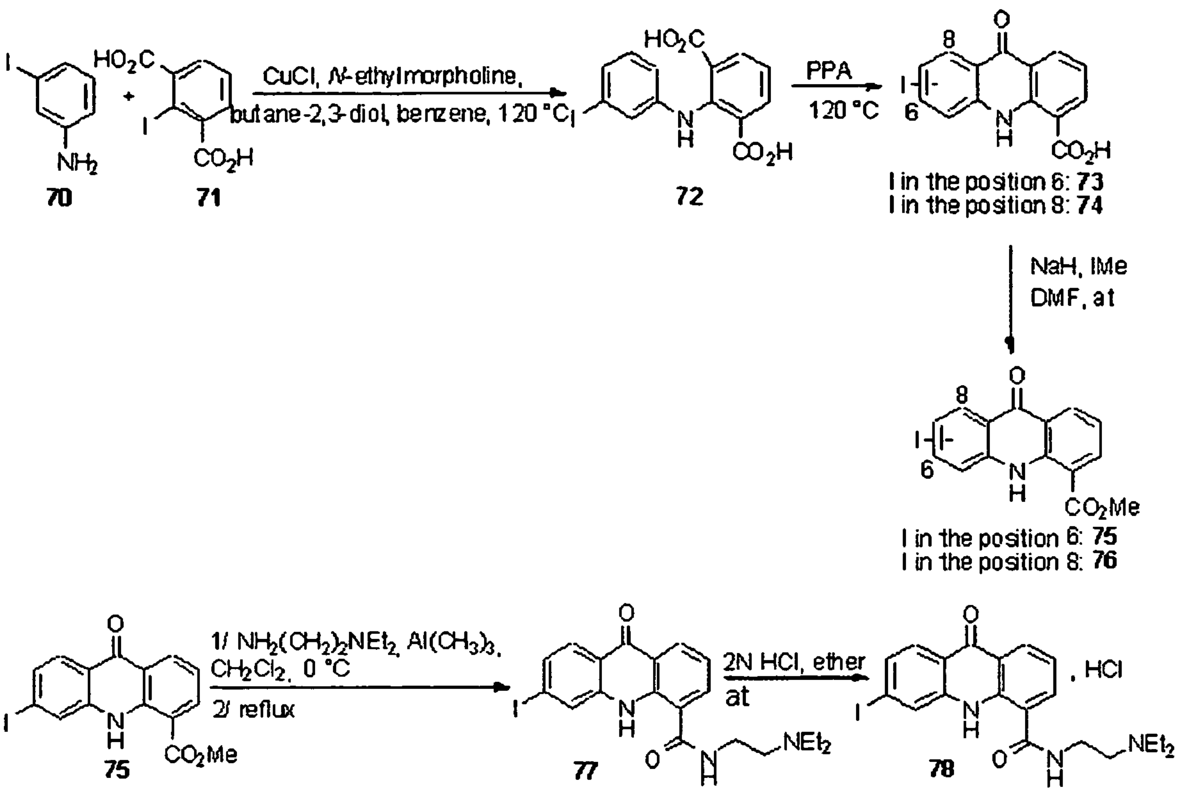


70 71 72

I in the position 6: 73
I in the position 8: 74

I in the position 6: 75
I in the position 8: 76

75 77 78

**Stage A: 2-(3-iodophenylamino)isophthalic acid (72)**

3-iodoaniline (**70**) (6.74 g, 30.8 mmol) and copper chloride (2.03 g, 20.5 mmol) are added, under argon, to a solution of 2-iodoisophthalic acid (**71**) (Rewcastle G. W. and Denny, W. A., Synthesis, 1985, 217-220) (6.00 g, 20.6 mmol) in a mixture of anhydrous butane-2,3-diol (25 ml) and anhydrous benzene (45 ml). The reaction mixture is immersed in an oil bath heated beforehand to 120˚C. When the internal temperature reaches approximately 100˚C, the anhydrous *N*-ethylmorpholine (5.88 ml, 45.9 mmol) is added and then the reaction mixture is stirred at 120˚C (external) for 4 hours. After returning to ambient temperature, the solution is treated with a 0.5N aqueous ammonia solution (100 ml) and then with active charcoal (6 g). The mixture is subsequently filtered through Celite® 521 and the filtered residue is washed several times with water (2 x 50 ml). The filtrates are acidified with 2N hydrochloric acid (80 ml) and then the aqueous phase is extracted with ethyl acetate (3 x 100 ml). The organic phase is filtered through Celite® 521 in order to remove an inorganic precipitate and is then extracted with a 0.5N aqueous ammonia solution (2 x 100 ml). The aqueous phase is acidified with concentrated hydrochloric acid and then concentrated to 2/3 under vacuum. The precipitate formed is filtered off and then washed with hot water. The precipitate is subsequently taken up in anhydrous ethanol (50 ml) to be dried under vacuum, to result in the acid **72** (2.12 g, 5.34 mmol). Yield: 26%; melting point: 214-216˚C; $^1$H NMR (400 MHz, $d_6$-DMSO) δ 6.89 (d, 1H, *J* = 8 Hz), 6.98 (t, 1H, *J* = 8 Hz), 7.08 (t, 1H, *J* = 8 Hz), 7.21 (d, 1H, *J* = 8 Hz), 7.25 (s, 1H), 7.95 (d, 2H, *J* = 8 Hz), 9.54 (bs, 1H), 13.10 (m, 2H); $^{13}$*C* NMR (100 MHz, $d_6$-DMSO) δ 95.1, 116.4, 120.2, 122.0 (2C), 125.2, 129.6, 130.9, 135.0 (2C), 142.8, 145.5, 168.4 (2C); IR (KBr) *v* $cm^{-1}$: 1249, 1432, 1584, 1665, 1693, 2800-3200; MS (m/z, %) 383 ($M^+$, 100), 321 (38), 194 (48), 166 (53), 139 (21).

**Stage B: 9,10-dihydro-6-iodo-9-oxoacridine-4-carboxylic acid (73) and 9,10-dihydro-8-iodo-9-oxoacridine-4-carboxylic acid (74)**

2-(3-iodophenylamino)isophthalic acid (**72**) (0.89 g, 23.2 mmol) is dissolved in polyphosphoric acid (PPA) (26 g) at 120˚C (internal). The mixture is stirred at 120˚C (internal) for 2 hours and then poured into boiling water (86 ml). The precipitate formed is filtered off and then dissolved in a 1/1 (v/v) mixture of methanol and a 1N aqueous sodium hydroxide solution (140 ml). The solution is heated to 60˚C (internal) and then filtered while hot. The filtrate is acidified (pH = 5) with glacial acetic acid (10 ml), concentrated to 2/3 under vacuum and cooled on an ice bath until precipitation occurs. The yellow precipitate formed is filtered off and then taken up in ethanol (20 ml) to be dried under vacuum, to result in a mixture (1/1, dislayed by $^1$H NMR) of the acids **73** and **74** (0.71 g, 1.95 mmol).

**Stage C: methyl 9,10-dihydro-6-iodo-9-oxoacridine-4-carboxylate (75) and methyl 9,10-dihydro-8-iodo-9-oxoacridine-4-carboxylate (76)**

60% sodium hydride in mineral oil (10.4 mg, 0.26 mmol) is added to a mixture of 9,10-dihydro-6-iodo-9-oxoacridine-4-carboxylic acid (**73**) and 9,10-dihydro-8-iodo-9-oxoacridine-4-carboxylic acid (**74**) (95.0 mg, 0.26 mmol) in anhydrous dimethylformamide (5 ml), stirred beforehand for 10 minutes, at ambient temperature under argon. The mixture is then stirred at ambient temperature for 30 minutes. Methyl iodide (16.2 μl, 0.26 mmol) is subsequently added to the solution, which is stirred at ambient temperature for 18 hours. The red solution obtained is then evaporated under vacuum. The mixture is subsequently diluted with water (10 ml), basified with a saturated aqueous sodium carbonate solution (10 ml) and extracted with ethyl acetate (3 x 20 ml). The organic phases obtained are combined, dried over magnesium sulphate, filtered and evaporated under vacuum. The residue is subsequently separated by chromatography on a column of silica eluted with dichloromethane, to result, in order of elution, in the following products :

**methyl 9,10-dihydro-8-iodo-9-oxoacridine-4-carboxylate (76)**

(50 mg, 0.13 mmol); yield: 51%; Rf: 0.58 ($SiO_2$, dichloromethane); melting point: 239-241°C; $^1$H NMR (400 MHz, $CDCl_3$ δ 4.01 (s, 3H), 7.23 (m, 2H), 7.34 (d, 1H, *J* = 8 Hz), 7.92 (d, 1H, *J* = 7.5 Hz), 8.37 (d, 1H, *J* = 7 Hz), 8.67 (d, 1H, *J* = 7.5 Hz), 11.64 (bs, 1H); $^{13}$C NMR (100 MHz, $CDCl_3$) δ 52.6, 92.5, 113.3, 118.6, 119.0, 120.5, 122.3, 133.8, 134.4, 136.6, 137.2, 140.7, 141.3, 168.4, 176.1; IR (KBr) *v* $cm^{-1}$: 1261, 1592, 1611, 1692, 2926, 2961; MS (m/z, %) 379 ($M^+$, 79), 347 (82), 220 (28), 192 (34), 164 (100), 75 (43), 63 (33).

**Methyl 9,10-dihydro-6-iodo-9-oxoacridine-4-carboxylate (75)**

(32 mg, 0.08 mmol); yield: 32%; Rf: 0.38 ($SiO_2$, dichloromethane); melting point: 258-260°C; $^1$H NMR (200 MHz, $CDCl_3$) δ 4.03 (s, 3H), 7.30 (t, 1H, *J* = 8 Hz), 7.61 (d, 1H, *J* = 8.5 Hz), 7.85 (s, 1 H), 8.13 (d, 1H, *J* = 8.5 Hz), 8.46 (d, 1H, *J* = 7.5 Hz), 8.71 (d, 1H, *J* = 7.5 Hz), 11.77 (bs, 1H); $^{13}$C NMR (50 MHz, $CDCl_3$) δ 52.7, 101.5, 120.5, 120.8, 121.3, 122.7, 126.4, 128.5, 131.6, 134.0, 136.9, 140.7, 141.7, 168.5, 174.6; IR (KBr) *v* $cm^{-1}$: 1280, 1589, 1609, 1640, 1685, 3271; MS (m/z, %) 379 ($M^+$, 95), 347 (100), 220 (32), 192 (29), 164 (99), 137 (26), 75 (58), 63 (42).

**Stage D: *N*-(2-diethylaminoethyl)-9,10-dihydro-6-iodo-9-oxoacridine-4-carboxamide (77)**

The compound **77** was prepared according to the procedure described for the preparation of the compound **2**, using methyl 9,10-dihydro-6-iodo-9-oxoacridine-4-carboxylate (**75**) as starting material and heating the reaction medium at reflux for 2 hours. Yield: 65%; Rf: 0.25 ($Al_2O_3$, dichloromethane / ethanol (99/1, v/v)); melting point: 208-210°C; $^1$H NMR (400 MHz, $CDCl_3$) δ 1.25 (t, 6H, *J* = 7 Hz), 2.95 (m, 4H), 3.04 (m, 2H), 3.72 (m, 2H), 7.22 (t, 1H, *J* = 7.5 Hz), 7.47 (d, *1H, J* = 8.5 Hz), 7.70 (s, 1H), 8.00 (d, 1H, *J* = 8.5 Hz), 8.25 (d, 1H, *J* = 7 Hz), 8.51 (d, 1H, *J* = 8 Hz), 8.67 (bs, 1H), 12.40 (s, 1H); $^{13}$C NMR (100 MHz, $CDCl_3$) δ 9.9 (2C), 35.9, 47.9 (2C), 52.5, 101.3, 116.7, 120.5, 120.6, 122.7, 126.5, 128.3, 131.0, 132.0, 133.0, 140.7, 141.2, 168.7, 177.7; IR (KBr) *v* $cm^{-1}$: 1301, 1449, 1522, 1560, 1580, 1610, 2966, 3343; MS (m/z, %) 463 ($M^+$, 1), 86 (100), 58 (12).

**Stage E: *N*-(2-diethylaminoethyl)-9,10-dihydro-6-iodo-9-oxoacridine-4-carboxamide hydrochloride (78).**

The compound **78** was prepared according to the procedure described for the preparation of the compound **3**, using *N*-(2-diethylaminoethyl)-9,10-dihydro-6-iodo-9-oxoacridine-4-carboxamide (**77**) as starting material. Yield: 46%; melting point: 266-268°C; $^1$H NMR (400 MHz, $d_6$-DMSO) δ 1.27 (t, 6H, *J* = 7 Hz), 3.23 (m, 4H), 3.34 (m, 2H), 3.76 (m, 2H), 7.37 (t, 1H, *J* = 7.5 Hz), 7.61 (t, 1H, *J* = 8.5 Hz), 7.93 (d, 1H, *J* = 8.5 Hz), 8.26 (s, 1H), 8.38 (d, 1H, *J* = 7.5 Hz), 8.42 (d, 1H, *J* = 8 Hz), 9.43 (m, 1H), 10.41 (bs, I H), 12.2 (bs, I H); $^{13}$C NMR (100 MHz, $d_6$-DMSO) δ 8.3 (2C), 34.2, 46.5 (2C), 49.7, 102.2, 118.6, 119.5, 120.4, 121.7, 126.8, 127.4, 130.2, 130.6, 133.6, 139.9, 140.6, 167.9, 176.2; IR (KBr) ν $cm^{-1}$: 1309, 1450, 1523, 1577, 1611, 3066, 3250-3450. Anal. calculated for $C_{20}H_{22}IN_3O_2 \cdot HCl \cdot 1.5H_2O$: C, 45.60; H, 4.97; N, 7.98. Found : C, 45.80; H, 4.71; N, 7.92.

**<u>EXAMPLE 18</u>**

Syntheses of *N*-(2-diethylaminoethyl)-9,10-dihydro-7-iodo-9-oxoacridine-4-carboxamide hydrochloride (**84**) and of *N*-(2-diethylaminoethyl)-9,10-dihydro-9-oxo-7-(tributylstannyl)acridine-4-carboxamide (**85**).

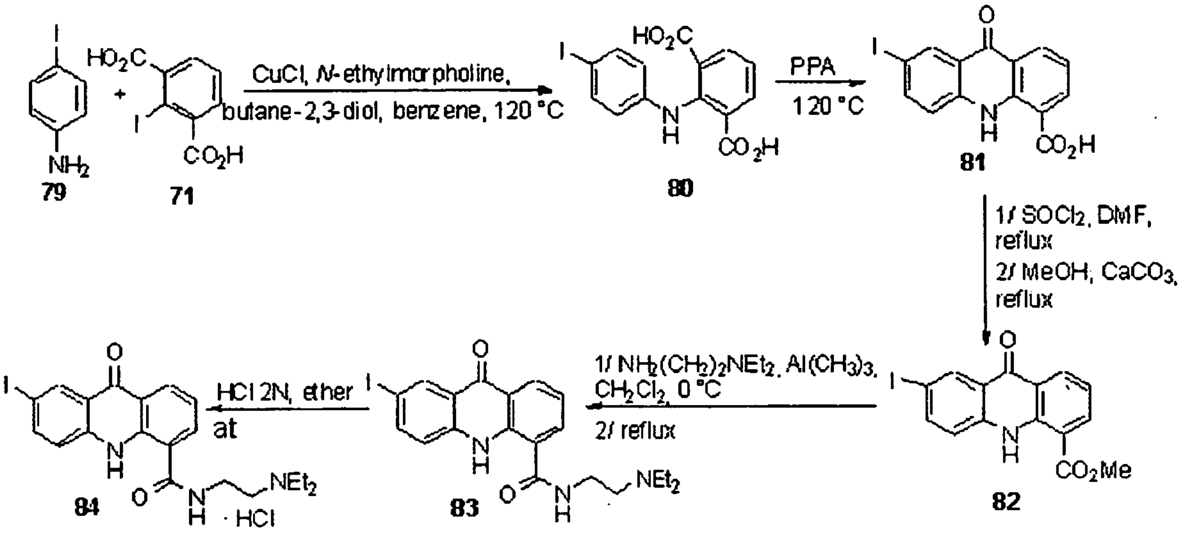


79 71 80 81 82 83 84

**Stage A: 2-(4-iodophenylamino)isophtalic acid (80)**

The compound **80** was prepared according to the procedure described for the preparation of the compound **72,** using 4-iodoaniline (**79**) as starting material. Yield: 62%; melting point: 241-243°C; $^1$H NMR (200 MHz, $d_6$-DMSO) δ 6.75 (d, 2H, *J* = 9 Hz), 7.04 (t, 1H, *J* = 8 Hz), 7.49 (d, 2H, *J* = 9 Hz), 7.94 (d, 2H, *J* = 8 Hz), 9.60 (s, 1 H); $^{13}$C NM R (100 MHz, $d_6$-DMSO) δ 83.8, 119.7 (2C), 119.8, 121.4 (2C), 135.1 (2C), 137.4 (2C), 143.3, 143.9, 168.5 (2C); IR (KBr) *v* cm$^{-1}$: 1243, 1408, 1505, 1592, 1689, 2800-3200; MS (m/z, %) 383 (M$^+$, 100), 194 (86), 166 (35), 139 (22).

**Stage B: 9,10-dihydro-7-iodo-9-oxoacridine-4-carboxylic acid (81)**

The compound **81** was prepared according to the procedure described for the preparation of the compound **73,** using 2-(4-iodophenylamino)isophtalic acid (**80**) as starting material. Yield: 63%; melting point: 355-357°C; $^1$H NMR (200 MHz, $d_6$-DMSO) δ 7.25 (t, 1H, *J* = 8 Hz), 7.46 (d, 1H, *J* = 9 Hz), 7.86 (d, 1H, *J* = 9 Hz), 8.34 (m, 3H), 11.93 (bs, 1H); $^{13}$C NMR (100 MHz, $d_6$-DMSO) δ 85.8, 115.9, 120.4, 121.0, 121.4, 122.2, 131.9, 134.0, 136.9, 139.0, 140.9, 141.6, 169.0, 175.1; IR (KBr) *v* cm$^{-1}$: 1148, 1446, 1518, 1609, 1693, 2800-3200; MS (m/z, %) 365 (M$^+$, 95), 347 (100), 319 (23), 164 (33).

**Stage C: methyl 9,10-dihydro-7-iodo-9-oxoacridine-4-carboxylate (82)**

The compound **82** was prepared according to the procedure described for the preparation of the compound **67**, using 9,10-dihydro-7-iodo-9-oxoacridine-4-carboxylic acid (**81**) as starting material. Yield: 60%; Rf 0.85 ($Al_2O_3$, dichloromethane); melting point: 224-226°C; NMR $^1$H (200 MHz, $CDCl_3$) δ 4.01 (s, 3H), 7.14 (d, 1H, *J* = 8.5 Hz), 7.25 (t, 1 H, *J* = 8 Hz), 7.89 (dd, 1 H, *J* = 8.5, 2 Hz), 8.41 (d, 1H, *J* = 8 Hz), 8.67 (d, I H, *J* = 8 Hz), 8.72 (d, 1H, J = 2 Hz), 11.76 (s, 1H); $^{13}$C NMR (100 MHz, $CDCl_3$) δ 52.7, 85.4, 113.9, 119.6, 120.4, 122.5, 123.2, 134.0, 136.0, 136.9, 139.3, 141.6, 142.3, 168.4, 176.5; IR (KBr) ν cm$^{-1}$: 1137, 1440, 1518, 1589, 1614, 1692, 3247; MS (m/z, %) 379 (M$^+$, 91), 347 (100), 220 (29), 164 (94), 75 (44), 63 (34).

**Stage D: *N*-(2-diethylaminoethyl)-9,10-dihydro-7-iodo-9-oxoacridine-4-carboxamide (83)**

The compound **83** was prepared according to the procedure described for the prcparation of the compound **2**, using methyl 9,10-dihydro-7-iodo-9-oxoacridine-4-carboxylate (**82**) as starting material and heating the reaction medium at reflux for 6 hours. Yield: 69%; Rf: 0.27 ($Al_2O_3$, dichloromethane / ethanol (99/1, v/v)); melting point: 140-142°C; $^1$H NMR (400 MHz, $CDCl_3$) δ 1.30 (t, 6H, *J* = 7 Hz), 3.01 (m, 4H), 3.10 (m, 2H), 3.76 (m, 2H), 7.07 (d, 1H, *J* = 9 Hz), 7.24 (t, 1H, *J* = 8 Hz), 7.81 (d, 1H, *J* = 9 Hz), 8.25 (d, 1H, *J* = 8 Hz), 8.53 (d, 1H, *J* = 8 Hz), 8.65 (m, 2H), 12.48 (s, 1H); $^{13}$C NMR (100 MHz, $CDCl_3$) δ 10.0 (2C), 35.9, 47.6 (2C), 52.2, 84.9, 116.8, 119.7, 120.4, 122.4, 122.8, 131.9, 132.9, 135.6, 139.2, 141.0, 141.9, 168.6, 176.5; IR (KBr) *v* cm$^{-1}$: 1300, 1516, 1613, 1647, 2800-3500; MS (m/z, %) 463 (M$^+$, 4), 86 (100), 58 (7).

Stage E: N-(2-diethylaminoethyl)-9,10-dihydro-7-iodo-9-oxoacridine-4-carboxamide hydrochloride (84)

The compound 84 was prepared according to the procedure described for the preparation of the compound 3, using N-(2-diethylaminoethyl)-9,10-dihydro-7-iodo-9-oxoacridine-4-carboxamide (83) as starting material. Yield: 99%; melting point: 220-222°C; $^1$H NMR (400 MHz, $d_6$-DMSO) δ 1.27 (t, 6H, J = 7 Hz), 3.23 (m, 4H), 3.34 (m, 2H), 3.76 (m, 2H), 7.38 (t, 1H, J = 8 Hz), 7.63 (d, 1H, J = 9 Hz), 8.00 (d, 1H, J = 9 Hz), 8.44 (m, 2H), 8.49 (s, 1H), 9.46 (bs, 1H), 10.40 (bs, 1H), 12.38 (bs, 1H); $^{13}$C NMR (100 MHz, $d_6$-DMSO) δ 8.3 (2C), 34.2, 46.5 (2C), 49.7, 85.7, 118.5, 120.4, 121.0, 121.6, 122.2, 130.4, 133.7, 134.1, 139.2, 140.1, 141.7, 168.0, 175.2; IR (KBr) ν $cm^{-1}$: 1303, 1517, 1617, 3200-3400. Anal. Calculated for $C_{20}H_{22}IN_3O_2\cdot HCl\cdot 2H_2O$ : C, 44.83; H, 5.08; N, 7.84. Found: C, 44.67; H, 4.43; N, 7.76.

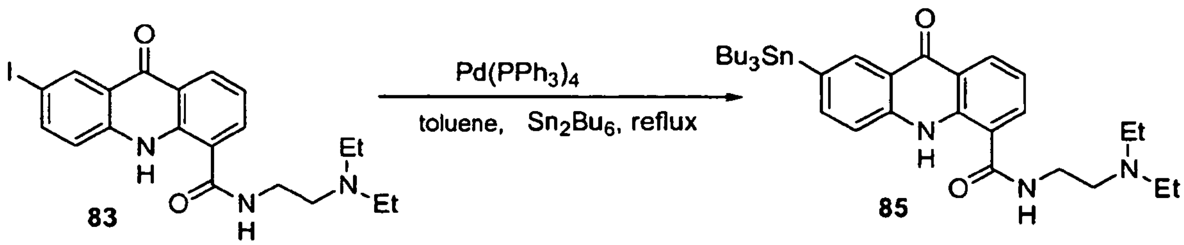


**Stage F : *N*-(2-diethylaminoethyl)-9,10-dihydro-9-oxo-7-(tributylstannyl)-acridine-4-carboxamide (85)**

The compound **85** was prepared according to the procedure described for the preparation of the compound **32**, using *N*-(2-diethylaminoethyl)-9,10-dihydro-7-iodo-9-oxoacridine-4-carboxamide (**83**) as starting material and heating the reaction medium at reflux for 4 hours. The residue obtained is chromatographed on a column of alumina eluted with ethyl acetate. Yield: 69%; Rf: 0.20 ($Al_2O_3$, ethyl acetate); viscous liquid; $^1$H NMR (200 MHz, $CDCl_3$) δ 0.88 (t, 9H, *J* = 7 Hz), 1.05 (m, 12H), 1.24 (m, 6H), 1.55 (m, 6H), 2.57 (q, 4H, *J* = 7 Hz), 2.70 (t, 2H, *J* = 7 Hz), 3.52 (m, 2H), 7.19 (t, 1H, *J* = 8 Hz), 7.37 (d, 1H, *J* = 8 Hz), 7.52 (bs, 1H), 7.75 (d, 1H, *J* = 8 Hz), 7.90 (d, 1H, *J* = 8 Hz), 8.54 (s, 1H), 8.65 (d, 1H, *J* = 8 Hz), 12.40 (bs, 1H); $^{13}$C NMR (50 MHz, $CDCl_3$) δ 9.9 (3C, $^1J_{Sn-c}$ = 324 Hz), 12.1 (2C), 13.7 (3C), 27.4 (3C, $^3J_{Sn-C}$ = 55 Hz), 29.2 (3C, $^2J_{Sn-C}$= 20 Hz), 37,3, 46.9 (2C), 51.1, 117.2, 117.8, 119.5, 121.1, 123.3, 131.3, 131.8, 134.8, 135.1, 140.4, 141.3, 141.4, 168.4, 178.2; IR ($CCl_4$) ν $cm^{-1}$: 1150, 1505, 1608, 1652, 2928, 2960; ESI-MS m/z 628.2 $[M+H]^+$.

<u>**EXAMPLE 19**</u>

Synthesis of *N*-(2-diethylaminoethyl)-9,10-dihydro-8-iodo-9-oxoacridine-4-carboxamide hydrochloride (**87**)

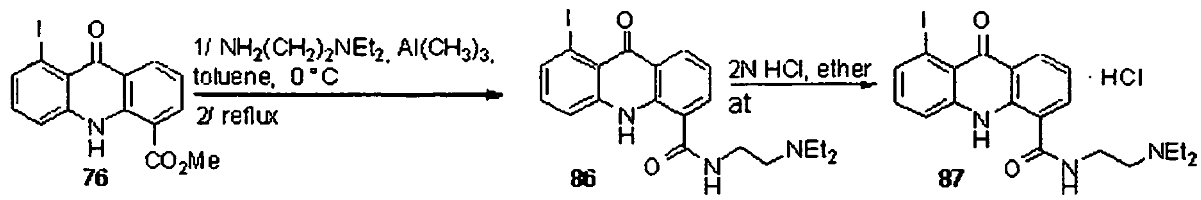


**Stage A: *N*-(2-diethylaminoethyl)-9,10-dihydro-8-iodo-9-oxoacridine-4-carboxamide (86)**

The compound **86** was prepared according to the procedure described for the preparation of the compound **2**, using methyl 9,10-dihydro-8-iodo-9-oxoacridine-4-carboxylate (**76**) as starting material and heating the reaction medium at reflux for 4 hours in anhydrous toluene. Yield: 63%; unstable product; Rf: 0.30 ($Al_2O_3$, dichloromethane / ethanol (99/1, v/v)); melting point: 144-146°C; $^1$H NMR (200 MHz, $CDCl_3$) δ 1.15 (t, 6H, *J* = 7 Hz), 2.76 (q, *4H, J* = 7 Hz), 2.86 (t, 2H, *J* = 6 Hz), 3.59 (m, 2H), 7.17 (m, 2H), 7.31 (d, 1H, *J* = 8 Hz), 7.80 (bs, I H), 7.85 (d, 1H, *J* = 7.5 Hz), 7.95 (d, 1 H, *J* = 7.5 Hz), 8.54 (d, 1H, *J* = 7 Hz), 11.30 (bs, 1H); $^{13}$C NMR (100 MHz, $CDCl_3$) δ 11.3 (2C), 36.6, 47.0 (2C), 51.3, 92.2, 116.8, 118.5, 118.7, 120.3, 122.4, 131.8, 132.1, 133.6, 136.6, 140.1, 141.3, 168.1, 176.1; IR (KBr) *ν* $cm^{-1}$: 1298, 1514, 1606, 1645, 2800-3500; MS (m/z, %) 463 ($M^+$, 1), 86 (100), 58 (12).

**Stage B: *N*-(2-diethylaminoethyl)-9,10-dihydro-8-iodo-9-oxoacridine-4-carboxamide hydrochloride (87)**

The compound **87** was prepared according to the procedure described for the preparation of the compound **3**, using *N*-(2-diethylaminoethyl)-9,10-dihydro-8-iodo-9-oxoacridine-4-carboxamide (**86**) as starting material. Yield: 91%; melting point: 228-230°C; $^1H$ NMR (400 MHz, $d_6$-DMSO) δ 1.25 (t, 6H, *J* = 7 Hz), 3.30 (m, 6H), 3.71 (m, 2H), 7.37 (m, 2H), 7.71 (d, 1H, *J* = 8 Hz), 7.90 (d, 1H, *J* = 7.5 Hz), 8.28 (d, 1H, *J* = 7.5 Hz), 8.43 (d, 1H, *J* = 7 Hz), 9.23 (bs, 1H), 9.44 (m, 1H), 12.26 (bs, 1H); $^{13}C$ NMR (100 MHz, $d_6$-DMSO) δ 8.3 (2C), 34.2, 46.5 (2C), 49.7, 92.4, 117.5, 117.8, 119.3, 120.4, 121.6, 130.9, 133.4, 134.2, 136.3, 139.3, 141.2, 168.0, 175.0; IR (KBr) ν $cm^{-1}$ : 1295, 1458, 1517, 1560, 1607, 2925, 3200-3400. Anal. Calculated for $C_{20}H_{22}IN_3O_2$-HCl·0.5$H_2O$ : C, 47.21; H, 4.74; N, 8.26. Found : C, 47.70; H, 5.12; N, 8.29.

## EXAMPLE 20

Synthesis of *N*-(2-diethylaminoethyl)-1-iodoacridine-4-carboxamide dihydrochloride **(91)**

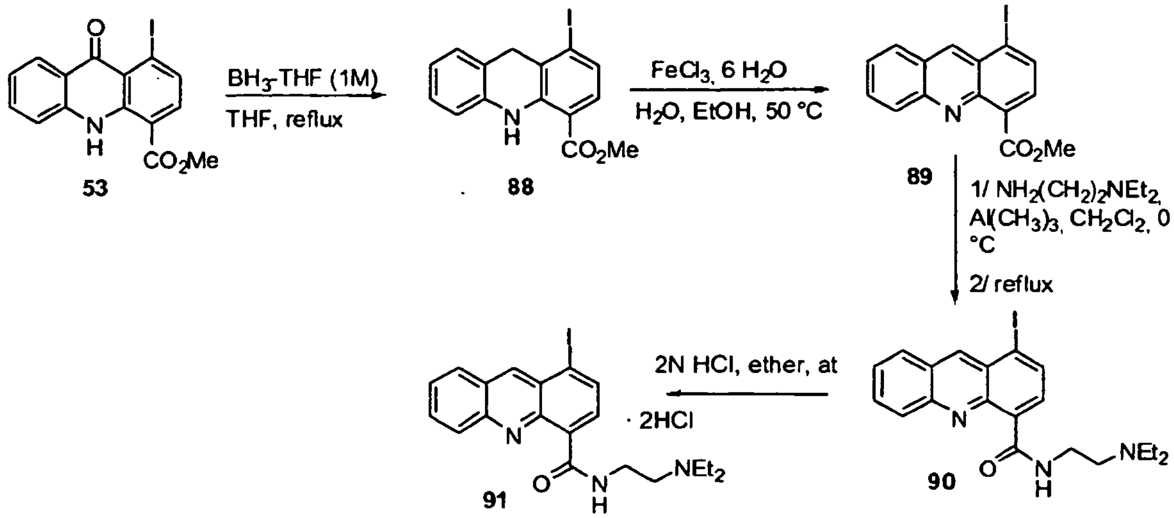


**Stage A: methyl I-iodoacridane-4-carboxylate (88)**

A 1M solution of $BH_3$-THF (0.82 ml, 0.82 mmol) is added dropwise at reflux, under argon, to a solution of methyl 9,10-dihydro-1-iodo-9-oxoacridine-4-carboxylate **(53)** (0.26 g, 0.69 mmol) in anhydrous THF (10 ml). The medium is heated at reflux for 45 minutes. After returning to ambient temperature, the reaction is halted with a 3N hydrochloric acid solution (5 ml) and then the medium is basified using a saturated aqueous sodium carbonate solution (pH = 8-9). The reaction mixture is extracted with dichloromethane (3 x 100 ml). The organic phase is dried over magnesium sulphate and evaporated under vacuum. The crude reaction product is purified by chromatography on silica eluted with dichloromethane, to result in the acridane **88** (188 mg, 0.52 mmol); yield: 75%; Rf: 0.91 ($SiO_2$, dichloromethane); melting point: 119-121°C; $^1H$ NMR (400 MHz, $CDCl_3$) δ 3.91 (s, 3H), 4.13 (s, 2H), 6.74 (d, 1H, *J* = 7.5 Hz), 6.88 (t, 1H, *J* = 7.5 Hz), 7.10 (m, 2H), 7.26 (d, 1H, *J* = 8.5 Hz), 7.41 (d, 1H, *J* = 8.5 Hz), 9.93 (bs, 1H); $^{13}C$ NMR (100 MHz, $CDCl_3$) δ 38.1, 52.0, 109.0, 109.8, 114.5, 119.5, 121.8, 124.3, 127.6, 128.6, 128.8, 130.1, 137.9, 143.6, 168.8; IR (KBr) ν $cm^{-1}$: 1252, 1269, 1431, 1498, 1585, 1690, 2948, 3304; MS (m/z, %) 365 ($M^+$, 100), 332 (78), 206 (52), 178 (53), 151 (23).

**Stage B: methyl 1-iodoacridine-4-carboxylate (89)**

Methyl 1-iodoacridane-4-carboxylate **(88)** (140 mg, 0.38 mmol) is dissolved in a mixture of water (2 ml) and ethanol (10 ml) and then $FeCl_3·6H_2O$ (0.30 g) is added. The solution is stirred at 50°C for 30 minutes and then a saturated aqueous sodium carbonate solution (20 ml) is added. The aqueous phase is subsequently extracted with dichloromethane (3 x 30 ml). The organic phase is dried over magnesium sulphate, filtered and evaporated to dryness. The product obtained **89** is very unstable and is used without purification (121 mg, 0.33 mmol); yield: 87%; $^1H$ NMR (400 MHz, $CDCl_3$) δ 4.00 (s, 3H), 7.53 (t, 1H, *J* = 7 Hz), 7.66 (d, 1H, *J* = 7 Hz), 7.76 (t, 1H, *J* = 7 Hz), 7.99 (d, 1H, *J* = 8 Hz), 8.07 (d, 1H, *J* = 7 Hz), 8.21 (d, 1H, *J* = 8 Hz), 8.91 (s, 1H).

**Stage C : *N*-(2-diethylaminoethyl)-1-iodoacridine-4-carboxamide (90)**

The compound **90** was prepared according to the procedure described for the preparation of the compound **2,** using methyl 1-iodoacridine-4-carboxylate **(89)** as starting material and heating the reaction medium at reflux for 4 hours. Yield: 80%; Rf: 0.40 ($Al_2O_3$, dichloromethane / ethanol (99/1, v/v)); melting point: 103-105 °C; $^1$H NMR (400 MHz, $CDCl_3$) δ 1.15 (t, 6H, *J* = 7 Hz), 2.78 (q, *4H, J* = 7 Hz), 2.88 (t, *2H, J* = 7 Hz), 3.83 (m, 2H), 7.63 (t, 1H, *J* = 7 Hz), 7.85 (t, 1H, *J* = 8 Hz), 8.07 (d, 1H, *J* = 8.5 Hz), 8.24 (d, 1H, *J* = 8 Hz), 8.29 (d, 1 H, *J* = 8.5 Hz), 8.60 (d, 1H, *J* = 7.5 Hz), 9.04 (s, 1H), 11.82 (bs, 1H); $^{13}$C NMR (100 MHz, $CDCl_3$) δ 11.6 (2C), 38.0, 47.1 (2C), 51.9, 103.8, 126.9, 127.0, 128.0, 128.3, 128.7, 129.4, 131.9, 135.4, 137.1, 142.6, 145.8, 148.1, 165.4; IR (KBr) ν $cm^{-1}$: 1517, 1649, 1654, 2967, 3186, 3300-3500; MS (m/z, %) 447 ($M^+$, 8), 332 (18), 177 (17), 86 (100), 58 (15).

**Stage D: *N*-(2-dietliylaminoetliyl)-1-iodoacridine-4-carboxamide dihydrochloride (91)**

The compound **91** was prepared according to the procedure described for the preparation of the compound **3,** using *N*-(2-diethylaminoethyl)-1-iodoacridine-4-carboxamide **(90)** as starting material. Yield: 71%; melting point: 219-221°C; $^1$H NMR (400 MHz, $d_6$-DMSO) δ 1.27 (t, 6H, *J* = 7 Hz), 3.27 (m, 4H), 3.43 (m, 2H), 4.00 (m, 2H), 7.80 (t, 1H, *J* = 7.5 Hz), 8.06 (d, 1H, *J* = 7.5 Hz), 8.40 (d, 1H, *J* = 7.5 Hz), 8.46 (m, 2H), 8.58 (d, 1H, *J* = 9 Hz), 9.34 (s, 1H), 10.25 (s, 1H), 11.30 (s, 1H); $^{13}$C NMR (100 MHz, $d_6$-DMSO) δ 8.5 (2C), 34.5, 46.9 (2C), 54.9, 105.4, 126.6, 127.4, 127.5, 128.3, 128.8, 132.7, 134.8, 136.8, 142.9, 144.7, 147.4, 165,4, one carbon not observed; IR (KBr) ν $cm^{-1}$: 1394, 1549, 1577, 1624, 2472, 2638, 2975, 3200-3500. Anal. Calculated for $C_{20}H_{22}IN_3O{\cdot}2HCl$: C, 46.18; H, 4.65; N, 8.08. Found : C, 46.38; H, 4.79; N, 7.99.

**<u>EXAMPLE 21</u>**

Synthesis of *N*-(2-diethylaminoethyl)-2-iodoacridine-4-carboxamide dihydrochloride **(95)**

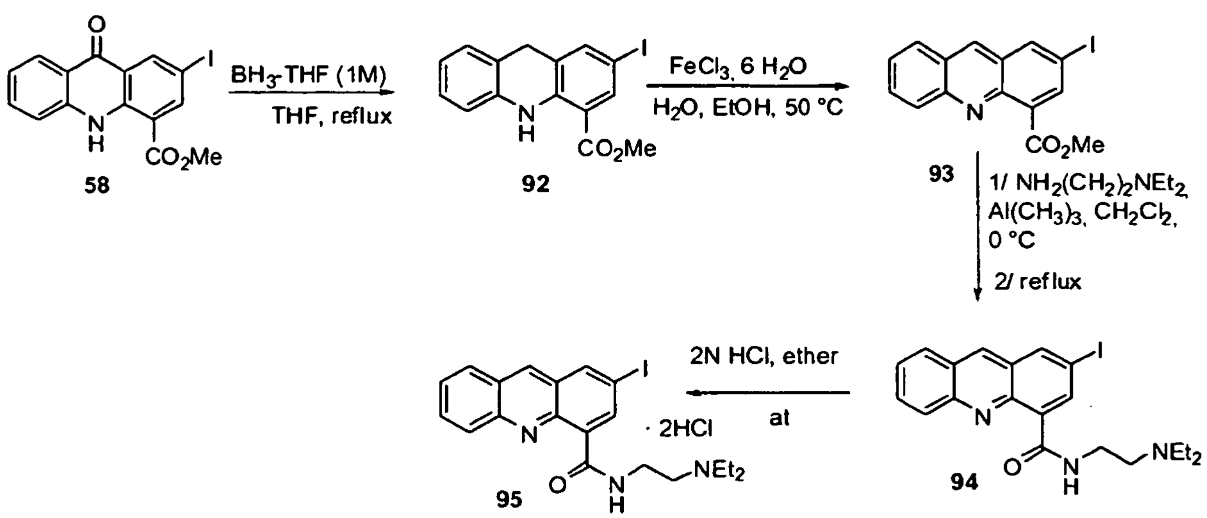


**Stage A: methyl 2-iodoacridane-4-carboxylate (92)**

The compound **92** was prepared according to the procedure described for the preparation of the compound **88,** using methyl 9,10-dihydro-2-iodo-9-oxoacridine-4-carboxylate **(58)** as starting material. Yield: 58%; Rf: 0.93 ($SiO_2$, dichloromethane); melting point: 148-150°C; $^1$H NMR (400 MHz, $CDCl_3$) δ 3.92 (s, 3H), 4.06 (s, 2H), 6.77 (d, 1H, *J* = 7.5 Hz), 6.90 (t, 1H, *J* = 7.5 Hz), 7.06 (d, 1H, *J* = 7.5 Hz), 7.12 (t, 1H, *J* = 7.5 Hz), 7.48 (s, 1H), 8.08 (s, 1H), 9.77 (bs, 1H); $^{13}$C NMR (100 MHz, $CDCl_3$) δ 31.0, 52.2, 79.2, 112.3, 114.9, 119.4, 121.9, 124.4, 127.5, 128.4, 137.5, 138.3, 141.3, 143.3, 167.7; IR (KBr) ν $cm^{-1}$: 1253, 1263, 1501, 1676, 2941, 3344; MS (m/z, %) 365 ($M^+$, 100), 332 (23), 178 (58), 151 (20), 127 (22).

**Stage B: methyl 2-iodoacridine-4-carboxylate (93)**

The compound **93** was prepared according to the procedure described for the preparation of the compound **89,** using methyl 2-iodoacridane-4-carboxylate **(92)** as starting material. Yield: 93%; very unstable product; $^1$H NMR (200 MHz, $CDCl_3$) δ 4.14 (s, 3H), 7.62 (t, 1H, *J* = 7.5 Hz), 7.88 (t, 1H, *J* = 7.5 Hz), 8.05 (d, 1H, *J* = 8 Hz), 8.36 (m, 2H), 8.59 (s, I H), 8.86 (s, I H).

**Stage C : *N*-(2-diethylaminoethyl)-2-iodoacridine-4-carboxamide (94)**

The compound **94** was prepared according to the procedure described for the preparation of the compound **2,** using methyl 2-iodoacridine-4-carboxylate **(93)** as starting material and heating the reaction medium at reflux for 4 hours. Yield: 80%; Rf: 0.41 ($Al_2O_3$, dichloromethane / ethanol (99/1, v/v)); melting point: 108-110˚C; $^1$H NMR (400 MHz, $CDCl_3$) δ 1.19 (t, 6H, *J* = 7 Hz), 2.85 (m, 4H), 2.96 (m, 2H),-3.88 (m, 2H), 7.61 (t, 1H, *J* = 7 Hz), 7.87 (t, 1H, *J* = 8 Hz), 8.01 (d, 1H, *J* = 8.5 Hz), 8.29 (d, 1H, *J* = 8.5 Hz), 8.51 (s, 1H), 8.71 (s, 1H), 9.10 (s, 1H), 11.88 (bs, 1H); $^{13}$C NMR (100 MHz, $CDCl_3$) δ 11.2 (2C), 37.7, 47.2 (2C), 51.7, 91.0, 126.2, 127.1, 128.2, 128.4, 129.4, 129.6, 131.8, 136.3, 140.7, 143.4, 145.0, 147.9, 164.8; IR (KBr) ν $cm^{-1}$: 1516, 1637, 2965, 3182; MS (m/z, %) 447 ($M^+$, 1), 177 (8), 86 (100), 58 (13).

**Stage D: *N*-(2-diethylaminoethyl)-2-iodoacridine-4-carboxamide dihydrochloride (95)**

The compound **95** was prepared according to the procedure described for the preparation of the compound **3,** using *N*-(2-diethylaminoethyl)-2-iodoacridinc-4-carboxamide **(94)** as starting material. Yield: 76%; melting point: 215-217˚C; $^1$H NMR (200 MHz, $d_6$-DMSO) δ 1.26 (t, 6H, *J* = 7 Hz), 3.24 (m, 4H), 3.41 (m, 2H), 3.83 (m, 2H), 7.73 (m, 1H), 8.01 (t, 1H, *J* = 8 Hz), 8.23 (d, 1H, *J* = 8.5 Hz), 8.52 (d, 1H, *J* = 8.5 Hz), 8.79 (s, 1H), 8.90 (s, 1H), 9.27 (s, 1H), 10.49 (m, 1H), 11.33 (bs, 1H); $^{13}$C NMR (100 MHz, $d_6$-DMSO) δ 8.5 (2C), 34.5, 46.9 (2C), 49.6, 91.2, 125.8, 127.4, 128.0, 128.6, 128.7, 129.1, 132.4, 138.0, 141.0, 142.0, 143.5, 146.9, 164.5; IR (KBr) ν $cm^{-1}$: 1572, 1625, 1649, 2651, 2975, 3200-3500. Anal. Calculated for $C_{20}H_{22}IN_3O{\cdot}2HCl{\cdot}1.5H_2O$ : C, 43.90; H, 4. 97; N,7.68. Found : C, 43.77; H, 4.77; N, 7.54.

__EXAMPLE 22__

Synthesis of *N*-(2-diethylaminoethyl)-3-iodoacridine-4-carboxamide dihydrochloride **(98)**

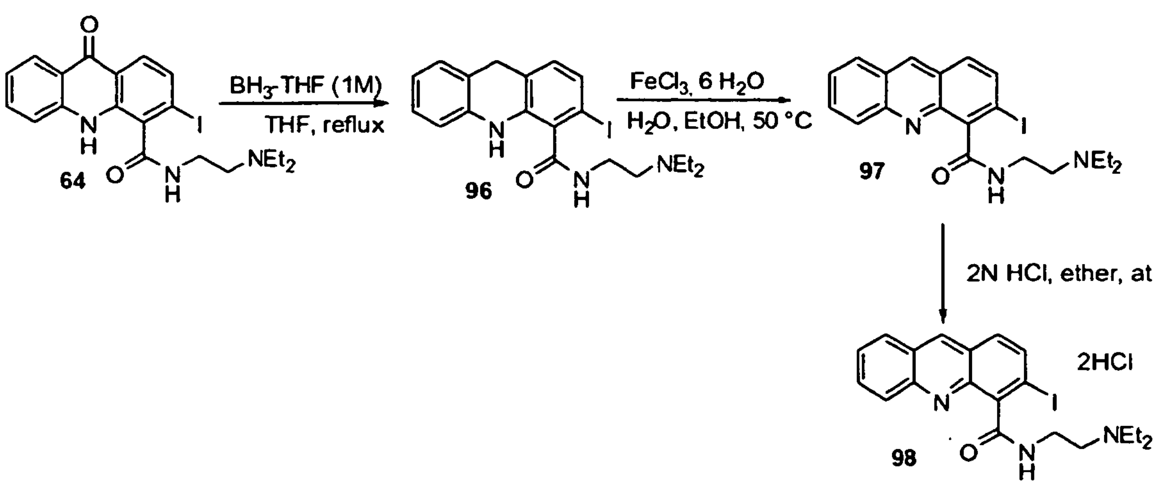


64 96 97 98

**Stage A : *N*-(2-diethylaminoethyl)-3-iodoacridane-4-carboxamide (96)**

The compound **96** was prepared according to the procedure described for the preparation of the compound **88,** using methyl *N*-(2-diethylaminoethyl)-9,10-dihydro-3-iodo-9-oxoacridine-4-carboxylate **(64)** as starting material. Yield: 60%; unstable product; melting point: 106-108˚C; $^1$H NMR (200 MHz, $CDCl_3$) δ 1.26 (t, 6H, *J* = 7 Hz), 2.99 (m, 6H), 3.91 (q, 2H, *J* = 6.5 Hz), 3.98 (s, 2H), 6.48 (m, 1H), 6.71 (d, 1H, *J* = 8 Hz), 6.79 (t, 1H, *J* = 8 Hz), 6.89 (m, 1H), 7.09 (m, 2H), 7.26 (m, 2H); $^{13}$C NMR (50 MHz, $CDCl_3$) δ 8 8.5 (2C), 31.1, 35.1, 53.4 (2C), 57.0, 90.1, 114.6, 119.2, 121.3,

121.5, 124.5, 127.3, 128.3, 130.9, 131.3, 138.8, 139.6, 168.8; IR (KBr) ν $cm^{-1}$: 1164, 1449; 1636, 2346, 3268; MS (m/z, %) 449 ($M^+$, 1), 86 (100), 58 (10).

**Stage B: *N*-(2-diethylaminoethyl)-3-iodoacridine-4-carboxamide (97)**

The compound **97** was prepared according to the procedure described for the preparation of the compound **89,** using *N*-(2-diethylaminoethyl)-3-iodoacridane-4-carboxamide **(96)** as starting material. Yield: 73%; Rf: 0.13 ($Al_2O_3$, ethyl acetate / pentane (1/1, v/v)); melting point: 81-83°C; $^1$H NMR (400 MHz, $CDCl_3$) δ 1.04 (t, 6H, *J* = 7 Hz), 2.69 (m, 4H), 2.91 (m, 2H), 3.81 (m, 2H), 6.95 (bs, 1H), 7.54 (d, 1H, *J* = 8 Hz), 7.65 (d, 1H, *J* = 9 Hz), 7.76 (t, 1H, *J* = 8Hz), 7.80 (d, 1H, *J* = 9 Hz), 7.94 (d, 1H, *J* = 8.5 Hz), 8.17 (d, 1H, *J* = 9 Hz), 8.68 (s, I H); $^{13}$C NMR (100 MHz, $CDCl_3$) δ 11.3 (2C), 37.2, 46.8 (2C), 51.6, 97.1, 125.3, 126.5, 126.6, 128.0, 129.5, 130.1, 130.7, 135.1, 136.0, 142.5, 146.2, 149.2, 169.2; IR (KBr) ν $cm^{-1}$: 1458, 1654, 2925, 3200-3500; MS (m/z, %) 447 ($M^+$, 1), 177 (11), 86 (100), 58 (12).

**Stage C: *N*-(2-diethylaminoethyl)-3-iodoacridine-4-carboxamide dihydrochloride (98)**

The compound **98** was prepared according to the procedure described for the preparation of the compound **3,** using *N*-(2-diethylaminoethyl)-3-iodoacridine-4-carboxamide **(97)** as starting material. Yield: 83%; melting point: 134-136°C; $^1$H NMR (200 MHz, $CD_3OD$) δ 1.47 (t, 6H, *J* = 7 Hz), 3.49 (q, 4H, *J* = 7 Hz), 3.62 (t, 2H, *J* = 7 Hz), 3.97 (t, 2H, *J* = 7Hz), 7.79 (m, 1H), 8.06 (m, 3H), 8.35 (m, 2H), 9.40 (s, 1H); $^{13}$C NMR (100 MHz, *$d_6$-DMSO*) δ 8.8 (2C), 34.1, 46.9 (2C), 49.4, 97.9, 124.9, 126.2, 126.6, 128.7, 128.9, 130.0, 131.6, 135.0, 137.0, 142.3, 145.6, 148.3 169.0; IR (KBr) ν $cm^{-1}$ : 1458, 1560, 1636, 1654, 2924, 3200-3500. Anal. Calculated for $C_{20}H_{22}IN_3O$, 2HCl, $H_2O$ : C, 44.63; H, 4.87; N, 7.81. Found : C, 44.58; H, 4.92; N, 7.42.

**EXAMPLE 23**

Syntheses of *N*-(2-diethylaminoethyl)-5-iodoacridine-4-carboxamide dihydrochloride **(102)** and of *N*-(2-diethylaminoethyl)-5-(tributylstannyl)acridine-4-carboxamide **(103)**

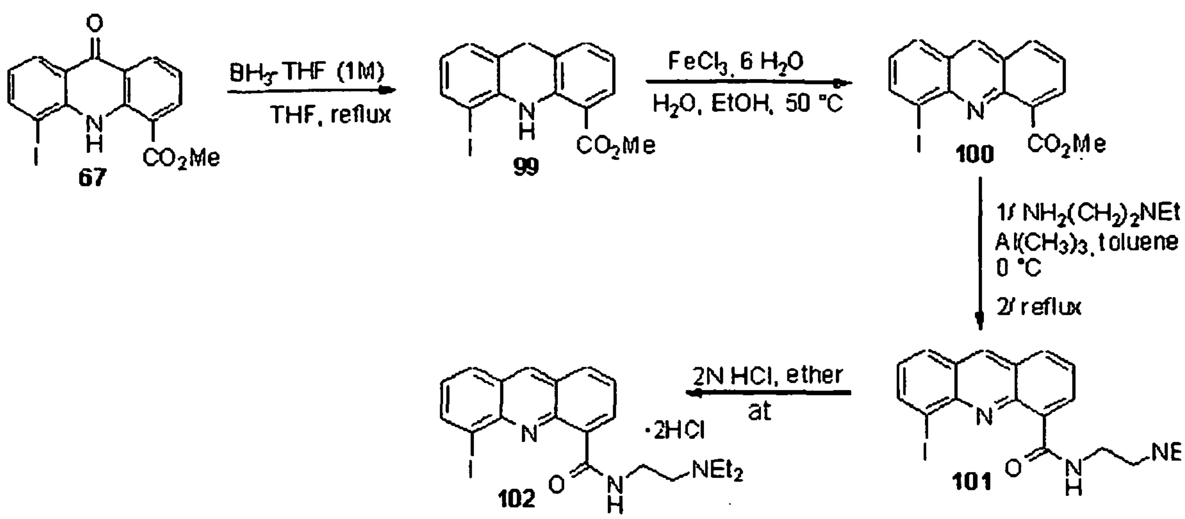


**Stage A: methyl 5-iodoacridane-4-carboxylate (99)**

The compound **99** was prepared according to the procedure described for the preparation of the compound **88,** using methyl 9,10-dihydro-5-iodo-9-Oxoacridine-4-carboxylate **(67)** as starting material. Yield: 98%; Rf: 0.94 ($SiO_2$ dichloromethane); melting point: 105-107°C; $^1$H NMR (400 MHz, $CDCl_3$) 8 3.97 (s, 3H), 4.08 (s, 2H), 6.61 (t, 1H, *J* = 7.5 Hz), 6.83 (t, 1H, *J* = 7.5 Hz), 7.03 (d, 1H, *J* = 7 Hz), 7.24 (d, 1H, *J* = 7 Hz), 7.58 (d, *1H, J* = 8 Hz), 7.84 (d, 1H, *J* = 8 Hz), 10.16 (bs, 1H); $^{13}$C NMR (100 MHz, $CDCl_3$) δ 32.2, 52.2, 84.3, 111.3, 119.6, 121.1, 121.7, 122.7, 128.3, 129.5, 133.3, 137.3, 140.1, 143.0, 168.5; IR (KBr) ν $cm^{-1}$ : 1259, 1443, 1491, 1689, 2924, 3260; MS (m/z, %) 365 ($M^+$, 100), 332 (56), 206 (53), 177 (81), 151 (63), 103 (36), 89 (37), 75 (46).

**Stage B: methyl 5-iodoacridine-4-carboxylate (100)**

The compound **100** was prepared according to the procedure described for the preparation of the compound **89,** using methyl 5-iodoacridane-4-carboxylate **(99)** as starting material. Yield: 97%; very unstable product; $^1H$ NMR (200 MHz, $CDCl_3$) δ 4.20 (s, 3H), 7.17 (dd, 1H, *J* = 8.5, 7 MHz), 7.51 (dd, 1H, *J* = 8.5, 7 Hz), 7.87 (d, 1H, *J* = 8.5 Hz), 8.03 (d, 1H, *J* = 8.5 Hz), 8.11 (d, 1H, *J* = 7 Hz), 8.39 (d, 1H, *J* = 7 Hz), 8.60 (s, 1H).

**Stage C : *N*-(2-diethylaminoethyl)-5-iodoacridine-4-carboxamide (101)**

The compound **101** was prepared according to the procedure described for the preparation of the compound **2,** using methyl 5-iodoacridine-4-carboxylate **(100)** as starting material and heating the reaction medium at reflux for 3 hours in anhydrous toluene. Yield: 73%; Rf: 0.17 ($Al_2O_3$, dichloromethane / ethanol (99/1, v/v)); melting point: 64-66°C; $^1H$ NMR (200 MHz, $CDCl_3$) δ 1.11 (t, 6H, *J* = 7 Hz), 2.69 (q, 4H, *J* = 7 Hz), 2.92 (m, 2H), 3.82 (m, 2H), 7.19 (dd, 1H, *J* = 8.5, 7 Hz), 7.56 (dd, 1H, *J* = 8.5, 7 Hz), 7.86 (d, 1H, *J* = 8.5 Hz), 7.98 (d, 1H, *J* = 8.5 Hz), 8.32 (d, 1H, *J* = 7 Hz), 8.60 (s, 1H), 8.88 (d, 1H, *J* = 7 Hz), 11.76 (s, 1H); $^{13}$NMR (100 MHz, $CDCl_3$) δ 11.8 (2C), 38.4, 47.5 (2C), 52.4, 102.6, 125.8, 126.1, 127.0, 127.1, 128.2, 129.5, 132.9, 136.0, 138.6, 141.6, 145.8, 146.3, 165.4; IR (KBr) ν $cm^{-1}$: 1648, 1654, 2925, 3200, 3300-3500; MS (m/z, %) 447 ($M^+$, 1), 177 (9), 86 (100), 58 (12).

**Stage D: *N*-(2-diethylaminoethyl)-5-iodoacridine-4-carboxamide dihydrochloride (102)**

The compound **102** was prepared according to the procedure described for the preparation of the compound **3,** using *N*-(2-diethylaminoethyl)-5-iodoacridine-4-carboxamide **(101)** as starting material. Yield: 69%; melting point: 144-146°C; $^1H$ NMR (400 MHz, $d_6$-DMSO) δ 1.27 (t, 6H, *J* = 7 Hz), 3.26 (m, 4H), 3.43 (m, 2H), 4.10 (m, 2H), 7.48 (t, 1H, *J* = 7.5 Hz), 7.83 (t, 1H, *J* = 7.5 Hz), 8.27 (d, 1H, *J* = 8.5 Hz), 8.46 (d, 1H, *J* = 8 Hz), 8.60 (d, 1H, *J* = 7 Hz), 8.84 (d, 1H, *J* = 7 Hz), 9.36 (s, 1H), 10.72 (bs, 1H), 12.27 (bs, 1H); $^{13}C$ NMR (100 MHz, *$d_6$*-DMSO) δ 8.4 (2C), 34.8, 46.4 (2C), 49.9, 104.0, 125.9, 126.0, 126.9, 127.0, 127.8, 129.3, 133.2, 135.7, 140.1, 141.8, 145.6, 145.8, 165.2; IR (KBr) ν $cm^{-1}$: 1546, 1578, 1654, 2660, 2928, 3300-3500. Anal. Calculated for $C_{20}H_{22}IN_3{\cdot}O\text{-}2HCl\text{-}H_2O$: C, 44.63; H, 4.87; N, 7.81. Found: C, 44.80; H, 4.73; N, 7.63.

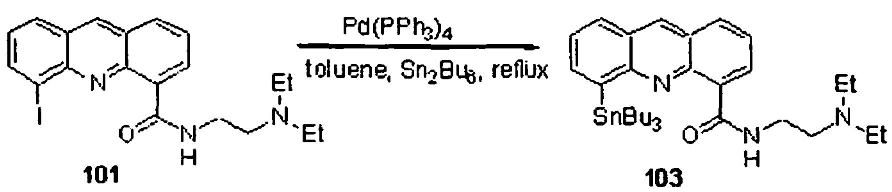


**101** **103**

**Stage E : *N*-(2-diethylaminoethyl)-5-(tributylstannyl)acridine-4-carboxamide(103)**

The compound **103** was prepared according to the procedure described for the preparation of the compound **32,** using *N*-(2-diethylaminoethyl)-5-iodoacridine-4-carboxamide **(101)** as starting material and heating at reflux for 32 hours. The residue obtained is chromatographed on a column of alumina eluted with a mixture of ethyl acetate and pentane (1/1, v/v), resulting, in order of elution, in:

***N*-(2-diethylaminoethyl)-5-(tributylstannyl)acridine-4-carboxamide (103)**

Yield: 25%; Rf: 0.79 ($Al_2O_3$, ethyl acetate / pentane (1/1, v/v)); viscous liquid; $^1H$ NMR (200 MHz, $CDCl_3$) δ 0.7 (t, 9H, *J* = 7 Hz), 1.3 (m, 18H), 1.45 (m, 6H), 2.76 (m, 4H), 2.88 (m, 2H), 3.73 (m, 2H), 7.54 (t, 1H, *J* = 7.5 Hz), 7.64 (dd, 1H, *J* = 7.5, 8.5 Hz), 8.00 (m, 2H), 8.15 (d, 1H, *J* = 8.5 Hz), 8.88 (s, 1H), 8.93 (d, 1H, *J* = 7.5 Hz), 10.59 (bs, 1H); $^{13}C$ NMR (50 MHz, $CDCl_3$) δ 10.1 (3C, $^1J_{Sn\text{-}C}$ = 343 Hz), 12.2 (2C), 13.7 (3C), 27.4 (3C, $^3J_{Sn\text{-}C}$ = 60 Hz), 29.3 (3C, $^2J_{sn\text{-}C}$= 20 Hz), 39.1, 47.8 (2C), 52.1, 125.1, 126.0, 126.5, 127.0, 128.8, 129.3, 132.7, 135.3, 139.3, 141.0, 145.0, 146.4, 153.0, 166.7; IR ($CCl_4$) ν $cm^{-1}$: 1286, 1662, 2927, 2959; ESI-MS m/z 612.3 $[M+H]^+$.

***N*-(2-diethylaminoethyl)acridine-4-carboxamide (**Atwell, G. J.; Rewcastle, G. W., Baguley, B. C. and Denny, W. A., J. Med. Chem., 1987, 30, 664-669). Yield 29%; Rf: 0.33 ($Al_2O_3$, ethyl acetate / pentane (1/1, v/v)); melting point 148-150°C.

**EXAMPLE 24.**

Synthesis of *N*-(2-diethylaminoethyl)-6-iodoacridine-4-carboxamide dihydrochloride (107)

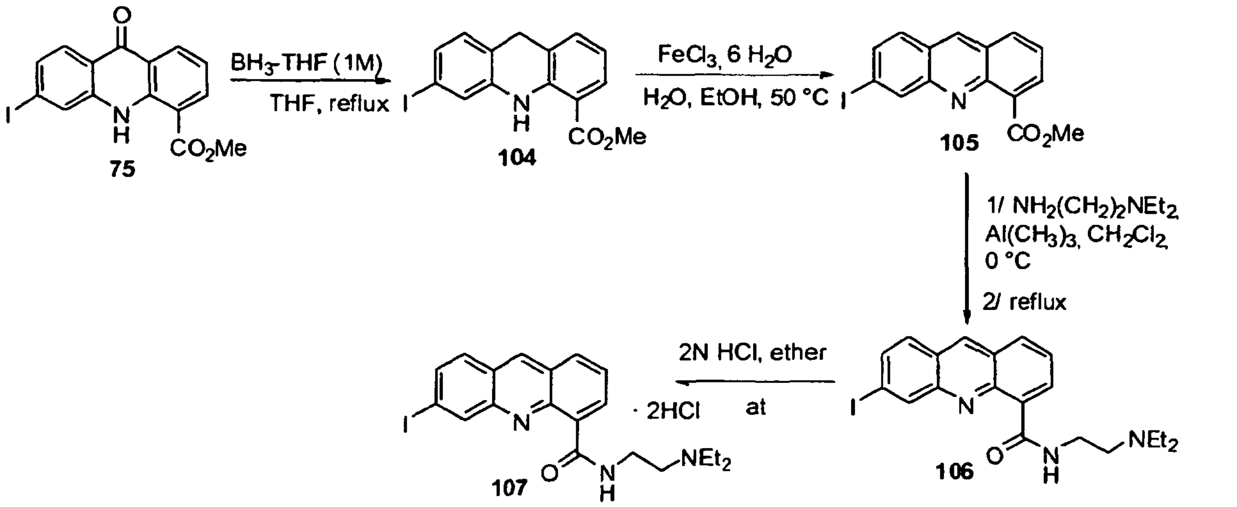


**Stage A: methyl 6-iodoacridane-4-carboxylate (104)**

The compound **104** was prepared according to the procedure described for the preparation of the compound **88,** using methyl 9,10-dihydro-6-iodo-9-oxoacridine-4-carboxylate (75) as starting material. Yield: 76%; unstable product; melting point: 108-110°C; $^1$H NMR (400 MHz, $CDCl_3$) δ 3.94 (s, 3H), 4.01 (s, 2H), 6.80 (m, 2H), 7.14 (s, 1H), 7.18 (d, 1H, J = 8 Hz), 7.22 (d, 1H, J = 7.5 Hz), 7.79 (d, 1H, J = 8 Hz), 9.82 (bs, I H); $^{13}$C NMR (100 MHz, $CDCl_3$) δ 31.2, 52.0, 91.6, 110.8, 119.2, 119.5, 121.3, 123.3, 129.4, 129.9, 130.2, 133.6, 140.5, 143.0, 168.8; IR (KBr) ν $cm^{-1}$: 1266, 1429, 1464, 1507, 1594, 1684,2942,3342.

Stage B: methyl 6-iodoacridine-4-carboxylate (105)

The compound 105 was prepared according to the procedure described for the preparation of the compound 89, using methyl 6-iodoacridane-4-carboxylate (104) as starting material. Yield: 88%; very unstable product; $^1$H NMR (200 MHz, $CDCl_3$) δ 4.12 (s, 3H), 7.54 (dd, 1H, *J* = 8.5, 7 Hz), 7.67 (m, 2H), 8.06 (d, 1H, *J* = 8.5 Hz), 8.12 (d, 1H, *J* = 7 Hz), 8.70 (s, 1H), 8.77 (s, 1H).

**Stage C : *N*-(2-diethylaminoethyl)-6-iodoacridine-4-carboxamide (106)**

The compound **106** was prepared according to the procedure described for the preparation of the compound **2,** using methyl 6-iodoacridine-4-carboxylate **(105)** as starting material and heating the reaction medium at reflux for 4 hours. Yield: 52%; Rf: 0.37 ($Al_2O_3$, dichloromethane / ethanol (99/1, v/v)); melting point: 80-82 °C; $^1$H NMR (200 MHz, $CDCl_3$) δ 1.16 (t, 6H, *J* = 7 Hz), 2.79 (m, 6H), 3.77 (q, 2H, *J* = 6 Hz), 7.65 (dd, 1H, *J* = 8.5, 7 Hz), 7.70 (d, 1H, *J* = 9 Hz), 7.78 (d, 1H, *J* = 9 Hz), 8.06 (d, 1H, *J* = 8.5 Hz), 8.78 (s, 1H), 8.8 (s, 1H), 8.97 (d, 1H, *J* = 7 Hz), 11.90 (bs, 1H); $^{13}$C NMR (50 MHz, CDCl;) δ 12.0 (2C), 38.0, 47.0 (2C), 51.9, 98.1, 124.6, 126.0, 127.0, 128.9, 129.0, 132.1, 134.8, 135.8, 137.6, 138.4, 146.6, 147.7, 165.5; IR (KBr) ν $cm^{-1}$: 1458, 1559, 1654, 2924, 3300-3500: MS (m/z, %) 447 ($M^+$, 3), 177 (9), 86 (100), 58 (15).

**Stage D: *N*-(2-diethylaminoethyl)-6-iodoacridine-4-carboxamide dihydrochloride(107)**

The compound **107** was prepared according to the procedure described for the preparation of the compound **3,** using *N*-(2-diethylaminoethyl)-6-iodoacridine-4-carboxamide **(106)** as starting material. Yield: 72%; melting point: 201-203°C; $^1$H NMR (400 MHz, *$d_6$*-DMSO) δ 1.27 (t, 6H, *J* = 7 Hz), 3.24 (m, 4H), 3.38 (m, 2H), 3.97 (q, 2H, *J* = 6 Hz), 7.79 (dd, I H, *J* = 8.5 and 7 Hz), 7.95 (d, 1H, J = 9 Hz), 8.02 (d, 1H, J = 9 Hz), 8.40 (d, I H, J = 8.5 Hz), 8.77 (d, 1H, J = 7 Hz), 9.11 (s, 1H), 9.37 (s, 1H), 10.47 (bs, I H), 11.33 (t, 1H, J = 6 Hz); $^{13}$C NMR (100 MHz, $d_6$-DMSO) δ 8.5 (2C), 34.4,

46.9 (2C), 49.8, 100.5, 124.5, 125.6, 126.5, 127.7, 129.8, 133.4, 134.9, 135.5, 137.2, 139.5, 145.3, 147.2, 165.6; IR (KBr) v cam$^{-1}$: 1609, 1637, 2970, 3300-3500. Anal. Calculated for $C_{20}H_{22}IN_3O{\cdot}2HCL{\cdot}0.5\ H_2O$ : C, 45.39; H, 4.76; N, 7.94. Found : C, 45.23; H, 4.70; N, 7.72.

EXAMPLE 25

Syntheses of N-(2-diethylaminoethyl)-7-iodoacridine-4-carboxamide dihydrochloride (111) and of N-(2-diethylaminoethyl)-7-(tributylstannyl)acridine-4-carboxamide (152).

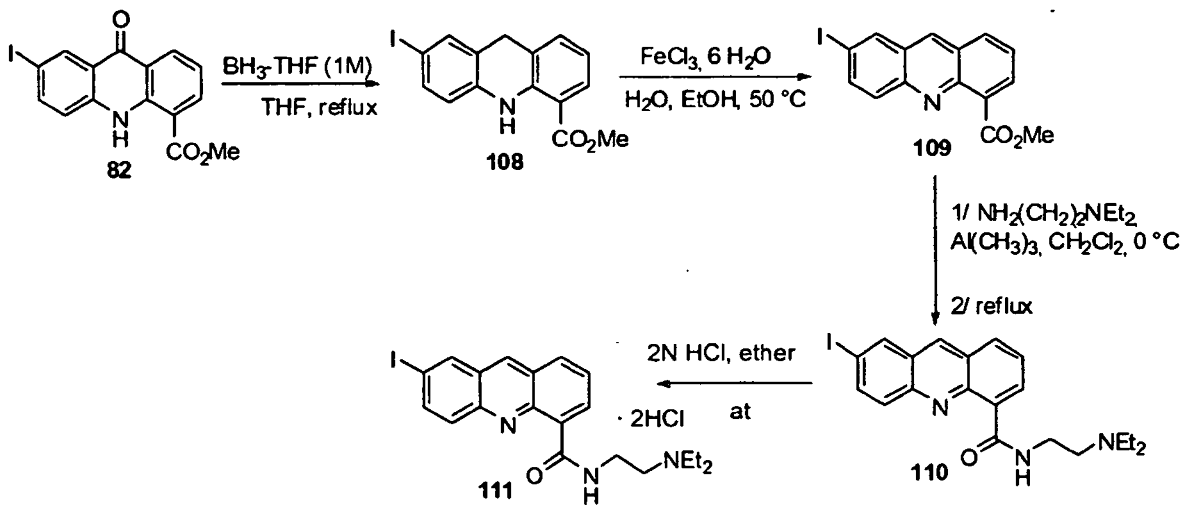


**Stage A: methyl 7-iodoacri(lane-4-carboxylate (108)**

The compound **108** was prepared according to the procedure described for the preparation of the compound **88,** using methyl 9,10-dihydro-7-iodo-9-oxoacridine-4-carboxylate **(82)** as starting material. Yield: 95%; Rf: 0.90 ($SiO_2$, dichloromethane); melting point: 122-124°C; $^1$H NMR (400 MHz, $CDCl_3$) δ 3.91 (s, 3H), 4.04 (s, 2H), 6.54 (d, 1H, *J* = 8.5 Hz), 6.79 (t, 1H, *J* = 7.5 Hz), 7.22 (d, 1H, *J* = 7 Hz), 7.37 (m, 2H), 7.77 (d, 1H, *J* = 7.5 Hz), 9.83 (bs, 1H); $^{13}$C NMR (100 MHz, $CDCl_3$) δ 30.4, 52.0, 82.9, 110.7, 116.7, 119.1, 121.1, 122.4, 129.4, 133.7, 136.0, 136.8, 138.8, 143.1, 168.9; IR (KBr) v cm$^{-1}$ : 1194, 1269, 1430, 1466, 1502, 1594, 1682, 2948, 3320; MS (m/z, %) 365 (M$^+$, 100), 333 (50), 206 (40), 177 (65), 151 (44), 127 (30), 103 (29), 89 (33), 75 (42).

**Stage B: methyl 7-iodoacridine-4-carboxylate (109)**

The compound **109** was prepared according to the procedure described for the preparation of the compound **89,** using methyl 7-iodoacridane-4-carboxylate **(108)** as starting material. Yield: 95%; very unstable product; $^1$H NMR (200 MHz, $CDCl_3$) δ 4.13 (s, 3H), 7.48 (dd, 1H, *J* = 8.5 and 7 Hz), 7.98 (m, 3H), 8.09 (d, 1H, *J* = 7 Hz), 8.29 (s, 1H), 8.53 (s, 1H).

**Stage C: *N*-(2-diethylaminoethyl)-7-iodoacridine-4-carboxamide (110)**

The compound **110** was prepared according to the procedure described for the preparation of the compound **2,** using methyl 7-iodoacridine-4-carboxylate **(109)** as starting material and heating the reaction medium at reflux for 6 hours. Yield: 53%; Rf: 0.33 ($Al_2O_3$, dichloromethane / ethanol (99/1, v/v)); melting point: 176-178°C; $^1$H NMR (200 MHz, $CDCl_3$) δ 1.26 (t, 6H, *J* = 7 Hz), 2.98 (m, 6H), 3.98 (m, 2H), 7.70 (dd, 1H, *J* = 8.5 and 7 Hz), 8.06 (d, 1H, *J* = 9 Hz), 8.16 (m, 2H), 8.47 (s, 1H), 8.78 (s, 1H), 8.98 (d, 1H, *J* = 7 Hz), 12.0.1 (bs, 1H); $^{13}$C NMR (100 MHz, $CDCl_3$) 6 10.8 (2C), 37.2, 47.2 (2C), 51.6, 92.2, 126.1, 127.0, 127.5, 128.3, 130.9, 132.6, 135.8, 136.4, 136.8, 139.8, 146.3, 146.5, 166.1; IR (KBr) v cm$^{-1}$: 1457, 1515, 1562, 2965, 1665, 3300-3500; MS (m/z, %) 447 (M$^+$, 2), 332 (10), 305 (12), 177 (8), 86 (100), 58 (8).

**Stage D: *N*-(2-diethylaminoethyl)-7-iodoacridine-4-carboxamide dihydrochloride (111)**

The compound **111** was prepared according to the procedure described for the preparation of the compound **3,** using *N*-(2-diethylaminoethyl)-7-iodoacridine-4-carboxamide **(110)** as starting material. Yield: 67%; melting point: 213-215°C; $^1$H NMR (400 MHz, *$d_6$-DMSO)* δ 1.27 (t, 6H, *J* = 7 Hz), 3.25 (m, 4H), 3.41 (m, 2H), 3.92 (m, 2H), 7.79 (t, 1H, *J* = 7.5 Hz), 8.19 (d, 1H, *J* = 9 Hz), 8.36 (d, 1H, *J* = 9 Hz), 8.41 (d, 1H, *J* = 8.5 Hz), 8.74 (m, 2H), 9.28 (s, 1H), 10.57 (bs, 1H), 11.32 (bs, 1H); $^{13}$C NMR (100 MHz, *$d_6$-DMSO*) δ 8.5 (2C), 34.4, 46.9 (2C), 49.7, 93.3, 125.8, 126.5, 127.2, 128.0, 130.6, 133.3, 135.1, 136.7, 137.8, 139.8, 145.3, 145.7; IR (KBr) ν $cm^{-1}$: 1403, 1585, 1628, 2665, 2953, 3222, 3200-3500. Anal. Calculated for $C_{20}H_{22}IN_3O{\cdot}2HCl{\cdot}2.5H_2O$ : C, 42.50; H, 5.17; N, 7.43. Found : C, 42.78; H, 4.96; N, 7.35.

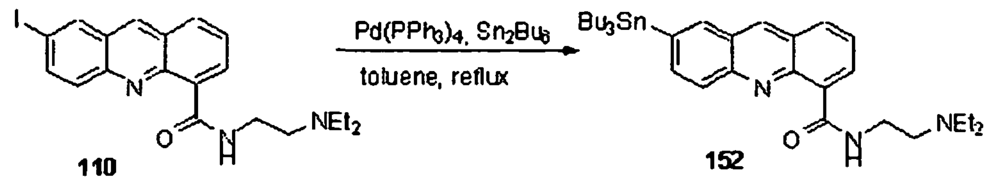


110 152

**Stage E : *N*-(2-diethylaminoethyl)-7-(tributylstannyl)acridine-4-carboxamide (12)**

The compound **152** was prepared according to the procedure described for the preparation of the compound **32,** using *N*-(2-diethylaminoethyl)-7-iodoacridine-4-carboxamide **(110)** as starting material and heating the reaction medium at reflux for 5 hours. The residue obtained is chromatographed on a column of alumina eluted with a mixture of ethyl acetate and pentane (7/3, v/v). Yield: 40%; Rf: 0.29 ($Al_2O_3$, ethyl acetate / pentane (7/3, v/v)); viscous liquid; $^1$H NMR (400 MHz, $CDCl_3$) δ 0.90 (t, 9H, *J* = 7 Hz), *1.13 (t, 6H, J= 7 Hz),* 1.21 (m, 6H), 1.37 (st, 6H, *J* = 7 Hz), 1.60 (m, 6H), 2.74 (q, 4H, *J* = 7 Hz), 2.85 (t, *2H, J* = 6.5 Hz), 3.80 (q, *2H, J* = 6.5 Hz), 7.62 (m, 1H), 7.90 (d, 1H, *J* = 8.5 Hz), 8.12 (m, 2H), 8.21 (d, 1H, *J* = 8.5 Hz), 8.82 (s, 1H), 8.96 (d, 1H, *J* = 7 Hz), 12.04 (bs, 1H); $^{13}$ C NMR (100 MHz, $CDCl_3$) δ 9.8 (3C, $^1JS_{n-C}$ = 333 Hz), 11.9 (2C), 13.7 (3C), 27.4 *(3C, $^3J_{Sn-C}$* = 55 Hz), 29.1 (3C, $^2J_{Sn-C}$ = 20 Hz), 38.1, 47.1 (2C), 52.0, 125.2, 126.0, 126.9, 127.7, 128.6, 132.4, 135.0, 136.7, 136.9, 138.0, 141.4, 146.3, 147.7, 166.0; 1R ($CCl_4$) ν $cm^{-1}$: 1464, 1514, 1559, 1657, 2854, 2873, 2928, 2961, 3150-3250; ESI-MS m/z 612.2 $[M+H]^+$.

<u>**EXAMPLE 26**</u>

Synthesis of *N*-(2-dicthylaminocthyl)-8-iodoacridine-4-carboxamide dihydrochloride **(115)**

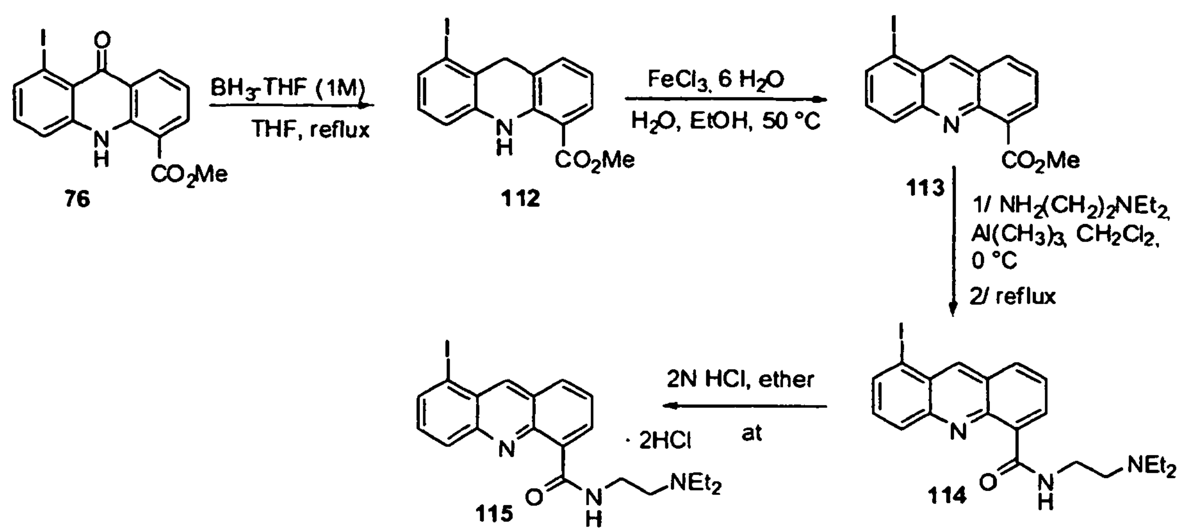


76 112 113 114 115

**Stage A: methyl 8-iodoacridane-4-carboxylate (112)**

The compound **112** was prepared according to the procedure described for the preparation of the compound **88,** using methyl 9,10-dihydro-8-iodo-9-oxoacridine-4-carboxylate **(76)** as starting material. Yield: 92%; Rf: 0.97 ($SiO_2$,

dichloromethane); melting point: 108-110°C; $^1$H NMR (400 MHz, CDCb) δ 3.90 (s, 3H), 4.09 (s, 2H), 6.69 (d, 1H, *J* = 8 Hz), 6.77 (m, 2H), 7.24 (d, 1H, *J* = 7.5 Hz), 7.36 (d, 1H, *J* = 8 Hz), 7.77 (d, 1H, *J* = 8 Hz), 9.77 (bs, 1H); $^{13}$C NMR (100 MHz, $CDCl_3$) δ 37.5, 51.9, 101.2, 110.3, 114.8, 115.7, 119.0, 122.5, 128.9, 129.4, 131.4, 133.9, 139.5, 142.8, 168.9; IR (KBr) ν $cm^{-1}$: 1262, 1442, 1501, 1603, 1685, 2960, 3319; MS (m/z, %) 365 ($M^+$, 100), 333 (53), 332 (55), 206 (81), 178 (90), 177 (95), 151 (71), 127 (42), 103 (42), 89 (32), 75 (66), 63 (42).

**Stage B: methyl 8-iodoacridine-4-carboxylate (113)**

The compound **113** was prepared according to the procedure described for the preparation of the compound **89,** using methyl 8-iodoacridane-4-carboxylate **(112)** as starting material. Yield: 90%; very unstable product; $^1$H NMR (200 MHz, $CDCl_3$) δ 4.11 (s, 3H), 7.51 (dd, 1H, *J* = 8.5 and 7 Hz), 7.62 (dd, 1H, *J* = 8.5 and 7 Hz), 8.22 (m, 3H), 8.32 (d, 1H, *J* = 8.5 Hz), 9.02 (s, 1H).

**Stage C: *N*-(2-diethylaminoethyl)-8-iodoacridine-4-carboxamide (114)**

The compound **114** was prepared according to the procedure described for the preparation of the compound **2,** using methyl 8-iodoacridine-4-carboxylate **(113)** as starting material and heating the reaction medium at reflux for 16 hours in anhydrous dichloromethane. Yield: 55%; Rf: 0.35 ($Al_2O_3$, dichloromethane / ethanol (99/1, v/v)); melting point: 78-80°C; $^1$H NMR (400 MHz, $CDCl_3$) δ 1.16 (t, 6H, *J* = 7 Hz), 2.80 (q, 4H, *J* = 7 Hz), 2.91 (m, 2H), 3.85 (m, 2H), 7.50 (t, 1H, *J* = 8 Hz), 7.69 (t, 1H, *J* = 7.5 Hz), 8.16 (m, 2H), 8.27 (d, 1H, *J* = 8.5 Hz), 8.96 (s, 1H), 8.98 (d, 1H, *J* = 7 Hz), 11.76 (bs, 1H); $^{13}$C NMR (100 MHz, $CDCl_3$) δ 11.4 (2C), 37.7, 47.1 (2C), 51.8, 98.4, 126.1, 127.5, 127.7, 128.2, 130.2, 131.6, 132.4, 135.9, 137.4, 142.4, 146.7, 147.4, 165.6; IR (KBr) ν $cm^{-1}$: 1508, 1546, 1654, 2925, 3200; MS (m/z, %) 447 ($M^+$, 1), 177 (11), 86 (100), 58 (11).

**Stage D: *N*-(2-diethylaminoethyl)-8-iodoacridine-4-carboxamide dihydrochloride (115)**

The compound **115** was prepared according to the procedure described for the preparation of the compound **3,** using *N*-(2-diethylaminoethyl)-8-iodoacridine-4-carboxamide **(114)** as starting material. Yield: 72%; melting point: 128-130°C; $^1$H NMR (400 MHz, *$d_6$-DMSO*) δ 1.28 (t, 6H, *J* = 7 Hz), 3.25 (m, 4H), 3.41 (m, 2H), 3.99 (m, 2H), 7.71 (t, 1H, *J* = 7.5 Hz), 7.82 (t, 1H, *J* = 7 Hz), 8.33 (d, 1H, *J* = 7 Hz), 8.60 (m, 2H), 8.76 (d, 1H, *J* = 7 Hz), 9.25 (s, 1H), 10.73 (m, 1H), 11.26 (bs, 1H); $^{13}$C NMR (100 MHz, *$d_6$-DMSO*) δ 8.5 (2C), 34.4, 46.9 (2C), 49.7, 99.2, 125.9, 127.1, 127.3, 127.6, 130.0, 132.6, 133.4, 135.3, 137.8, 142.7, 145.6, 147.1, 165.5; IR (KBr) ν $cm^{-1}$: 1507, 1559, 1653, 3300-3500. Anal. Calculated for $C_{20}H_{22}IN_3O{\cdot}2HCl{\cdot}I.5H_2O$ : C, 43.90; H, 4.97; N, 7.68. Found : C, 43.87; H, 4.77; N, 7.38.

**EXAMPLE 27**

Synthesis of *N*-(2-diethylaminoethyl)-2-iodoacridine-9-carboxamide dihydrochloride **(120)**

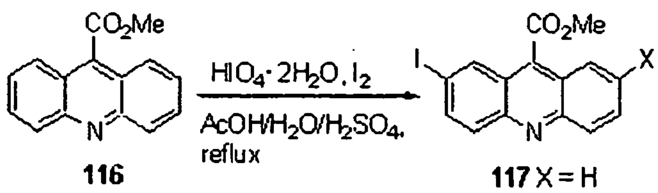


**116** **117** X = H **118** X = I

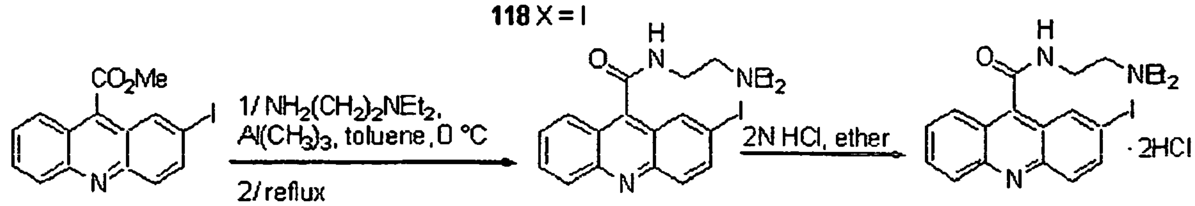


**117** **119** **120**

**Stage A : methyl 2-iodoacridine-9-carboxylate (117) and methyl 2,7-diiodoacridine-9-carboxylate (118)**

Periodic acid dihydrate (76 mg, 0.33 mmol) and diiodine (216 mg, 0.85 mmol) are added to a solution of methyl

acridine-9-carboxylate **(116)** (Renotte, R., Sarlet, G., Thunus, L. and Lejeune, R., Luminescence, 2000, 15, 311-320) (0.40 g, 1.69 mmol) in a mixture of concentrated sulphuric acid (80 μl), water (320 μl) and acetic acid (1 ml). The solution is stirred at reflux for 8 hours. After returning to ambient temperature, water (2 ml) and then a saturated aqueous sodium carbonate solution (10 ml) are added. The medium is extracted with dichloromethane (3 x 20 ml). The organic phase is dried with magnesium sulphate, filtered and then evaporated to dryness. The residue obtained is purified by chromatography on a column of silica eluted with dichloromethane, to result, in order of elution, in the following products:

**Methyl 2,7-diiodoacridine-9-carboxylate (118)**

(50 mg, 0.10 mmol); yield: 6%; Rf: 0.50 ($SiO_2$, dichloromethane); melting point: 201-203°C; $^1$H NMR (400 MHz, $CDCl_3$) δ 4.24 (s, 3H), 8.04 (m, 4H), 8.42 (d, 2H, *J* = 2 Hz); $^{13}$C NMR (100 MHz, $CDCl_3$) δ 53.5, 94.7 (2C), 123.9 (2C), 130.8 (2C), 134.1 (2C), 139.8, 146.8 (2C), 166.7; IR (KBr) ν $cm^{-1}$: 1220, 1720; MS (m/z, %) 489 ($M^+$, 100), 458 (29), 430 (18), 347 (10), 303 (17), 164 (14).

**Methyl 2-iodoacridine-9-carboxylate (117)**

(98 mg, 0.27 mmol); yield: 16%; Rf: 0.30 ($SiO_2$, dichloromethane); melting point: 106-108°C; $^1$H NMR (400 MHz, $CDCl_3$) δ 4.20 (s, 3H), 7.58 (m, 1H), 7.79 (m, 1H), 7.95 (m, 2H), 7.98 (d, 1H, *J* = 8 Hz), 8.20 (d, 1H, *J* = 8.5 Hz), 8.39 (s, 1H); $^{13}$C NMR (100 MHz, $CDCl_3$) δ 53.2, 93.6, 122.5, 123.7, 125.3, 127.8, 130.0, 130.7, 131.3, 134.0, 135.4, 138.9, 147.1, 148.8, 167.4; IR (KBr) ν $cm^{-1}$: 1218, 1728; MS (m/z, %) 363 ($M^+$, 100), 332 (33), 304 (28), 177 (33).

**Stage B : *N*-(2-diethylaminoethyl)-2-iodoacridine-9-carboxamide (119)**

The compound **119** was prepared according to the procedure described for the preparation of the compound **2,** using methyl 2-iodoacridine-9-carboxylate **(117)** as starting material and heating the reaction medium at reflux for 24 hours in anhydrous toluene. Yield: 58%; Rf: 0.61 ($Al_2O_3$, dichloromethane / ethanol (99/1, v/v)); melting point: 83-85°C; $^1$H NMR (400 MHz, $CDCl_3$) δ 1.00 (t, 6H, *J* = 7 Hz), 2.58 (q, 4H, *J* = 7 Hz), 2.78 (t, 2H, *J* = 6 Hz), 3.79 (q, 2H, *J* = 6 Hz), 6.85 (m, 1H), 7.55 (m, 1H), 7.80 (m, 1H), 7.91 (d, 1H, *J* = 9 Hz), 7.96 (d, 1H, *J* = 9 Hz), 8.08 (d, 1H, *J* = 9 Hz), 8.17 (d, 1H, *J* = 8.5 Hz), 8.48 (s, 1H); $^{13}$C NMR (50 MHz, $CDCl_3$) δ 11.6 (2C), 37.6, 46.8 (2C), 51.8, 93.1, 122.3, 123.6, 125.6, 127.3, 129.7, 130.7, 131.1, 134.2, 138.9, 140.1, 147.2, 148.7, 166.5; IR (KBr) ν $cm^{-1}$: 1636, 2956, 3200-3400; MS (m/z, %) 447 ($M^+$, 1), 177 (3), 86 (100), 58 (9).

**Stage C: *N*-(2-diethylaminoethyl)-2-iodoacridine-9-carboxamide dihydrochloride (120)**

The compound **120** was prepared according to the procedure described for the preparation of the compound **3,** using *N*-(2-diethylaminoethyl)-2-iodoacridine-9-carboxamide **(119)** as starting material. Yield: 78%; melting point: 121-123°C; $^1$H NMR (400 MHz, *$d_6$-DMSO*) δ 1.29 (t, 6H, *J* = 7 Hz), 3.22 (m, 4H), 3.41 (m, 2H), 3.96 (m, 2H), 7.79 (m, 1H), 8.06 (t, 1H, *J* = 7.5 Hz), 8.13 (m, 2H), 8.24 (d, 1H, *J* = 9 Hz), 8.33 (d, 1H, *J* = *9* Hz), 8.45 (s, 1H), 9.55 (t, 1H, *J* = 6 Hz), 10.79 (m, 1H); $^{13}$C NMR (50 MHz, $d_6$-DMSO) δ 8.5 (2C), 34.3, 46.6 (2C), 49.3, 94.6, 122.0, 123.2, 126.2, 126.7, 128.8, 128.5, 133.0, 133.9, 140.6, 142.9, 144.4, 146.0, 165.3; IR (KBr) ν $cm^{-1}$: 1420, 1464, 1654, 2400-2600, 2976, 3200-3500. Anal. Calculated for $C_{20}H_{22}IN_3O\text{-}_2HCl\cdot 1.5H_2O$ : C, 43.90; H, 4.97; N, 7.68. Found : C, 43.73; H, 4.72; N, 7.58.

**EXAMPLE 28**

Synthesis of *N*-(2-diethylaminoethyl)-2,7-diiodoacridine-9-carboxamide dihydrochloride **(122)**

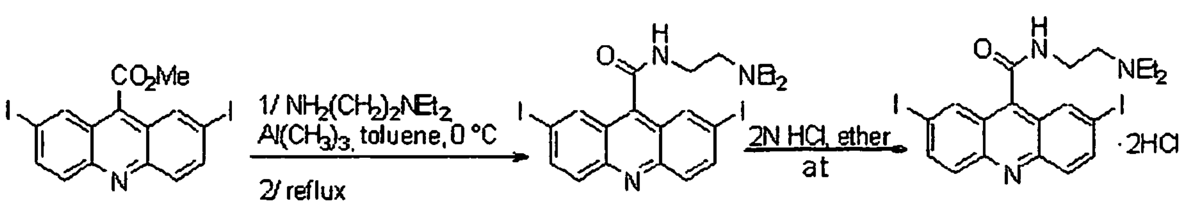


**118** **121** **122**

**Stage A: *N*-(2-diethylaminoethyl)-2,7-diiodoacridine-9-carboxamide (121)**

The compound **121** was prepared according to the procedure described for the preparation of the compound

**2,** using methyl 2,7-diiodoacridine-9-carboxylate **(118)** as starting compound and heating the reaction medium at reflux for 20 hours in anhydrous toluene. Yield: 57%; Rf: 0.72 ($Al_2O_3$, dichloromethane / ethanol (99/1, v/v)); melting point: 209-211 °C; $^1$H NMR (400 MHz, $CDCl_3$) δ 1.03 (t, 6H, *J* = 7 Hz), 2.63 (q, 4H, *J* = 7 Hz), 2.80 (t, 2H, *J* = 6 Hz), 3.81 (q, 2H, *J* = 6 Hz), 6.90 (m, 1H), 7.88 (d, 2H, *J* = 9 Hz), 7.98 (d, 2H, *J* = 9 Hz), 8.46 (s, 2H); $^{13}$C NMR (100 MHz, $CDCl_3$) δ 11.7 (2C), 37.8, 46.7 (2C), 51.7, 94.1 (2C), 123.6 (2C), 130.9 (2C), 134.4 (2C), 138.9, 139.3 (2C), 147.1 (2C), 165.9; IR (KBr) ν $cm^{-1}$: 1637, 2924, 3300-3500; MS (m/z, %) 573 ($M^+$, 1), 458 (2), 430 (3), 303 (7), 86 (100), 58 (12).

**Stage B: *N*-(2-diethylaminoethyl)-2,7-diiodoacridine-9-carboxamide dihydrochloride (122)**

The compound **122** was prepared according to the procedure described for the preparation of the compound **3,** using *N*-(2-diethylaminoethyl)-2,7-diiodoacridine-9-carboxamide **(121)** as starting material. Yield: 92%; melting point: 224-226°C; $^1$H NMR (200 MHz, $d_6$-DMSO) δ 1.31 (t, 6H, *J* = 7 Hz), 3.24 (m, 4H), 3.42 (m, 2H), 3.96 (m, 2H), 8.03 (d, 2H, *J* = 9 Hz), 8.21 (d, 2H, *J* = 9 Hz), 8.42 (s, 2H), 9.56 (m, 1H), 10.74 (bs, 1H); $^{13}$C NMR (50 MHz, $d_6$-DMSO) δ 8.6 (2C), 34.4, 46.6 (2C), 49.3, 95.3 (2C), 123.4 (2C), 129.6 (2C), 133.9 (3C), 140.3 (2C), 145.5 (2C), 165.0; IR (KBr) ν $cm^{-1}$: 1420, 1663, 2575, 3200-3500. Anal. Calculated for $C_{20}H_{22}1N_3O.2HCl$ : C, 37.18; H, 3.59; N, 6.50. Found : C, 37.57; H, 3.76; N, 6.18.

**EXAM PLE 29**

Synthesis of *N*-(2-diethylaminoethyl)-2-iodo-9-(4-methanesulphonamido-2-methoxyanilino)acridine-4-carboxamide dihydrochloride **(125)**

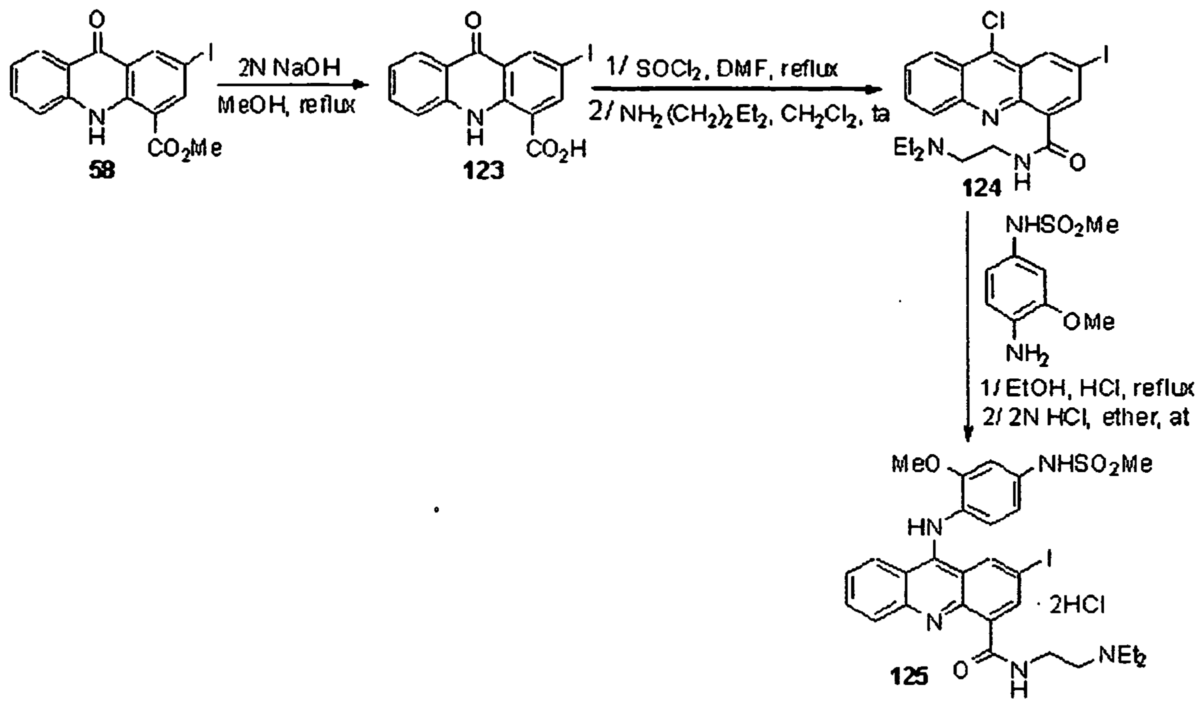

58
123
124
125

**Stage A : 9,10-dihydro-2-iodo-9-oxoacridine-4-carboxylic acid (123)**

A 2N aqueous sodium hydroxide solution (30 ml) is added to a solution of ester **58** (0.30 g, 0.79 mmol) in ethanol (30 ml). The reaction mixture is brought to reflux for 10 minutes and then filtered while hot. After returning to ambient temperature, the filtrate is acidified with glacial acetic acid (pH = 5), concentrated to 2/3 and cooled in an ice bath until precipitation has occurred. The precipitate formed is filtered off and washed with water, then taken up in anhydrous ethanol (30 ml) and, finally, dried under vacuum, to result in the acid **123** (0.26 g, 0.71 mmol); yield: 90%; melting point: > 400°C; $^1$H NMR (400 M Hz, $d_6$-DMSO) δ 7.31 (t, 1H, *J* = 7.5 Hz), 7.64 (d, 1H, *J* = 7.5 Hz), 7.77 (t, 1H, *J* = 7.5 Hz), 8.25 (d, 1H, *J* = 7.5 Hz), 8.59 (m, 2H), 14.45 (bs, 1H); $^{13}$C NMR (100 MHz, $d_6$-DMSO) δ 83.3, 118.4, 120.4, 121.5, 123.1, 124.8, 126.0, 133.9, 136.5, 139.9, 140.7, 143.2, 167.8, 175.5; IR (KBr) ν $cm^{-1}$ : 1165, 1514, 1615, 3000-3400; MS (m/z, %) 365 ($M^+$, 5), 164 (10), 84 (27), 69 (64), 55 (100).

**Stage B: 9-chloro-*N*-(2-diethylaminoethyl)-2-iodoacridine-4-carboxamide (124)**

A suspension comprising 9,10-dihydro-2-iodo-9-oxoacridine-4-carboxylic acid (**123**) (0.88 g, 2.41 mmol), thionyl chloride (5 ml) and dimethylformamide (1 drop) is brought to reflux for 45 minutes. The thionyl chloride is driven off by evaporating under vacuum (temperature of the bath of the evaporator < 40˚C). The acid chloride thus formed is taken up in anhydrous toluene (10 ml) in order to be once again evaporated to dryness. It is subsequently cooled to -5˚C and then a solution of *N*,*N*-diethylethylenediamine (1.32 ml, 9.24 mmol) in anhydrous dichloromethane (10 ml) is added, under argon, at -5˚C. The reaction medium is stirred at ambient temperature for 2 hours. The organic phase is washed with a 10% aqueous sodium carbonate solution (2 x 20 ml) and a saturated aqueous sodium chloride solution (20 ml). The organic phase is dried over magnesium sulphate, filtered and evaporated to dryness. The chlorinated derivative **124** obtained is very unstable and is used without further purification (0.93 g, 1.93 mmol); yield: 80%; $^1$H NMR (200 MHz, $CDCl_3$) δ 1.23 (t, 6H, *J* = 7 Hz), 2.95 (m, 6H), 3.93 (m, 2H), 7.70 (t, 1H, *J* = 7.5 Hz), 7.91 (t, 1H, *J* = 7.5 Hz), 8.38 (m, 2H), 8.98 (d, 1H, *J* = 2 Hz), 9.12 (d, 1H, *J* = 2 Hz), 11.77 (bs, 1H).

**Stage C: *N*-(2-diethylaminoethyl)-2-iodo-9-(4-methanesulphonamido-2-methoxyanilino)acridine-4-carboxamide dihydrochloride (125)**

4-Methanesulphonamido-2-methoxyaniline (Ferlin, M. G., Marzano, C., Chiarelotto, G., Baccichetti, F. and Bordin, F., Eur. J. Med. Chem., 2000, 35, 827-837) (97 mg, 0.45 mmol) and concentrated hydrochloric acid (40 μl) are added to the compound 9-chloro-*N*-(2-diethylaminoethyl)-2-iodoacridine-4-carboxamide (**124**) (0.22 g, 0.46 mmol) in solution in anhydrous ethanol (10 ml). The reaction medium is stirred at reflux for 17 hours. After returning to ambient temperature, a saturated aqueous sodium carbonate sodium (20 ml) is added. The aqueous phase is subsequently extracted with dichloromethane (3 x 50 ml). The organic phases are combined, dried over magnesium sulphate, filtered and then evaporated to dryness. The precipitate obtained is purified on a column of alumina eluted with a dichloromethane / ethanol, 99/1 (v/v), mixture. The red solid obtained is taken up in dichloromethane (5 ml) and then a solution of hydrochloric acid (2N) in anhydrous ether (5 ml). The mixture is stirred at ambient temperature for 5 minutes and then the solvents are driven off by evaporation under vacuum. The residue is taken up in anhydrous ether and stirred at ambient temperature under argon overnight. The hydrochloride obtained is filtered off and then washed with anhydrous ether, to result in the compound **125** (96 mg, 0.13 mmol). Yield: 28%; melting point: 235-237˚C; $^1$H NMR (400 MHz, $d_6$-DMSO) δ 1.29 (t, 6H, *J* = 7 Hz), 3.14 (s, 3H), 3.24 (m, 4H), 3.37 (m, 2H), 3.48 (s, 3H), 3.80 (m, 2H), 7.04 (d, 1H, *J* = 8.5 Hz), 7.09 (s, 1H), 7.46 (t, 1H, *J* = 7.5 Hz), 7.52 (d, 1H, *J* = 8 Hz), 8.00 (t, 1H, *J* = 7.5 Hz), 8.16 (d, 1H, *J* = 8.5 Hz), 8.20 (bs, 1H), 8.85 (s, 1H), 8.89 (bs, 1H), 9.80 (bs, 1H), 10.20 (s, 1H), 10.75 (bs, 1H), 11.75 (m, 1H), 13.83 (bs, 1H); $^{13}$C NMR (50 MHz, $d_6$-DMSO) δ 8.5 (2C), 34.7, 39.5, 46.6 (2C), 49.8, 56.0, 87.0, 104.1, 112.0, 113.7, 116.0, 120.4, 121.8, 123.8, 124.8, 125.2, 127.7, 136.2, 137.3, 137.6, 139.1, 140.4, 142.7, 153.6, 155.3, 166.3; IR (KBr) ν $cm^{-1}$: 1150, 1323, 1510, 1618, 2933, 3200-3500; MS (of the basic form) (m/z, %) 661 ($M^+$, 1), 582 (11), 509 (11), 86 (100), 58 (21). Anal. Calculated for $C_{28}H_{32}IN_5O_4S{\cdot}2HCl{\cdot}2.5H_2O$ : C, 43.14; H, 5.04; N, 8.98. Found : C, 43.06; H, 4.74; N, 8.90.

<u>**EXAMPLE 30**</u>

Synthesis of *N*-(2-diethylaminoethyl)-5-iodo-9-(4-methanesulphonamido-2-methoxyanilino)acridine-4-carboxamide dihydrochloride **(127)**

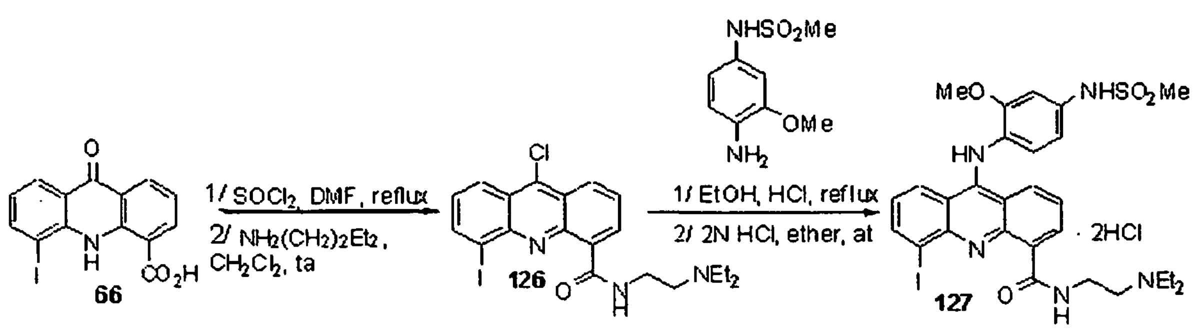

66 126 127

**Stage A: 9-chloro-*N*-(2-diethylaminoethyl)-5-iodoacridine-4-carboxamide (126)**

The compound **126** was prepared according to the procedure described for the preparation of the compound **124**, using 9,10-dihydro-5-iodo-9-oxoacridine-4-carboxylic acid (**66**) as starting material. Yield: 93%; very unstable compound; $^1$H NMR (200 MHz, $CDCl_3$) δ 1.38 (t, 6H, *J* = 7 Hz), 3.17 (q, 4H, *J* = 7 Hz), 3.37 (m, 2H), 4.22 (m, 2H), 7.41 (dd, 1H, *J* = 9 and 7 Hz), 7.78 (dd, 1H, *J* = 8.5 and 7 Hz), 8.44 (d, 1H, *J* = 8.5 Hz), 8.51 (d, 1H, *J* = 7 Hz), 8.60 (d, 1H, *J* = 8.5 Hz), 8.99 (d, 1H, *J* = 7 Hz), 12.40 (m, 1H).

**Stage B: *N*-(2-diethylaminoethyl)-5-iodo-9-(4-methanesulphonamido-2-methoxyanilino)acridine-4-carboaamide dihydrochloride (127)**

The compound **127** was prepared according to the procedure described for the preparation of the compound **125**, using 9-chloro-*N*-(2-diethylaminoethyl)-5-iodoacridine-4-carboxamide (**126**) as starting material. Yield: 38%; melting point: 212-214°C; $^1$H NMR (400 MHz, $d_6$-DMSO) δ 1.28 (t, 6H, *J* = 7 Hz), 3.13 (s, 3H), 3.25 (q, 4H, *J* = 7 Hz), 3.40 (m, 2H), 3.48 (s, 3H), 3.85 (m, 2H), 7.02 (m, 2H), 7.21 (t, 1H, *J* = 8 Hz), 7.55 (m, 2H), 8.39 (m, 1H), 8.54 (m, 2H), 8.89 (d, 1H, *J* = 7 Hz), 10.06 (s, 1H), 10.25 (bs, 1H), 10.81 (m, 1H), 12.00 (bs, 1H), 14.70 (bs, 1H); $^{13}$C NMR (100 MHz, $d_6$-DMSO) δ 8.3 (2C), 34.4, 39.5, 46.4 (2C), 49.5, 55.7, 89.5, 103.7, 111.6, 113.8, 114.2, 117.8, 122.7, 123.5, 125.2, 125.4, 127.5, 129.6, 135.9, 138.9, 139.3, 140.0, 146.0, 153.2, 156.9, 167.6; IR (KBr) ν $cm^{-1}$: 1151, 1325, 1513, 1585, 1613, 2900-3100, 3200-3500; MS (of the basic form) (m/z, %) 661 ($M^+$, 1), 582 (9), 509 (15), 446 (13), 383 (12), 86 (100). Anal. Calculated for $C_{28}H_{32}IN_5O_4S \cdot 2HCl \cdot 3H_2O$ : C, 42.65; H, 5.11; N, 8.88. Found : C, 42.72; H, 4.81; N, 8.68.

**EXAMPLE 31**

Syntheses of *N*-(2-diethylaminoethyl)-7-iodo-9-(4-methanesulphonamido-2-methoxyanilino)acridine-4-carboxamide dihydrochloride (**129**) and of *N*-(2-diethylaminoethyl)-7-(tributylstannyl)-9-(4-methanesulphonamido-2-methoxyanilino) acridine-4-carboxamide (**131**)

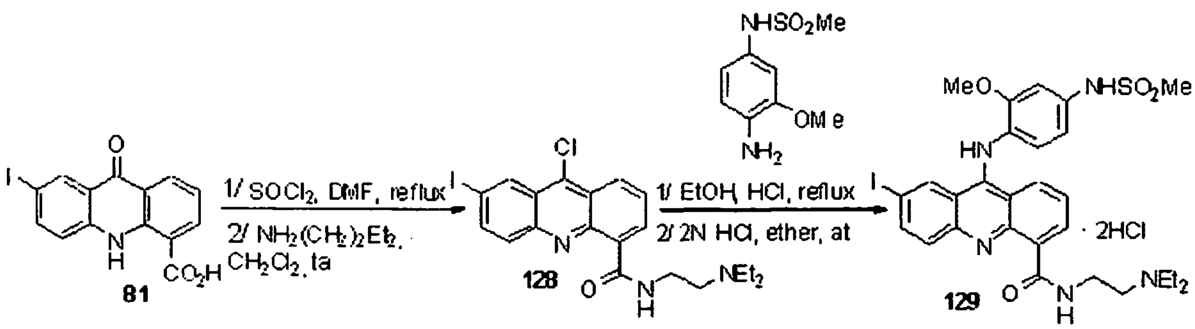


81 128 129

**Stage A: 9-chloro-*N*-(2-diethylaminoethyl)-7-iodoacridine-4-carboxamide (128)**

The compound **128** was prepared according to the procedure described for the preparation of the compound **124**, using 9,10-dihydro-7-iodo-9-oxoacridine-4-carboxylic acid (**81**) as starting material. Yield: 96%; very unstable compound; $^1$H NMR (200 MHz, $CDCl_3$) δ 1.12 (t, 6H, *J* = 7 Hz), 2.75 (m, 6H), 3.77 (q, 2H, *J* = 6 Hz), 7.72 (dd, 1H, *J* = 8.5 and 7 Hz), 8.00 (m, 2H), 8.49 (d, 1H, *J* = 8.5 Hz), 8.74 (s, 1H), 8.98 (d, 1H, *J* = 7 Hz), 11.60 (m, 1H).

**Stage B: *N*-(2-diethylaminoethyl)-7-iodo-9-(4-methanesulphonamido-2-methoayanilino)acridine-4-carboxarnide dihydrochloride (129)**

The compound **129** was prepared according to the procedure described for the preparation of the compound **125**, using 9-chloro-*N*-(2-diethylaminoethyl)-7-iodoacridine-4-carboxamide (**128**) as starting material. Yield: 33%; melting point: 208-210°C; $^1$H NMR (400 MHz, $d_6$-DMSO) δ 1.28 (t, 6H, *J* = 7 Hz), 3.13 (s, 3H), 3.24 (m, 4H), 3.37 (m, 2H), 3.49 (s, 3H), 3.80 (m, 2H), 7.06 (d, 1H, *J* = 8.5 Hz), 7.09 (s, 1H), 7.54 (m, 2H), 7.97 (d, 1H, *J* = 9 Hz), 8.19 (d, 1H, *J* = 9 Hz), 8.58 (m, 2H), 8.70 (d, 1H, *J* = 7.5 Hz), 9.95 (t, 1H, *J* = 6 Hz), 10.26 (s, 1H), 10.85 (m, 1H), 11.80 (m, 1H), 14.04 (bs, 1H); $^{13}$C NMR (100 MHz, $d_6$-DMSO) δ 8.3 (2C), 34.3, 39.5, 46.4 (2C), 49.5, 55.7, 89.2, 103.7, 111.7, 114.2, 114.8, 119.9, 121.9, 122.7, 123.4, 127.5, 129.1, 133.3, 135.7, 137.9, 138.2, 140.2, 143.2, 153.3, 155.0, 167.2; IR (KBr) ν $cm^{-1}$:

1150, 1324, 1511, 1586, 1618, 2926, 3300-3500; MS (of the basic form) (m/z, %) 661 ($M^+$, 7), 582 (47), 509 (62), 466 (29), 439 (21), 383 (17), 86 (100), 58 (13). Anal. Calculated for $C_{29}H_{32}IN_5O_4S{\cdot}2HCl{\cdot}2H_2O$: C, 43.65; H, 4.97; N, 9.09. Found: C, 43.37; H, 4.64; N, 8.93.

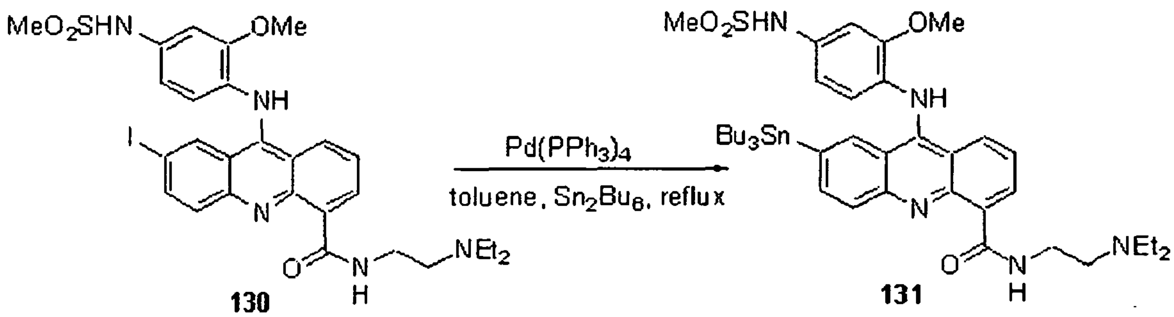


130 131

**Stage C: *N*-(2-diethylaminoethyl)-7-(tributylstannyl)-9-(4-methanesulphonamido-2-methoxyanilino)acridine-4-carboxamide (131)**

The compound **131** was prepared according to the procedure described for the preparation of the compound **32**, using *N*-(2-diethylaminoethyl)-7-iodo-9-(4-methanesulphonamido-2-methoxyanilino)acridine-4-carboxamide (**130**) (freshly prepared from the dihydrochloride **129** treated with a saturated aqueous sodium carbonate solution and then extracted with dichloromethane) as starting material and heating at reflux for 4.5 hours. The residue obtained is chromatographed on a column of alumina eluted with a mixture of ethyl acetate and methanol (96/4, v/v). Yield: 34%; Rf: 0.48 ($Al_2O_3$, ethyl acetate / ethanol (96/4, v/v)); melting point: 139-141°C; IR (KBr) v $cm^{-1}$ : 1152, 1457, 1507, 1592, 2924; ESI-MS m/z 826.5 $[M+H]^+$.

## EXAMPLE 32

Synthesis of *N*-(2-diethylaminoethyl)-9-(5-iodo-4-methanesulphonamido-2-methoxyanilino)acridine-4-carboxamide dihydrochloride (**137**)

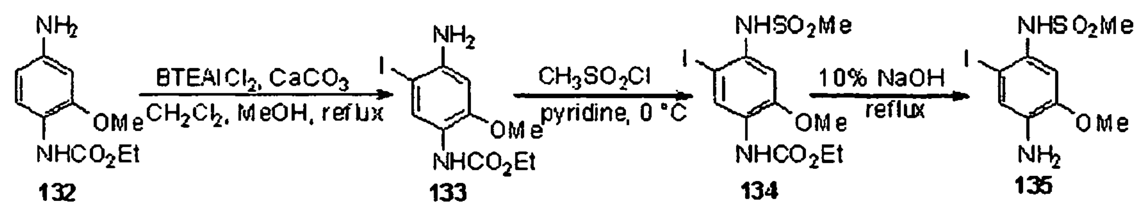


132 133 134 135

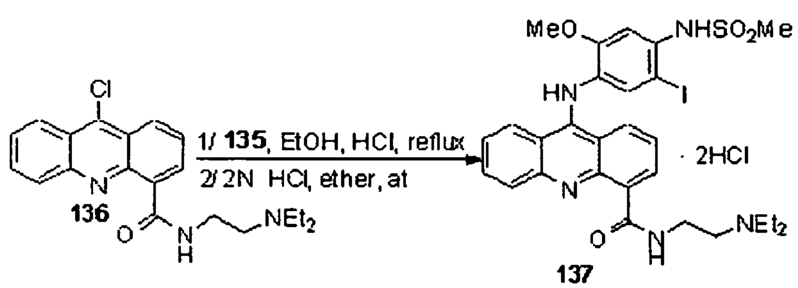


136 137

**Stage A: ethyl *N*-(4-amino-5-iodo-2-methoxyphenyl)carbamate (133)**

$BTEAlCl_2$ (Kajigaeshi, S., Kakinami, T., Yamasaki, H., Fujisaki, S. and Okamoto, T., Bull. Chem. Soc. Jpn., 1988, 61, 600-602) (0.81 g, 2.09 mmol) and calcium carbonate (0.32 mg, 3.08 mmol) are added to the aniline derivative **132** (Ferlin, M. G., Marzano, C., Chiarelotto, G., Baccichetti, F. and Bordin, F., Eur. J. Med. Chem., 2000, 35, 827-837) (0.40 g, 1.90 mmol) in solution in a mixture of methanol (15 ml) and dichloromethane (40 ml). The reaction medium is stirred at reflux for 45 minutes. After returning to ambient temperature, the mixture is filtered through Celite® 521. The filtrate is subsequently concentrated to 1/3 under vacuum and then the organic phase is washed with a 5% aqueous

sodium bisulphite solution (10 ml), a saturated aqueous sodium bicarbonate solution (10 ml), water (10 ml) and finally a saturated aqueous NaCl solution (10 ml). The organic phase is dried over magnesium sulphate, filtered and then evaporated to dryness. The residue obtained is chromatographed on a column of alumina eluted with an acetate / pentane (7/3, v/v) mixture, to result in the iodinated compound **133** (102 mg, 0.30 mmol). Yield: 16%; viscous liquid; Rf: 0.79 ($Al_2O_3$, acetate / pentane (7/3, v/v)); $^1H$ NMR (200 MHz, $CDCl_3$) δ 1.29 (t, 3H, *J* = 7 Hz), 3.78 (s, 3H), 4.01 (m, 2H), 4.19 (q, 2H, *J* = 7 Hz), 6.31 (s, 1H), 6.84 (bs, 1H), 8.25 (bs, 1H); $^{13}C$ NMR (100 MHz, $CDCl_3$) δ 14.7, 55.8, 61.2, 72.8, 97.8, 120.5, 128.4, 142.8, 149.8, 153.7; IR ($CCl_4$) ν $cm^{-1}$: 1221, 1529, 3200-3500; MS (m/z, %) 336 ($M^+$, 100), 290 (16), 264 (18), 249 (18), 108 (32), 94 (20), 52 (20).

**Stage B: ethyl *N*-(5-iodo-4-methanesulphonamido-2-methoxyphenyl)-carbamate (134)**

To the aminated derivative **133** (0.64 g, 1.90 mmol) in solution in anhydrous pyridine (5 ml) is added dropwise, at -15°C, under argon, methanesulphonyl chloride (0.16 ml, 2.09 mmol) (internal temperature < -5°C). The reaction medium is stored overnight in a refrigerator and then concentrated to 2/3 under vacuum. The mixture is subsequently taken up with water and then the remaining pyridine is neutralized by addition of concentrated hydrochloric acid. The aqueous phase is extracted with dichloromethane (3 × 30 ml). The organic phase is dried over sodium sulphate, filtered and then evaporated to dryness. The residue obtained is chromatographed on a column of alumina eluted with dichloromethane, to result in the compound **134** (0.43 g, 1.05 mmol). Yield: 55%; melting point: 153-155°C; Rf: 0.08 ($Al_2O_3$, dichloromethane); $^1H$ NMR (400 MHz, $CDCl_3$) δ 1.31 (t, 3H, *J* = 7 Hz), 2.94 (s, 3H), 3.87 (s, 3H), 4.21 (q, 2H, *J* = 7 Hz), 6.45 (s, 1H), 7.14 (s, 1H), 7.19 (s, 1H), 8.53 (bs, 1H); $^{13}C$ NMR (100 MHz, $CDCl_3$) δ 14.5, 39.8, 56.1, 61.6, 82.4, 106.8, 126.8, 127.4, 132.1, 148.6, 153.2; IR (KBr) ν $cm^{-1}$: 1150, 1337, 1526, 1708, 3195, 3353; MS (m/z, %) 414 ($M^+$, 40), 335 (100), 291 (13), 263 (40), 136 (12).

**Stage C: 5-iodo-4-methanesulphonamido-2-methoxyaniline (135)**

The ethyl carbamate **134** (0.60 g, 1.45 mmol) is brought to reflux for 1.5 hours in the presence of a 10% aqueous sodium hydroxide solution (10 ml). After returning to ambient temperature, the reaction medium is adjusted to pH = 8 by addition of concentrated hydrochloric acid. The aqueous phases are extracted with dichloromethane (4 × 50 ml). The organic phases are combined, dried, filtered and then evaporated to dryness, to result in the amine **135** (0.44 g, 1.29 mmol). Yield: 89%; melting point: 110-112°C; Rf: 0.08 ($Al_2O_3$, dichloromethane); $^1H$ NMR (400 MHz, $d_6$-DMSO) δ 2.96 (s, 3H), 3.75 (s, 3H), 5.07 (s, 2H), 6.76 (s, 1H), 7.08 (s, 1H), 8.94 (s, 1H); $^{13}C$ NMR (100 MHz, $d_6$-DMSO) δ 40.9, 55.5, 89.7, 111.7, 121.7, 126.0, 138.8, 146.3; IR (KBr) ν $cm^{-1}$ : 1221, 1529, 1723, 3356, 3430; MS (m/z, %) 342 ($M^+$, 19), 263 (100), 136 (18), 121 (12).

**Stage D: *N*-(2-diethylaminoethyl)-9-(5-iodo-4-methanesulphonamido-2-methoxyanilino)acridine-4-carboxamide dihydrochloride (137)**

The compound **137** was prepared according to the procedure described for the preparation of the compound **125**, using 9-chloro-*N*-(2-diethylaminoethyl)acridine-4-carboxamide (**136**) (Atwell, G. J., Cain, B. F., Baguley, B. C., Finlay, G. J. and Denny, W. A., J. Med. Chem., 1984, 27, 1481-1485) and 5-iodo-4-methanesulphonamido-2-methoxyaniline (**135**) as starting materials. Yield: 33%; melting point: 213-215°C; $^1H$ NMR (400 MHz, $d_6$-DMSO) δ 1.28 (t, 6H, *J* = 7 Hz), 3.16 (s, 3H), 3.24 (q, 4H, *J* = 7 Hz), 3.38 (m, 2H), 3.47 (s, 3H), 3.82 (m, 2H), 7.15 (s, 1H), 7.48 (m, 1H), 7.57 (m, 1H), 8.02 (m, 1H), 8.11 (s, 1H), 8.19 (d, 1H, *J* = 8 Hz), 8.28 (d, 1H, *J* = 8 Hz), 8.61 (d, 1H, *J* = 8Hz), 8.74 (d, 1H, *J* = 7Hz), 9.50 (bs, 1H), 9.95 (bs, 1H), 10.85 (bs, 1H), 11.80 (bs, 1H), 14.20 (bs, 1H); $^{13}C$ NMR (50 MHz, $d_6$-DMSO) δ 8.3 (2C), 34.3, 41.4 (2C), 46.4, 49.5, 56.0, 86.4, 111.1, 113.6, 114.5, 119.7, 120.2, 122.6, 124.5, 124.9, 128.0, 129.1, 133.6, 135.9 (2C), 138.3, 138.8, 139.2, 153.0, 156.0, 167.3; IR (KBr) ν $cm^{-1}$: 1151, 1320, 1522, 1572, 1623, 2927, 3300-3500; MS (of the basic form) (m/z, %) 661 ($M^+$, 2), 582 (24), 509 (25), 456 (74), 383 (73), 340 (40), 128 (27), 86 (100), 58 (19). Anal. Calculated for $C_{28}H_{32}IN_5O_4S{\cdot}2HCl{\cdot}2H_2O$: C, 43.65; H, 4.97; N, 9.09. Found : C, 43.51; H, 4.76; N, 9.03.

**EXAMPLE 33**

Synthesis of *N*-(2-diethylaminoethyl)-8-iodo-1,6-naphthyridine-2-carboxamide dihydrochloride (**142**)

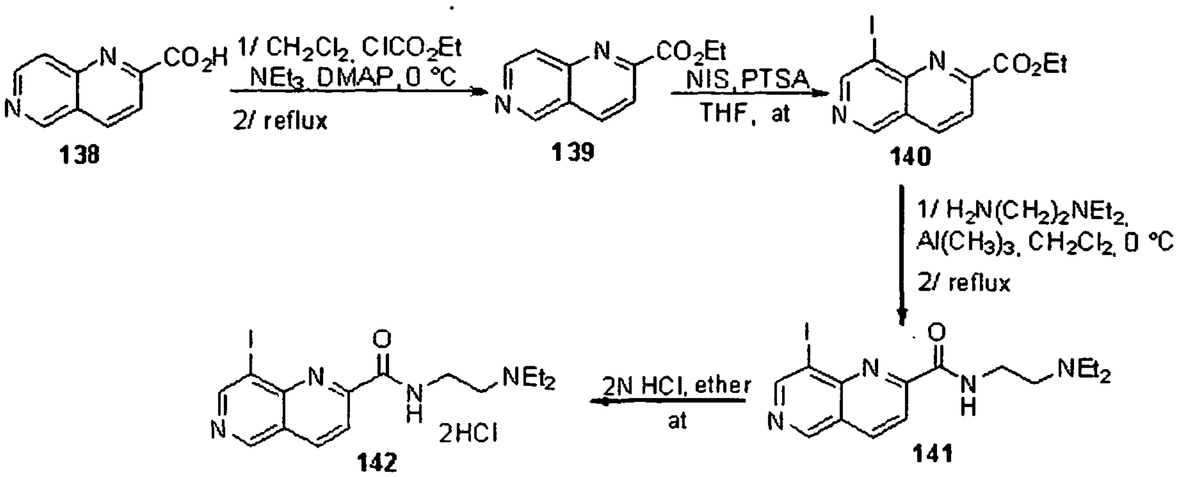


138 139 140 141 142

**Stage A: ethyl 1,6-naphthyridine-2-carboxylate (139)**

Triethylamine (3.03 ml, 21.8 mmol), ethyl chloroformate (3.73 ml, 39.0 mmol) and 4-dimethylaminopyridine (DMAP) (2.40 g, 19.6 mmol) are added, at 0˚C, to a solution of 1,6-naphthyridine-2-carboxylic acid (**138**) (Chan, L., Jin, H., Stefanac, T., Lavallée, J.F., Falardeau, G., Wang, W., Bédard, J., May, S. and Yuen, L., J. Med. Chem., 1999, 42, 3023-3025) (1.00 g, 5.75 mmol) in anhydrous dichloromethane (75 ml). After returning to ambient temperature, the reaction mixture is brought to reflux for 5 hours, the solution is evaporated to dryness and then the residue is purified on a column of alumina eluted with ethyl acetate to result in the ester **139** (517 mg, 2.56 mmol). Yield: 45%; Rf: 0.86 ($Al_2O_3$, ethyl acetate); melting point: 106-108˚C; $^1H$ NMR (400 MHz, $CDCl_3$) δ 1.41 (t, 3H, *J* = 7 Hz), 4.49 (q, 2H, *J* = 7 Hz), 8.02 (d, 1H, *J* = 6 Hz), 8.21 (d, 1H, *J* = 8.5 Hz), 8.39 (d, 1H, *J* = 8.5 Hz), 8.75 (d, 1H, *J* = 6 Hz), 9.29 (s, 1H); $^{13}C$ NMR (100 MHz, $CDCl_3$) δ 14.3, 62.0, 122.4, 122.7, 124.0, 137.2, 147.5, 149.7, 152.3, 152.9, 164.6; IR (KBr) ν $cm^{-1}$: 1256, 1734; MS (m/z, %) 202 ($M^+$, 3), 158 (18), 130 (100), 102 (15), 75 (22), 51 (16).

**Stage B: ethyl 8-iodo-1,6-naphthyridine-2-carboxylate (140)**

*N*-iodosuccinimide (NIS) (691 mg, 3.07 mmol) and para-toluenesulphonic acid (PTSA) (176 mg, 1.02 mmol) are added, at -10˚C, to a solution of the ester **139** (517 mg, 2.56 mmol) in tetrahydrofuran (50 ml). The reaction mixture is stirred at -10˚C for 10 minutes and then continued, after returning to ambient temperature, for 24 hours. *N*-iodosuccinimide (576 mg, 2.56 mmol) is then added to the solution. After stirring at ambient temperature for 21 hours, the solution is evaporated under vacuum and then the residue obtained is taken up in a saturated aqueous sodium carbonate solution (50 ml). The aqueous phase is extracted with ethyl acetate (3 × 20 ml). The organic phases are combined, dried over magnesium sulphate, filtered and then evaporated under vacuum. The residue is purified on a column of alumina eluted with an ethyl acetate/cyclohexane (7/3, v/v) mixture, to result in the iodinated ester **140** (527 mg, 1.61 mmol). Yield: 63%; Rf: 0.83 ($Al_2O_3$, ethyl acetate / cyclohexane (7/3, v/v)); melting point: 135-137˚C; $^1H$ NMR (200 MHz, $CDCl_3$) δ 1.51 (t, 3H, *J* = 7 Hz), 4.55 (q, 2H, *J* = 7 Hz), 8.32 (d, 1H, *J* = 8 Hz), 8.39 (d, 1H, *J* = 8 Hz), 9.23 (s, 1H), 9.28 (s, 1H); $^{13}C$ NMR (100 MHz, $CDCl_3$) δ 14.2, 62.7, 100.9, 123.4, 125.2, 137.9, 149.3, 152.8, 153.2, 155.2, 164.3; IR ($CCl_4$) ν $cm^{-1}$: 1126, 1274, 1742; MS (m/z, %) 328 ($M^+$, 18), 284 (18), 256 (100), 228 (8), 129 (29), 101 (52), 75 (25).

**Stage C: N-(2-diethylaminoethyl)-8-iodo-1,6-naphthyridine-2-carboxamide (141)**

The compound **141** was prepared according to the procedure described for the preparation of the compound **2**, using ethyl 8-iodo-1,6-naphthyridine-2-carboxylate (**140**) as starting material and heating the reaction medium at reflux for 3 hours. The residue is chromatographed on a column of alumina eluted with ethyl acetate. Yield: 73%; melting point: 44-46 ˚C; Rf: 0.67 ($Al_2O_3$, AcOEt); $^1H$ NMR (200 MHz, $CDCl_3$) δ 1.07 (t, 6H, *J* = 7 Hz), 2.60 (q, 4H, *J* = 7 Hz), 2.70 (t, 2H, *J* = 6 Hz), 3.57 (q, 2H, *J* = 6 Hz), 8.35 (d, I H, *J* = 8.5 Hz), 8.44 (d, 1H, *J* = 8.5 Hz), 8.83 (m, 1H), 9.17 (s, 1H), 9.19 (s, 1H); $^{13}C$ NMR (50 MHz, $CDCl_3$) δ 12.3 (2C), 37.4, 46.9 (2C), 51.4, 100.2, 121.5, 125.3, 138.1, 148.1, 152.8, 154.7, 154.8, 162.8; IR (KBr) ν $cm^{-1}$: 1460, 1516, 1689, 2801, 2926, 2965, 3300-3400; MS (m/z, %) 398 ($M^+$, 1), 86 (100), 58 (15).

**Stage D: *N*-(2-diethylaminoethyl)-8-iodo-1 ,6-naphthyridine-2-carboxamide dihydrochloride (142)**

The compound **142** was prepared according to the procedure described for the preparation of the compound **3**, using *N*-(2-diethylaminoethyl)-8-iodo-1,6-naphthyridine-2-carboxamide (**141**) as starting material. Yield: 72%; melting point: 215-217°C; $^1$H NMR (200 MHz, $d_6$-DMSO) δ 1.27 (t, 6H, *J* = 7 Hz), 3.25 (m, 6H), 3.82 (m, 2H), 8.34 (d, I H, *J* = 8.5 Hz), 8.81 (d, 1H, *J* = 8.5 Hz), 8.95 (m, 1H), 9.29 (s, 1H), 9.29 (s, I H), 10.75 (bs, 1H); IR (KBr) ν $cm^{-1}$: 1450, 1528, 1624, 1681, 2043, 2240-2750, 2939, 3045, 3250-3550. Anal. Calculated for $C_{15}H_{19}IN_4O{\cdot}2HCl{\cdot}1.5H_2O$: C, 36.16; H, 4.86; N, 11.25. **Found**: C, 36.50; H, 4.56; N, 11.03.

**EXAMPLE 34**

Synthesis of *N*-(4-dipropylaminobutyl)-6-iodoquinoxaline-2-carboxamide dihydrochloride (**144**)

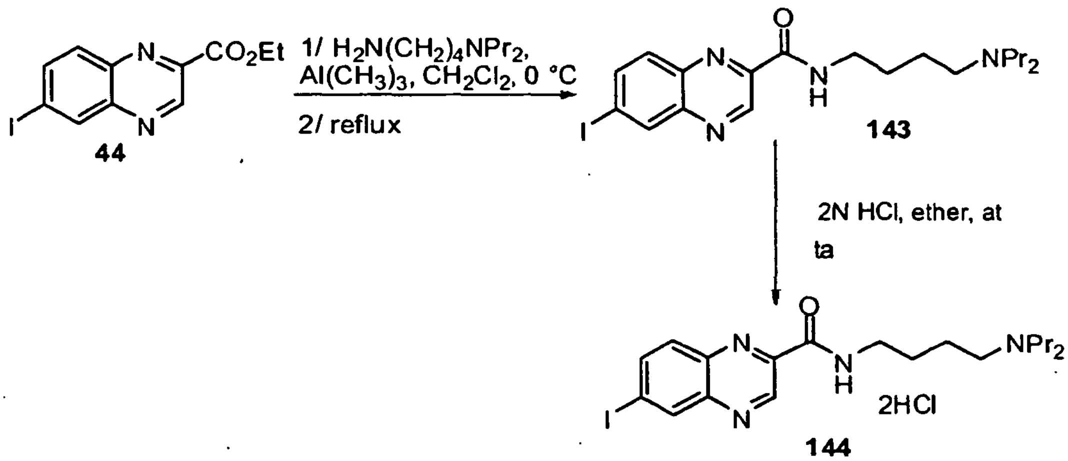


**Stage A: *N*-(4-dipropylaminobutyl)-6-iodoquinoxaline-2-carboxamide (143)**

The compound **143** was prepared according to the procedure described for the preparation of the compound **2**, using ethyl 6-iodoquinoxaline-2-carboxylate (**44**) and 4-amino-*N*,*N*-dipropylbutylamine (Seguin, H., Gardette, D., Moreau, M. F., Madelmont, J. C. and Gramain, J. C., Synth. Commun., 1998, 28, 4257-4272) as starting materials. The reaction medium is heated at reflux for 20 hours. Chromatography results, by order of elution, in the starting ethyl 6-iodoquinoxaline-2-carboxylate (**44**). Yield: 30%; and then in the carboxamide derivative **143**. Yield: 60%; melting point: 74-76°C; Rf: 0.83 ($Al_2O_3$, dichloromethane / ethanol (97/3, v/v)); $^1$H NMR ($CDCl_3$, 200 MHz) δ 0.80 (t, 6H, *J* = 7 Hz), 1.48 (m, 8H), 2.38 (m, 6H), 3.50 (q, 2H, *J* = 6.5 Hz), 7.72 (d, 1H, *J* = 9 Hz), 7.99 (dd, 1H, *J* = 9 and 2 Hz), 8.07 (m, 1H), 8.51 (d, 1H, *J* = 2 Hz), 9.57 (s, 1H); $^{13}$C NMR ($CDCl_3$, 50 MHz) δ 11.9 (2C), 19.8 (2C), 24.6, 27.5, 39.5, 53.6, 56.0 (2C), 98.0, 130.5, 138.5, 139.3, 139.6, 143.9, 144.2, 144.6, 162.9; 1R (KBr) ν $cm^{-1}$: 1539, 1685, 2802, 2869, 2955, 3240-3350; MS (m/z, %) 454 ($M^+$, 4), 425 (23), 354 (7), 255 (13), 128 (23), 114 (100), 72 (21), 70 (22).

**Stage B: *N*-(4-dipropylaminobutyl)-6-iodoquinoxaline-2-carboxamide dihydrochloride (144)**

The compound **144** was prepared according to the procedure described for the preparation of the compound **3**, using *N*-(4-dipropylaminobutyl)-6-iodoquinoxaline-2-carboxamide (**143**) as starting material. Yield: 81%; melting point: 175-177°C; $^1$H NMR (200 MHz, $d_6$-DMSO) δ 0.88 (t, 6H, *J* = 7 Hz), 1.67 (m, 8H), 2.95 (m, 6H), 3.39 (m, 2H), 7.93 (d, 1H, *J* = 9 Hz), 8.24 (dd, 1H, *J* = 9 and 2 Hz), 8.63 (d, 1H, *J* = 2 Hz), 9.19 (t, 1H, *J* = 5.5 Hz), 9.45 (s, 1H), 10.13 (bs, 1H); IR (KBr) ν $cm^{-1}$: 1475, 1541, 1683, 2600-2750, 2800-3000, 3273. Anal. Calculated for $C_{19}H_{27}IN_4O{\cdot}2HCl$: C, 43.28; H, 5.54; N, 10.63. Found: C, 44.33; H, 5.59; N, 10.75.

**EXAMPLE 35**

Synthesis of *N*-(2-diethylaminoethyl)-7-iodophenazine-1-carboxamide dihydrochloride (**151**)

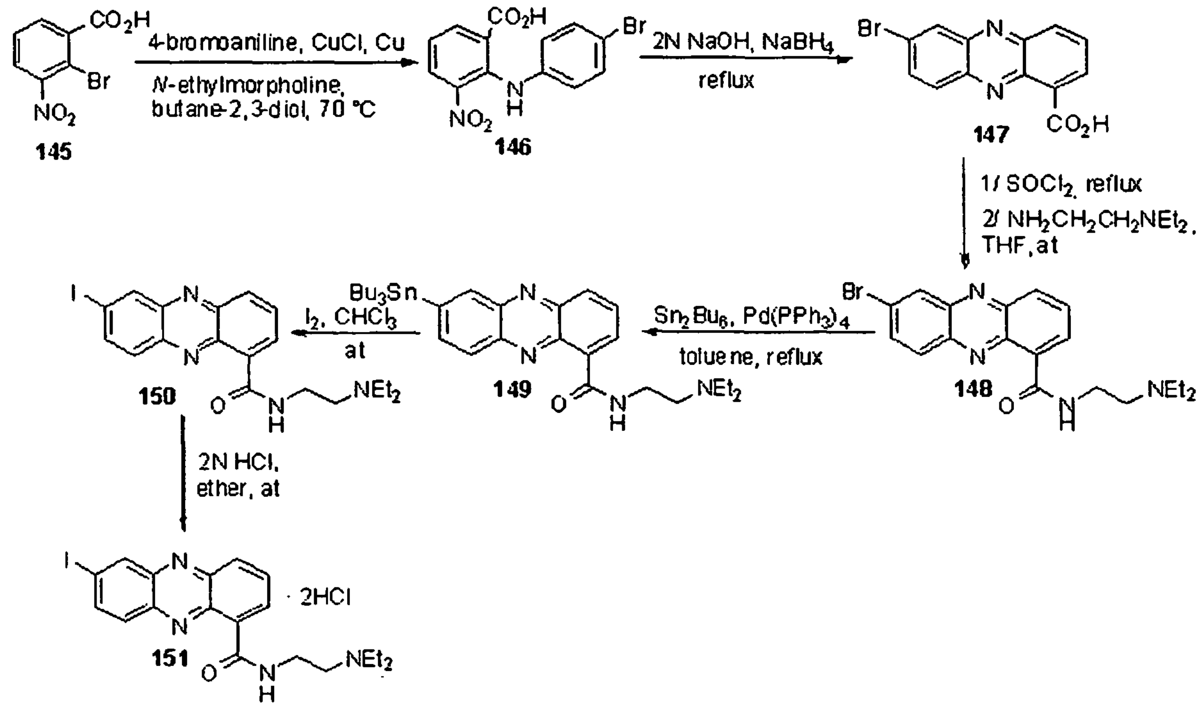


## Stage A: *N*-(4-bromophenyl)-3-nitroanthranilic acid (146)

Butane-2,3-diol (10 ml), 2-bromo-3-nitrobenzoic acid (**145**) (Culhane, P.J., Organic Syntheses, Wiley, New York, 1944, Collect. Vol I, 125) (4.10 g, 16.7 mmol), cuprous chloride (206 mg) and copper powder (410 mg) are successively added to a solution of 4-bromoaniline (4.30 g, 24.5 mmol) in *N*-ethylmorpholine (6 ml, 46.9 mmol). The solution is heated at 70°C for 7.5 hours. Heating is halted and a 0.5M aqueous ammonia solution (140 ml) is added with stirring. The medium is then rapidly filtered through Celite® 545. The Celite is washed with 0.5M aqueous ammonia solution (20 ml). The filtrate is poured, with vigorous stirring, onto a 2N aqueous hydrochloric acid solution (140 ml). After stirring for 10 minutes, the solution loses its colour and a pale yellow precipitate is formed. The solution is placed in a refrigerator overnight and the precipitate formed is filtered off and then dried in a desiccator, to result in the diphenylamine **146** (1.76 g, 5.22 mmol) Yield: 31%; melting point: 194-196°C; $^{+}$H NMR (400 MHz, $d_6$-DMSO) δ 6.86 (d, 2H, $J$ = 9 Hz), 7.16 (t, 1H, $J$ = 8 Hz), 7.38 (d, 2H, $J$ = 9 Hz), 8.09 (d, 1H, $J$ = 8 Hz), 8.18 (d, 1H, $J$ = 8 Hz), 9.69 (bs, 1H); $^{13}$C NMR (100 MHz, $CDCl_3$) δ 114.1, 119.8 (2C), 120.0, 122.0, 130.4, 131.8 (2C), 136.5, 137.8, 140.5, 141.4, 168.1; IR (KBr) ν $cm^{-1}$: 1259, 1437, 1526, 1668, 3328; ESI-MS m/z 334.7 $[M-H]^+$.

## Stage B:7-bromophenazine-1-carboxylic acid (147)

Sodium borohydride (505 mg, 13.3 mmol) is added to a solution of *N*-(4-bromophenyl)-3-nitroanthranilic acid (**146**) (1.50 g, 4.45 mmol) in 2N sodium hydroxide solution (75 ml). The reaction medium is heated at reflux for 2 hours. After returning to ambient temperature, the solution is cooled by means of an ice bath and then filtered. The precipitate is washed with precooled 2N sodium hydroxide solution (10 ml). The precipitate is taken up in water (100 ml) and the solution is acidified with stirring by addition of glacial acetic acid until precipitation has occurred (pH = 5). The precipitate obtained is filtered off and then dried in a desiccator, to result in the acid **147** (807 mg, 2.66 mmol). Yield: 60%; melting point: 268-269°C; $^{1}$H NMR (200 MHz, $d_6$-DMSO) δ; 8.12 (m, 2H), 8.34 (d, 1H, $J$ = 9 Hz), 8.45 (m, 2H), 8.60 (d, 1H, $J$ = 2H); IR (KBr) ν $cm^{-1}$: 1396, 1739, 2600-2800; ESI-MS m/z 300.8 $[M+H]^+$.

## Stage C: *N*-(2-diethylaminoethyl)-7-bromophenazine-1-carboxamide (148)

A solution of the acid **147** (885 mg, 2.92 mmol) in thionyl chloride (20 ml) under argon is heated at reflux for 30 minutes. The solution is evaporated under vacuum, then the residue is taken up in anhydrous toluene (5 ml) and again evaporated under vacuum. The acid chloride thus obtained is dissolved in anhydrous dichloromethane (30 ml). A solution of *N*,*N*-diethylethylenediamine (2.05 ml, 14.6 mmol) in anhydrous dichloromethane (15 ml) is added, under argon and at 0°C, to the solution. After returning to ambient temperature, the reaction medium is stirred for 22 hours. The solution is evaporated under vacuum and then the residue is chromatographed on a column of alumina eluted with

ethyl acetate, to result in the compound **148** (1.13 g, 2.82 mmol). Yield: 96%; melting point: 107-109°C; Rf: 0.57 ($Al_2O_3$, ethyl acetate); $^1H$ NMR (200 MHz, $CDCl_3$) δ 1.27 (t, 6H, *J* = 7 Hz), 2.96 (q, 4H, *J* = 7 Hz), 3.06 (m, 2H), 4.00 (m, 2H), 7.97 (m, 2H), 8.43 (m, 3H), 8.98 (d, 1H, *J* = 7 Hz), 11.18 (bs, 1H); $^{13}C$ NMR (50 MHz, $CDCl_3$) δ 11.3 (2C), 37.7, 47.1 (2C), 51.7, 125.6, 129.4, 130.5, 130.7, 131.5, 133.6, 135.2, 135.6, 140.1, 140.8, 143.1, 143.6, 164.7; IR (KBr) ν $cm^{-1}$: 1515, 1653, 2874, 2936, 2966, 3260; ESI-MS m/z 400.7 $[M+H]^+$.

**Stage D: *N*-(2-diethylaminoethyl)-7-(tributylstannyl)phenazine-1-carboxamide (149)**

The compound **149** was prepared according to the procedure described for the preparation of the compound **32**, using *N*-(2-diethylaminoethyl)-7-bromophenazine-1-carboxamide (**148**) as starting material. The residue obtained is chromatographed on a column of alumina eluted with an ethyl acetate / cyclohexane (7/3, v/v) mixture; yield: 61%; viscous liquid; Rf: 0.84 ($Al_2O_3$, ethyl acetate / cyclohexane (7/3, v/v)); $^1H$ NMR (400 MHz, $CDCl_3$) δ 0.89 (t, 9H, *J* = 7.5 Hz), 1.17 (t, 6H, *J* = 7 Hz), 1.21 (m, 6H), 1.35 (st, 6H, *J* = 7.5 Hz), 1.59 (m, 6H), 2.81 (q, 4H, *J* = 7 Hz), 2.91 (t, 2H, *J* = 6 Hz), 3.85 (q, 2H, *J* = 6 Hz), 7.91 (dd, 1H, *J* = 7 and 8.5 Hz), 7.98 (d, 1H, *J* = 8.5 Hz), 8.25 (d, 1H, *J* = 8.5 Hz), 8.36 (d, I H, *J* = 8.5 Hz), 8.39 (s, 1 H), 8.95 (d, 1 H, *J* = 7 Hz), 11.29 (bs, 1 H); $^{13}C$ NMR (100 MHz, $CDCl_3$) δ 10.0 (3C, $^1J_{Sn\text{-}C}$= 335 Hz), 11.4 (2C), 13.7 (3C), 27.4 (3C, $^3J_{Sn\text{-}C}$= 56 Hz), 29.1 (3C, $^2J_{Sn\text{-}C}$= 20 Hz), 37.7, 47.1 (2C), 51.7, 127.5, 129.2, 129.8, 133.7, 135.0, 138.2, 138.4, 140.9, 141.7, 142.2, 143.3, 148.7, 165.1; IR ($CCl_4$) ν $cm^{-1}$: 1465, 1508, 1660, 2854, 2873 2928, 2960; ESI-MS m/z 613.1 $[M+H]^+$.

**Stage E: *N*-(2-diethylaminoethyl)-7-iodophenazine-1-carboxamide (150)**

A solution of diiodine in chloroform (0.95 g, 3.74 mmol, 70 ml) is added dropwise over a period of 5 hours to a solution of the compound **149** (1.14 g, 1.87 mmol) in chloroform (30 ml). The reaction medium is stirred at ambient temperature for 18 hours. Diiodine (0.27 g, 1.06 mmol) is added and stirring is continued for 7 hours. A 5% aqueous sodium carbonate solution (100 ml) is added to the reaction medium. After separating by settling, the organic phase is washed with a 5% aqueous sodium bisulphite solution (2 × 40 ml), dried over magnesium sulphate, filtered and evaporated under vacuum. The residue obtained is chromatographed on a column of alumina eluted with an ethyl acetate / cyclohexane (7/3, v/v) mixture, to result in the iodonated derivative **150** (397 mg, 0.89 mmol); yield: 47%; melting point: 140-142°C; Rf: 0.56 ($Al_2O_3$, ethyl acetate / cyclohexane (7/3, v/v)); $^1H$ NMR (200 MHz, $CDCl_3$) δ 1.12 (t, 6H, *J* = 7 Hz), 2.76 (q, 4H, *J* = 7 Hz), 2.85 (m, 2H), 3.79 (q, 2H, *J* = 6 Hz), 7.93 (dd, 1H, *J* = 7 and 8.5 Hz), 8.07 (m, 2H), 8.30 (d, 1H, *J* = 8.5 Hz), 8.68 (s, 1H), 8.97 (d, 1H, *J* = 7 Hz), 11.0 (m, 1 H); $^{13}C$ NMR (50 MHz, $CDCl_3$) δ 11.6 (2C), 37.8, 47.1 (2C), 51.7, 97.8, 129.5, 130.2, 130.7, 133.6, 135.7, 138.7, 140.1, 140.4, 141.0, 143.2, 143.5, 164.6; IR (KBr) ν $cm^{-1}$: 1510, 1646, 2872, 2936, 2963, 3243; ESI-MS m/z 448.9 $[M+H]^+$.

**Stage F: *N*-(2-diethylaminoethyl)-7-iodophenazine-1-carboxamide dihydrochloride (151)**

The compound **151** was prepared according to the procedure described for the preparation of the compound **3**, using *N*-(2-diethylaminoethyl)-7-iodophenazine-1-carboxamide (**150**) as starting material. Yield: 84%; melting point: 212-214°C; $^1H$ NMR (200 MHz, $d_6$-DMSO) δ 1.26 (t, 6H, *J* = 7 Hz), 3.24 (m, 4H), 3.39 (m, 2H), 3.91 (m, 2H), 8.10 (dd, 1H, *J* = 7 and 8.5 Hz), 8.37 (m, 3H), 8.69 (d, 1 H, *J* = 7 Hz), 8.78 (s, 1 H), 10.26 (bs, 1H), 10.46 (m, 1H); IR (KBr) ν $cm^{-1}$: 1508, 1654, 2361, 2588, 2948, 3255. Anal. Calculated for $C_{19}H_{21}INO\cdot 2HCl$: C, 43.78; H, 4.45; N, 10.75. Found: C, 44.85; H, 4.42; N, 10.76.

The chemical structures and physical data of some precursor (unlabelled) iodinated compounds of formulae (1), (1'), (I"), (la), (lb), (Ic), (Id), (Ie), (If), (Ig), (Ih), (Ii), (Ij), (Ik), (Im), (In), (Io), (Ip), (Iq) and (Ir) of the invention before their labelling described below and of some compounds of formula (II) according to the invention are illustrated in the following Table I.

**Table I**

<table>
<tr><td colspan="7">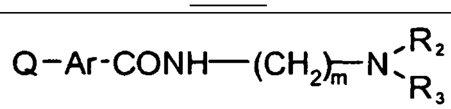</td></tr>
<tr><th>No.</th><th>Q-Ar-</th><th>m</th><th>$R_2$</th><th>$R_3$</th><th>Salt</th><th>M.p. (°C)</th></tr>
<tr><td>2</td><td rowspan="2">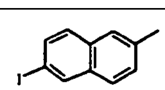</td><td rowspan="2">2</td><td rowspan="2">Et</td><td rowspan="2">Et</td><td>-</td><td>105-107</td></tr>
<tr><td>3</td><td>HCl</td><td>151-152</td></tr>
</table>

(continued)

| No. | Q-Ar- | m | $R_2$ | $R_3$ | Salt | M.p. (˚C) |
|---|---|---|---|---|---|---|
| 6 | 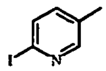 | 2 | Et | Et | - | Liq |
| 7 | | | | | 2HCl | 127-129 |
| 9 | 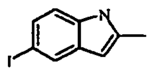 | 2 | Et | Et | - | 208-210 |
| 10 | | | | | HCl | 217-219 |
| 12 | 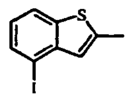 | 2 | Et | Et | - | 76-78 |
| 13 | | | | | HCl | 163-165 |
| 15 | 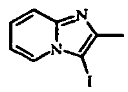 | 2 | Et | Et | - | Liq |
| 16 | | | | | 2HCl | 128-130 |
| 20 | 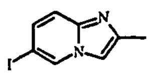 | 2 | Et | Et | - | Liq |
| 21 | | | | | 2HCl | 208-210 |
| 25 | 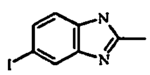 | 2 | Et | Et | - | 136-138 |
| 26 | | | | | 2HCl | 217-219 |
| 30 | 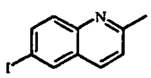 | 2 | Et | Et | - | 75-77 |
| 31 | | | | | 2HCl | 104-106 |
| 34 | 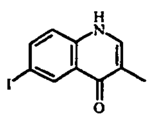 | 2 | Et | Et | - | 234-236 |
| 35 | | | | | 2HCl | 164-166 |
| 38 | 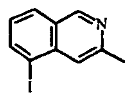 | 2 | Et | Et | - | Liq |
| 39 | | | | | 2HCl | 152-154 |
| 45 | 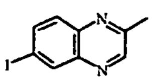 | 2 | Et | Et | - | 60-62 |
| 46 | | | | | 2HCl | 201-203 |
| 48 | 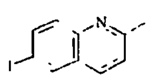 | 4 | Pr | Pr | - | Liq |
| 49 | | | | | 2HCl | 133-135 |
| 54 | 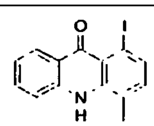 | 2 | Et | Et | - | 248-250 |
| 55 | | | | | 2HCl | 269-271 |
| 59 | 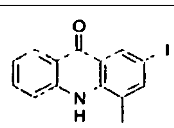 | 2 | Et | Et | - | 250-252 |
| 60 | | | | | 2HCl | 298-300 |
| 64 | 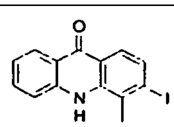 | 2 | Et | Et | - | 215-217 |
| 65 | | | | | 2HCl | 262-264 |
| 68 | 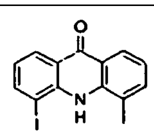 | 2 | Et | Et | - | 148-150 |
| 69 | | | | | 2HCl | 251-253 |
| 77 | 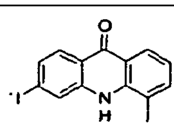 | 2 | Et | Et | - | 208-210 |
| 78 | | | | | 2HCl | 266-268 |

(continued)

| No. | Q-Ar- | m | $R_2$ | $R_3$ | Salt | M.p. (˚C) |
|---|---|---|---|---|---|---|
| 83 | 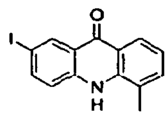 | 2 | Et | Et | - | 140-142 |
| 84 | | | | | 2HCl | 220-222 |
| 86 | 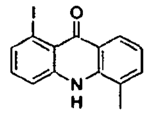 | 2 | Et | Et | - | 144-146 |
| 87 | | | | | 2HCl | 228-230 |
| 90 | 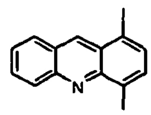 | 2 | Et | Et | - | 103-105 |
| 91 | | | | | 2HCl | 219-221 |
| 94 | 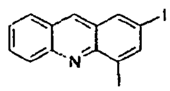 | 2 | Et | Et | - | 108-110 |
| 95 | | | | | 2HCl | 215-217 |
| 97 | 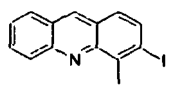 | 2 | Et | Et | - | 81-83 |
| 98 | | | | | 2HCl | 134-136 |
| 101 | 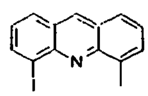 | 2 | Et | Et | - | 64-66 |
| 102 | | | | | 2HCl | 144-146 |
| 106 | 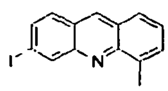 | 2 | Et | Et | - | 80-82 |
| 107 | | | | | 2HCl | 201-203 |
| 110 | 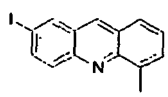 | 2 | Et | Et | - | 176-178 |
| 111 | | | | | 2HCl | 213-215 |
| 114 | 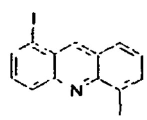 | 2 | Et | Et | - | 78-80 |
| 115 | | | | | 2HCl | 128-130 |
| 119 | 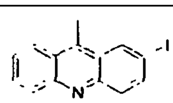 | 2 | Et | Et | - | 83-85 |
| 120 | | | | | 2HCl | 121-123 |
| 121 | 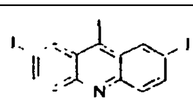 | 2 | Et | Et | - | 209-211 |
| 122 | | | | | 2HCl | 224-226 |
| 125 | 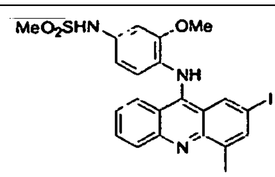 | 2 | Et | Et | 2HCl | 235-237 |
| 128 | 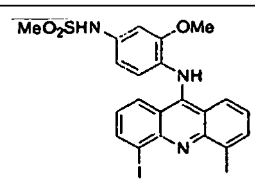 | 2 | Et | Et | 2HCl | 212-214 |

(continued)

| No. | Q-Ar- | m | $R_2$ | $R_3$ | Salt | M.p. (°C) |
|---|---|---|---|---|---|---|
| **129** | 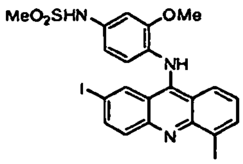 | 2 | Et | Et | 2HCl | 208-210 |
| **137** | 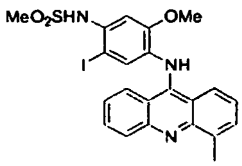 | 2 | Et | Et | 2HCl | 213-215 |
| **141** | 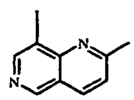 | 2 | Et | Et | - | Liq |
| **142** | | | | | 2HCl | 215-217 |
| **143** | 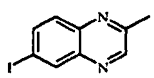 | 4 | Pr | Pr | - | 74-76 |
| **144** | | | | | 2HCl | 175-177 |
| **150** | 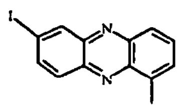 | 2 | Et | Et | -- | 140-142 |
| **151** | | | | | 2HCl | 212-214 |

- Et denotes an ethyl radical,
- Pr denotes a propyl radical,
- "Liq" means that the product exists in the form of a liquid,
- M.p. denotes the melting point.

The structures of compounds of formula (VII) which are precursors of the compounds of formulae (I), (I'), (I"), (Ia), (Ib), (Ic), (Id), (Ie), (Ig), (Ih), (Ii), (Ik), (Im), (In) and (II) are illustrated in the following Table II.

**Table II**

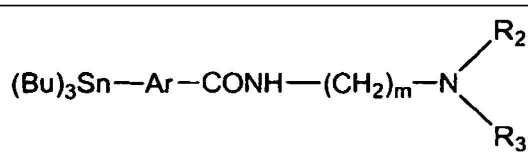 (VII)

| No. | $(Bu)_3Sn$-Ar- | m | $R_2$ | $R_3$ | M.p. (°C) |
|---|---|---|---|---|---|
| **17** | 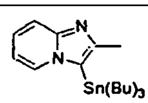 | 2 | Et | Et | Liq |
| **32** | 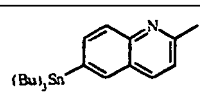 | 2 | Et | Et | Liq |
| **47** | 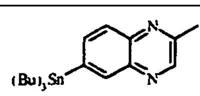 | 2 | Et | Et | Liq |
| **85** | 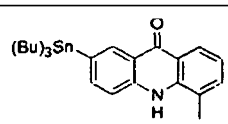 | 2 | Et | Et | Liq |

(continued)

| No. | $(Bu)_3Sn$-Ar- | m | $R_2$ | $R_3$ | M.p. (°C) |
|---|---|---|---|---|---|
| **103** | 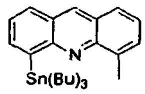 | 2 | Et | Et | Liq |
| **131** | 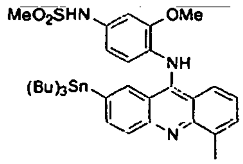 | 2 | Et | Et | Liq |
| **152** | 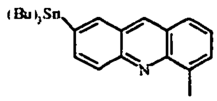 | 2 | Et | Et | Liq |
| - Bu denotes a butyl radical | | | | | |

**EXAMPLE 36 : Labelling**

33.1. First alternative for the preparation of the labelled compounds of formula (II)

MATERIALS, EQUIPMENT AND METHODS

The $Na^{125}I$ and the $Na^{131}I$ are supplied by Amersham. The Extrelut® and the sodium citrate/hydrochloric acid pH = 4 buffer originate from Merck. The radioactivity measurements were carried out, in the case of direct exchange, on an AMBIS 400 (Scanalytics, CSPI, San Diego, CA, USA). In the case of labellings of high specific activity, HPLC purifications were carried out on a Shimadzu device (LC 6A pump, SCL 6B controller, $CR_5A$ integrator) equipped with a Zorbax 80Å extend $C_{18}$ column (4.6 × 150 mm) connected in series with a UV spectrophotometer of Shimadzu SPD 6AV type and a Raytest Steffi gamma detector. The column is eluted with a water/methanol/0.2% aqueous ammonia mixture with a flow rate of 1 ml / minute and a linear elution gradient for methanol from 70 to 100% over a period of 10 minutes. The radiochemical purities were determined after HPLC analysis (HP1100, Hewlett Packard, Les Ulis, France). The Purospher $RP_{18}$ e column (5 μm) is coupled to a diode array detector and a Flow one $A_{500}$ Radiomatic radioactivity detector (Packard, Canberra, Australia). Chromatography is carried out using a water/methanol/0.2% aqueous ammonia mixture with a flow rate of 0.5 ml/minute and a linear elution gradient for methanol from 70 to 100% over a period of 10 minutes. The labelled products were identified by comparing their TLC (Rf) or HPLC (Rt) values with those obtained for the corresponding unlabelled compounds.

**General procedure for the preparation of the products labelled with $^{125}I$ iodine by direct exchange**

The hydrochloride (2-3 mg) is dissolved in 500 μl of a citrate/hydrochloric acid (pH = 4) buffer solution comprising 100 μl of an aqueous copper sulphate solution (5 mg/ml). An aqueous solution of sodium iodide $Na^{125}I$ exhibiting an activity of between 111 and 148 MBq is subsequently added to this mixture. The reaction mixture is heated at 120-150°C for one hour. The crude reaction product is taken up in 500 μl of deionized water and the reaction is monitored by thin layer chromatography (alumina, dichloromethane/ethanol (97/3, v/v)). A plate analyzer then makes it possible to count the radioactivity and to determine the percentage incorporation of $^{125}I$ in the molecule. After changing to basic pH (>10) using a IN sodium hydroxide solution (100 μl), the mixture is purified on an Extrelut® cartridge (elution with 5 times 3 ml of dichloromethane). After evaporation, the residue is taken up in anhydrous dichloromethane (2 ml) and then in a 2N solution of hydrochloric acid in ether (5 ml), and then evaporated. The hydrochloride obtained is taken up in anhydrous ether (5 ml) and evaporated under vacuum. The radiolabelled compound is thus obtained with a yield and a radiochemical purity given in the following Table III:

**Table III**

| Starting hydrochloride | Heating temperature (Time) | Compound of formula (II) labelled with iodine-125 | Yield (%) | Purity[a] (%) |
|---|---|---|---|---|
| 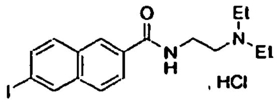 compound **3** | 150 °C (1 hour) | 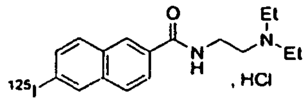 | 77 | 97 |
| 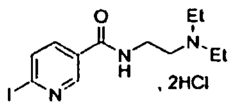 compound **7** | 120 °C (1 hour) | 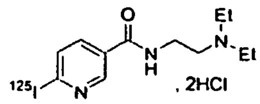 | 39 | 98 |
| 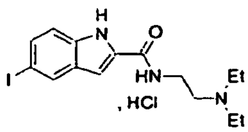 compound **10** | 150 °C (1 hour) | 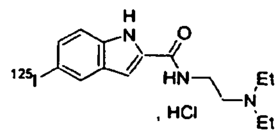 | 75 | 98 |
| 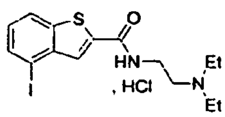 compound **13** | 150 °C (1 hour) | 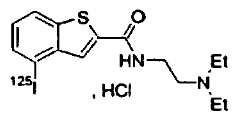 | 73 | 99 |
| 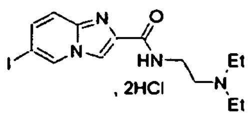 compound **21** | 150 °C (1 hour) | 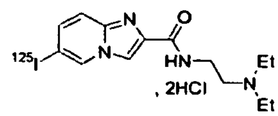 | 68 | 98 |
| 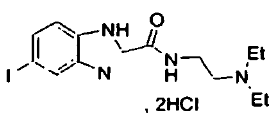 compound **26** | 150 °C (1 hour) | 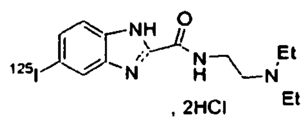 | 22 | 95 |
| 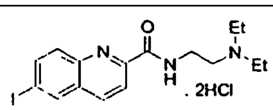 compound **31** | 150°C (1 hour) | 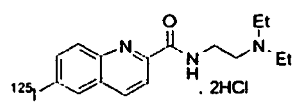 | 92 | 99 |
| 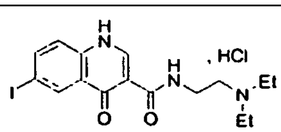 compound **35** | 150 °C (1 hour) | 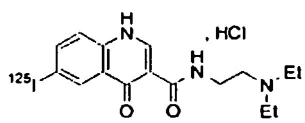 | 54 | 99[b] |
| 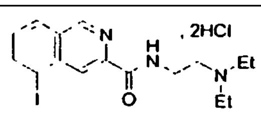 compound **39** | 150 °C (1 hour) | 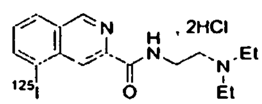 | 43 | 96 |
| 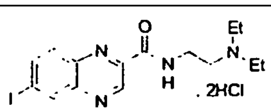 compound **46** | 150°C (1 hour) | 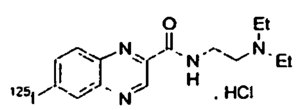 | 68 | 99 |

(continued)

| Starting hydrochloride | Heating temperature (Time) | Compound of formula (II) labelled with iodine-125 | Yield (%) | Purity[a] (%) |
|---|---|---|---|---|
| 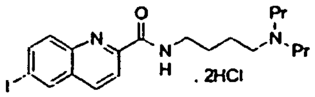 compound **49** | 150 °C (hour) | 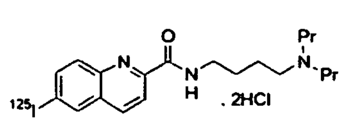 | 69 | 99 |
| 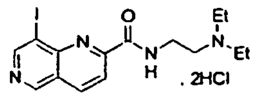 compound **142** | 130 °C (30 minutes) | 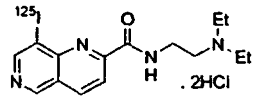 | 69 | 98[c] |
| 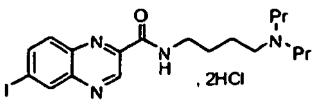 compound **144** | 140 °C (18 minutes) | 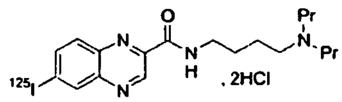 | 30[d] | 99 |
| 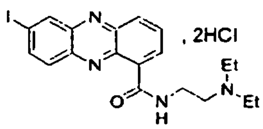 compound **151** | 130 °C (30 minutes) | 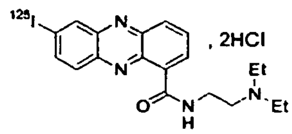 | 58 | 99[c] |

[a]Radiochemical purity after Extrelut®
[b]TLC, $Al_2O_3$, dichloromethane / ethanol (9/1, v/v)
[c]TLC, $Al_2O_3$, ethyl acetate
[d]purification by Extrelut® column and then by HPLC : retention time : 10.6 minutes (compound **144**)

33.2. Second alternative for the preparation of the labelled compounds of formula (II)

According to the second alternative, the compounds of formula (VII) are used as intermediates.

**Preparation of [$^{125}$I]-*N*-(2-diethylaminoethyl)-3-iodoimidazo[1,2-*a*]pyridine-2-carboxamide dihydrochloride [$^{125}$I]-(16) (labelling of high specific activity)**

An acetic acid/ethanol (1/1, v/v, 20 µl) mixture, Na$^{125}$I (5 µl, 18.9 MBq) and peracetic acid (20 µl) are added to a solution of the trialkylstannane **17** (0.43 mg) in ethanol (100 µl) in a vial. The vial is scaled and stirred, at ambient temperature, with a vortex for 30 minutes. A solution of sodium metabisulphite (20 mg) in water (100 µl) and then a 5N sodium hydroxide solution (100 µl) are added and the mixture is stirred with a vortcx for a few minutes. The solution is deposited on an Extrelut® column and the vial is rinsed with a water/ethanol (1/1, v/v, 2 × 100 µl) mixture. After ten minutes of contact, the column is eluted with dichloromethane (5 × 2 ml). The organic phase is evaporated under vacuum and the residue is separated on an HPLC column (elution rate: 1 ml /minute). The retention times obtained are : 7.22 minutes (compound **15** labelled with $^{125}$I) and 16.7 minutes (compound **17**). The various fractions collected are evaporated under vacuum. The residue is taken up in anhydrous dichloromethane (1 ml) and then in a 2N solution of hydrochloric acid in ether (2 ml). The solution is evaporated under vacuum. The residue is taken up in anhydrous ether (2 ml) and evaporated under vacuum, to result in [$^{125}$I]-*N*-(2-diethylaminoethyl)-3-iodoimidazo[1,2-*a*]pyridine-2-carboxamide dihydrochloride ([$^{125}$I] **16**) (7.96 MBq). Radiochemical yield: 57%; radiochemical purity: 99%.

**Preparation of [$^{131}$I]-*N*-(2-diethylaminoethyl)-6-iodoquinoline-2-carboxamide dihydrochloride [$^{131}$I]-(31) (labelling of high specific activity)**

A 0.5N hydrochloric acid solution (100 µl), Na$^{131}$I (100 µl, 1.30 GBq) and an aqueous solution of chloramine-T monohydrate (1 mg/ml, 100 µl) are successively added to a solution of the trialkylstannane **32** (0.45 mg) in ethanol (100 µl) in a vial. The vial is sealed and stirred, at ambient temperature, with a vortex for 30 minutes. A solution of sodium metabisulphite (20 mg) in water (100 µl) and then a 3N sodium hydroxide solution (150 µl) are added and the mixture is stirred with a vortex for a few minutes. The solution is deposited on an Extrelut® column and the vial is rinsed with a

water/ethanol (l/l, v/v, 2 × 100 μl) mixture. After ten minutes, the column is eluted with dichloromethane (5 × 2 ml). The organic phase is evaporated under vacuum and the residue is chromatographed on an HPLC column (elution rate : 1 ml /minute). The retention times obtained are : 9.75 minutes (compound **30** labelled with $^{131}$I) and 19.9 minutes (compound **32**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{131}$I]-*N*-(2-diethylaminoethyl)-6-iodoquinoline-2-carboxamide dihydrochloride [$^{131}$I]-(**31**) (677 MBq). Radiochemical yield: 52%; radiochemical purity: 99%.

**Preparation of [$^{131}$I]-*N*-(2-diethylaminoethyl)-6-iodoquinoxaline-2-carboxamide hydrochloride [$^{131}$I]-(46) (labelling of high specific activity)**

A 0.5N hydrochloric acid solution (100 μl), Na$^{131}$I (120 μl, 1.49 GBq) and an aqueous solution of chloramine-T monohydrate (1 mg/ml, 100 μl) are successively added to a solution of the trialkylstannane **47** (0.45 mg) in ethanol (100 μl) in a vial. The vial is sealed and stirred, at ambient temperature, with a vortex for 20 minutes. A solution of sodium metabisulphite (20 mg) in water (100 μl) and then a 3N sodium hydroxide solution (150 μl) are added and the mixture is stirred with a vortex for a few minutes. The solution is deposited on an Extrelut® column and the vial is rinsed with a water/ethanol (l/l, v/v, 2 × 100 μl) mixture. After ten minutes, the column is eluted with dichloromethane (5 × 2 ml). The organic phase is evaporated under vacuum and the residue is chromatographed on an HPLC column (elution rate: 1 ml /minute). The retention times obtained are: 7.8 minutes (compound **45** labelled with $^{131}$I) and 17.2 minutes (compound **47**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{131}$I]-*N*-(2-diethylaminoethyl)-6-iodoquinoxaline-2-carboxamide hydrochloride [$^{131}$I]-(**46**) (1.02 GBq). Radiochemical yield: 68%; radiochemical purity: 98%.

**Preparation of [$^{125}$I]-*N*-(2-diethylaminoethyl)-9,10-dihydro-7-iodo-9-oxoacridine-4-carboxamide dihydrochloride [$^{125}$I]-(84) (labelling of high specific activity)**

A 1% ethanolic acetic acid solution (30 μl), Na$^{125}$I in sodium hydroxide solution (5 μl, 11.1 MBq) and an aqueous solution of chloramine-T monohydrate (0.4 mg/ml, 15 μl) are successively added to a solution of the trialkylstannane **85** (0.12 mg) in ethanol (30 μl) in a vial. The vial is sealed and stirred, at ambient temperature, with a vortex for 10 minutes. A 0.1N sodium hydroxide solution (20 μl) is added and the mixture is stirred with a vortex for a few minutes. The solution is deposited on an Extrelut® column and the vial is rinsed with a water/ethanol (l/l, v/v, 2 × 100 μl) mixture. After ten minutes, the column is eluted with dichloromethane (5 × 2 ml). The organic phase is evaporated under vacuum and the residue is chromatographed on an HPLC column (elution rate: 1 ml /minute). The retention times obtained are: 11.5 minutes (compound **83** labelled with $^{125}$I) and 19.8 minutes (compound **85**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{125}$I]-*N*-(2-diethylaminoethyl)-9,10-dihydro-7-iodo-9-oxoacridine-4-carboxamide dihydrochloride [$^{125}$I]-(**84**) (6.33 MBq). Radiochemical yield: 57%; radiochemical purity: 99.9%.

**Preparation of [$^{125}$I]-*N*-(2-diethylaminoethyl)-5-iodoacridine-4-carboxamide dihydrochloride [$^{125}$I]-(102) (labelling of high specific activity)**

A citrate/hydrochloric acid (pH = 4) buffer solution (20 μl), Na$^{125}$I in sodium hydroxide solution (8 μL, 29.6 MBq) and an aqueous solution of chloramine-T monohydrate (0.5 mg/ml, 15 μl) are successively added to a solution of the trialkylstannane **103** (0.12 mg) in ethanol (30 μl) in a vial. The vial is sealed and stirred, at ambient temperature, with a vortex for 10 minutes. A 0.1N sodium hydroxide solution (20 μl) is added and the mixture is stirred with a vortex for a few minutes. The solution is deposited on an Extrelut® column and the vial is rinsed with a water/ethanol (l/l, v/v, 2 × 100 μl) mixture. After ten minutes, the column is eluted with dichloromethane (5 × 2 ml). The organic phase is evaporated under vacuum and the residue is chromatographed on an HPLC column (elution rate: I ml /minute). The retention times obtained are: 11.0 minutes (compound **101** labelled with $^{125}$I) and 17.7 minutes (compound **103**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{125}$I]-*N*-(2-diethylaminoethyl)-5-iodoacridine-4-carboxamidc dihydrochloride [$^{125}$I]-(**102**) (19.6 MBq). Radiochemical yield: 66%; radiochemical purity:

99%.

**Preparation of [$^{125}$I]-*N*-(2-diethylaminoethyl)-7-iodoacridine-4-carboxamide dihydrochloride [$^{125}$I]-(111) (labelling of high specific activity)**

A citrate/hydrochloric acid (pH = 4) buffer solution (20 μl), $Na^{125}I$ in sodium hydroxide solution (70 μl, 221.3 MBq) and an aqueous solution of chloramine-T monohydrate (0.5 mg/ml, 10 μl) are successively added to a solution of the trialkylstannane **152** (0.12 mg) in ethanol (30 μl) in a vial. The vial is sealed and stirred, at ambient temperature, with a vortex for 10 minutes. A 0.1N sodium hydroxide solution (20μ l) is added and the mixture is stirred with a vortex for a few minutes. The solution is deposited on an Extrelut® column and the vial is rinsed with a water/ethanol (l/l, v/v, 2 × 100 μl) mixture. After ten minutes, the column is eluted with dichloromethane (5 × 2 ml). The organic phase is evaporated under vacuum and the residue is chromatographed on an HPLC column eluted with a water/methanol/0.2% aqueous ammonia mixture with a flow rate of 1 ml/minute and a linear elution gradient for methanol from 70 to 100% over a period of 20 minutes. The retention times obtained are: 15.1 minutes (compound **110** labelled with $^{125}$I) and 23.3 minutes (compound **152**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{125}$I]-*N*-(2-diethylaminoethyl)-7-iodoacridine-4-carboxamide dihydrochloride [$^{125}$I]-(**111**) (66.6MBq). Radiochemical yield: 31%; radiochemical purity: 99%.

**Preparation [$^{125}$I]-*N*-(2-diethylaminoethyl)-7-iodo-9-(4-methanesulphonamido-2-methoxyanilino)acridine-4-carboxamide dihydrochloride [$^{125}$I]-(129) (labelling of high specific activity)**

A 1% ethanolic acetic acid solution (30 μl), $Na^{125}I$ in sodium hydroxide solution (50 μl, 125.4 MBq) and an aqueous solution of chloramine-T monohydrate (0.25 mg/ml, 15 μl) are successively added to a solution of the trialkylstannane **131** (0.12 mg) in ethanol (30 μl) in a vial. The vial is sealed and stirred, at ambient temperature, with a vortex for 10 minutes. A 0.1N sodium hydroxide solution (20 μl) is added and the mixture is stirred with a vortex for a few minutes. The solution is deposited on an Extrelut® column and the vial is rinsed with a water/ethanol (l/l, v/v, 2 × 100 μl) mixture. After ten minutes, the column is eluted with dichloromethane (5 × 2 ml). The organic phase is evaporated under vacuum and the residue is chromatographed on an HPLC column (elution rate: 1 ml /minute). The retention times obtained are: 12.4 minutes (compound **130** labelled with $^{125}$I) and 17.8 minutes (compound **131**). The various fractions collected are evaporated under vacuum. The residue is taken up in anhydrous dichloromethane (1 ml) and then in a 2N solution of hydrochloric acid in ether (2 ml). The solution is evaporated under vacuum. The precipitate obtained is taken up in anhydrous ether (2 ml) and evaporated under vacuum, to result in [$^{125}$I]-*N*-(2-diethylaminoethyl)-7-iodo-9-(4-methanesulphonamido-2-methoxyanilino)acridine-4-carboxamide dihydrochloride [$^{125}$I]-(**129**) (67.0 MBq). Radiochemical yield: 53%; radiochemical purity: 97%.

The compounds in accordance with the invention were subjected to pharmacological tests which demonstrated their usefulness in the treatment and/or diagnosis of malignant melanoma.

**EXAMPLE 37: Biodistribution of the molecules in the melanoma-bearing mouse**

*Experimental protocol*: The selection of the molecules with the pharmacokinetic profile best suited to an application in diagnosis or in therapy is based on the study of their biodistribution after labelling with $^{125}$I in the male C57BL6 mouse bearing grafted B16 F0 murine melanoma tumour. The distribution of the radioactivity of the various compounds in the body (tumour and other organs) is quantified using an Ambis 4000 image analyzer on sections of whole animals. While using a smaller number of animals than the conventional counting method, this technique appears to be more predictive of the scintigraphic imaging. All the *in vivo* experimentation is carried out within the context defined by French legislation relating to animal experimentation.

The B16 F0 melanoma cells are provided by the ATCC and cultured in monolaycrs in culture medium (MEM, Invitrogen) supplemented with 10% foetal calf sacrum and antibiotics. The cultures are maintained by subculturing after trypsinization. The cells of the first passages are frozen and stored in liquid nitrogen. For the transplanting, the cells in culture at confluence are detached by trypsinization and washed with phosphate buffer (PBS). They are resuspended in PBS and injected subcutaneously into the mice (3 × $10^5$ cells; 0.1 ml) in the left flank. After ten days, the tumours are palpables with a percentage of uptake of 98-100%.

The molecule labelled with $^{125}$I is injected via the caudal vein (0.1 μmol, 0.5-3.6 MBq/animal) at ten animals per product. At various times after administration (1, 3, 6, 24 or 72 hours), two mice are sacrificed by inhalation of $CO_2$ and rapidly frozen in liquid nitrogen. The animal is then cryosectioned at -22°C using a Reichert-Jung cryomicrotome (Leica Instruments, Rueil Malmaison, France) into sections with a thickness of 40 μm which are left to dehydrate under

cold conditions for 48 hours.

The distribution of the radioactivity present in the sections is analyzed using an AMBIS 4000 analyzer (Scanalytics, CSPI, San Diego, CA), which is a proportional counter with a multiwire chamber validated and calibrated beforehand for the monitoring of iodine in the mouse. The measurements require acquisition times of 1000 minutes. The quantification of the radioactivity in the various organs is carried out from the two-dimensional image of the mouse cross section displayed on the screen, in the defined and delimited regions of interest. The value of radioactivity per unit of surface area (cpm/$mm^2$) is converted to concentration (kBq/g) and expressed as percentage of the injected dose/g of tissue (%ID/g).

In parallel with the autoradiographic study, the kinetics of the products are monitored by scintigraphic imaging of the mice, after injection of the labelled compound (3.7 MBq), using a dedicated gamma camera (Biospace gamma imager) which makes possible *in vivo* imaging and its repetition in the same animal.

*Results:*

The biodistribution of the compounds labelled with $^{125}$**I,** measured by the %ID/g, in the various organs of the melanoma-bearing mouse is represented in the following **Tables 1-19** (only the organs where the presence of radioactivity was displayed and quantified are given).

The radioactivity concentrations measured from 1 to 72 hours in the various organs after the i.v. administration of a compound labelled with $^{125}$**I** in the C57BL6 mouse bearing a subcutaneously grafted B 16 melanoma are summarized in **Tables 1 to 19.**

Concentration of radioactivity in the tissues: tumour (B 16), intestinal contents (Ic), stomach contents (Sc), skeletal muscle (muscle), blood (measured in the heart cavity), urine (bladder), pigmented tissues of the eye (uvea), gall bladder (GB), seminal vesicles (SV) or bone marrow (BM).

Table I : Biodistribution compound **3** (dose=1.50 MBq)

| | 1H | | 3H | | 6H | | 24H | | 72H | |
|---|---|---|---|---|---|---|---|---|---|---|
| | Mean | Standard deviation | Mean | Standard deviation | Mean | Standard deviation | Mean | Standard deviation | Mean | Standard deviation |
| **B 16** | **12.99** | **2.72** | **1138** | **2.20** | **8.95** | **0.72** | **6.97** | **3.87** | **1.52** | **0,46** |
| **brain** | 4.37 | 0.92 | 0.78 | 0.24 | 0.25 | 0.05 | - | - | - | - |
| **lc** | 7.82 | | 25.85 | 16.22 | 18.72 | 7.67 | 3.03 | 1.75 | - | - |
| **Sc** | 27.82 | 8.46 | 24.69 | 8.37 | 10.79 | 3.52 | 3.10 | 1.97 | - | - |
| **liver** | 9.46 | 1.18 | 4.75 | 0.60 | 2.73 | 0.36 | - | - | - | - |
| **muscle** | 1.92 | 0.55 | 0.83 | 0.17 | 0.38 | 0.05 | - | - | - | - |
| **pancreas** | 10.18 | 1.66 | 8.19 | - | - | - | - | - | - | - |
| **lunges** | 11.82 | 2.16 | 4.43 | 0.88 | 2.12 | 0.29 | - | - | - | - |
| **spleen** | 9.70 | 3.28 | 2.59 | 0.13 | 8.05 | 0.20 | - | - | - | - |
| **kidney** | 13.30 | 2.45 | 5.65 | 0.77 | 3.07 | 0.29 | - | - | - | - |
| **blood** | 2.97 | 0.55 | 1.77 | 0.09 | 1.74 | - | - | - | - | - |
| **uvea** | 13.21 | 1.68 | 13.90 | 1.79 | 12.76 | 2.26 | 12.28 | 6.22 | 7.84 | 9.38 |

Table 2 : Biodistribution compound **7** (dose=0.94 MBq)

| | 1H | | 3H | | 6H | | 24H | | 72H | |
|---|---|---|---|---|---|---|---|---|---|---|
| | **Mean** | **Stanard deviation** | **Mean** | **Standard deviation** | **Mean** | **Standard deviation** | **Mean** | **Standard deviation** | **Mean** | **Standard deviation** |
| **B 16** | **15.49** | 1.70 | **9.27** | 1.50 | **6.97** | 0.96 | **2.69** | 0.74 | **0.87** | 0.38 |
| **brain** | 0.64 | 0.20 | 0.22 | 0.14 | 0.12 | 0.05 | - | - | - | - |
| **lc** | 13.44 | 9.46 | 4.94 | 1.84 | 2.98 | 1.04 | - | - | - | - |
| **SV** | 6.79 | 0.95 | 3.78 | 0.38 | 3.55 | 0.55 | - | - | - | - |
| **Sc** | 34.80 | 8.68 | 42.71 | 10.70 | 50.21 | 5.45 | 1.46 | 0.51 | - | - |
| **liver** | 5.14 | 0.92 | 3.01 | 0.30 | 2.09 | 0.32 | - | - | - | - |
| **muscle** | 1.48 | 0.18 | - | - | 0.53 | 0.12 | - | - | - | - |
| **pancreas** | 3.94 | 0.83 | 2.53 | 0.63 | 2.27 | 0.28 | - | - | - | - |
| **lungs** | 7.59 | 1.58 | 3.96 | 0.40 | 3.21 | 0.49 | - | - | - | - |
| **spleen** | 5.44 | 4.42 | 2.68 | 0.31 | 2.35 | 0.50 | - | - | - | - |
| **kidney** | 6.08 | 1.13 | 3.26 | 0.46 | 3.05 | 0.32 | - | - | 1.09 | 0.32 |
| **blood** | 6.67 | 1.00 | 4.22 | 0.29 | 3.71 | 0.62 | - | - | - | - |
| **testicles** | 4.11 | 0.01 | 2.43 | 0.39 | 2.01 | 0.24 | - | - | - | - |
| **thyroid** | 25.29 | | 59.09 | 34.36 | 7.92 | | 111.30 | | 178.80 | 86.38 |
| **uvea** | 16.66 | 4.94 | 12.69 | 2.16 | 11.50 | 1.21 | 6.07 | 1.70 | 2.54 | 0.32 |
| **GB** | 8.68 | | 6.78 | 2.13 | 4.90 | 1.34 | - | - | - | - |

Table 3 : Biodistribution compound **10** (dose=1.52 MBq)

| | 1H | | 3H | | 6H | | 24H | | 72H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard demiation** |
| **B 16** | **8.31** | 3.10 | **10.22** | 1.37 | **9.84** | 0.86 | **8.16** | 4.39 | **0.84** | 0.26 |
| **brain** | 4.84 | 0.69 | 1.73 | 0.29 | 0.74 | - | 0.00 | 0.00 | - | - |
| **lc** | 76.74 | 137.12 | 112.56 | 80.06 | 79.85 | 62.59 | 3.31 | 2.34 | - | - |
| **Sc** | 35.41 | 21.04 | 8.52 | 16.68 | 21.84 | 16.62 | 2.10 | 1.10 | 0.04 | |
| **liver** | 11.86 | 1.23 | 5.21 | 0.59 | 3.63 | 0.41 | 1.61 | 0.38 | 0.61 | 0.10 |
| **muscle** | 1.50 | 0.47 | 0.49 | 0.13 | 0.31 | 0.18 | 0.19 | - | - | - |
| **pancreas** | 7.15 | 2.66 | 2.48 | 0.05 | 1.46 | - | 0.09 | 0.10 | - | - |
| **lungs** | 14.41 | 1.87 | 3.46 | 0.74 | 1.70 | 0.29 | 0.37 | 0.26 | - | - |
| **spleen** | 8.09 | 1.35 | 2.79 | 0.42 | 1.68 | 0.32 | 1.22 | 0.30 | - | - |
| **kidney** | 10.87 | 1.83 | 4.19 | 0.66 | 2.50 | 0.38 | 0.83 | 0.28 | 0.71 | - |
| **blood** | 1.60 | 0.27 | 0.79 | 0.24 | 0.67 | 0.18 | 0.10 | 0.11 | - | - |
| **uvea** | 11.70 | 1.29 | 12.78 | 3.31 | 13.20 | 2.81 | 8.81 | 2.48 | 5.86 | 0.63 |

Table 4 : Biodistribution compound **13** (dose= 0.71 MBq)

| | 1H | | 3H | | 6H | | 24H | | 72H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **12..47** | 4.78 | **9.98** | 4.29 | **8.72** | 4.08 | **7.28** | 2.29 | **1.73** | 0.47 |
| **brain** | 0.81 | 0.43 | - | - | - | - | - | - | - | - |
| **Ic** | 71.13 | 64.79 | 73.02 | 61.60 | 37.70 | 35.50 | - | - | - | - |
| **Sc** | 20.00 | 12.68 | 5.93 | 3.24 | 18.77 | 8.42 | - | - | - | - |
| **liver** | 2.21 | 0.58 | 1.25 | 0.37 | - | - | - | - | - | - |
| **muscle** | 0.77 | 0.23 | - | - | - | - | - | - | - | - |
| **lungs** | 3.69 | 1.02 | - | - | - | - | - | - | - | - |
| **spleen** | 3.88 | 0.83 | - | - | - | - | - | - | - | - |
| **kidney** | 5.61 | 1.08 | - | - | - | - | - | - | - | - |
| **blood** | 1.37 | 0.30 | - | - | - | - | - | - | - | - |
| **thyroid** | 24.03 | - | 67.21 | 48.99 | 139.12 | 41.99 | 79.82 | 66.09 | - | - |
| **uvea** | 16.09 | 8.29 | 19.51 | 6.38 | 16.29 | 4.53 | 11.06 | 3.00 | 9.22 | 3.38 |
| **GB** | - | - | 65.56 | 47.19 | - | - | - | - | - | - |

Table 5 : Biodistribution compound **16** (dose=0.74 MBq)

| | 1H | | 3H | | 6H | | 24H | | 72H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **2.96** | 1.79 | **5.40** | 3.14 | **9.87** | 1.09 | **5.22** | 1.61 | - | - |
| **brain** | 0.33 | 0.07 | 0.36 | 0.29 | 0.65 | 0.41 | - | - | - | - |
| **Ic** | 58.60 | 60.15 | 52.06 | 17.32 | 26.14 | 15.95 | - | - | - | - |
| **Sc** | 24.51 | 4.39 | 30.71 | 13.69 | 48.21 | 12.16 | - | - | 3.42 | |
| **liver** | 4.86 | 1.51 | 2.56 | 0.31 | - | - | - | - | - | - |
| **muscle** | 1.10 | 0.26 | 1.30 | 0.42 | - | - | - | - | - | - |
| **lungs** | 6.48 | - | 3.38 | 0.84 | 4.56 | 0.36 | - | - | - | - |
| **spleen** | 8.16 | 4.03 | 12.67 | 6.38 | | | - | - | - | - |
| **kidney** | 7.12 | 3.31 | 5.04 | 1.18 | 4.47 | 0.10 | - | - | - | - |
| **blood** | 2.20 | 2.20 | 3.93 | 1.01 | - | - | - | - | - | - |
| **uvea** | 28.30 | 11.11 | 16.60 | 3.65 | 22.16 | 11.57 | 11.17 | 1.79 | 2.41 | 0.05 |

Table 6 : Biodistribution compound **21** (dose=1.36 MBq)

| | 1H | | 3H | | 6H | | 24H | | 72H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **5.66** | 2.96 | **7.14** | 1.92 | **8.12** | 3.35 | **8.02** | 2.36 | **2.67** | 1.10 |
| **Ic** | 9.48 | 10.62 | 78.03 | 33.12 | 56.39 | 46.99 | 2.68 | 0.64 | - | - |
| **Sc** | 4.55 | 1.23 | 5.19 | 1.22 | 16.57 | 12.41 | - | - | - | - |
| **liver** | 3.97 | 0.69 | 0.99 | 0.16 | - | - | - | - | - | - |
| **muscles** | 0.51 | 0.17 | 0.09 | 0.02 | - | - | - | - | - | - |
| **pancreas** | 7.52 | - | - | - | - | - | - | - | - | - |
| **lungs** | 2.42 | 1.16 | - | - | - | - | - | - | - | - |
| **spleen** | 13.07 | 4.58 | - | - | - | - | - | - | - | - |
| **kidney** | 2.99 | 0.71 | - | - | - | - | - | - | - | - |
| **blood** | 0.68 | 0.28 | - | - | . | - | - | - | - | - |
| **uvea** | 17.43 | 4.63 | 19.18 | 5.76 | 26.81 | 10.20 | 11.54 | 4.32 | 9.55 | 2.12 |

Table 7 : Biodistribution compound 26 (dose=0.47 MBq)

| | 1H | | 3H | | 6H | | 24H | | 72H | |
|---|---|---|---|---|---|---|---|---|---|---|
| | Mean | Standard deviation | Mean | Standard deviation | Mean | Standard deviation | Mean | Standard deviation | Mean | Standard deviation |
| **B 16** | **14.78** | 4.81 | **16.13** | 2.94 | **9.90** | 3.76 | **7.06** | 1.97 | **4.54** | 2.10 |
| **Ic** | 105.55 | 95.85 | 72.05 | 50.76 | 95.86 | 47.28 | 3.19 | 2.13 | - | - |
| **Sc** | 24.46 | 10.07 | 17.98 | 5.70 | 8.62 | 1.67 | 2.32 | 2.90 | - | - |
| **liver** | 13.08 | 1.74 | 7.15 | 1.67 | 3.38 | 0.77 | - | - | - | - |
| **muscle** | 0.55 | - | - | - | - | - | - | - | - | - |
| **pancreas** | 6.17 | - | - | - | - | - | - | - | - | - |
| **lungs** | 5.16 | 2.07 | 3.74 | 0.31 | - | - | - | - | - | - |
| **spleen** | 11.51 | 7.04 | - | - | 14.97 | 2.44 | - | - | - | - |
| **kidney** | 6.90 | 1.09 | 4.57 | 0.00 | - | - | - | - | - | - |
| **blood** | 3.31 | 1.68 | 1.33 | 0.26 | - | - | - | - | - | - |
| **thyroid** | | | 63.44 | 47.68 | 34.17 | 38.00 | 142.88 | 1.28 | 174.73 | 87.84 |
| **uvea** | 12.87 | 3.92 | 24.40 | 17.72 | 10.09 | 2.69 | 10.44 | | 7.26 | 1.59 |
| **GB** | - | - | 160.60 | 26.81 | 38.11 | - | - | - | - | - |

Table 8 : Biodistribution compound **31** (dose=1.47 MBq)

| | **1H** | | **3H** | | **6H** | | **24H** | | **72H** | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **19.63** | 8.99 | **25.35** | 9.58 | **17.21** | 4.64 | **12.13** | 5.18 | **5.93** | 1.68 |
| **brain** | 1.96 | 0.42 | 0.83 | 0.32 | 0.37 | 0.16 | - | - | - | - |
| **lc** | 15.78 | 17.68 | 20.65 | 11.13 | 23.36 | 10.68 | 1.45 | | - | - |
| **Sc** | 48.03 | 19.55 | 34.72 | 14.30 | 65.36 | 19.81 | 2.33 | 0.27 | - | - |
| **liver** | 10.92 | 1.52 | 8.89 | 1.74 | 7.11 | 1.48 | 2.25 | 0.77 | 1.23 | 0.45 |
| **muscle** | 1.79 | 0.22 | 1.14 | 0.37 | 0.95 | 0.21 | - | - | - | - |
| **pancreas** | 9.80 | 0.82 | 6.17 | 1.34 | - | - | - | - | - | - |
| **lungs** | 9.41 | 1.43 | 5.58 | 0.72 | 5.32 | 0.89 | 0.53 | - | - | - |
| **spleen** | 11.56 | 2.59 | 10.63 | 6.37 | 7.60 | 2.79 | 2.66 | 0.28 | 1.85 | 0.24 |
| **kidney** | 11.82 | 2.14 | 12.59 | 4.08 | 7.60 | 1.84 | 0.34 | - | - | - |
| **blood** | 7.01 | 0.82 | 5.57 | 0.98 | 5.91 | 0.78 | - | - | - | - |
| **uvea** | 32.71 | 14.88 | 33.10 | 8.22 | 36.62 | 10.60 | 22.27 | 4.72 | 18.30 | 6.97 |

Table 9 : Biodistribution compound 35 (dose=0,56 MBq)

| | 1H | | 3H | | 6H | | 24H | | 72H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **4.69** | **1.23** | **4.05** | **0.78** | **5.81** | **1.94** | **2.74** | **1.08** | **2.35** | **0.75** |
| **brain** | 0.07 | 0.08 | - | - | 0.08 | 0.14 | 0.04 | 0.07 | 0.10 | 0.12 |
| **Ic** | 52.94 | 54.99 | 90.12 | 66.99 | 61.06 | 37.95 | 1.62 | 0.79 | 0.70 | 0.29 |
| **Sc** | 12.74 | 7.63 | 9.93 | 3.52 | 22.27 | 25.17 | 0.13 | 0.15 | - | - |
| **liver** | 5.16 | 0.72 | 1.84 | 0.78 | 0.32 | 0.29 | 0.07 | 0.08 | - | - |
| **muscle** | 0.92 | 0.32 | 0.13 | 0.00 | 0.01 | 0.02 | - | - | - | - |
| **pancreas** | 5.74 | 1.32 | 0.96 | 0.02 | 1.08 | 0.00 | - | - | - | - |
| **lungs** | 2.06 | 0.44 | 0.88 | 0.46 | 0.15 | 0.15 | 0.04 | 0.09 | - | - |
| **spleen** | 1.24 | 0.37 | 0.28 | 0.28 | 0.26 | 0.30 | 0.15 | 0.00 | - | - |
| **kidney** | 2.80 | 0.56 | 0.80 | 0.52 | 0.20 | 0.20 | 0.03 | 0.07 | - | - |
| **blood** | 0.42 | 0.14 | 0.23 | 0.22 | 0.17 | 0.22 | 0.22 | 0.36 | - | - |
| **thyroid** | | | 4.33 | 0.00 | 8.55 | 2.96 | 5.09 | 2.72 | 9.83 | 1.77 |
| **uvea** | 5.12 | 0.61 | 5.66 | 1.9 | 5.18 | 1.56 | 3.5 | 1.42 | 6.79 | 4.16 |

Table 10 : Biodistribution compound **39** (dose= 3.63 MBq)

| | 1H | | 3H | | 6H | | 24H | | 72H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B16** | **7.27** | **2.43** | **7.12** | **5.45** | **7.92** | **2.17** | **7.05** | **2.42** | **3.13** | **1.92** |
| **brain** | 0.30 | 0.05 | 0.09 | - | - | - | - | - | - | - |
| **lc** | 42.97 | 59.32 | 68.64 | 52.65 | 29.16 | **11.21** | 1.70 | 0.46 | - | - |
| **Sc** | 23.01 | 17.75 | 19.05 | 7.60 | 28.44 | 20.08 | 0.75 | 0.29 | - | - |
| **liver** | 3.86 | 0.28 | 2.02 | 0.37 | 1.54 | 0.44 | 0.43 | - | - | - |
| **muscle** | 0.74 | 0.17 | 0.43 | 0.14 | - | - | - | - | - | - |
| **pancreas** | 2.39 | 0.31 | - | - | - | - | - | - | - | - |
| **lungs** | 3.68 | 0.28 | 2.76 | 0.41 | 2.05 | 0.55 | - | - | - | - |
| **spleen** | 2.83 | 0.25 | - | - | - | - | - | - | 1.62 | 1.43 |
| **kidney** | 4.62 | 0.47 | 2.66 | 0.35 | 2.11 | 0.47 | - | - | - | - |
| **blood** | 2.15 | 0.23 | 2.65 | 0.19 | 3.17 | 0.43 | - | - | - | - |
| **thyroid** | 16.89 | 2.66 | 35.71 | 6.74 | 48.93 | 38.15 | 88.65 | 66.65 | 132.79 | - |
| **uvea** | 18.08 | 6.80 | 13.87 | 4.59 | 18.02 | 4.89 | 14.55 | 5.32 | 9.73 | 1.39 |

Table 11: Biodistribution compound **46** (dose=1.17 MBq)

| | 1H | | 3H | | 6H | | 24H | | 72H | |
|---|---|---|---|---|---|---|---|---|---|---|
| | Mean | Standard deviation | Mean | Standard deviation | Mean | Standard deviation | Mean | Standard deviation | Mean | Standard deviation |
| **B 16** | **17.00** | 11.06 | **27.68** | 7.01 | **40.70** | 7.76 | **21.67** | 10.77 | **12.45** | 1.61 |
| **brain** | 2.34 | 0.46 | 1.03 | 0.18 | - | - | - | - | - | - |
| **lc** | 15.22 | 9.50 | 14.63 | 3.88 | 17.45 | 8.05 | - | - | - | - |
| **Sc** | 24.33 | 6.48 | 20.33 | 5.19 | 21.09 | 6.85 | - | - | 2.23 | 0.38 |
| **liver** | 13.18 | 1.33 | 8.87 | 1.11 | 7.53 | 1.40 | 1.49 | 0.18 | - | - |
| **BM** | 4.58 | 0.90 | - | - | - | - | - | - | - | - |
| **muscle** | 2.04 | 0.37 | 0.86 | 0.22 | 0.87 | 0.23 | - | - | - | - |
| **pancreas** | 7.60 | 0.76 | - | - | - | - | - | - | - | - |
| **lungs** | 7.83 | 0.59 | 5.68 | 1.05 | 4.46 | 0.60 | - | - | - | - |
| **spleen** | 14.21 | 14.32 | 4.69 | - | - | - | - | - | - | - |
| **kidney** | 16.46 | 2.98 | 9.30 | 3.31 | 7.69 | 0.80 | - | - | - | - |
| **blood** | 3.45 | 0.44 | 2.91 | 0.33 | 3.09 | 0.52 | - | - | - | - |
| **thyroid** | - | - | 39.76 | 23.47 | 83.77 | 26.14 | 94.22 | 27.48 | | |
| **uvea** | 33.82 | 3.47 | 24.79 | 6.78 | 32.30 | 6.52 | 32.44 | 5.98 | 25.26 | 8.38 |
| **GB** | - | - | - | - | 33.38 | 7.70 | - | - | - | - |

Table 12: Biodistribution compound **49** (dose=1.09 MBq)

| | 1H | | 3H | | 6H | | 24H | | 72H | |
|---|---|---|---|---|---|---|---|---|---|---|
| | **Mean** | **Standard deviation** | **Mean** | **Stand ard deviati on** | **Mean** | **Standard deviation** | **Mean** | **Standard deviation** | **Mean** | **Standard deviation** |
| **B 16** | **19.07** | 1.29 | **20.34** | 8.64 | **27.38** | 9.38 | **18.28** | 6.20 | **10.86** | 3.32 |
| **brain** | - | - | - | - | - | - | - | - | - | - |
| **lc** | 24.45 | 11.24 | 23.54 | 8.25 | 26.72 | 9.87 | 1.21 | 0.22 | - | - |
| **Sc** | 25.53 | 12.50 | 24.89 | 9.33 | 22.21 | 9.54 | 2.16 | 0.44 | - | - |
| **liver** | 13.79 | 1.37 | 7.77 | 0.67 | 3.89 | 0.72 | - | - | - | - |
| **muscle** | 2.00 | 0.51 | 1.41 | 0.38 | 0.45 | 0.36 | - | - | - | - |
| **pancreas** | 21.47 | 4.78 | 9.71 | 0.94 | - | - | - | - | - | - |
| **lungs** | 8.33 | 1.07 | 6.88 | 1.23 | 3.34 | 0.56 | - | - | - | - |
| **spleen** | 18.88 | 7.30 | 16.71 | 15.26 | - | - | - | - | - | - |
| **kidney** | 18.29 | 3.49 | 13.10 | 2.90 | 7.98 | 1.65 | - | - | - | - |
| **blood** | 3.31 | 0.52 | 3.47 | 0.46 | 2.50 | | - | - | - | - |
| **thyroid** | 28.75 | | 125.51 | 113.55 | 16.38 | - | - | - | 53.26 | |
| **uvea** | 21.04 | 4.02 | 31.76 | 6.25 | 28.78 | 8.39 | 19.22 | 5.28 | 19.78 | 5.33 |
| **GB** | - | - | 50.82 | 14.16 | 55.80 | 3.92 | - | - | - | - |

Table 13: Biodistribution compound **84** (dose=1.60 MBq)

| | 1H | | 3H | | 6H | | 24H | | 72H | |
|---|---|---|---|---|---|---|---|---|---|---|
| | **Mean** | **Standard deviation** | **Mean** | **Standard deviation** | **Mean** | **Standard demiation** | **Mean** | **Standard deviation** | **Mean** | **Standard deviation** |
| **B 16** | **12.64** | 2.85 | **21.05** | 8.81 | **26.27** | 8.64 | **27.45** | 8.26 | **17.49** | 2.67 |
| **brain** | 1.61 | 0.25 | 1.20 | 0.28 | 0.77 | 0.23 | 0.15 | 0.12 | - | - |
| **lc** | 22.68 | 19.59 | 27.80 | 10.94 | 32.99 | 8.82 | 5.16 | 2.77 | 0.31 | - |
| **Sc** | 23.12 | 5.13 | 13.44 | 3.07 | 24.42 | 4.89 | 3.13 | 1.34 | - | - |
| **liver** | 14.00 | 2.78 | 9.33 | 0.62 | 7.37 | 0.67 | 1.23 | 0.18 | 0.59 | 0.19 |
| **muscle** | 7.51 | 0.86 | 5.84 | 1.33 | 4.61 | 0.92 | 1.27 | 1.37 | - | - |
| **pancreas** | 18.67 | 3.31 | 23.73 | 7.93 | 23.46 | 4.68 | 19.96 | 6.32 | 16.83 | 8.20 |
| **lungs** | 1.87 | 0.37 | 1.39 | 0.19 | 0.92 | 0.16 | 0.22 | - | - | - |
| **spleen** | 8.69 | 0.64 | 6.66 | 1.05 | 5.14 | 0.70 | - | - | - | - |
| **kidney** | 23.94 | 2.76 | 13.99 | 2.72 | 9.99 | 1.47 | 0.68 | 0.09 | - | - |
| **blood** | 13.91 | 1.42 | 9.95 | 0.62 | 7.24 | 0.90 | 0.71 | 0.32 | - | - |
| **thyroid** | 17.60 | 2.28 | 12.97 | 1.99 | 8.95 | 1.38 | 0.65 | 0.38 | - | - |
| **uvea** | 12.88 | 5.29 | 16.32 | 3.71 | 36.25 | 8.52 | 76.78 | 44.05 | 14.93 | 2.68 |
| **GB** | 15.23 | 12.36 | 16.85 | 6.21 | 19.51 | 2.08 | 27.54 | 7.08 | 26.42 | 4.21 |

Table 14: Biodistribution compound **102** (dose=1.11 MBq)

| | 1H | | 3H | | 6H | | 24H | | 72H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **18.81** | 5.94 | **21.41** | 4.96 | **23.62** | 7.65 | **13.82** | 2.53 | **6.99** | 2.89 |
| **brain** | 1.05 | 0.44 | 0.50 | 0.22 | 0.38 | 0.13 | - | - | - | - |
| **Ic** | 155.76 | 134.50 | 77.08 | 125.04 | 109.20 | 64.67 | 4.65 | 1.93 | - | - |
| **Sc** | 87.29 | 55.99 | 51.74 | 23.04 | 60.55 | 3.62 | 3.47 | 1.84 | - | - |
| **liver** | 17.89 | 1.82 | 11.30 | 1.38 | 7.64 | 1.19 | 1.07 | 0.19 | - | - |
| **muscles** | 2.34 | 0.49 | 1.34 | 0.53 | 1.36 | 0.60 | - | - | - | - |
| **pancreas** | 11.71 | 2.62 | 6.70 | 1.33 | z.36 | 0.72 | - | - | - | - |
| **lungs** | 9.62 | 1.69 | 6.83 | 0.97 | 6.22 | 1.23 | - | - | - | - |
| **spleen** | 21.63 | 4.57 | 16.03 | 11.52 | 6.20 | 0.59 | - | - | - | - |
| **kidney** | 16.80 | 2.96 | 8.22 | 1.09 | 6.36 | 1.59 | - | - | - | - |
| **blood** | 4.07 | 0.86 | 5.37 | 0.72 | 6.46 | 1.90 | - | - | - | - |
| **thyroid** | 21.91 | 13.58 | 23.93 | - | 194.64 | - | 254.61 | - | 82.10 | 4.64 |
| **uvea** | 20.00 | 6.56 | 20.14 | 4.52 | 16.16 | 4.88 | 14.44 | 3.12 | 16.59 | 5.35 |
| **GB** | 281.34 | 63.99 | 150.10 | 55.85 | 381.20 | - | - | - | - | - |

Table 15 : Biodistribution compound **111** (dose = 1,54 MBq)

| | 1H | | 3H | | 6H | | 24H | | 72H | |
|---|---|---|---|---|---|---|---|---|---|---|
| | **Mean** | **Standard deviation** | **Mean** | **Standard deviation** | **Mean** | **Standard deviation** | **Mean** | **Standard deviation** | **Mean** | **Standard deviation** |
| **B 16** | **4.78** | 0.64 | **3.98** | 1.16 | **4.57** | 0.70 | **2.77** | 1.16 | **1.67** | 1.06 |
| **Brain** | 0.28 | 0.15 | 0.27 | - | 0.11 | 0.08 | - | - | - | - |
| **Ic** | 23.92 | 19.42 | 30.20 | 9.86 | 28.54 | 11.25 | 1.83 | 0.80 | 0.75 | - |
| **Sc** | 10.16 | 4.07 | 9.56 | 2.96 | 6.06 | 3.06 | 0.73 | 0.27 | - | - |
| **Liver** | 5.38 | 1.12 | 3.62 | 0.80 | 2.16 | 0.19 | 0.48 | 0.21 | 0.42 | 0.10 |
| **muscles** | 0.30 | 0.07 | - | - | - | - | - | - | - | - |
| **pancreas** | 1.38 | 0.65 | 0.70 | 0.22 | - | | - | - | - | - |
| **lungs** | 1.70 | 0.48 | 0.88 | 0.17 | 0.59 | 0.02 | - | - | - | - |
| **spleen** | 3.11 | 0.97 | 1.29 | 0.39 | 0.87 | 0.27 | - | - | - | - |
| **kidney** | 3.07 | 0.64 | 1.27 | 0.19 | 0.77 | 0.15 | - | - | - | - |
| **blood** | 0.75 | 0.15 | 0.54 | 0.21 | 0.42 | 0.09 | - | - | - | - |
| **thyroid** | 21.00 | 15.49 | - | - | - | - | 40.73 | 45.83 | 38.07 | 76.44 |
| **uvea** | 3.83 | 0.62 | 4.69 | 3.01 | 3.51 | 0.60 | 3.45 | 1.87 | 1.96 | 1.14 |
| **GB** | 43.71 | - | - | - | 12.79 | - | - | - | - | - |

Table 16: Biodistribution compound **129** (dose=2.04 MBq)

| | 1H | | 3H | | 6H | | 24H | | 72H | |
|---|---|---|---|---|---|---|---|---|---|---|
| | Mean | Standard deviation | Mean | Standard deviation | Mean | Standard deviation | Mean | Standard deviation | Mean | Standard deviation |
| **B 16** | **0.95** | **0.43** | **1.55** | **0.11** | **1.25** | **0.29** | **1.19** | **0.25** | **1.05** | **0.39** |
| **brain** | 0.12 | 0.15 | 0.03 | - | - | - | - | - | - | - |
| **Ic** | 43.18 | 34.20 | 57.76 | 45.94 | 68.46 | 45.27 | 2.82 | 1.02 | - | - |
| **Sc** | 10.52 | 6.03 | 3.49 | 0.88 | 3.85 | 2.19 | 1.17 | 0.51 | 0.89 | - |
| **liver** | 8.88 | 1.04 | 4.96 | 0.50 | 4.95 | 0.35 | 1.53 | 0.31 | 1.11 | 0.08 |
| **muscles** | 0.84 | 0.24 | 0.48 | 0.12 | 0.44 | 0.14 | - | - | - | - |
| **pancreas** | 3.06 | 0.19 | 2.50 | - | 2.96 | 1.14 | - | - | - | - |
| **lungs** | 3.43 | 0.29 | 2.91 | 0.29 | 2.47 | 0.31 | 0.69 | 0.03 | - | - |
| **spleen** | 6.28 | 0.96 | 7.36 | 5.03 | 4.12 | 0.52 | 1.65 | 0.29 | 0.66 | - |
| **kidney** | 13.82 | 4.09 | 8.65 | 3.11 | 9.85 | 4.55 | 3.57 | 2.06 | 0.47 | 0.24 |
| **blood** | 0.53 | 0.12 | 0.24 | 0.05 | 0.24 | 0.05 | - | - | - | - |
| **thyroid** | - | - | - | - | 34.81 | 19.33 | 3.00 | - | 7.65 | - |
| **uvea** | 1.80 | 0.51 | 1.45 | - | 1.44 | 0.40 | 1.45 | 0.33 | 2.09 | 0.64 |
| **GB** | 20.19 | | 44.60 | 11.20 | 166.43 | 162.30 | - | - | - | - |

Table 17 : Biodistribution compound **142** (dose = 1.50 MBq)

| | 1H | | 3H | | 6H | | 24H | | 72H | |
|---|---|---|---|---|---|---|---|---|---|---|
| | **Mean** | **Standard deviation** | **Mean** | **Standard deviation** | **Mean** | **Standard-deviation** | **Mean** | **Standard-deviation** | **Mean** | **Standard-deviation** |
| **B 16** | **23.22** | 4.26 | **13.87** | 2.71 | **11.42** | 3.02 | **8.37** | 2.08 | **3.31** | 1.02 |
| **brain** | 0.35 | 0.05 | - | - | - | - | - | - | - | - |
| **lc** | 32.83 | 29.89 | 47.65 | 29.30 | 43.48 | 20.11 | 0.82 | - | - | - |
| **Sc** | 17.58 | 5.69 | 9.76 | 3.78 | 9.17 | 2.81 | 0.62 | - | 2.13 | - |
| **liver** | 3.92 | 0.83 | 2.27 | 0.25 | 1.49 | 0.31 | - | - | - | - |
| **muscle** | 0.86 | 0.15 | 0.43 | 0.20 | 0.24 | 0.07 | - | - | - | - |
| **pancreas** | 1.78 | 0.09 | - | - | - | - | - | - | - | - |
| **ungs** | 2.36 | 0.31 | 1.87 | 0.28 | 1.26 | 0.12 | - | - | - | - |
| **spleen** | 5.38 | 5.84 | 0.93 | - | - | - | - | - | - | - |
| **kidney** | 4.01 | 1.78 | 1.51 | 0.20 | 1.20 | 0.07 | - | - | - | - |
| **blood** | 2.21 | 0.31 | 1.88 | 0.21 | 1.36 | 0.06 | - | - | - | - |
| **thyroid** | 20.16 | 14.46 | 41.44 | 5.23 | 56.95 | 50.29 | 115.66 | 60.68 | - | - |
| **uvea** | 24.1 | 3.35 | 20.30 | 2.36 | 13.15 | 5.74 | 10.00 | 5.91 | 13.06 | 7.12 |
| **GB** | - | - | 41.51 | 20.52 | 28.67 | 10.68 | - | - | - | - |

Table 18 : Biodistribution compound **144** (dose = 1.09 MBq)

| | 1H | | 3H | | 24H | | 72H | |
|---|---|---|---|---|---|---|---|---|
| | Mean | Standard deviation | Mean | Standard-deviation | Mean | Standard-deviation | Mean | Standard-deviation |
| **B 16** | **14.69** | 3.82 | **44.92** | 8.05 | **37.46** | 9.04 | **24.02** | 7.83 |
| **brain** | 0.75 | 0.12. | 0.46 | 0.10 | - | - | - | - |
| **lc** | 27.28 | 10.15 | 29.12 | 15.08 | - | - | - | - |
| **Sc** | 24.11 | 9.79 | 34.86 | 19.88 | - | - | - | - |
| **liver** | 12.84 | 0.70 | 7.84 | 0.76 | - | - | - | - |
| **muscle** | - | - | 0.79 | 0.10 | - | - | - | - |
| **pancreas** | 23.88 | 2.52 | 13.06 | 4.65 | - | - | - | - |
| **lungs** | 5.81 | 0.62 | 4.91 | 0.71 | - | - | - | - |
| **spleen** | 18.70 | 12.36 | 6.08 | 0.96 | - | - | - | - |
| **kidney** | 24.42 | 1.80 | 17.73 | 5.71 | - | - | - | - |
| **blood** | 1.85 | 0.18 | 1.61 | 0.30 | - | - | - | - |
| **thyroid** | - | - | 60.78 | - | 44.74 | - | - | - |
| **uvea** | 28.75 | 15.47 | 41.75 | 14.40 | 32.62 | 7.44 | 23.28 | 9.66 |
| **GB** | - | - | - | - | - | - | - | - |

Table 19: Biodistribution compound **151** (dose= 2.1 MBq)

| | 1H | | 3H | | 6H | | 24H | | 72H | |
|---|---|---|---|---|---|---|---|---|---|---|
| | **Mean** | **Standard deviation** | **Mean** | **Standard deviation** | **Mean** | **Standard-deviation** | **Mean** | **Standard-deviation** | **Mean** | **Standard-deviation** |
| **B 16** | **20.96** | 2.45 | **23.65** | 2.08 | **27.70** | 6.08 | **11.47** | 3.67 | **6.26** | 2.18 |
| **brain** | 1.79 | 0.07 | 0.44 | 0.11 | - | - | - | - | - | - |
| **lc** | 56.33 | 51.00 | 52.72 | 34.17 | 52.58 | 28.94 | 2.30 | 1.57 | - | - |
| **Sc** | 11.25 | 3.96 | 14.52 | 6.14 | 16.09 | 7.94 | 2.95 | 1.28 | - | - |
| **liver** | 10.94 | 1.15 | 5.83 | 0.44 | 4.04 | 0.73 | 1.44 | 0.18 | - | - |
| **muscle** | 1.27 | 0.30 | 0.45 | 0.11 | 0.42 | - | - | - | - | - |
| **pancreas** | 6.63 | 1.40 | 1.53 | 0.05 | - | - | - | - | - | - |
| **lungs** | 8.77 | 2.62 | 2.84 | 0.46 | 1.40 | 0.17 | - | - | - | - |
| **spleen** | 14.02 | 7.40 | 13.84 | 8.93 | 18.88 | 3.12 | - | - | - | - |
| **kidney** | 9.45 | 1.53 | 3.42 | 0.52 | 1.55 | 0.22 | - | - | - | - |
| **blood** | 3.92 | 0.60 | 1.39 | 0.38 | - | - | - | - | - | - |
| **thyroid** | - | - | 8.63 | - | 54.71 | 21.06 | 214.42 | | 12.74 | 4.09 |
| **uvea** | 14.19 | 2.86 | 17.49 | 3.09 | 14.74 | 5.73 | 11.64 | 3.34 | 11.19 | 4.07 |
| **GB** | - | - | 107.29 | 41.74 | - | - | - | - | - | - |

Several molecules exhibit high tumour concentrations which are much higher than that of *N*-(2-diethylarninoethyl)-4-iodobenzamide (BZA), the reference compound already mentioned above. These values are particularly high for the compounds **26, 31, 46, 49, 84, 102, 144** and **151,** this being the case from the first hour with respective concentrations of 14.8, 19.6, 17, 19.1, 12.6, 18.8, 14.7 and 21% of the injected dose per gram of tumour (1D/g) whereas, for BZA, this value is 9.5% 1D/g.

These tumour concentrations are much higher in comparison with the other organs and reflect a specific affinity for the melanoma, in particular with respect to the organs which are potentially the site of metastases. These data are illustrated in Figure 1 respectively at 3 hours (A) and at 72 hours (B) after the administration of 10 compounds **21, 26, 31, 46, 49, 84, 102, 142, 144** and **151,** in comparison with $^{125}$I-BZA.

For example, the compound **21** exhibits a novel behaviour as, while its tumour concentration is slightly less than that of BZA, the concentration is particularly specific (**Table 6** reported above, **Figure 1**). The product disappears very rapidly from the nontarget organs, which is in favour of use in imaging, this being the case even within a short period of time after the injection. The compound **142** is also noteworthy in this application, with a very high tumour concentration from one hour.

Furthermore, the compounds **26, 31, 46, 49, 84, 102, 144** and **151** are retained in the tumour with the greatest persistance with, at 72 hours, concentrations respectively 6, 8, 16, 14, 22, 9, 31 and 8 times higher than with BZA (**Figure 1B**).

**EXAMPLE 38: Elimination of the molecules**

For this study, the two mice monitored up to 72 hours are kept in metabolic cages in order to collect the urine and faeces for counting and determination of the cumulative urinary and faecal excretions (**Table 20**).

**Table 20**: Urinary and faecal elimination 0-72 hours

| | Urine | Faeces | Total |
|---|---|---|---|
| **BZA** | 83.1% | 4.8% | 87.9% |
| **3** | 43.7% | 26.7% | 70.6% |
| **7** | 67.3% | 5.3% | 72.6% |
| **10** | 21.5% | 61.7% | 83.2% |
| **13** | 52.6% | 46.7% | 99.3% |
| **16** | 52.6% | 24.0% | 76.6% |
| **21** | 36.8% | 43.5% | 80.3% |
| **26** | 17.1% | 40.7% | 57.8% |
| **31** | 80.7% | 11.4% | 91.2% |
| **35** | 23.8% | 69.2% | 93.0% |
| **39** | 49.9% | 21.2% | 71.1% |
| **46** | 70.2% | 18.0% | 88.2% |
| **49** | 67.2% | 19.9% | 87.1% |
| **84** | 10.4% | 38.4% | 48.8% |
| **102** | 52.8% | 46.1% | 98.9% |
| **111** | 9.5% | 23.2% | 31.7% |
| **129** | 4.20% | 33.2% | 37.4% |
| **142** | 68.9% | 29.4% | 98.3% |
| **144** | 45.7% | 34.8% | 80.5% |
| **151** | 5.8% | 61% | 66.8% |

The elimination from the body of the compounds studied is highly disparate first in terms of kinetics. It is found that, for some molecules, the elimination kinetics are complete at 72 hours (e.g.: compound **13** or compound **102**). If,

for the majority of the compounds, significant elimination is observed at 72 hours (>70%), a lower elimination was measured in the case of the molecules **26**, **84**, **111** and **129**. This fact, for the compound **84**, is in agreement with the strong tumour retention demonstrated.

Furthermore, in terms of elimination route, a great disparity also exists according to the compounds. Some exhibit, like BZA, elimination predominantly in the urine; this is the case more particularly of the compounds **7**, **31** and **46**. The two routes are about equivalent for **13** and **102**. On the other hand, elimination by the faecal route predominates for the compounds **10**, **35** or **151** and for the compound **84**, which is the slowest eliminated.

## EXAMPLE 39: Dosimetry

The dosimetry parameters with regard to the tumour were evaluated, from the experimental data of biodistribution of each molecule, using the MIRD programme and extrapolated to the case of use of the molecules labelled with $^{131}I$. The results given in **Table 21** below show for several novel compounds, due to their kinetics, an increased potentiality with respect to BZA in terms of dose delivered to the tumour and in particular with the compounds **26, 31, 46, 49, 84, 102** and **151** (× 3, 3.7, 4.9, 5.8, 8.4, 3.9 and 3.7 respectively).

**Table 21**

| | Biological period hours | Effective period hours | Dose absorbed cGy / μCi injected |
|---|---|---|---|
| **BZA** | 19.6 | 17.8 | 1.00 |
| 3 | 24.0 | 21.4 | 1.63 |
| 7 | 19.0 | 17.3 | 1.58 |
| 10 | 20.1 | 18.2 | 0.89 |
| 13 | 26.7 | 23.5 | 1.72 |
| 16 | 7.8 | 7.5 | 0.13 |
| 21 | 52.6 | 41.3 | 1.38 |
| **26** | **42.9** | **35.1** | **3.05** |
| **31** | **38.0** | **31.8** | **3.67** |
| 35 | 66.3 | 49.4 | 1.36 |
| 39 | 56.3 | 43.6 | 1.86 |
| **46** | **66.1** | **49.3** | **4.93** |
| **49** | **69.7** | **51.2** | **5.75** |
| **84** | **268.9** | **112.5** | **8.37** |
| **102** | **43.3** | **35.4** | **3.91** |
| **111** | **nd** | **nd** | **nd** |
| **129** | **162.3** | **88.3** | **0.49** |
| **142** | **30.2** | **26.1** | **3.56** |
| **144** | **nd** | **nd** | **nd** |
| **151** | **35.5** | **30.0** | **3.70** |
| nd = not determined | | | |

## EXAMPLE 40: Antitumour effectiveness

37.1. Antitumour effectiveness of the compound **31** by systemic administration after labelling with $^{131}I$:

The study relates to 20 C57B16 mice bearing a melanoma grafted subcutaneously by injection of 50 000 B16 F0 cells (0.1 ml). On the 17th day, the animals are weighed and the tumours are measured in two dimensions (L, 1), the tumour volume being expressed by $L \times 1^2 \times 1/2$. On the 18t day (D18), the mice are divided into two groups, the

labelled compound [$^{131}$I]-**31** with a high specific activity is administered by the i.v. route (37 MBq; 0.2 ml) to 10 mice and 10 constitute the control batch. The monitoring of the animals is carried out with all the necessary radioprotection measures and in particular the removal of the contaminated bedding, the animals arc weighed and the tumours are measured every two days. Taking into account the volume of the tumours and ethical considerations, the surviving animals were euthenized on the 20th day after injection, i.e. 38 days after the tumour graft. Tumour growth is markedly slowed down in the treated animals and the difference from the controls, significant from the 6th day after the single administration, persists throughout the duration of the study. The evaluation of the effectiveness of an i.v. administration of [$^{131}$I]-**31** on tumour growth of C57BL6 mice which have received a subcutaneous graft of B 16 melanoma 18 days before treatment is represented in **Figure 2**. The times on the abscissa are given with respect to the day of inoculation of the tumours and with respect to the day of treatment. Under these conditions, a significant difference regarding the median of survival was not demonstrated.

37.2. Antitumour effectiveness of the compound **46** by systemic administration after labelling with $^{131}$I :

Administered with regard to the same model, in two doses of 18.5 MBq respectively 6 and 10 days after induction of the tumours, that is to say with regard to lesions which are less developed, growth is clearly slowed down. The evaluation of the effectiveness of an i.v. administration of [$^{131}$I]-**46** on tumour growth of C57BL6 mice which have received a subcutaneous graft of B16 melanoma is represented in **Figure 3**. The times on the abscissa are given with respect to the day of inoculation of the tumours and with respect to the first day of treatment. Under these conditions, the median of survival is extended and changes from 32 days for the control animals to 39 days for the treated animals. This experiment was repeated according to the same protocol and fully superimposable results were obtained. Unquestionably, effectiveness of the treatment is definitely demonstrated and should be made clear by other models and protocols.

**EXAMPLE 41 : Determination of the cytotoxicity of the compounds of the invention**

The compounds of the invention have formed the subject of a cytotoxicity study on murine melanoma (B16 $F_0$), human melanoma (M4Beu) and human fibroblast cell lines in comparison with DACA and with amsacrine, according to the Hoechst test.

*Experimental protocol:* the human fibroblasts were purchased from Biopredic International (Rennes, France). The M4Beu melanoma line originates from the laboratory of Dr. J. F. Doré (INSERM, Unit 218, Lyons, France). The B16 Fo, M4Beu and fibroblast lines are cultured in 75 $cm^2$ dishes comprising 12 ml of Eagle's essential medium with Earle's salts and Glutamax (Gibco-BRL, Paisley, Scotland) supplemented with 10% foetal calf serum, a solution of vitamins at 1X (Gibco), 1 mM sodium pyruvate (Gibco), a solution of nonessential amino acids at 1X (Gibco) and 4 $\mu$g/ml of gentamicin (antibiotic).

The cells are maintained at 37°C in an incubator under an atmosphere comprising 5% $CO_2$.

The cells ($5 \times 10^3$) in 150 $\mu$l of culture medium are inoculated in 96-well plates (Nunclon™, Nunc, Roskilde, Denmark). The plates are incubated for 16 hours (attachment of the cells) before their treatment. The stock solutions (200 X) are prepared with DMSO and are then stored at -20°C. 50 $\mu$l of a solution comprising stock solution and culture medium are subsequently added to the various wells (taking into account the different dilutions). Each test is carried out in triplicate.

After incubating for 48 hours, the plates are turned over on an absorbent paper and then frozen at -80 °C. The amount of cellular DNA is subsequently measured by the Hoechst test. The plates are then defrosted at ambient temperature for 10 minutes. 100 $\mu$l of a 0.01 % (w/v) solution of SDS (sodium dodecyl sulphate) in sterile distilled water are subsequently added using a microvolume dispenser. The plates (96 well) are incubated with stirring at ambient temperature for 30 minutes and are then frozen at -80 °C for one hour. The plates are subsequently defrosted at ambient temperature for 20 minutes. 100 $\mu$l of a solution of Hoechst 33 342 (30 $\mu$g/ml) in TNE 2X (10 mM tris-HCl, 1 mM EDTA, 2 M NaCl, pH = 7.4) are subsequently added. The plates are then incubated for one hour at ambient temperature, with stirring and with the exclusion of light. The fluorescence obtained is measured using a Fluoroskan 96-well at the excitation wavelength at 360 nm and omission wavelength at 460 nm. Under these conditions, the fluorescence is proportional to the cell biomass in each well. The percentage of survival is defined by the fluorescence in each well (treated) with respect to the fluorescence of the control wells (drug-free), the blanks being subtracted (drug-free and cell-free wells).

The $IC_{50}$ values ($\mu$M) for the compounds of the invention are summarized in the following **Table 22**, in comparison with DACA and with amsacrine.

Table 22

| Compound | M4Beu | B 16 | Fibroblast |
|---|---|---|---|
| **DACA** | 1.8 | 0.19 | 7.1 |
| **amsacrine** | 0.40 | 0.035 | 2.0 |
| **55** | 4.3 | 2.0 | 2.7 |
| **60** | 3.7 | 1.5 | 1.9 |
| **65** | 30 | 13 | 56 |
| **69** | 4.1 | 3.0 | 3.7 |
| **78** | 3.6 | 1.7 | 2.8 |
| **84** | 4.1 | 3.6 | 3.2 |
| **87** | 3.5 | 5.1 | 9.5 |
| **91** | 2.4 | 1.1 | 2.2 |
| **95** | 2.3 | 0.28 | 1.8 |
| **98** | 44 | 23 | nd |
| **102** | 0.79 | 0.44 | 0.71 |
| **107** | 3.5 | 2.1 | 2.2 |
| **111** | 3.9 | 2.2 | 3.3 |
| **115** | 3.1 | 1.2 | 3.0 |
| **120** | 28 | 9.5 | nd |
| **122** | 5.6 | 21 | 3.9 |
| **125** | 2.0 | 1.0 | 2.0 |
| **127** | 2.3 | 0.45 | 0.78 |
| **129** | 0.68 | 1.0 | 0.91 |
| **137** | 2.4 | 1.0 | 1.4 |
| **151** | 3.6 | 3.6 | 2.7 |
| nd = not determined | | | |

Except for the compound **65**, the acridonc derivatives **55** to **87** exhibit a mean cytotoxicity of the order of 3 μM without obvious specificity for the melanoma cells.

In the case of the iodinated analogues of DACA **91-122**, the compound **102**, with a greater activity than the parent compound with regard to the various cell lines studied, exhibits a noteworthy profile.

Among the structures of amsacrine type **125** to **137**, it is the compound **129** which exhibits the best effectiveness with regard to the M4Beu human cells, close to the values obtained for amscrine.

Finally, for the phenazine derivative **151**, activities similar to that of DACA were observed.

**EXAMPLE 42: *in vitro* study of the chemotherapeutic and radiotherapeutic effects of the compounds 84, 102 and 151:**

An *in vitro* study was carried out in order to evaluate the concept of radiotherapy by irradiation of the Auger electron ($^{125}I$) and to study the chemotherapeutic effects of the compounds **84, 102** and **151**. The colony forming technique is involved, that is to say the growth of colonies of clones starting from tumour cells (B16 $F_0$ murine melanoma cells) inoculated at a low concentration. This method makes it possible to visualize the cytotoxic and cytostatic properties of the compounds studied. The study was carried out in three parts :

1/ determination of the effects/doses range of the cold compound

2/ determination of the effects/doses range of the compound labelled with iodine-125 with a high specific activity

(HSA) in comparison with $Na^{125}I$ (without effect under these conditions)

3/ study of the overall activity of a moderately active dose of cold product in combination with a range of radiotoxic doses of the same product labelled with iodine-125.

The experiment is carried out on 6-well plates where 200 cells are inoculated in 2 ml of DMEM medium. After 20 hours, the medium is withdrawn and replaced with 2 ml of medium comprising the labelled compound at the desired concentration. This study is carried out in comparison with control wells comprising DMEM medium alone. After 48 hours of contact, the medium is withdrawn and 2 ml of DMEM medium are added. The plates are incubated for 8 days and then the medium is withdrawn. The wells are rinsed with PBS and methanol is added for 3 minutes. Once attached, the cells are coloured with crystal violet with a contact time of 3 minutes. Subsequently, the colonies comprising more than 50 cells are counted using a dedicated counter. The growth of the treated colonies of cells is quantified in comparison with the controls. The activity of the product is expressed by a percentage of inhibition :

$$100-[(\text{number of treated colonies})/(\text{number of control colonies})] \times 100$$

The results obtained are summarized in the following Tables 23 to 31 :

**Table 23 (Chemotherapeutic effects alone)**

| Concentration cold compound **84** | 47 nM | 187.5 nM | 750 nM | 3.0 μM |
|---|---|---|---|---|
| % of inhibition | 8 | 11 | 25 | 100 |

**Table 24 (Radiotherapeutic effects alone)**

| Activity compound [$^{125}$I]-**84** | 1.5 kBq | 6 kBq | 12 kBq | 24 kBq | 48 kBq |
|---|---|---|---|---|---|
| Concentration of the compound [$^{125}$I]-**84** | 9.31 pM | 37.23 pM | 74.5 pM | 148.9 pM | 297.8 pM |
| % of inhibition | 4.5 | 12.4 | 29 | 30 | 69 |

**Table 25 (Radiotherapeutic and chemotherapeutic effects)**

| Activity compound [$^{125}$I]-**84** | 0 | 1.5 kBq | 6 kBq | 12 kBq | 24 kBq | 48 kBq |
|---|---|---|---|---|---|---|
| Cumulative effect * (% inhib) | 17.3 | 18.3 | 22 | 40.3 | 54 | 88 |
| *Cumulative effect: Cold compound **84** at the dose of 0.75 μM with labelled [$^{125}$I]-**84** with a high specific activity. | | | | | | |

**Table 26 (Chemotherapeutic effects alone)**

| Concentration cold compound **102** | 3.125 nM | 6.25 nM | 12.5 nM | 25 nM | 50 nM |
|---|---|---|---|---|---|
| % of inhibition | 9.5 | 21 | 40.5 | 78.5 | 89.9 |

Table 27 (Radiotherapeutic effects alone)

| Activity compound [$^{125}$I]-**102** | 1.39 kBq | 2.78 kBq | 5.55 kBq | 11.1 kBq | 16.7 kBq | 22.2 kBq |
|---|---|---|---|---|---|---|
| Concentration of the compound [$^{125}$I]-**102** | 8.625 pM | 17.25 pM | 34.5 pM | 69 pM | 103.5 pM | 138 pM |
| % of inhibition | 11 | 14.3 | 40.3 | 65.5 | 79 | 96.1 |

**Table 28 (Radiotherapeutic and chemotherapeutic effects)**

| Activity compound [$^{125}$I]-**102** | 0 | 1.39 kBq | 2.78 MBq | 5.55 kBq | 11.1 kBq | 16.7 kBq |
|---|---|---|---|---|---|---|

(continued)

| | | | | | | |
|---|---|---|---|---|---|---|
| Cumulative effect * (% inhib) | 18.3 | 30 | 37.5 | 59.5 | 76.5 | 89 |
| *Cumulative effect: cold compound **102** at a dose of 6.25 nM with labelled [$^{125}$I]-**102** with a high specific activity. | | | | | | |

**Table 29 (Chemotherapeutic effects alone)**

| Concentration cold compound **151** | 2 μM | 4 μM | 6 μM | 8 μM | 10 μM |
|---|---|---|---|---|---|
| % of inhibition | 21 | 74 | 87 | 97 | 100 |

**Table 30 (Radiotherapeutic effects alone)**

| Activity compound [$^{125}$I]-**151** | 1.5 kBq | 3 kBq | 6 kBq | 12 kBq | 24 kBq | 48 kBq | 96 kBq |
|---|---|---|---|---|---|---|---|
| Concentration of the compound [$^{125}$I]-**151** | 9.31 pM | 18.6 pM | 37.2 pM | 74.5 pM | 148.9 pM | 297.8 pM | 595.6 pM |
| % of inhibition | 7 | 21 | 25 | 28 | 36 | 45 | 74 |

**Table 31 (Radiotherapeutic and chemotherapeutic effects)**

| Activity compound [$^{125}$I]-**151** | 0 | 1.5 kBq | 3 kBq | 6 kBq | 12 kBq | 24kBq | 48 kBq | 96 kBq |
|---|---|---|---|---|---|---|---|---|
| Cumulative effect * (% inhib) | 19 | 28 | 29 | 32 | 39 | 40 | 49 | 82 |
| *Cumulative effect: cold compound **151** at the dose of 2 μM with labelled [$^{125}$I]-**151** with a high specific activity. | | | | | | | | |

A dose/effect study on the compounds **84, 102** and **151** was carried out, on the one hand with the cold products and, on the other hand, with the labelled products with a high specific activity (**Tables 23 and 24, 26 and 27, and 29 and 30**). It is apparent, with regard to the study model, which takes into account the inhibition of growth, that the activity of the cold compound **102** is much greater in comparison with the derivatives **84** and **151**, with a more marked difference than that observed during the acute cytotoxicity study (from 15 to 40×). For the group of the labelled compounds with an HSA, the activity observed is clearly related to a radiotoxicity mechanism as the ranges of concentrations differ by a factor of between 300 and $10^5$.

The third part of the *in vitro* study, in which a specific dose with an inhibiting effect of approximately 20% is combined with increasing concentrations of labelled compound with an HSA, clearly shows the effects to be additive (**Tables 25,28 and 31**).

According to another of its aspects, the present invention relates to a radiopharmaceutical composition comprising, as active principle, a compound of formula (I) wherein $R_2$ and $R_3$ represent, independently of one another, a ($C_1$-$C_6$)alkyl group or a ($C_1$-$C_6$)alkenyl group or of formula (I'), (I"), (Ia), (Ib), (Ic), (Id), (Ie), (If), (Ig), (Ih), (Ii), (Ij), (Ik), (Im), (In), (Io), (Ip), (Iq) or (Ir) or a compound of formula (II) comprising a radionuclide according to the invention or one of its pharmaceutically acceptable salts.

The said radiopharmaceutical composition advantageously comprises an effective amount of such a compound of formula (I), (I'), (I"), (Ia), (Ib), (Ic), (Id), (Ie), (If), (Ig), (Ih), (Ii), (Ij), (Ik), (Im), (In), (Io), (Ip), (Iq) or (Ir) or of a compound of formula (II) comprising a radionuclide, and also one or more excipients. Such excipients are chosen according to the type of formulation.

The present invention also relates to a product chosen from a labelled compound of formula (Ip), (Ir), (Ii), (In); (Ih), (Im), (Ig), (Ik), (Io) or (Iq) or a labelled compound of formula (II) or one of its pharmaceutically acceptable salts for use in a

radiopharmaceutical composition intended for medical imaging and in particular for the diagnosis of melanomas.

The present invention also relates to a product chosen from a labelled compound of formula (Ic), (If), (Ij), (Ib), (Ie), (Ia), (Id) or (I"), or a labelled compound of formula (II) or one of its pharmaceutically acceptable salts for use in a radiopharmaceutical composition intended for treatment of melanomas.

Lastly, a subject-matter of the present invention is a noninvasive method for the determination of the tissue

distribution of tumour cells of melanomas on the human body, comprising the stages of at least one injection of a radiopharmaceutical composition comprising at least one compound of formula (I), (I'), (I"), (Ia), (Ib), (Ic), (Id), (Ie), (If), (Ig), (Ih), (Ii), (Ij), (Ik), (Im), (In), (Io), (Ip), (Iq) or (Ir) or one compound of formula (II) comprising a radionuclide or one of its pharmaceutically acceptable salts and of at least one determination of the concentration of the radioactivity.

## Claims

1. Compound of formula (I):

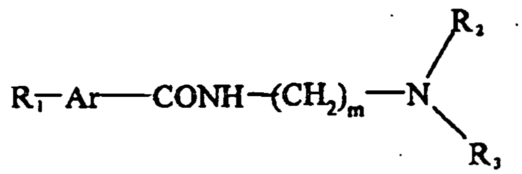

(I)

in which
$R_1$ represents a radionuclide,
Ar is a heteroaryl group,
m is an integer varying from 2 to 4,
$R_2$ and $R_3$ represent, independently of one another, a hydrogen atom, a $(C_1-C_6)$alkyl group or a $(C_1-C_6)$alkenyl group, in which the heteroaryl group is a 5- or 6-membered aromatic ring comprising 1 or 2 nitrogen atoms or a bi- or tricyclic aromatic nucleus comprising from 1 to 4 nitrogen atoms or comprising a sulphur atom, at least one of the rings of which has 6 ring members, the other fused ring or rings having 5 or 6 ring members, it being possible for the said heteroaryl group to be monosubstituted by:

- an optionally labelled halogen atom,
- a $(C_1-C_4)$alkoxy group,
- a $(C_1-C_4)$alkyl group,
- an oxo group or
- an anilino group which can itself be substituted by 1 to 3 groups which can be chosen from a $(C_1-C_4)$alkyl or $(C_1-C_4)$alkoxy group, a hydroxyl group, a halogen atom or an $NHR^e$ group where $R^e$ represents a hydrogen, or a $COR^a$ group, a $COOR^a$ group or an $SO_2R^a$ group, where $R^a$ represents an aryl group or a $(C_1-C_{10})$alkyl group optionally substituted by an oxo group;

and in which $R_1$ is bonded to the aromatic nucleus as such or, when the substituent of the aromatic nucleus is an anilino group, $R_1$ can be bonded to the phenyl group of the anilino group,
and in which Ar is chosen from a pyridyl, phenazinyl, naphthyridinyl, indolyl, imidazopyridyl, benzimidazolyl, quinolyl, quinolonyl, isoquinolyl, quinoxalinyl,
benzothienyl, acridinyl or acridonyl group, it being possible for the said group to be monosubstituted by a methyl group, a methoxy group or an optionally labelled halogen atom, and an acridiyl group substituted by an anilino group which can itself be substituted by 1 to 3 groups which can be chosen from a $(C_1-C_4)$alkyl or $(C_1-C_4)$alkoxy group, a hydroxyl group, a halogen atom or an $NHR^c$ group where $R^e$ represents a hydrogen, or a $COR^a$ group, a $COOR^a$ group or an $SO_2R^a$ group, where $R^a$ represents an aryl group or a $(C_1-C_{10})$alkyl group optionally substituted by an oxo group,
and their addition salts with pharmaceutically acceptable acids, for use in a radiopharmaceutical composition in the diagnosis and/or treatment of melanoma.

2. Compound for use according to Claim 1, **characterized in that** Ar is a bi- or tricyclic aromatic nucleus and the $R_1$ group is bonded to one of these rings and the group

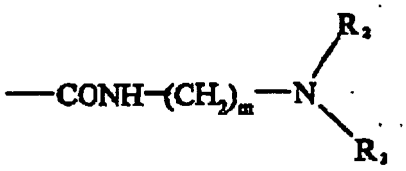

is bonded to the other ring or to one of the other rings constituting the bi- or tricyclic group.

**3.** Compound for use according to any one of the preceding claims, characteized in that alternatively:

- the $R_1$ group is in the para position with respect to the group

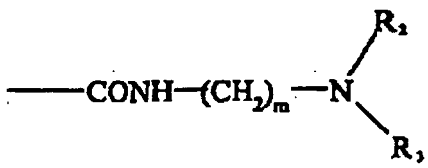

when Ar comprises only one ring and
in that the $R_1$ group is bonded to one of the rings and the group

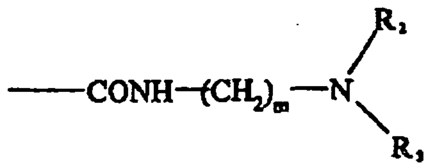

is bonded to the other ring or to one of the other rings when Ar is a bi- or tricycle, or
- Ar is a bi- or tricyclic heteroaryl and in that $R_1$ is bonded to the ring, taken in isolation, not comprising a heteroatom or comprising the least thereof and the group

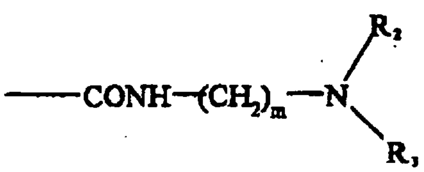

is bonded to another ring comprising the greater number of heteroatom(s).

**4.** Compound of formula (I')

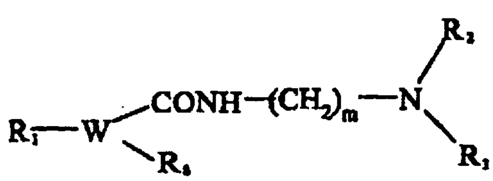

(I')

in which
W is chosen from a phenazinyl, imidazopyridyl, quinolyl, quinoxalinyl, acridinyl and acridonyl group,
it being possible for the said acridinyl group to be substituted by an aniline group itself substituted by three groups,

- at least one of the substituents representing the ($C_1$-$C_4$)alkoxy group,
- at least one of the substituents being chosen from an $NHR^c$ group where $R^c$ represents a hydrogen, or a $COR^a$ group, a $COOR^a$ group or an $SO_2R^a$ group, where $R^a$ represents an aryl group or a ($C_1$-$C_{10}$)alkyl group optionally substituted by an oxo group, and
- the remaining substituent representing a hydrogen or halogen atom,

$R_1$, $R_2$, $R_3$ and m have the same meaning as that defined in any one of Claims 1 to 3, and
$R_8$ represents a hydrogen atom, a ($C_1$-$C_4$)alkyl or ($C_1$-$C_4$)alkoxy group, an optionally labelled halogen atom, an -SH group, an -OH group or an $-NR_5R_6$ group where $R_5$ and $R_6$ can independently represent a hydrogen atom or a ($C_1$-$C_4$)alkyl group,
for use in a composition in the treatment of melanoma.

**5.** Compound for use in according to any one of the preceding claims, **characterized in that** the radionuclide is a radioisotope chosen from $^{113}I$, $^{124}I$, $^{125}I$, $^{131}I$, $^{75}Br$, $^{76}Br$, $^{77}Br$, $^{18}F$, $^{210}At$ and $^{211}At$ and is in particular $^{123}I$, $^{124}I$, $^{125}I$, or $^{131}I$.

**6.** Compound of formula (II)

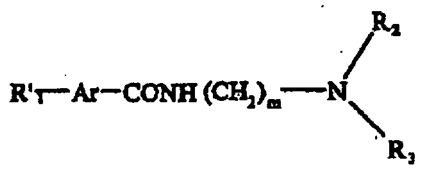

(II)

in which

R'$_1$ represents a labelled halogen atom,

m has the same meaning as that defined in Claim 1,
$R_2$ and $R_3$ represent, independently of one another, a ($C_1$-$C_6$)alkyl or a ($C_1$-$C_6$)alkenyl group, and
Ar is chosen from the pyridyl, indolyl, imidazopyridinyl, benzimidazolyl, quinolyl, quinolonyl, isoquinolyl, quinoxalinyl, naphthyridinyl and benzothienyl group.

**7.** Compound of formula (I")

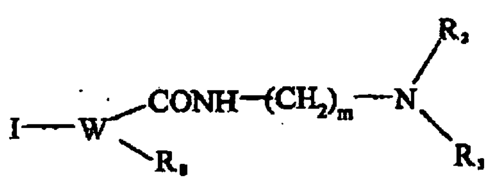

(I")

in which
$R_2$, $R_3$ and m are as defined according to Claim 1, $R_8$ and W are as defined in Claim 4 and the iodine atom, being optionally a $^{123}I$, $^{124}I$, $^{125}I$ or $^{131}I$.

**8.** Compound chosen from :

- *N*-(2-diethylaminoethyl)-6-iodonicotinamide dihydrochloride;
- *N*-(2-diethylaminoethyl)-5-iodoindole-2-carboxamide hydrochloride;
- *N*-(2-diethylaminoethyl)-4-iodobenzo[*b*]thiophene-2-carboxamide hydrochloride;
- *N*-(2-diethylaminoethyl)-3-iodoimidazo[1,2-*a*]pyridine-2-carboxamide dihydrochloride;
- *N*-(2-diethylaminoethyl)-6-iodoimidazo[1,2-*a*]pyridine-2-carboxamide dihydrochloride;
- *N*-(2-diethylaminoethyl)-5-iodobenzimidazole-2-carboxamide dihydrochloride;
- *N*-(2-diethylaminoethyl)-6-iodoquinoline-2-carboxamide dihydrochloride;
- *N*-(2-diethylaminoethyl)-1,4-dihydro-6-iodo-4-oxoquinoline-3-carboxamide hydrochloride;

- *N*-(2-diethylaminoethyl)-5-iodoisoquinolino-3-carboxamide dihydrochloride;
- *N*-(2-diethylaminoethyl)-6-iodoquinoxaline-2-carboxamide dihydrochloride;
- *N*-(4-dipropylaminobutyl)-6-iodoquinoline-2-carboxamide dihydrochloride;
- *N*-(2-diethyllaminoethyl)-9,10-dihydro-1-iodo-9-oxacridine-4-carboxamide hydrochloride;
- *N*-(2-diethylaminoethyl)-9,10-dihydro-2-iodo-9-oxoacridine-4-carboxamide hydrochloride;
- *N*-(2-diethylaminoethyl)-9,10-dihydro-3-iodo-9-oxoacridine-4-carboxamide hydrochloride:
- *N*-(2-diethylaminoethyl)-9,10-dihydro-5-iodo-9-oxoacridine-4-carboxamide hydrochloride;
- *N*-(2-diethylaminoethyl)-9,10-dihydro-6-iodo-9-oxoacridine-4-carboxamide hydrochloride;
- *N*-(2-(diethylaminoethyl)-9,10-dihydro-7-iodo-9-oxoacridine-4-carboxamide hydrochloride;
- *N*-(2-diethylaminoethyl)-9,10-dihydro-8-iodo-9-oxoacridine-4-carboxamide hydrochloride;
- *N*(2-diethylaminoethyl)-1-iodoacridine-4-carboxamide dihydrochloride;
- *N*-(2-diethylaminoethyl)-2-iodoacridine-4-carboxamide dihydrochloride;
- *N*-(2-diethylaminoethyl)-3-iodoacridine-4-carboxamide dihydrochloride;
- *N*-(2-diethylaminoethyl)-5-iodoacridine-4-carboxamide dihydrochloride ;
- *N*-(2-diethylaminoethyl)-6-iodoacridine-4-carboxamide dihydrochloride;
- *N*-(2-diethylaminoethyl)-7-iodoacridine-4-carboxamide dihydrochloride;
- *N*-(2-diethylaminoethyl)-8-iodoacridine-4-carboxamide dihydrochloride;
- *N*-(2-diethylaminoethyl)-2-iodoacridine-9-carboxamide dihydrochloride:
- *N*-(2-diethylaminoethyl)-2,7-diiodoacridine-9-carboxamide dihydrochloride;
- *N*-(2-diethylaminoethyl)-2-iodo-9-(4-methanesulphonamido-2-methoxyanilino)acridine-4-carboxamide dihydrochloride;
- *N*-(2-diethylaminoethyl)-5-iodo-9-(4-methanesulphonamido-2-methoxyanilino)acridine-4-carboxamide dihydrochloride;
- *N*-(2-diethylaminoethyl)-7-iodo-9-(4-methanesulphonamido-2-methoxyanilino)acridine-4-carboxamide dihydrochloride;
- *N*-(2-diethylaminoethyl)-9-(5-iodo-4-methanesulphonamido-2-methoxyanilino)acridine-4-carboxamide dihydrochloride;
- *N*-(2-diethylaminoethyl)-8-iodo-1,6-naphthyridine-2-carboxamide dihydrochloride:
- *N*-(4-dipropylaminobutyl)-6-iodoquinoxaline-2-carboxamide dihydrochloride;
- *N*-(2-diethylaminoethyl)-7-iodophenazine-1-carboxamide dihydrochloride;
- and their pharmaceutically acceptable salts.

**9.** Process for the preparation of the compound of formula (I") according to Claim 7 or of the compound of formula (II) according to Claim 6, **characterized in that** it consists in condensing an ester of formula (III)

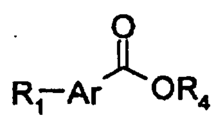

(III)

with a diamine of formula (IV)

$H_2N-(CH_2)_m-NR_2R_3$ (IV)

in which $R_2$, $R_3$, m and $R_1$ are as defined in Claim 1 and $R_4$ represents a $(C_1-C_6)$alkyl, aryl or heteroaryl group.

**10.** Compound of formula (VII)

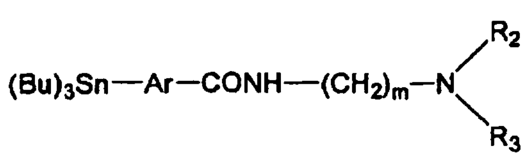

(VII)

in which
Ar, m, $R_2$ and $R_3$ are as defined in Claim 1 to 3.

**11.** Radiopharmaceutical composition comprising, as active principle, a compound of formula (I), wherein $R_2$ and $R_3$ represent, independently of one another, a $(C_1-C_6)$alkyl group or a $(C_1-C_6)$alkenyl group or of formula (I'), (I") or (II) or one of its pharmaceutically acceptable salts as defined according to any one of Claims 1 to 8.

**12.** Compound of formula (I") as defined according to Claim 7 or compound of formula (II) or of one of its pharmaceutically acceptable salts according to Claim 6, the said compound comprising a radionuclide, for use in a radiopharmaceutical composition in medical imaging.

**13.** Compound for use according to claim 12, wherein the radiopharmaceutical composition is intended for the diagnosis of melanomas.

**14.** Compound (I") as defined according to Claim 7 or compound of formula (II) or of one of its pharmaceutically acceptable salts according to Claim 6 for use in a radiopharmaceutical composition in the treatment of melanomas.

**15.** Process for the preparation of a compound of formula (VI).

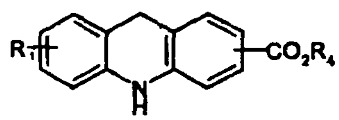

in which $R_1$ is a halogen atom and $R_4$ represents a $(C_1-C_4)$alkyl, aryl or heteroaryl group, of use as synthetic intermediate in the preparation of the compounds of formula (I') in which W is an acridonyl group, **characterized in that** it comprises a stage of reduction of the acridone of formula (IIIa)

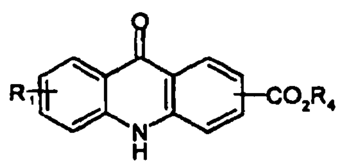

in which $R_1$ and $R_4$ are as defined above, in the presence of a complexing agent.

## Patentansprüche

**1.** Verbindung der Formel (I):

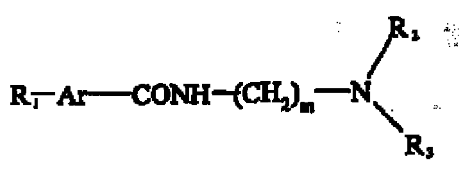 (I)

worin $R^1$ ein Radionuklid darstellt,
Ar eine Heteroarylgruppe ist,
m eine ganze Zahl von 2 bis 4 ist,
$R_2$ und $R_3$ unabhängig voneinander ein Wasserstoffatom, eine $(C_1-C_6)$-Alkylgruppe oder eine $(C_1-C_6)$-Alkenylgruppe darstellen, wobei die Heteroarylgruppe ein 5- oder 6-gliedriger aromatischer Ring, der 1 oder 2 Stickstoffatome enthält, oder ein bi- oder tricyclischer aromatischer Kern ist, der 1 bis 4 Stickstoffatome oder ein Schwefelatom enthält, wobei mindestens einer der Ringe 6 Ringmitglieder aufweist, wobei der andere fusionierte Ring bzw. die anderen fusionierten Ringe 5 oder 6 Ringmitglieder aufweist bzw. aufweisen, wobei die Heteroarylgruppe mit einem der folgenden Elemente monosubstituiert sein kann:

- ein gegebenenfalls markiertes Halogenatom,

- eine ($C_1$-$C_4$)-Alkoxygruppe,
- eine ($C_1$-$C_4$)-Alkylgruppe
- eine Oxogruppe oder
- eine Anilingruppe, die ihrerseits mit 1 bis 3 Gruppen substituiert sein kann, die unter einer ($C_1$-$C_4$)-Alkylgruppe oder einer ($C_1$-$C_4$)-Alkoxygruppe, einer Hydroxygruppe, einem Halogenatom oder einer $NHR^e$-Gruppe, wobei $R^e$ ein Wasserstoffatom darstellt, oder einer $COR^a$-Gruppe, einer $COOR^a$-Gruppe oder einer $SO_2R^a$-Gruppe ausgewählt ist, wobei $R^a$ eine Arylgruppe oder eine gegebenenfalls mit einer Oxogruppe substituierte $C_1$-$C_{10}$)-Alkylgruppe darstellt;

und worin $R^1$ an den aromatischen Kern als solchen gebunden ist oder, wenn der Substituient des aromatischen Kerns eine Anilingruppe ist, $R^1$ an die Phenylgruppe der Anilingruppe gebunden sein kann,
und worin Ar unter einer Pyridyl-, Phenyzinyl-, Naphthyridinyl-, Indolyl-, Imidazopyridyl-, Benzimidazolyl-, Chinolyl-, Chinolonyl-, Isochinolyl-, Chinoxalinyl-, Benzothienyl-, Acridinyl- oder Acridonylgruppe, wobei die Gruppe mit einer Methylgruppe, Methoxygruppe oder einem gegebenenfalls markierten Halogenatom monosubstituiert sein kann, und einer Acridinylgruppe ausgewählt ist, die mit einer Anilingruppe substituiert ist, die ihrerseites mit 1 bis 3 Gruppen substituiert sein kann, die unter einer ($C_1$-$C_4$)-Alkylgruppe oder einer ($C_1$-$C_4$)-Alkoxygruppe, einer Hydroxygruppe, einem Halogenatom oder einer $NHR^e$-Gruppe, wobei $R^e$ ein Wasserstoffatom darstellt, oder einer $COR^a$-Gruppe, einer $COOR^a$-Gruppe oder einer $SO_2R^a$-Gruppe ausgewählt ist, wobei $R^a$ eine Arylgruppe oder eine ($C_1$-$C_{10}$)-Alkylgruppe ist, gegebenenfalls mit einer Oxogruppe substituiert ist,
und ihre Additionssalze mit pharmazeutisch geeigneten Säuren, für die Verwendung in einer radiopharmazeutischen Zusammensetzung in der Diagnose und/oder der Behandlung von Melanomen.

2. Verbindung für die Verwendung nach Anspruch 1, **dadurch gekennzeichnet, dass** Ar einen aromatische Kern bi- oder tricyclisch ist, die $R^1$-Gruppe an einen dieser Ringe gebunden ist und die Gruppe

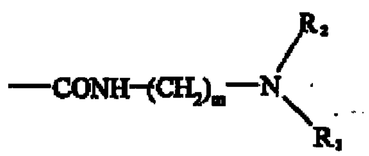

an den anderen Ring oder an einen der anderen Ringe der bi- oder tricyclischen Gruppe gebunden ist.

3. Verbindung für die Verwendung nach einem der vorstehenden Ansprüche, **dadurch gekennzeichnet, dass** alternativ

- die $R^1$-Gruppe in der para-Position in Bezug auf die Gruppe

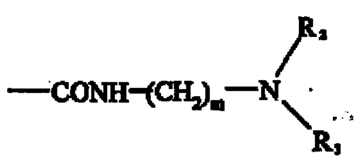

ist, wenn Ar nur einen Ring enthält, und
dadurch, dass die $R_1$-Gruppe an einen der Ringe gebunden ist und
die Gruppe

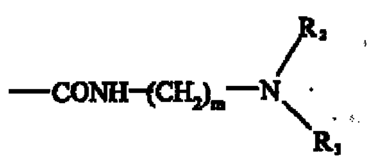

an den anderen Ring oder an einen der anderen Ringe gebunden ist, wenn Ar bi- oder tricyclisch ist, oder

- Ar eine bi- oder tricyclische Heteroarylgruppe ist und $R_1$ an den Ring gebunden ist, der isoliert betrachtet kein Heteroatom aufweist oder die geringste Anzahl aufweist und die Gruppe

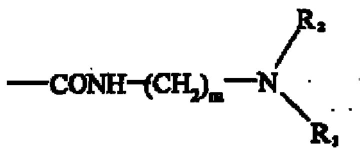

an einen anderen Ring gebunden ist, der die größere Anzahl an Heteroatomen enthält.

**4.** Verbindung der Formel (I')

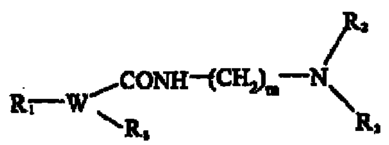

(I')

worin
W aus einer Phenazinyl-, Imidazopyridyl-, Chinolyl-, Chinoxalinyl-, Acridinyl- und Acridonylgruppe ausgewählt ist, wobei die Acridinylgruppe mit einer Anilingruppe substituiert sein kann, die ihrerseits mit drei Gruppen substituiert ist,

- wobei mindestens einer der Substituenten die $(C_1-C_4)$-Alkxoygruppe darstellt,
- wobei mindestens einer der Substituenten unter einer $NHR^e$-Gruppe, wobei $R^e$ ein Wasserstoffatom darstellt, oder einer $COR^a$-Gruppe, einer $COOR^a$-Gruppe oder einer $SO_2R^a$-Gruppe ausgewählt ist, wobei $R^a$ eine Arylgruppe oder eine $(C_1-C_{10})$-Alkylgruppe darstellt, die gegebenenfalls mit einer Oxogruppe substituiert ist, und
- wobei der verbleibende Substituent Wasserstoff oder ein Halogenatom darstellt,

$R_1$, $R_2$, $R_3$ und m dieselbe Bedeutung wie in einem der Ansprüche 1 bis 3 haben, und
$R_8$ ein Wasserstoffatom, eine $(C_1-C_4)$-Alkylgruppe oder eine $(C_1-C_4)$-Alkoxygruppe, ein gegebenenfalls markiertes Halogenatom, eine -SH-Gruppe, eine -OH-Gruppe oder eine $NR_5R_6$-Gruppe darstellt, wobei $R_5$ und $R_6$ unabhängig voneinander ein Wasserstoffatom oder eine $(C_1-C_4)$-Alkylgruppe darstellen, für die Verwendung in einer Zusammensetzung zur Behandlung.von Melanomen.

**5.** Verbindung für die Verwendung nach einem der vorstehenden Ansprüche, **dadurch gekennzeichnet, dass** das Radionuklid ein Radioisotop ist, das unter $^{123}I$, $^{124}I$, $^{125}I$, $^{131}I$, $^{75}Br$, $^{76}Br$, $^{77}Br$, $^{18}F$, $^{210}At$ und $^{211}At$ ausgewählt ist und es insbesondere $^{123}I$, $^{124}I$, $^{125}I$ oder $^{131}I$ ist.

**6.** Verbindung der Formel (II)

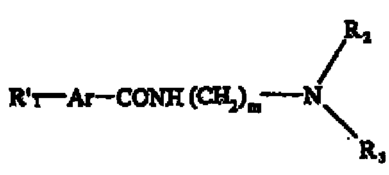

(II)

worin

R'$_1$ ein markiertes Halogenatom darstellt,
m dieselbe Bedeutung wie in Anspruch 1. hat,
$R_2$ und $R_3$ unabhängig voneinander eine $(C_1-C_6)$-Alkylgruppe oder eine $(C_1-C_6)$-Alkenylgruppe darstellen und
Ar unter einer Pyridyl-, Indoxyl-, Imidazopyridinyl-, Benzimidazolyl-, Chinolyl-, Chinolonyl-, Isochinolyl-, Chinoxalinyl-, Naphthyridinyl- und Benzothienylgruppe ausgewählt ist.

**7.** Verbindung der Formel (I")

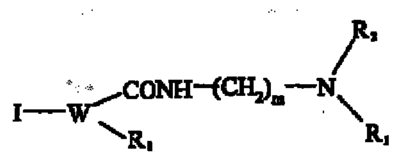

(I")

worin $R_2$, $R_3$ und m dieselbe Bedeutung wie in Anspruch 1 haben, $R_8$ und W wie in Ansrpuch 4 definiert sind und das Iodatom gegebenenfalls $^{123}I$, $^{124}I$, $^{125}I$ oder $^{131}I$ ist.

**8.** Unter folgenden Verbindungen ausgewählte Verbindung:

- N-(2-Diethylaminoethyl)-6-iodnicotinamiddihydrochlorid;
- N-(2-Diethylaminoethyl)-5-iodindol-2-carboxamidhydrochlorid;
- N-(2-Diethylaminoethyl)-4-iodbenzo[b]thiophen-2-carboxamidhydrochlorid;
- N-(2-Diethylaminoethyl)-3-iodimidazo[1,2-a]-pyridin-2-carboxamiddihydrochlorid;
- N-(2-Diethylaminoethyl)-6-iodimidazo[1,2-a]-pyridin-2-carboxamiddihydrochlorid;
- N-(2-Diethylaminoethyl)-5-iodbenzimidazol-2-carboxamiddihydrochlorid;
- N-(2-Diethylaminoethyl)-6-iodchinolin-2-carboxamiddihydrochlorid;
- N-(2-Diethylaminoethyl)-1,4-dihydro-6-iod-4-oxochinolin-3-carboxamidhydrochlorid;
- N-(2-Diethylaminoethyl)-5-iodisochinolin-3-carboxamid-dihydrochlorid;
- N-(2-Diethylaminoethyl)-6-iodchinoxalin-2-carboxamid-dihydrochloride;
- N-(4-Dipropylaminobutyl)-6-iodchinolin-2-carboxamiddihydrochloride;
- N-(2-Diethylaminoethyl)-9,10-dihydro-1-iod-9-oxoacridin-4-carboxamidhydrochlorid;
- N-(2-Diethylaminoethyl)-9,10-dihydro-2-iod-9-axoacridin-4-carboxamidhydrochlorid;
- N-(2-Diethylaminoethyl)-9,10-dihydro-3-iod-9-oxoacridin-4-carboxamidhydrochlorid;
- N-(2-Diethylaminoethyl)-9,10-dihydro-5-iod-9-oxoacridin-4-carboxamidhydrochlorid;
- N-(2-Diethylaminoethyl)-9,10-dihydxo-6-iod-9-oxoacridin-4-carboxamidhydrochlorid;
- N-(2-Diethylaminoethyl)-9,10-dihydro-7-iod-9-oxoacridin-4-carboxamidhydrochlorid;
- N-(2-Diethylaminoethyl)-9,10-.dihydro-8-iod-9-oxoacridin-4-carboxamidhydrochlorid;
- N-(2-Diethylaminoethyl)-1-iodacridin-4-carboxamiddihydrochlorid;
- N-(2-Diethylaminoethyl)-2-iodacridin-4-carboxamiddihydrochloride;
- N-(2-Diethylaminoethyl)-3-iodacridin-4-carboxamiddihydrochlorid;
- N-(2-Diethylaminoethyl)-5-iodacridin-4-carboxamiddihydrochlorid;
- N-(2-Diethylaminoethyl)-6-iodacridin-4-carboxamiddihydrochlorid;
- N-(2-Diethylaminoethyl)-7-iodacridin-4-carboxamiddihydrochlorid;
- N-(2-Diethylaminoethyl)-8-iodacridin-4-carboxamiddihydrochlorid;
- N-(2-Diethylaminoethyl)-2-iodacridin-9-carboxamiddihydrochlorid;
- N-(2-Diethylaminoethyl)-2,7-diiodacridin-9-carboxamid-dihydrochlorid;
- N-(2-Diethylaminoethyl)-2-iod-9-(4-methansulfonamido-2-methoxyanilino)acridin-4-carboxamiddihydrochlorid;
- N-(2-Diethylaminoethyl)-5-iod-9-(4-methansulfonamido-2-methoxyanilino)acridin-4-carboxamiddihydrochlorid;
- N-(2-Diethylaminoethyl)-7-iod-9-(4-methansulfonamido-2-methoxyanilino)acridin-4-carboxamiddihydrochlorid;
- N-(2-Diethylaminoethyl)-9-(5-iod-(4-methansulfonamido-2-methoxyanilino)acridin-4-carboxamiddihydrochlorid;
- N-(2-Diethylaminoethyl)-8-iod-1,6-naphthyridin-2-carbox amiddihydrochlorid;
- N-(4-Dipropylaminobutyl)-6-iodchinoxalin-2-carboxamid-dihydrochloride;
- N-(2-Diethylaminoethyl)-7-iodphenazin-1-carboxamiddihydrochlorid;

und ihre pharmazeutisch geeigneten Salze.

**9.** Verfahren zum Herstellen einer Verbindung der Formel (I") nach Anspruch 7 oder der Verbindung der Formel (II) nach Anspruch 6, durch gekennzeichnet, dass es das Kondensieren eines Esters der Formel (III)

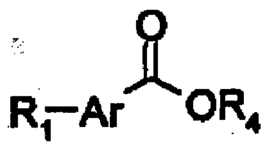

(III)

mit einem Diamin der Formel (IV)

$H_2N-(CH_2)_m-NR_2R_3$ (IV)

umfasst, worin $R_2$, $R_3$, m und $R_1$ wie in Anspruch 1 definiert sind und $R_4$ eine ($C_1$-$C_6$)-Alkyl-, Aryl- oder Heteroarylgruppe darstellt.

**10.** Verbindung der Fornmel (VII)

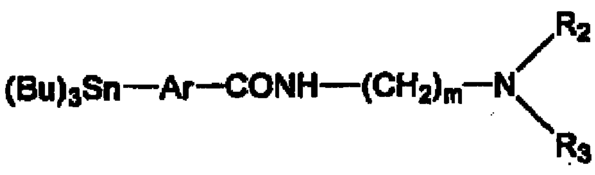

(VII)

worin Ar, m, $R_2$ und $R_3$ wie in Anspruch 1 bis 3 definiert sind.

**11.** Radiopliarmazeutische Zusammensetzung, die als aktiven Hauptbestandteil eine Verbindung der Formel (I), worin $R_2$ und $R_3$ unabhängig voneinander eine ($C_1$-$C_1$)-Alkylgruppe oder eine ($C_1$-$C_6$) - Alkenylgruppe darstellen, oder der Formel (I'), (I") oder (II) oder eines ihrer pharmazeutisch geeigneten Salze nach einem der Ansprüche 1 bis 8 umfasst.

**12.** Verbindung der Formel (I") nach Anspruch 7 oder Verbindung der Formel (II) oder eines ihrer pharmazeutisch geeigneten Salze nach Anspruch 6, wobei die Verbindung ein Radionuklid enthält, für die Verwendung in einer radiopharmazeutischen Zusammensetzung für medizinische Bildgebung.

**13.** Verbindung für die Verwendung nach Anspruch 12, wobei die radiopharmazeutische Zusammensetzung für die Diagnose von Melanomen vorgesehen ist.

**14.** Verbindung (I") nach Anspruch 7 oder Verbindung der Formel (II) oder eines ihrer pharmazeutisch geeigneten Salze nach Anspruch 6 für die Verwendung in einer radiopharmazeutischen Zusammensetzung für die Behandlung von Melanomen.

**15.** Verfahren zur Herstellung einer Verbindung der Formel (VI)

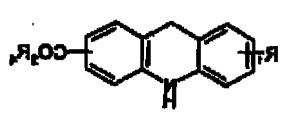

worin $R_1$, ein Halogenatom ist und $R_4$ eine($C_1$-$C_4$)-Alkyl-, Aryl- oder Heteroarylgruppe ist, für die Verwendung als synthetisches Intermediat in der Herstellung der Verbindungen der Formel (I'), wobei W eine Acridonylgruppe ist, dadurch-gekennzeichnet, dass es eine Stufe umfasst, in der das Acridon der Formel (IIIa)

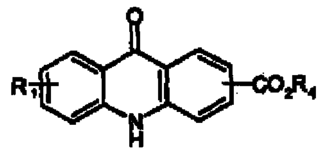

worin $R_1$ und $R_4$ wie vorstehend definiert sind, in Anwesenheit eines Komplexierungsmittels reduziert wird.

## Revendications

**1.** Composé de formule (I):

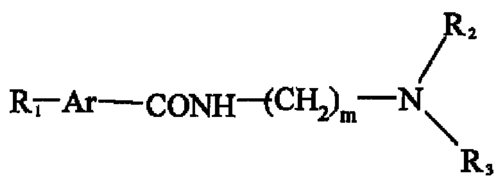

(I)

dans laquelle
$R_1$ représente un radionucléide,
Ar représente un groupe hétéroaryle,
m est un entier variant de 2 à 4,
$R_2$ et $R_3$ représentent, indépendamment l'un de l'autre, un atome d'hydrogène, un groupe ($C_1$-$C_6$)alkyle ou un groupe ($C_1$-$C_6$)alkényle,
le groupe hétéroaryle étant un cycle aromatique à 5 ou 6 chaînons comprenant 1 ou 2 atomes d'azote ou un noyau aromatique bi- ou tricyclique comprenant de 1 à 4 atomes d'azote ou comprenant un atome de soufre, et dont au moins l'un des cycles est formé de 6 chaînons, l'autre ou les autres cycle(s) fusionné(s) étant formé(s) de 5 ou 6 chaînons, ledit groupe hétéroaryle pouvant être monosubstitué par :

- un atome d'halogène éventuellement marqué,
- un groupe ($C_1$-$C_4$)alcoxy,
- un groupe ($C_1$-$C_4$)alkyle,
- un groupe oxo ou,
- un groupe anilino pouvant lui-même être substitué par 1 à 3 groupes pouvant être choisis parmi un groupe ($C_1$-$C_4$)alkyle, ($C_1$-$C_4$)alcoxy, un groupe hydroxy, un atome d'halogène ou un groupe $NHR^e$ dans lequel $R^e$ représente un hydrogène ou un groupe $COR^a$, un groupe $COOR^a$ ou un groupe $SO_2R^a$, dans lesquels $R^a$ représente un groupe aryle ou un groupe ($C_1$-$C_{10}$) alkyle optionnellement substitué par un groupe oxo; et dans lequel $R_1$ est lié au noyau aromatique en tant que tel ou, lorsque le substituant du noyau aromatique est un groupe anilino, $R_1$ peut être lié au groupe phényle du groupe anilino,
- et dans lequel Ar est choisi parmi le groupe pyridyle, phenazinyle, naphthyridinyle, indolyle, imidazopyridyle, benzimidazolyle, quinolyle, quinolonyle, isoquinolyle, quinoxalinyle, benzothienyle, acridinyle ou acridonyle, ledit groupe pouvant être monosubstitué par un groupe méthyle, un groupe méthoxy ou un atome d'halogène éventuellement marqué, et un groupe acridinyle substitué par un groupe aniline pouvant lui-même être substitué par 1 à 3 groupes pouvant être choisis parmi un groupe ($C_1$-$C_4$)alkyle ou ($C_1$-$C_4$)alcoxy, un groupe hydroxy, un atome d'halogène ou un groupe $NHR^e$ dans lequel $R^e$ représente un hydrogène ou un groupe $COR^a$, un groupe $COOR^a$ ou un groupe $SO_2R^a$, dans lesquels $R^a$ représente un groupe aryle ou un groupe ($C_1$-$C_{10}$) alkyle optionnellement substitué par un groupe oxo ;

ainsi que leurs sels d'addition à des acides pharmaceutiquement acceptable, pour leur utilisation dans une composition radiopharmaceutique dans le diagnostic et/ou le traitement du mélanome.

**2.** Composé pour son utilisation conforme à la revendication 1, **caractérisé en ce que Ar** est **un noyau aromatique** bi- ou tricyclique et **en ce que** le groupe $R_1$ est lié à l'un de ces cycles et le groupe

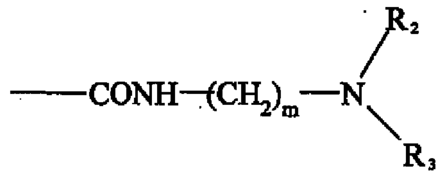

est lié à un autre cycle ou à l'un des autres cycles constituant le groupe bi- ou tricyclique.

**3.** Composé pour son utilisation selon l'une quelconque des revendications précédentes, **caractérisé en ce que** alternativement :

- le groupe R1 est en position para du groupe

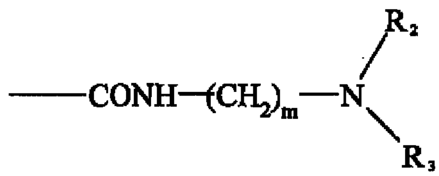

lorsque Ar ne comporte qu'un cycle et
**en ce que** le groupe $R_1$ est lié à l'un des cycles et le groupe

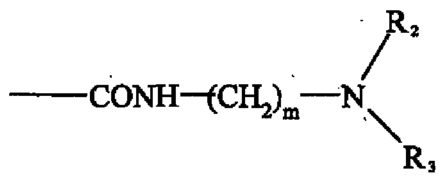

est lié à l'autre cycle ou à l'un des autres cycles lorsque Ar est un bi-ou tricycle, ou
- Ar est un hétéroaryle bi- ou tri-cyclique et **en ce que** R1 est lié au cycle, pris isolément, ne comprenant pas d'hétéroatome ou comprenant le moins et le groupe

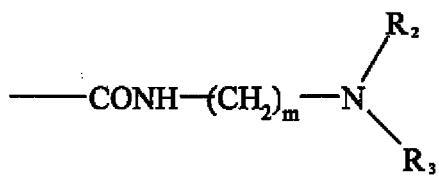

est lié à un autre cycle comprenant le ou les hétéroatome(s) en plus grand nombre.

**4.** Composé de formule (I')

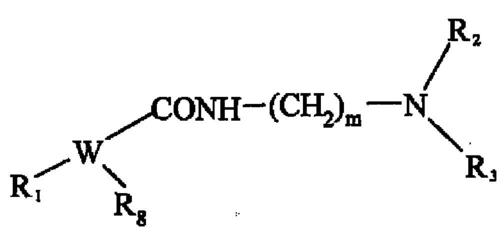

(I')

dans lequel
W est choisi parmi un groupe phenazinyle, imidazopyridyle, quinolyle, quinoxalinyle, acridinyle et acridonyle,

étant possible pour ledit groupe acridinyle d'être substitué par un groupe anilino lui-même substitué par 3 groupes,

- au moins l'un des substituants représentant un groupe ($C_1$-$C_4$)alkoxy,
- au moins l'un des substituants étant choisi par un groupe $NHR^e$ dans lequel $R^e$ représente un hydrogène ou un groupe $COR^a$, un groupe $COOR^a$ au un groupe $S0_2R^a$, dans lesquels $R^a$ représente un groupe aryle ou un groupe ($C_1$-$C_{10}$)alkyle optionnellement substitué par un groupe oxo ; et
- le substituant restant représentant un atome d'hydrogène ou d'halogéne,

R1, R2, R3 et m étant tels que définis dans l'une quelconques des revendications 1 à 3, et R8 représente un atome d'hydrogène, un groupe ($C_1$-$C_4$)alkyle ou ($C_1$-$C_4$)alkoxy, un atome d'halogène optionnellement marqué, un groupe -SH, un groupe -OH ou un groupe - NR5R6 où R5 et R6 peuvent indépendemment représenter un atome d'hydrogène ou un groupe ($C_1$-$C_4$)alkyle,
pour son utilisation dans une composition dans le traitement du mélanome.

**5.** Composé pour son utilisation selon l'une quelconque des revendications précédentes, **caractérisée en ce que** le radionucléide est un radioisotope choisi parmi $^{123}$I $^{124}$I, $^{125}$I, $^{131}$1, $^{75}$Br, $^{76}$Br, $^{77}$Br, $^{18}$F, $^{210}$At et $^{211}$A et est en particulier $^{123}$I, $^{124}$I, $^{125}$I ou $^{131}$I.

**6.** Composé de formule (II)

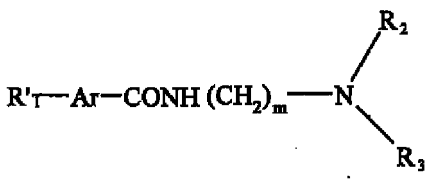

(II)

dans laquelle

R'$_1$ représente un atome d'halogène marqué,

m a la même signification que celle définie en revendication 1,
$R_2$ et $R_3$ représentent, indépendemment l'un de l'autre, un groupe ($C_1$-$C_6$)alkyle ou un groupe ($C_1$-$C_6$)alkényle, et Ar est choisi parmi le groupe pyridyle, benzothiényle, indolyle, quinolyle, quinolonyle, isoquinolyle, quinoxalinyle, benzimidazolyle, naphtyridinyle et imidazopyridinyle,

**7.** Composé de formule (I")

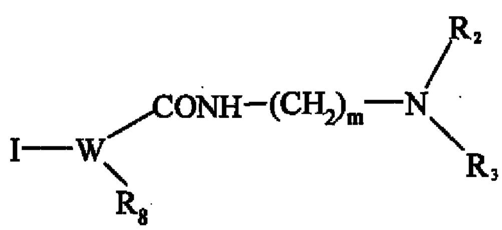

(I")

dans lequel
R2, R3 et m sont tels que définis en revendication 1, R8 et W sont tels que définis en revendication 4 et l'atome d'iode étant optionnellement un $^{123}$I, $^{124}$I, $^{125}$I ou $^{131}$I.

**8.** Composé choisi parmi :

- *Dichlorhydrate de N-(2-diéthylaminoéthyl)-6-iodonicotinamide;*
- *Chlorhydrate de N-(2-diéthylaminoéthyl)-5-iodoindole-2-carboxamide;*
- *Chlorhydrate de N-(2-diéthylaminoéthyl)-4-iodobenzo[b]thiophène-2-carboxamide;*
- *Dichlorhydrate de N-(2-diéthylaminoéthyl)-3-iodoimidazo[1,2-α]pyridine-2-carboxamide;*

- *Dichlorhydrate de N-(2-diéthylaminoéthyl)-6-iodoimidazol[1,2-a]pyridïne-2-carboxamide;*
- *Dichlorhydrate de N-(2-diéthylaminoéthyl)-5-iodobenzimidazole-2-carboxamide;*
- *Dichlorhydrate de N-(2-diéthylaminoéthyl)-6-iodoquinoléine-2-carboxamide;*
- *Chlorhydrate de N-(2-diéthylaminoéthyl)-1,4-dihydro-6-iodo-4-oxoquinoléine-3-carboxamide;*
- *Dichlorhydrate de N-(2-diéthylaminoéthyl)-5-iodoisoquinoléine-3-carboxamide;*
- *Dichlorhydrate de N-(2-diéthylaminoéthyl)-6-iodoquinoxaline-2-carboxamide ;*
- *Dichlorhydrate du N-(4-dipropylaminobutyl)-6-iodoquinaléine-2-carboxamide;*
- *Chlorhydrate de N-(2-diéthylaminoéthyl)-9,10-dihydro-1-iodo-9-oxoacridine-4-carboxamide;*
- *Chlorhydrate de N-(2,diéthylaminoéthyl)-9,10-dihydro-2-iodo-9-oxoacridine-4-carboxamide;*
- *Chlorhydrate de N-(2-diéthylaminoéthyl)-9,10-dihydro-3-iodo-9-oxoacridine-4-carboxamide;*
- *Chlorhydrate de N-(2-diéthylaminaéthyl)-9,10-dihydro-5-iodo-9-oxoacridine-4-carboxamide;*
- *Chlorhydrate de N-(2-diéthylaminoéthyl)-9,10-dihydro-6-iodo-9-oxoacridine-4-carboxamide;*
- *Chlorhydrate de N-(2-diéthylaminoéthyl)-9,10-dihydro-7-iodo-9-oxoacridïne-4-carboxamide;*
- *Chlorhydrate de N-(2-diéthylaminoéthyl)-9,10-dihydro-8-iodo-9-oxoacridine-4-carboxamide;*
- *Dichlorhydrate N-(2-diéthylaminoéthyl)-1-iodoacridine-4-carboxamide;*
- *Dichlorhydrate N-(2-diéthylaminoéthyl)-2-iodoacridine-4-carboxamide;*
- *Dichlorhydrate N-(2-diéthylaminoéthyl)-3-iodoacridine-4-carboxamide;*
- *Dichlorhydrate N-(2-diéthylaminoéthyl)-5-iodoacridine-4-carboxamide;*
- *Dichlorhydrate N-(2-diéthylaminoéthyl)-6-iodoacridine-4-carboxamide;*
- *Dichlorhydrate N-(2-diéthylaminoéthyl)-7-iodoacridine-4-carboxamide;*
- *Dichlorhydrate N-(2-diéthylaminoéthyl)-8-iodoacridine-4-carboxamide;*
- *Dichlorhydrate N-(2-diéthylaminoéthyl)-2-iodoacridine-9-carboxamide*
- *Dichlorhydrate de N-(2-diéthylaminaéthyl)-2,7-diiodoacridine-9-carboxamide;*
- *Dichlorhydrate de N-(2-diéthylaminoéthyl)-2-iodo-9-(4-méthanesulfonamido-2-méthoxyanilino)acridine-4-carboxamide ;*
- *Dichlorhydrate de N-(2-diéthylaminoéthyl)-5-iodo-9-(4-méthanesulfonamido-2-méthoxyanilino)acridine-4-carboxamide;*
- *Dichlorhydrate de N-(2-diéthylaminoéthyl)-7-iodo-9-(4-méthanesulfonamido-2-méthoxyanilino)acridine-4-carboxamide;*
- *Dichlorhydrate de N-(2-diéthylaminoéthyl)-9-(5-iodo-4-méthanesulfonamido-2-méthoxyanilino)acridine-4-carboxamide;*
- *Dichlorhydrate de N-(2-diéthylaminoéthyl)-8-iodo-1,6-naphthyridine-2-carboxamide;*
- *Dichlorhydrate de N-(4-dipropylaminobutyl)-6-iodoquinoxaline-2-carboxamide;*
- *Dichlorhydrate de N-2(-diethylaminoethyl)-7-iodophenazine-1-carboxamide;*
- ainsi que leurs sels pharmaceutiquement acceptables.

**9.** Procédé de préparation du composé de formule (I") selon la revendication 7 ou du composé de formule (II) selon la revendication 6, **caractérisé en ce qu'**il consiste à condenser un ester.de formule (III)

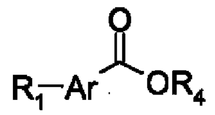

(III)

avec une diamine de formule (IV)

$$H_2N\text{-}(CH_2)_m\text{-}NR_2R_3 \qquad \text{(IV)}$$

dans lesquelles $R_2$, $R_3$, m et $R_1$ sont tels que définis en revendication 1 et $R_4$ représente un groupe ($C_1$-$C_6$)alkyle, aryle ou hétéroaryle.

**10.** Composé de formule (VII)

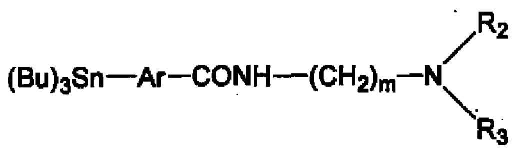

(VII)

dans laquelle
Ar, m, $R_2$ et $R_3$ sont tels que définis en revendications 1 à 3.

**11.** Composition radiopharmaceutique comprenant à titre de principe actif un composé de formule (I), dans lequel R2 et R3 représentent, indépendemment l'une de l'autre, un groupe ($C_1$-$C_6$)alkyle ou un groupe ($C_1$-$C_6$)alkenyle ou un composé de formule (I'), (I") ou (II) ou l'un de ses sels pharmaceutiquement acceptable, tel que défini dans l'une quelconque des revendications 1 à 8.

**12.** Composé de formule (1") tel que défini en revendication 7 ou composé de formule (II) ou l'un de ses sels pharmaceutiquement acceptable selon la revendication 6, ledit composé comprenant un radionucléide, pour son utilisation dans une composition radiopharmaceutique dans l'imagerie médicale.

**13.** Composé pour son utilisation selon la revendication 12, **caractérisé en ce que** la composition radiopharmaceutique est destinée au diagnostic des mélanomes.

**14.** Composé de formule (I") tel que défini selon la revendication 7, ou composé de formule (II) ou l'un de ses sels pharmaceutiquement acceptable selon la revendication 6, pour son utilisation dans une composition radiopharmaceutique dans le traitement des mélanomes.

**15.** Procédé de préparation d'un composé de formule (VI)

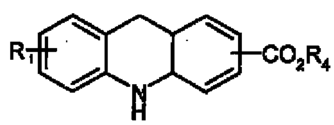

dans laquelle $R_1$ est un atome d'halogène et $R_4$ représente un groupe ($C_1$-$C_4$)alkyle, aryle ou hétéroaryle utile en tant qu'intermédiaire de synthèse dans la préparation des composés de formule (I') dans lequel W est un groupe acridonyle,
**caractérisé en ce qu'**il comprend une étape de réduction de l'acridone de formule (IIIa)

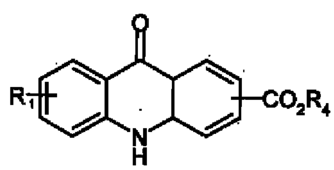

dans laquelle $R_1$ et $R_4$ sont tels que définis précédemment, en présence d'un agent complexant.

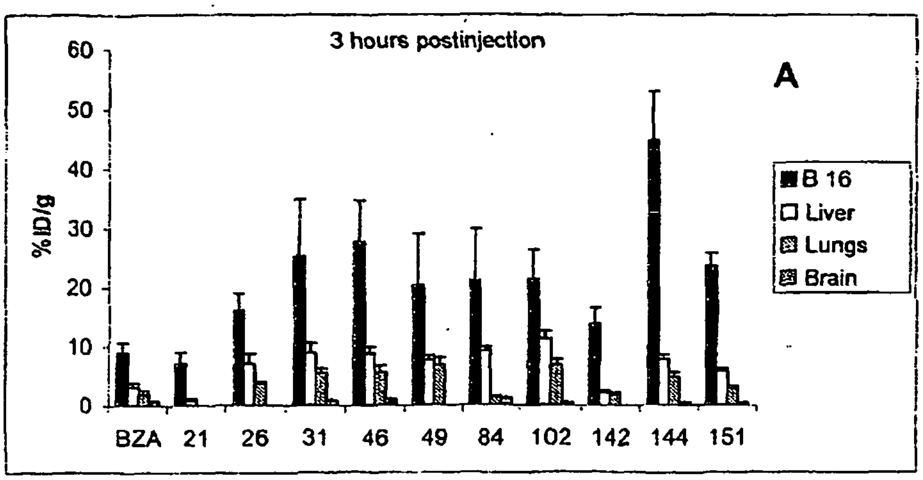


Figure 1A

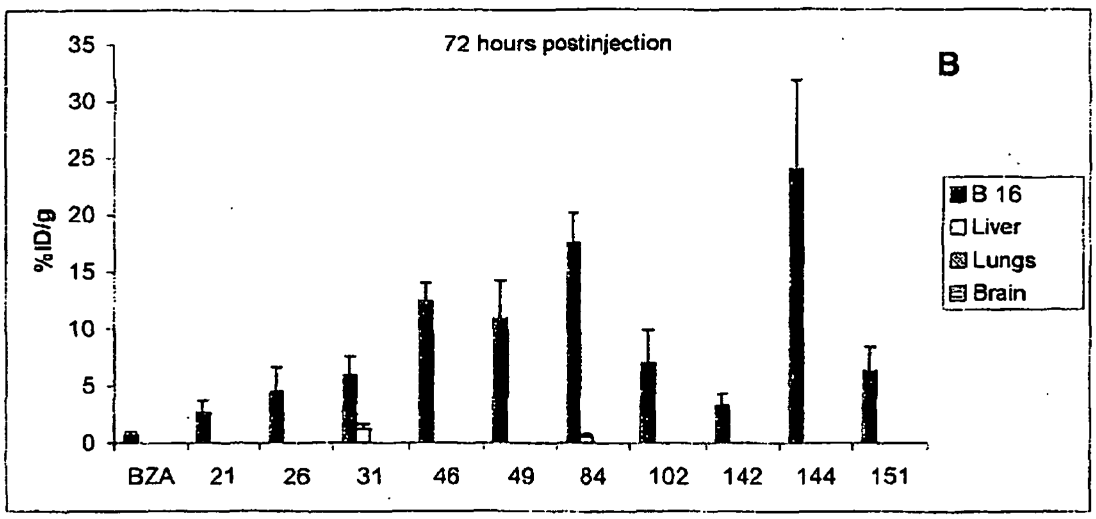


Figure 1B

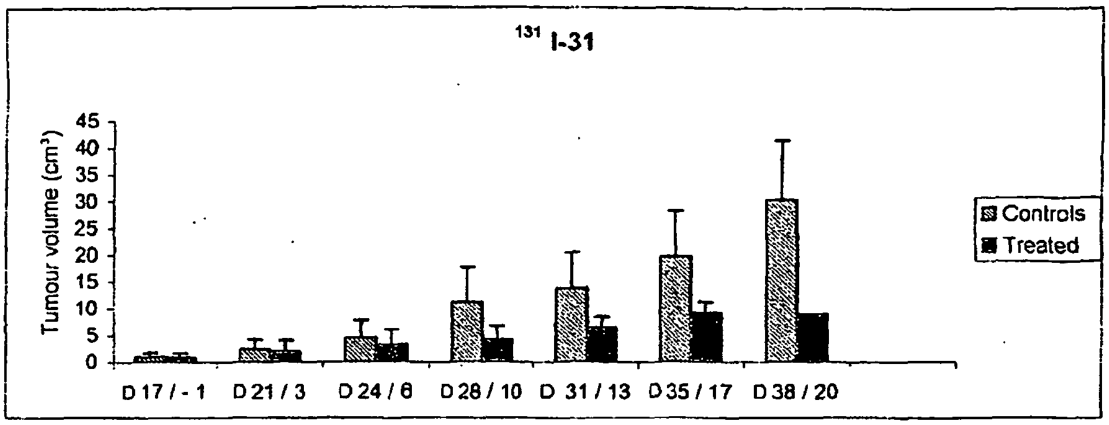


Figure 2

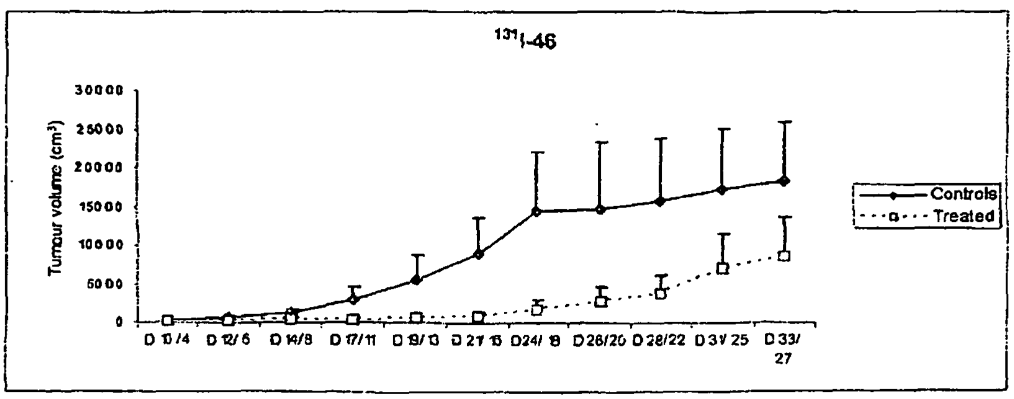


Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 458886 A
- US 5911970 A
- WO 9324096 A
- WO 9817649 A
- US 6821983 B
- WO 2005118580 A
- US 656417 A, Lee, C. H., Bayburt, E. K., DiDomenico, S., Drizin, I., Gomtsyan, A. R., Koenig, J. R., Pemer, R. J., Schmidt, R. G., Turner, S. C., White, T. K. and Zheng, G. Z.
- US 2003 A
- EP 0587311 A, Higashida, S., Sakurai, M., Yabe, Y., Nishihgaki, T., Komai, T. and Handa, H.
- US 20050038032 A, Allison, B. D., Hack, M. D., Phuong, V. K., Rabinowitz, M. H. and Rosen, M. D.


### Non-patent literature cited in the description


- **Osman, S. ; Rowlinson-Busza, G. ; Luthra, K. S. ; Aboagye, E. O. ; Brown, G. D. ; Brady, F. ; Myers, R. ; Gamage, S. A. ; Denny, W. A. ; Baguley, B. C.** *Cancer Res.,* 2001, vol. 61, 2935-2944
- **Saleem, A. ; Harte, R. J. ; Matthews, J. C. ; Osman, S. ; Brady, F. ; Luthra, S. K. ; Brown, G. D. ; Bleehen, N. ; Connors, T. ; Jones, T.** *J. Clin. Oncology,* 2001, vol. 19, 1421-1429
- **S. A. Gamage et al.** *Bioorganic & Medicinal Chemistry,* 2006, vol. 14, 1160-1168
- **Adcock, W. ; Wells, P. R.** *Aust. J. Chem.,* 1965, vol. 18, 1351-1364
- **Newkome, G. R. ; Moorfield, C. N. ; Sabbaghian, B.** *J. Org. Chem.,* 1986, vol. 51, 953-954
- **Beshore, D. C. ; Dinsmore, C: J.** *Synth. Commun.,* 2003, vol. 33, 2423-2427
- **Bridges, A. J. ; Lee, A. ; Maduakor, E. C. ; Schwartz, C. E.** *Tetrahedron Lett.,* 1992, vol. 33, 7499-7502
- **Enguchard, C. ; Renou, J. L. ; Collot, V. ; Hervet, M. ; Rault, S. ; Gueiffier, A.** *J. Org. Chem.,* 2000, vol. 65, 6572-6575
- **Sunberg, R. J. ; Biswas, S. ; Murthi, K. K. ; Rowe, D. ; McDall, J. W. ; Dzimianski, M. T.** *J. Med. Chem.,* 1998, vol. 41, 4317-4328
- **Petrow, V. ; Sturgeon, B.** *J. Chem. Soc.,* 1954, 570-574
- **Lin, A. J. ; Loo, T. L.** *J. Med. Chem.,* 1978, vol. 21, 268-272
- **Seguin, H. ; Gardette, D. ; Moreau , M. F. ; Madelmont, J. C. ; Gramain, J. C.** *Synth. Commun.,* 1998, vol. 28, 4257-4272
- **Fieser, L. F. ; Haddadin, M. J.** *Org. Synth.,* 1966, vol. 46, 107-112
- **Sy, W. W.** *Synth. Commun.,* 1992, vol. 22, 3215-3219
- **Rewcasttle, G. W. ; Denny, W. A.** *Synthesis,* 1985, 220-222
- **Seltzman, H. H. ; Berrang, B. D.** *Tetrahedron Lett.,* 1993, vol. 34, 3083-3086
- **Rewcastle, G. W. ; Denny, W. A.** *Synthesis,* 1985, vol. 2, 217-220
- **Rewcastle G. W. ; Denny, W. A.** *Synthesis,* 1985, 217-220
- **Atwell, G. J. ; Rewcastle, G. W. ; Baguley, B. C. ; Denny, W. A.** *J. Med. Chem.,* 1987, vol. 30, 664-669
- **Renotte, R. ; Sarlet, G. ; Thunus, L. ; Lejeune, R.** *Luminescence,* 2000, vol. 15, 311-320
- **Ferlin, M. G. ; Marzano, C. ; Chiarelotto, G. ; Baccichetti, F. ; Bordin, F.** *Eur. J. Med. Chem.,* 2000, vol. 35, 827-837
- **Kajigaeshi, S. ; Kakinami, T ; Yamasaki, H. ; Fujisaki, S. ; Okamoto, T.** *Bull. Chem. Soc. Jpn.,* 1988, vol. 61, 600-602
- **Atwell, G. J. ; Cain, B. F. ; Baguley, B. C. ; Finlay, G. J. ; Denny, W. A.** *J. Med. Chem.,* 1984, vol. 27, 1481-1485
- **Chan, L. ; Jin, H. ; Stefanac, T. ; Lavallée, J.F. ; Falardeau, G. ; Wang, W. ; Bédard, J. ; May, S. ; Yuen, L.** *J. Med. Chem.,* 1999, vol. 42, 3023-3025
- **Seguin, H. ; Gardette, D. ; Moreau, M. F. ; Madelmont, J. C. ; Gramain, J. C.** *Synth. Commun.,* 1998, vol. 28, 4257-4272
- **Culhane, P.J.** Organic Syntheses. Wiley, 1944
- *Collect.,* vol. I, 125